(19) **Europäisches Patentamt**
**European Patent Office**
**Office européen des brevets**

(11) **EP 3 988 522 A1**

(12) **EUROPEAN PATENT APPLICATION**
published in accordance with Art. 153(4) EPC

(43) Date of publication:
**27.04.2022 Bulletin 2022/17**

(21) Application number: **20827208.8**

(22) Date of filing: **19.06.2020**

(51) International Patent Classification (IPC):
*C07C 21/18* (2006.01)     *F25B 1/00* (2006.01)
*C09K 5/04* (2006.01)

(52) Cooperative Patent Classification (CPC):
**B60H 1/22; C07C 17/42; C07C 21/18; C09K 5/04;**
**F25B 1/00**

(86) International application number:
**PCT/JP2020/024198**

(87) International publication number:
**WO 2020/256129 (24.12.2020 Gazette 2020/52)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **19.06.2019   JP 2019114154**
**21.06.2019   JP 2019115584**

(71) Applicant: **Daikin Industries, Ltd.**
**Osaka-shi, Osaka 530-8323 (JP)**

(72) Inventors:
• **GOTOU, Tomoyuki**
**Osaka-shi, Osaka 530-8323 (JP)**
• **YOSHIMURA, Takashi**
**Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **METHOD FOR COEXISTING 1,2-DIFLUOROETHYLENE (HFO-1132) AND OXYGEN IN GAS PHASE, AND STORAGE CONTAINER AND REFRIGERATOR CONTAINING HFO-1132 AND OXYGEN**

(57)     An object is to suppress the polymerization reaction or self-decomposition reaction of 1,2-difluoroethylene. Provided as a means for achieving the object is a method for allowing 1,2-difluoroethylene (HFO-1132) and oxygen to coexist in a gas phase, the concentration of oxygen in the gas phase at a temperature of 25°C being 1000 volume ppm or less.

Fig. 2

**Description**

Technical Field

The present disclosure relates to a method for allowing 1,2-difluoroethylene (HFO-1132) and oxygen to coexist in a gas phase, and a storage container and refrigerating machine that comprise HFO-1132 and oxygen.

Background Art

Various mixed refrigerants containing HFO-1132 have been proposed as low-GWP mixed refrigerants that can replace R410A (Patent Literature 1).

Citation List

Patent Literature

PTL 1: WO2015/141678

Summary of Invention

Technical Problem

An object is to improve the stability of 1,2-difluoroethylene (HFO-1132) in a refrigerant containing HFO-1132 during storage and during operation of a refrigerating machine comprising the refrigerant.

Solution to Problem

Item 1. A method for allowing a refrigerant comprising 1,2-difluoroethylene (HFO-1132) and oxygen to coexist in a gas phase, the concentration of oxygen in the gas phase at a temperature of 25°C being 1000 volume ppm or less.
Item 2. A method for storing a refrigerant comprising HFO-1132 by allowing the refrigerant and oxygen to coexist in a gas phase in a closed container, the concentration of oxygen in the gas phase at a temperature of 25°C being 1000 volume ppm or less.
Item 3. The method according to Item 1 or 2, wherein the temperature of the gas phase is less than 80°C.
Item 4. The method according to any one of Items 1 to 3, wherein the gas phase coexists with a liquid phase containing the refrigerant.
Item 5. The method according to Item 1 or 2, wherein a refrigerating machine is operated using the refrigerant as a working fluid, the method comprises performing the coexistence in the refrigerating machine, and the concentration of oxygen in the gas phase at a temperature of 25°C is 1000 volume ppm or less.
Item 6. The method according to Item 5, wherein the gas phase coexists with a liquid phase containing the refrigerant in at least part of the refrigerating machine.
Item 7. A method for stabilizing a refrigerant comprising HFO-1132, the method comprising, in a state that allows the refrigerant and oxygen to coexist in a gas phase, maintaining the concentration of oxygen in the gas phase at a temperature of 25°C to 1000 volume ppm or less to thereby stabilize the refrigerant.
Item 7. A storage container of a refrigerant comprising HFO-1132, in which the refrigerant and oxygen coexist in a gas phase, the concentration of oxygen in the gas phase at a temperature of 25°C being 1000 volume ppm or less.
Item 8. A refrigerating machine comprising, as a working fluid, a gas phase containing a refrigerant comprising HFO-1132 and oxygen, the concentration of oxygen in the gas phase at a temperature of 25°C being 1000 volume ppm or less.
Item 9. The method, storage container, or refrigerating machine according to Items 1 to 8, wherein the refrigerant comprises trans-1,2-difluoroethylene (HFO-1132(E)), trifluoroethylene (HFO-1123), and 2,3,3,3-tetrafluoro-1-propene (R1234yf) .
Item 10. The method, storage container, or refrigerating machine according to Items 1 to 8, wherein in the refrigerant, when the mass% of HFO-1132(E), HFO-1123, and R1234yf based on their sum is respectively represented by x, y, and z,

coordinates (x,y,z) in a ternary composition diagram in which the sum of HFO-1132(E), HFO-1123, and R1234yf is 100 mass% are within the range of a figure surrounded by line segments OD, DG, GH, and HO that connect the following 4 points:

point D (87.6, 0.0, 12.4),
point G (18.2, 55.1, 26.7),
point H (56.7, 43.3, 0.0), and
point O (100.0, 0.0, 0.0),
or on the line segments OD, DG, and GH (excluding the points O and H) ;

the line segment DG is represented by coordinates $(0.0047y^2-1.5177y+87.598, y, -0.0047y^2+0.5177y+12.402)$,
the line segment GH is represented by coordinates $(-0.0134z^2-1.0825z+56.692, 0.0134z^2+0.0825z+43.308, z)$, and
the lines HO and OD are straight lines.

Item 11. The method, storage container, or refrigerating machine according to Items 1 to 8, wherein in the refrigerant, when the mass% of HFO-1132(E), HFO-1123, and R1234yf based on their sum is respectively represented by x, y, and z,

coordinates (x,y,z) in a ternary composition diagram in which the sum of HFO-1132(E), HFO-1123, and R1234yf is 100 mass% are within the range of a figure surrounded by line segments LG, GH, HI, and IL that connect the following 4 points: point L (72.5, 10.2, 17.3),

point G (18.2, 55.1, 26.7),
point H (56.7, 43.3, 0.0), and
point I (72.5, 27.5, 0.0),
or on the line segments LG, GH, and IL (excluding the points H and I);

the line segment LG is represented by coordinates $(0.0047y^2-1.5177y+87.598, y, -0.0047y^2+0.5177y+12.402)$,
the line segment GH is represented by coordinates $(-0.0134z^2-1.0825z+56.692, 0.0134z^2+0.0825z+43.308, z)$, and
the line segments HI and IL are straight lines.

Item 12. The method, storage container, or refrigerating machine according to Items 1 to 8, wherein the refrigerant further comprises difluoromethane (R32).

Item 13. The method, storage container, or refrigerating machine according to Items 1 to 8, wherein in the refrigerant, when the mass% of HFO-1132(E), HFO-1123, R1234yf, and R32 based on their sum is respectively represented by x, y, z, and a,

if $0<a\leq10.0$, coordinates (x,y,z) in a ternary composition diagram in which the sum of HFO-1132(E), HFO-1123, and R1234yf is 100 mass% are within the range of a figure surrounded by straight lines that connect the following 4 points: point A $(0.02a^2-2.46a+93.4, 0, -0.02a^2+2.46a+6.6)$,

point B' $(-0.008a^2-1.38a+56, 0.018a^2-0.53a+26.3, -0.01a^2+1.91a+17.7)$,
point C $(-0.016a^2+1.02a+77.6, 0.016a^2-1.02a+22.4, 0)$, and point O (100.0, 0.0, 0.0),
or on the straight lines OA, AB', and B'C (excluding the points O and C);

if $10.0<a\leq16.5$, coordinates (x,y,z) in the ternary composition diagram are within the range of a figure surrounded by straight lines that connect the following 4 points:

point A $(0.0244a^2-2.5695a+94.056, 0, -0.0244a^2+2.5695a+5.944)$,
point B' $(0.1161a^2-1.9959a+59.749, 0.014a^2-0.3399a+24.8, -0.1301a^2+2.3358a+15.451)$,
point C $(-0.0161a^2+1.02a+77.6, 0.0161a^2-1.02a+22.4, 0)$, and
point O (100.0, 0.0, 0.0),
or on the straight lines OA, AB', and B'C (excluding the points O and C); or

if $16.5<a\leq21.8$, coordinates (x,y,z) in the ternary composition diagram are within the range of a figure surrounded by straight lines that connect the following 4 points:

point A $(0.0161a^2-2.3535a+92.742, 0, -0.0161a^2+2.3535a+7.258)$,
point B' $(-0.0435a^2-0.0435a+50.406, -0.0304a^2+1.8991a-0.0661, 0.0739a^2-1.8556a+49.6601)$,
point C $(-0.0161a^2+0.9959a+77.851, 0.0161a^2-0.9959a+22.149, 0)$, and
point O (100.0, 0.0, 0.0),
or on the straight lines OA, AB', and B'C (excluding the points O and C).

Item 14. The method, storage container, or refrigerating machine according to Items 1 to 8, wherein the refrigerant comprises HFO-1132(E) and HFO-1123 in a total amount of 99.5 mass% or more based on the entire refrigerant, and the refrigerant comprises HFO-1132(E) in an amount of 62.5 mass% to 72.5 mass% based on the entire refrigerant.

Item 15. The method, storage container, or refrigerating machine according to Items 1 to 8, wherein the refrigerant comprises HFO-1132(E), R32, and R1234yf, and

in the refrigerant, when the mass% of HFO-1132(E), R32, and R1234yf based on their sum is respectively represented by x, y, and z,
coordinates (x,y,z) in a ternary composition diagram in which the sum of HFO-1132(E), R32, and R1234yf is 100 mass% are within the range of a figure surrounded by line segments AC, CF, FD, and DA that connect the following 4 points:

point A (71.1, 0.0, 28.9),
point C (36.5, 18.2, 45.3),
point F (47.6, 18.3, 34.1), and
point D (72.0, 0.0, 28.0),
or on the line segments AC, CF, FD, and DA;

the line segment AC is represented by coordinates $(0.0181y^2-2.2288y+71.096, y, -0.0181y^2+1.2288y+28.904)$,
the line segment FD is represented by coordinates $(0.02y^2-1.7y+72, y, -0.02y^2+0.7y+28)$, and
the line segments CF and DA are straight lines.

Item 16. The method, storage container, or refrigerating machine according to Items 1 to 8, wherein the refrigerant comprises HFO-1132(E), R32, and R1234yf, and

in the refrigerant, when the mass% of HFO-1132(E), R32, and R1234yf based on their sum is respectively represented by x, y, and z,
coordinates (x,y,z) in a ternary composition diagram in which the sum of HFO-1132(E), R32, and R1234yf is 100 mass% are within the range of a figure surrounded by line segments AB, BE, ED, and DA that connect the following 4 points:

point A (71.1, 0.0, 28.9),
point B (42.6, 14.5, 42.9),
point E (51.4, 14.6, 34.0), and
point D (72.0, 0.0, 28.0),
or on the line segments AB, BE, ED, and DA;

the line segment AB is represented by coordinates $(0.0181y^2-2.2288y+71.096, y, -0.0181y^2+1.2288y+28.904)$,
the line segment ED is represented by coordinates $(0.02y^2-1.7y+72, y, -0.02y^2+0.7y+28)$, and

Item 17. The method, storage container, or refrigerating machine according to Items 1 to 8, wherein the refrigerant comprises HFO-1132(E), R32, and R1234yf, and

in the refrigerant, when the mass% of HFO-1132(E), R32, and R1234yf based on their sum is respectively represented by x, y, and z,
coordinates (x,y,z) in a ternary composition diagram in which the sum of HFO-1132(E), R32, and R1234yf is 100 mass% are within the range of a figure surrounded by line segments GI, IJ, and JK that connect the following 3 points:

point G (77.5, 6.9, 15.6),
point I (55.1, 18.3, 26.6), and
point J (77.5. 18.4, 4.1),
or on the line segments GI, IJ, and JK;

the line segment GI is represented by coordinates $(0.02y^2-2.4583y+93.396, y, -0.02y^2+1.4583y+6.604)$, and
the line segments IJ and JK are straight lines.

Item 18. The method, storage container, or refrigerating machine according to Items 1 to 8, wherein the refrigerant comprises HFO-1132(E), R32, and R1234yf, and

in the refrigerant, when the mass% of HFO-1132(E), R32, and R1234yf based on their sum is respectively represented by x, y, and z,
coordinates (x,y,z) in a ternary composition diagram in which the sum of HFO-1132(E), R32, and R1234yf is 100 mass% are within the range of a figure surrounded by line segments GH, HK, and KG that connect the following 3 points:

point G (77.5, 6.9, 15.6),
point H (61.8, 14.6, 23.6), and
point K (77.5, 14.6, 7.9),
or on the line segments GH, HK, and KG;

the line segment GH is represented by coordinates $(0.02y^2-2.4583y+93.396, y, -0.02y^2+1.4583y+6.604)$, and
the line segments HK and KG are straight lines.

Item 19. The method, storage container, or refrigerating machine according to Items 1 to 8, wherein the refrigerant comprises HFO-1132(E), HFO-1123, and R32, and

in the refrigerant, when the mass% of HFO-1132(E), HFO-1123, and R32 based on their sum is respectively represented by x, y, and z,
coordinates (x,y,z) in a ternary composition diagram in which the sum of HFO-1132(E), HFO-1123, and R32 is 100 mass% are within the range of a figure surrounded by line segments OC', C'D', D'E', E'A', and A'O that connect the following 5 points: point O (100.0, 0.0, 0.0),

point C' (56.7, 43.3, 0.0),
point D' (52.2, 38.3, 9.5),
point E' (41.8, 39.8, 18.4), and
point A' (81.6, 0.0, 18.4),
or on the line segments C'D', D'E', and E'A' (excluding the points C' and A');

the line segment C'D' is represented by coordinates $(-0.0297z^2-0.1915z+56.7, 0.0297z^2+1.1915z+43.3, z)$,
the line segment D'E' is represented by coordinates $(-0.0535z^2+0.3229z+53.957, 0.0535z^2+0.6771z+46.043, z)$, and
the line segments OC', E'A', and A'O are straight lines.

Item 20. The method, storage container, or refrigerating machine according to Items 1 to 8, wherein the refrigerant comprises HFO-1132(E), HFO-1123, and R32, and

in the refrigerant, when the mass% of HFO-1132(E), HFO-1123, and R32 based on their sum is respectively represented by x, y, and z,
coordinates (x,y,z) in a ternary composition diagram in which the sum of HFO-1132(E), HFO-1123, and R32 is 100 mass% are within the range of a figure surrounded by line segments OC, CD, DE, EA', and A'O that connect the following 5 points:

point O (100.0, 0.0, 0.0),
point C (77.7, 22.3, 0.0),
point D (76.3, 14.2, 9.5),
point E (72.2, 9.4, 18.4), and
point A' (81.6, 0.0, 18.4),
or on the line segments CD, DE, and EA' (excluding the points C and A');

the line segment CDE is represented by coordinates $(-0.017z^2+0.0148z+77.684, 0.017z^2+0.9852z+22.316, z)$, and
the line segments OC, EA' and A'O are straight lines.

Item 21. The method, storage container, or refrigerating machine according to Items 1 to 8, wherein the refrigerant comprises HFO-1132(E), HFO-1123, and R32, and

in the refrigerant, when the mass% of HFO-1132(E), HFO-1123, and R32 based on their sum is respectively represented by x, y, and z,
coordinates (x,y,z) in a ternary composition diagram in which the sum of HFO-1132(E), HFO-1123, and R32 is 100 mass% are within the range of a figure surrounded by line segments OC', C'D', D'A, and AO that connect the following 5 points:

point O (100.0, 0.0, 0.0),
point C' (56.7, 43.3, 0.0),
point D' (52.2, 38.3, 9.5), and
point A (90.5, 0.0, 9.5),
or on the line segments C'D' and D'A (excluding the points C' and A) ;

the line segment C'D' is represented by coordinates $(-0.0297z^2-0.1915z+56.7, 0.0297z^2+1.1915z+43.3, z)$, and the line segments OC', D'A, and AO are straight lines.

Item 22. The method, storage container, or refrigerating machine according to Items 1 to 8, wherein the refrigerant comprises HFO-1132(E), HFO-1123, and R32, and

in the refrigerant, when the mass% of HFO-1132(E), HFO-1123, and R32 based on their sum is respectively represented by x, y, and z,
coordinates (x,y,z) in a ternary composition diagram in which the sum of HFO-1132(E), HFO-1123, and R32 is 100 mass% are within the range of a figure surrounded by line segments OC, CD, DA, and AO that connect the following 5 points:

point O (100.0, 0.0, 0.0),
point C (77.7, 22.3, 0.0),
point D (76.3, 14.2, 9.5), and
point A (90.5, 0.0, 9.5),
or on the line segments CD and DA (excluding the points C and A);

the line segment CD is represented by coordinates $(-0.017z^2+0.0148z+77.684, 0.017z^2+0.9852z+22.316, z)$, and the line segments OC, DA, and AO are straight lines.

Item 23. The method, storage container, or refrigerating machine according to Items 1 to 8, wherein the refrigerant comprises $CO_2$, trans-1,2-difluoroethylene (HFO-1132(E)), difluoromethane (R32), and 2,3,3,3-tetrafluoro-1-propene (R1234yf), and

when the mass% of $CO_2$, R32, HFO-1132(E), and R1234yf based on their sum is respectively represented by w, x, y, and z,
if $0<w\leq1.2$, coordinates (x,y,z) in a ternary composition diagram in which the sum of R32, HFO-1132(E), and R1234yf is (100-w) mass% are within the range of a figure surrounded by curves IJ, JK, and KL, as well as straight lines LB", B"D, DC, and CI that connect the following 7 points: point I (0.0, 72.0, 28.0-w),

point J (18.3, 48.5, 33.2-w),
point K (36.8, 35.6, 27.6-w),
point L (51.7, 28.9, 19.4-w),
point B" $(-1.5278w^2+2.75w+50.5, 0.0, 1.5278w^2-3.75w+49.5)$,
point D (-2.9167w+40.317, 0.0, 1.9167w+59.683), and
point C (0.0, -4.9167w+58.317, 3.9167w+41.683),
or on the above line segments (excluding the points on the straight lines B"D and CI);

if $1.2<w\leq4.0$, coordinates (x,y,z) in the ternary composition diagram are within the range of a figure surrounded by curves IJ, JK, and KL, as well as straight lines LB", B"D, DC, and CI that connect the following 7 points:

point I (0.0, 72.0, 28.0-w),
point J (18.3, 48.5, 33.2-w),
point K (36.8, 35.6, 27.6-w),
point L (51.7, 28.9, 19.4-w),
point B" (51.6, 0.0, 48.4-w),
point D (-2.8226w+40.211, 0.0, 1.8226w+59.789), and
point C $(0.0, 0.1081w^2-5.169w+58.447, -0.1081w^2+4.169w+41.553)$,

or on the above line segments (excluding the points on the straight lines B"D and CI); or

if $4.0<w\leq7.0$, coordinates (x,y,z) in the ternary composition diagram are within the range of a figure surrounded by curves IJ, JK, and KL, as well as straight lines LB", B"D, DC, and CI that connect the following 7 points:

point I (0.0, 72.0, 28.0-w),
point J (18.3, 48.5, 33.2-w),
point K (36.8, 35.6, 27.6-w),
point L (51.7, 28.9, 19.4-w),
point B" (51.6, 0.0, 48.4-w),
point D (-2.8w+40.1, 0.0, 1.8w+59.9), and
point C (0.0, $0.0667w^2$-4.9667w+58.3, $-0.0667w^2$+3.9667w+41.7),
or on the above line segments (excluding the points on the straight lines B"D and CI);

the curve IJ is represented by coordinates (x, $0.0236x^2$-1.716x+72, $-0.0236x^2$+0.716x+28-w),
the curve JK is represented by coordinates (x, $0.0095x^2$-1.2222x+67.676, $-0.0095x^2$+0.2222x+32.324-w), and
the curve KL is represented by coordinates (x, $0.0049x^2$-0.8842x+61.488, $-0.0049x^2$-0.1158x+38.512).

Item 24. The method, storage container, or refrigerating machine according to Items 1 to 8, wherein the refrigerant comprises $CO_2$, trans-1,2-difluoroethylene (HFO-1132(E)), difluoromethane (R32), and 2,3,3,3-tetrafluoro-1-propene (R1234yf), and

when the mass% of $CO_2$, R32, HFO-1132(E), and R1234yf based on their sum is respectively represented by w, x, y, and z,
if $0<w\leq1.2$, coordinates (x,y,z) in a ternary composition diagram in which the sum of R32, HFO-1132(E), and R1234yf is (100-w) mass% are within the range of a figure surrounded by curves IJ and JK, as well as straight lines KF, FC, and CI that connect the following 5 points:

point I (0.0, 72.0, 28.0-w),
point J (18.3, 48.5, 33.2-w),
point K (36.8, 35.6, 27.6-w),
point F (-0.0833w+36.717, -4.0833w+5.1833, 3.1666w+58.0997), and point C (0.0, -4.9167w+58.317, 3.9167w+41.683),
or on the above line segments (excluding the points on the straight line CI);

if $1.2<w\leq1.3$, coordinates (x,y,z) in the ternary composition diagram are within the range of a figure surrounded by curves IJ and JK, as well as straight lines KF, FC, and CI that connect the following 5 points:

point I (0.0, 72.0, 28.0-w),
point J (18.3, 48.5, 33.2-w),
point K (36.8, 35.6, 27.6-w),
point F (36.6, -3w+3.9, 2w+59.5), and
point C (0.0, $0.1081w^2$-5.169w+58.447, $-0.1081w^2$+4.169w+41.553),
or on the above line segments (excluding the points on the straight line CI);

if $1.3<w\leq4.0$, coordinates (x,y,z) in the ternary composition diagram are within the range of a figure surrounded by curves IJ and JK, as well as straight lines KB', B'D, DC, and CI that connect the following 6 points:

point I (0.0, 72.0, 28.0-w),
point J (18.3, 48.5, 33.2-w),
point K (36.8, 35.6, 27.6-w),
point B' (36.6, 0.0, -w+63.4),
point D (-2.8226w+40.211, 0.0, 1.8226w+59.789), and
point C (0.0, $0.1081w^2$-5.169w+58.447, $-0.1081w^2$+4.169w+41.553),
or on the above line segments (excluding the points on the straight line CI); or

if $4.0<w\leq7.0$, coordinates (x,y,z) in the ternary composition diagram are within the range of a figure surrounded by curves IJ and JK, as well as straight lines KB', B'D, DC, and CI that connect the following 6 points:

point I (0.0, 72.0, 28.0-w),
point J (18.3, 48.5, 33.2-w),
point K (36.8, 35.6, 27.6-w),
point B' (36.6, 0.0, -w+63.4),
point D (-2.8w+40.1, 0.0, 1.8w+59.9), and
point C (0.0, $0.0667w^2$-4.9667w+58.3, $-0.0667w^2$+3.9667w+41.7),
or on the above line segments (excluding the points on the straight line CI);

the curve IJ is represented by coordinates (x, $0.0236x^2$-1.716x+72, $-0.0236x^2$+0.716x+28-w), and
the curve JK is represented by coordinates (x, $0.0095x^2$-1.2222x+67.676, $-0.0095x^2$+0.2222x+32.324-w).

Item 25. The method, storage container, or refrigerating machine according to Items 1 to 8, wherein the refrigerant comprises $CO_2$, R32, HFO-1132(E), and R1234yf, and

when the mass% of $CO_2$, R32, HFO-1132(E), and R1234yf based on their sum is respectively represented by w, x, y, and z,
if 0<w≤1.2, coordinates (x,y,z) in a ternary composition diagram in which the sum of R32, HFO-1132(E), and R1234yf is (100-w) mass% are within the range of a figure surrounded by curves IJ and JK, as well as straight lines KF, FC, and CI that connect the following 4 points:

point I (0.0, 72.0, 28.0-w),
point J (18.3, 48.5, 33.2-w),
point E (18.2, $-1.1111w^2$-3.1667w+31.9, $1.1111w^2$+2.1667w+49.9), and
point C (0.0, -4.9167w+58.317, 3.9167w+41.683),
or on the above line segments (excluding the points on the straight line CI);

if 1.2<w≤4.0, coordinates (x,y,z) in the ternary composition diagram are within the range of a figure surrounded by curves IJ and JK, as well as straight lines KF, FC, and CI that connect the following 4 points:

point I (0.0, 72.0, 28.0-w),
point J (18.3, 48.5, 33.2-w),
point E (-0.0365w+18.26, $0.0623w^2$-4.5381w+31.856,$-0.0623w^2$+3.5746w+49.884), and
point C (0.0, $0.1081w^2$-5.169w+58.447, $-0.1081w^2$+4.169w+41.553),
or on the above line segments (excluding the points on the straight line CI); or

if 4.0<w≤7.0, coordinates (x,y,z) in the ternary composition diagram are within the range of a figure surrounded by curves IJ and JK, as well as straight lines KF, FC, and CI that connect the following 4 points:

point I (0.0, 72.0, 28.0-w),
point J (18.3, 48.5, 33.2-w),
point E (18.1, $0.0444w^2$-4.3556w+31.411, $-0.0444w^2$+3.3556w+50.489), and
point C (0.0, $0.0667w^2$-4.9667w+58.3, $-0.0667w^2$+3.9667w+41.7),
or on the above line segments (excluding the points on the straight line CI); and

the curve IJ is represented by coordinates (x, $0.0236x^2$-1.716x+72, $-0.0236x^2$+0.716x+28-w).

Item 26. The method, storage container, or refrigerating machine according to Items 1 to 8, wherein the refrigerant comprises $CO_2$, R32, HFO-1132(E), and R1234yf, and

when the mass% of $CO_2$, R32, HFO-1132(E), and R1234yf based on their sum is respectively represented by w, x, y, and z,
if 0<w≤0.6, coordinates (x,y,z) in a ternary composition diagram in which the sum of R32, HFO-1132(E), and R1234yf is (100-w) mass% are within the range of a figure surrounded by curves GO and OP, as well as straight lines PB", B"D, and DG that connect the following 5 points:

point G ($-5.8333w^2$-3.1667w+22.2, $7.0833w^2$+1.4167w+26.2,$-1.25w^2$+0.75w+51.6),
point O (36.8, $0.8333w^2$+1.8333w+22.6, $-0.8333w^2$-2.8333w+40.6),
point P (51.7, $1.1111w^2$+20.5, $-1.1111w^2$-w+27.8),

point B" (-1.5278w$^2$+2.75w+50.5, 0.0, 1.5278w$^2$-3.75w+49.5), and
point D (-2.9167w+40.317, 0.0, 1.9167w+59.683),
or on the above line segments (excluding the points on the straight line B"D);

if 0.6<w≤1.2, coordinates (x,y,z) in the ternary composition diagram are within the range of a figure surrounded by curves GN, NO, and OP, as well as straight lines PB",B"D, and DG that connect the following 6 points:

point G (-5.8333w$^2$-3.1667w+22.2, 7.0833w$^2$+1.4167w+26.2,-1.25w$^2$+0.75w+51.6),
point N (18.2, 0.2778w2+3w+27.7, -0.2778w2-4w+54.1),
point O (36.8, 0.8333w$^2$+1.8333w+22.6, -0.8333w$^2$-2.8333w+40.6),
point P (51.7, 1.1111w$^2$+20.5, -1.1111w$^2$-w+27.8),
point B" (-1.5278w$^2$+2.75w+50.5, 0.0, 1.5278w$^2$-3.75w+49.5), and
point D (-2.9167w+40.317, 0.0, 1.9167w+59.683),
or on the above line segments (excluding the points on the straight line B"D),

if 0<w≤0.6, the curve GO is represented by coordinates (x, (0.00487w$^2$-0.0059w+0.0072)x$^2$+(-0.279w$^2$+0.2844w-0.6701)x+3.7639w$^2$-0.2467w+37.512, 100-w-x-y),
if 0.6<w≤1.2, the curve GN is represented by coordinates (x, (0.0122w$^2$-0.0113w+0.0313)x$^2$+(-0.3582w$^2$+0.1624w-1.4551)x+2.7889w$^2$+3.7417w+43.824, 100-w-x-y),
if 0.6<w≤1.2, the curve NO is represented by coordinates (x, (0.00487w$^2$-0.0059w+0.0072)x$^2$+(-0.279w$^2$+0.2844w-0.6701)x+3.7639w$^2$-0.2467w+37.512, 100-w-x-y), and
if 0<w≤1.2, the curve OP is represented by coordinates (x, (0.0074w$^2$-0.0133w+0.0064)x$^2$+(-0.5839w$^2$+1.0268w-0.7103)x+11.472w$^2$-17.455w+40.07, 100-w-x-y) ;
if 1.2<w≤4.0, coordinates (x,y,z) in the ternary composition diagram are within the range of a figure surrounded by curves MW, WN, NO, and OP, as well as straight lines PB", B"D, DC, and CM that connect the following 8 points:

point M (0.0, -0.3004w$^2$+2.419w+55.53, 0.3004w$^2$-3.419w+44.47),
point W (10.0, -0.3645w$^2$+3.5024w+44.422, 0.3645w$^2$-4.5024w+55.578),
point N (18.2, -0.3773w$^2$+3.319w+28.26, 0.3773w$^2$-4.319w+53.54),
point O (36.8, -0.1392w$^2$+1.4381w+24.475, 0.1392w$^2$-2.4381w+38.725),
point P (51.7, -0.2381w$^2$+1.881w+20.186, 0.2381w$^2$-2.881w+28.114),
point B" (51.6, 0.0, -w+48.4),
point D (-2.8226w+40.211, 0.0, 1.8226w+59.789), and
point C (0.0, 0.1081w$^2$-5.169w+58.447, -0.1081w$^2$+4.169w+41.553),
or on the above line segments (excluding the points on the straight lines B"D and CM),

the curve MW is represented by coordinates (x, (0.0043w$^2$-0.0359w+0.1509)x$^2$+(-0.0493w$^2$+0.4669w-3.6193)x-0.3004w$^2$+2.419w+55.53, 100-w-x-y),
the curve WN is represented by coordinates (x, (0.0055w$^2$-0.0326w+0.0665)x$^2$+(-0.1571w$^2$+0.8981w-2.6274)x+0.6555w$^2$-2.2153w+54.044, 100-w-x-y),
the curve NO is represented by coordinates (x, (-0.00062w$^2$+0.0036w+0.0037)x$^2$+(0.0375w$^2$-0.239w-0.4977)x-0.8575w$^2$+6.4941w+36.078, 100-w-x-y), and
the curve OP is represented by coordinates (x, (-0.000463w$^2$+0.0024w-0.0011)x$^2$+(0.0457w$^2$-0.2581w-0.075)x-1.355w$^2$+8.749w+27.096, 100-w-x-y); or
if 4.0<w≤7.0, coordinates (x,y,z) in the ternary composition diagram are within the range of a figure surrounded by curves MW, WN, NO, and OP, as well as straight lines PB", B"D, DC, and CM that connect the following 8 points:

point M (0.0, -0.0667w$^2$+0.8333w+58.133, 0.0667w$^2$-1.8333w+41.867),
point W (10.0, -0.0667w$^2$+1.1w+39.267, 0.0667w$^2$-2.1w+50.733),
point N (18.2, -0.0889w$^2$+1.3778w+31.411, 0.0889w$^2$-2.3778w+50.389),
point O (36.8, -0.0444w$^2$+0.6889w+25.956, 0.0444w$^2$-1.6889w+37.244),
point P (51.7, -0.0667w$^2$+0.8333w+21.633, 0.0667w$^2$-1.8333w+26.667),
point B" (51.6, 0.0, -w+48.4),
point D (-2.8w+40.1, 0.0, 1.8w+59.9), and
point C (0.0, 0.0667w$^2$-4.9667w+58.3, -0.0667w$^2$+3.9667w+41.7),
or on the above line segments (excluding the points on the straight lines B"D and CM),

the curve MW is represented by coordinates (x, (0.00357w$^2$-0.0391w+0.1756)x$^2$+(-0.0356w$^2$+0.4178w-3.6422)x-

0.0667w²+0.8333w+58.103, 100-w-x-y),

the curve WN is represented by coordinates (x, (-0.002061w²+0.0218w-0.0301)x²+(0.0556w²-0.5821w-0.1108)x-0.4158w²+4.7352w+43.383, 100-w-x-y),

the curve NO is represented by coordinates (x, 0.0082x²+(0.0022w²-0.0345w-0.7521)x-0.1307w²+2.0247w+42.327, 100-w-x-y), and

the curve OP is represented by coordinates (x, (-0.0006258w²+0.0066w-0.0153)x²+(0.0516w²-0.5478w+0.9894)x-1.074w²+11.651w+10.992, 100-w-x-y).

Item 27. The method, storage container, or refrigerating machine according to Items 1 to 8, wherein the refrigerant comprises $CO_2$, R32, HFO-1132(E), and R1234yf, and

when the mass% of $CO_2$, R32, HFO-1132(E), and R1234yf based on their sum is respectively represented by w, x, y, and z,

if 0<w≤0.6, coordinates (x,y,z) in a ternary composition diagram in which the sum of R32, HFO-1132(E), and R1234yf is (100-w) mass% are within the range of a figure surrounded by curve GO and straight lines OF and FG that connect the following 3 points:

point G (-5.8333w²-3.1667w+22.2, 7.0833w²-1.4167w+26.2,-1.25w²+3.5834w+51.6),
point O (36.8, 0.8333w²+1.8333w+22.6, -0.8333w²-2.8333w+40.6), and
point F (-0.0833w+36.717, -4.0833w+5.1833, 3.1666w+58.0997),
or on the above line segments, and

the curve GO is represented by coordinates (x, (0.00487w²-0.0059w+0.0072)x²+(-0.279w²+0.2844w-0.6701)x+3.7639w²-0.2467w+37.512, 100-w-x-y);

if 0.6<w≤1.2, coordinates (x,y,z) in the ternary composition diagram are within the range of a figure surrounded by curves GN and NO, as well as straight lines OF and FG that connect the following 4 points:

point G (-5.8333w²-3.1667w+22.2, 7.0833w²-1.4167w+26.2,-1.25w²+3.5834w+51.6),
point N (18.2, 0.2778w²+3.0w+27.7, -0.2778w²-4.0w+54.1),
point O (36.8, 0.8333w²+1.8333w+22.6, -0.8333w²-2.8333w+40.6), and
point F (-0.0833w+36.717, -4.0833w+5.1833, 3.1666w+58.0997),
or on the above line segments,

if 0.6<w≤1.2, the curve GN is represented by coordinates (x, (0.0122w²-0.0113w+0.0313)x²+(-0.3582w²+0.1624w-1.4551)x+2.7889w²+3.7417w+43.824, 100-w-x-y), and

if 0.6<w≤1.2, the curve NO is represented by coordinates (x, (0.00487w²-0.0059w+0.0072)x²+(-0.279w²+0.2844w-0.6701)x+3.7639w²-0.2467w+37.512, 100-w-x-y);

if 1.2<w≤1.3, coordinates (x,y,z) in the ternary composition diagram are within the range of a figure surrounded by curves MW, WN, and NO, as well as straight lines OF, FC, and CM that connect the following 6 points:

point M (0.0, -0.3004w²+2.419w+55.53, 0.3004w²-3.419w+44.47),
point W (10.0, -0.3645w²+3.5024w34.422, 0.3645w²-4.5024w+55.578),
point N (18.2, -0.3773w²+3.319w+28.26, 0.3773w²-4.319w+53.54),
point O (36.8, -0.1392w²+1.4381w+24.475, 0.1392w²-2.4381w+38.725),
point F (36.6, -3w+3.9, 2w+59.5), and
point C (0.1081w²-5.169w+58.447, 0.0, -0.1081w²+4.169w+41.553),
or on the above line segments (excluding the points on straight line CM),

the curve MW is represented by coordinates (x, (0.0043w²-0.0359w+0.1509)x²+(-0.0493w²+0.4669w-3.6193)x-0.3004w²+2.419w+55.53, 100-w-x-y),

the curve WN is represented by coordinates (x, (0.0055w²-0.0326w+0.0665)x²+(-0.1571w²+0.8981w-2.6274)x+0.6555w²-2.2153w+54.044, 100-w-x-y), and

the curve NO is represented by coordinates (x, (-0.00062w²+0.0036w+0.0037)x²+(0.0375w²-0.239w-0.4977)x 0.8575w²+6.4941w+36.078, 100-w-x-y);

if 1.3<w≤4.0, coordinates (x,y,z) in the ternary composition diagram are within the range of a figure surrounded by curves MW, WN, and NO, as well as straight lines OB', B'D, DC, and CM that connect the following 7 points:

point M (0.0, -0.3004w²+2.419w+55.53, 0.3004w²-3.419w+44.47),

point W (10.0, $-0.3645w^2+3.5024w+34.422$, $0.3645w^2-4.5024w+55.578$),
point N (18.2, $-0.3773w^2+3.319w+28.26$, $0.3773w^2-4.319w+53.54$),
point O (36.8, $-0.1392w^2+1.4381w+24.475$, $0.1392w^2-2.4381w+38.725$),
point B' (36.6, 0.0, $-w+63.4$),
point D ($-2.8226w+40.211$, 0.0, $1.8226w+59.789$), and
point C (0.0, $0.1081w^2-5.169w+58.447$, $-0.1081w^2+4.169w+41.553$),
or on the above line segments (excluding the points on the straight line CM),

the curve MW is represented by coordinates (x, $(0.0043w^2-0.0359w+0.1509)x^2+(-0.0493w^2+0.4669w-3.6193)x-0.3004w^2+2.419w+55.53$, 100-w-x-y),
the curve WN is represented by coordinates (x, $(0.0055w^2-0.0326w+0.0665)x^2+(-0.1571w^2+0.8981w-2.6274)x+0.6555w^2-2.2153w+54.044$, 100-w-x-y), and
the curve NO is represented by coordinates (x, $(-0.00062w^2+0.0036w+0.0037)x^2+(0.0457w^2-0.2581w-0.075)x-1.355w^2+8.749w+27.096$, 100-w-x-y); or
if $4.0<w\leq7.0$, coordinates (x,y,z) in the ternary composition diagram are within the range of a figure surrounded by curves MW, WN, and NO, as well as straight lines OB', B'D, DC, and CM that connect the following 7 points:

point M (0.0, $-0.0667w^2+0.8333w58.133$, $0.0667w^2-1.8333w+41.867$),
point W (10.0, $-0.0667w^2+1.1w+39.267$, $0.0667w^2-2.1w+50.733$),
point N (18. 2, $-0.0889w^2+1.3778w+31.411$, $0.0889w^2-2.3778w+50.389$),
point O (36.8, $-0.0444w^2+0.6889w+25.956$, $0.0444w^2-1.6889w+37.244$),
point B' (36.6, 0.0, $-w+63.4$),
point D ($-2.8w+40. 1$, 0.0, $1.8w+59.9$), and
point C (0.0, $0.0667w^2-4.9667w+58.3$, $-0.0667w^2+3.9667w+41.7$),
or on the above line segments (excluding the points on the straight line CM),

the curve MW is represented by coordinates (x, $(0.00357w^2-0.0391w+0.1756)x^2+(-0.0356w^2+0.4178w-3.6422)x-0.0667w^2+0.8333w+58.103$, 100-w-x-y),
the curve WN is represented by coordinates (x, $(-0.002061w^2+0.0218w-0.0301)x^2+(0.0556w^2-0.5821w-0.1108)x-0.4158w^2+4.7352w+43.383$, 100-w-x-y), and
the curve NO is represented by coordinates (x, $(0.0082x^2+(0.0022w^2-0.0345w-0.7521)x-0.1307w^2+2.0247w+42.327$, 100-w-x-y).

Item 28. The method, storage container, or refrigerating machine according to Items 1 to 8, wherein the refrigerant comprises $CO_2$, R32, HFO-1132(E), and R1234yf, and

when the mass% of $CO_2$, R32, HFO-1132(E), and R1234yf based on their sum is respectively represented by w, x, y, and z,
if $1.2<w\leq4.0$, coordinates (x,y,z) in a ternary composition diagram in which the sum of R32, HFO-1132(E), and R1234yf is (100-w) mass% are within the range of a figure surrounded by curves MW and WN, as well as straight lines NE, EC, and CM that connect the following 5 points:

point M (0.0, $-0.3004w^2+2.419w+55.53$, $0.3004w^2-3.419w+44.47$),
point W (10.0, $-0.3645w^2+3.5024w+34.422$, $0.3645w^2-4.5024w+55.578$),
point N (18.2, $-0.3773w^2+3.319w+28.26$, $0.3773w^2-4.319w+53.54$),
point E ($-0.0365w+18.26$, $0.0623w^2-4.5381w+31.856$, $-0.0623w^2+3.5746w+49.884$), and
point C (0.0, $0.1081w^2-5.169w+58.447$, $-0.1081w^2+4.169w+41.553$),
or on the above line segments (excluding the points on the straight line CM),

the curve MW is represented by coordinates (x, $(0.0043w^2-0.0359w+0.1509)x^2+(-0.0493w^2+0.4669w-3.6193)x-0.3004w^2+2.419w+55.53$, 100-w-x-y), and
the curve WN is represented by coordinates (x, $(0.0055w^2-0.0326w+0.0665)x^2+(-0.1571w^2+0.8981w-2.6274)x+0.6555w^2-2.2153w+54.044$, 100-w-x-y); or
if $4.0<w\leq7.0$, coordinates (x,y,z) in the ternary composition diagram are within the range of a figure surrounded by curves MW and WN, as well as straight lines NE, EC, and CM that connect the following 5 points:

point M (0.0, $-0.0667w^2+0.8333w+58.133$, $0.0667w^2-1.8333w+41.867$),
point W (10.0, $-0.0667w^2+1.1w+39.267$, $0.0667w^2-2.1w+50.733$),

point N (18.2, $-0.0889w^2+1.3778w+31.411$, $0.0889w^2-2.3778w+50.389$),
point E (18.1, $0.0444w^2-4.3556w+31.411$, $-0.0444w^2+3.3556w+50.489$), and
point C (0.0, $0.0667w^2-4.9667w+58.3$, $-0.0667w^2+3.9667w+41.7$),
or on the above line segments (excluding the points on the straight line CM),

the curve MW is represented by coordinates (x, $(0.00357w^2-0.0391w+0.1756)x^2+(-0.0356w^2+0.4178w-3.6422)x-0.0667w^2+0.8333w+58.103$, 100-w-x-y), and
the curve WN is represented by coordinates (x, $(-0.002061w^2+0.0218w-0.0301)x^2+(0.0556w^2-0.5821w-0.1108)x-0.4158w^2+4.7352w+43.383$, 100-w-x-y).

Item 29. The method, storage container, or refrigerating machine according to Items 1 to 8, wherein the refrigerant comprises HFO-1132(E) and HFO-1234yf, and

HFO-1132(E) is present in an amount of 35.0 to 65.0 mass%, and HFO-1234yf is present in an amount of 65.0 to 35.0 mass%, based on the total mass of HFO-1132(E) and HFO-1234yf.

Item 30. The method, storage container, or refrigerating machine according to Item 29, wherein in the refrigerant, HFO-1132(E) is present in an amount of 41.3 to 53.5 mass%, and HFO-1234yf is present in an amount of 58.7 to 46.5 mass%, based on the total mass of HFO-1132(E) and HFO-1234yf.

Item 31. The method, storage container, or refrigerating machine according to Items 1 to 8, wherein the refrigerant comprises HFO-1132(E) and HFO-1234yf, and

HFO-1132(E) is present in an amount of 40.5 to 49.2 mass%, and HFO-1234yf is present in an amount of 59.5 to 50.8 mass%, based on the total mass of HFO-1132(E) and HFO-1234yf.

Item 32. The method, storage container, or refrigerating machine according to Items 1 to 8, wherein the refrigerant comprises HFO-1132(E) and HFO-1234yf, and

HFO-1132(E) is present in an amount of 31.1 to 39.8 mass%, and HFO-1234yf is present in an amount of 68.9 to 60.2 mass%, based on the total mass of HFO-1132(E) and HFO-1234yf.

Item 33. The method, storage container, or refrigerating machine according to Item 32, wherein in the refrigerant, HFO-1132(E) is present in an amount of 31.1 to 37.9 mass%, and HFO-1234yf is present in an amount of 68.9 to 62.1 mass%, based on the total mass of HFO-1132(E) and HFO-1234yf.

Item 34. The method, storage container, or refrigerating machine according to any one of Items 29 to 33, wherein the refrigerant consists of HFO-1132(E) and HFO-1234yf.

Item 35. The refrigerating machine according to any one of Items 29 to 34, which has a refrigeration cycle in which the evaporating temperature is -75 to -5°C.

Item 36. The method, storage container, or refrigerating machine according to Items 1 to 8, wherein the refrigerant comprises HFO-1132(E) and HFO-1234yf, and

HFO-1132(E) is present in an amount of 21.0 to 28.4 mass%, and HFO-1234yf is present in an amount of 79.0 to 71.6 mass%, based on the total mass of HFO-1132(E) and HFO-1234yf.

Item 37. The method, storage container, or refrigerating machine according to Item 36, wherein the refrigerant consists of HFO-1132(E) and HFO-1234yf.

Item 38. The method, storage container, or refrigerating machine according to Items 1 to 8, wherein the refrigerant comprises HFO-1132(E) and HFO-1234yf, and

HFO-1132(E) is present in an amount of 12.1 to 72.0 mass%, and HFO-1234yf is present in an amount of 87.9 to 28.0 mass%, based on the total mass of HFO-1132(E) and HFO-1234yf.

Item 39. The method, storage container, or refrigerating machine according to Item 38, wherein the refrigerant consists of HFO-1132(E) and HFO-1234yf.

Item 40. The refrigerating machine according to Item 38 or 39, which is an air-conditioning system for vehicles.

Item 41. The air-conditioning system for vehicles according to Item 40, wherein the air-conditioning system for vehicles is for gasoline vehicles, hybrid vehicles, electric vehicles, or hydrogen vehicles.

Advantageous Effects of Invention

The present disclosure improves the stability of HFO-1132 in a refrigerant containing HFO-1132 during storage and during operation of a refrigerating machine comprising the refrigerant.

Brief Description of Drawings

Fig. 1 is a schematic view of an apparatus used in a flammability test.

Fig. 2 is a diagram showing points A to M and O, and line segments that connect these points in a ternary composition diagram in which the sum of HFO-1132(E), HFO-1123, and R1234yf is 100 mass%.

Fig. 3 is a diagram showing points A to C, B', and O, and line segments that connect these points in a ternary composition diagram in which the sum of HFO-1132(E), HFO-1123, and R1234yf is 100 mass%.

Fig. 4 is a diagram showing points A to C, B', and O, and line segments that connect these points in a ternary composition diagram in which the sum of HFO-1132(E), HFO-1123, and R1234yf is 95 mass% (R32 is present in an amount of 5 mass%).

Fig. 5 is a diagram showing points A to C, B', and O, and line segments that connect these points in a ternary composition diagram in which the sum of HFO-1132(E), HFO-1123, and R1234yf is 90 mass% (R32 is present in an amount of 10 mass%).

Fig. 6 is a diagram showing points A to C, B', and O, and line segments that connect these points in a ternary composition diagram in which the sum of HFO-1132(E), HFO-1123, and R1234yf is 85.7 mass% (R32 is present in an amount of 14.3 mass%).

Fig. 7 is a diagram showing points A to C, B', and O, and line segments that connect these points in a ternary composition diagram in which the sum of HFO-1132(E), HFO-1123, and R1234yf is 83.5 mass% (R32 is present in an amount of 16.5 mass%).

Fig. 8 is a diagram showing points A to C, B', and O, and line segments that connect these points in a ternary composition diagram in which the sum of HFO-1132(E), HFO-1123, and R1234yf is 80.8 mass% (R32 is present in an amount of 19.2 mass%).

Fig. 9 is a diagram showing points A to C, B', and O, and line segments that connect these points in a ternary composition diagram in which the sum of HFO-1132(E), HFO-1123, and R1234yf is 78.2 mass% (R32 is present in an amount of 21.8 mass%).

Fig. 10 is a diagram showing points A to K and O to R, and line segments that connect these points in a ternary composition diagram in which the sum of HFO-1132(E), R32, and R1234yf is 100 mass%.

Fig. 11 is a diagram showing points A to D, A' to D', and O, and line segments that connect these points in a ternary composition diagram in which the sum of HFO-1132(E), HFO-1123, and R32 is 100 mass%.

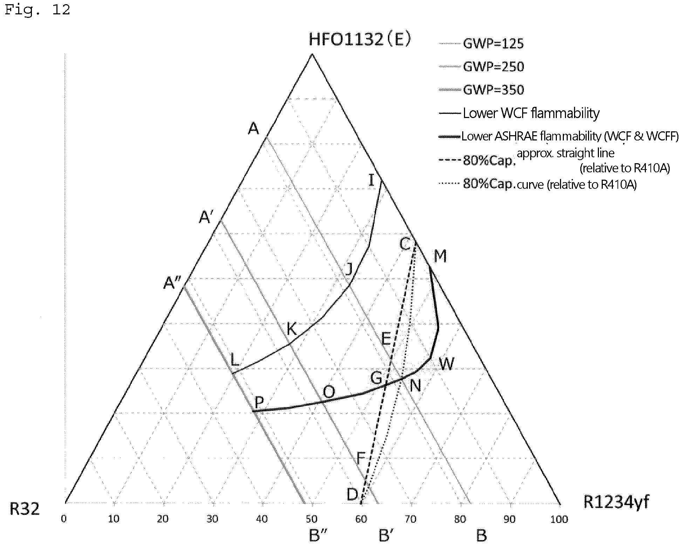

Fig. 12 is a diagram showing points and line segments that define the refrigerant according to the present disclosure in a ternary composition diagram in which the sum of R32, HFO-1132(E), and R1234yf is 100 mass%.

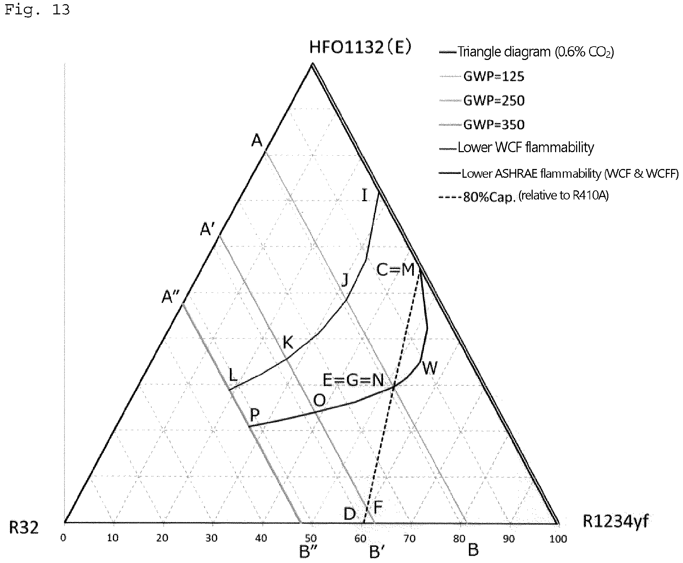

Fig. 13 is a diagram showing points and line segments that define the refrigerant according to the present disclosure in a ternary composition diagram in which the sum of R32, HFO-1132(E), and R1234yf is 99.4 mass% ($CO_2$ is present in an amount of 0.6 mass%).

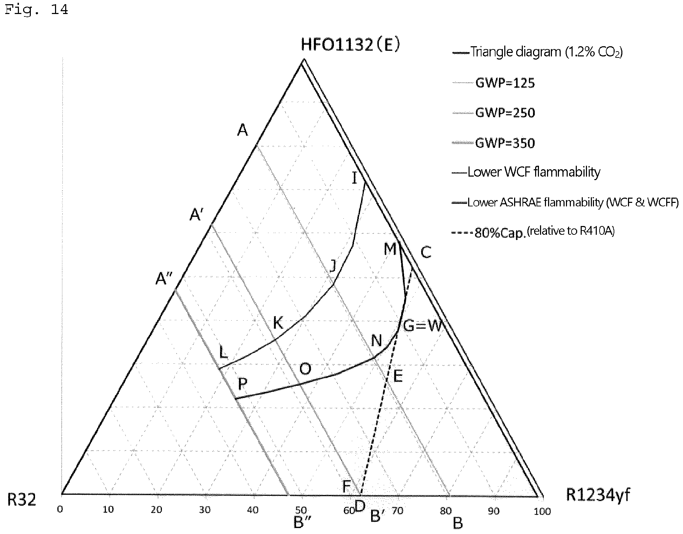

Fig. 14 is a diagram showing points and line segments that define the refrigerant according to the present disclosure in a ternary composition diagram in which the sum of R32, HFO-1132(E), and R1234yf is 98.8 mass% ($CO_2$ is present in an amount of 1.2 mass%).

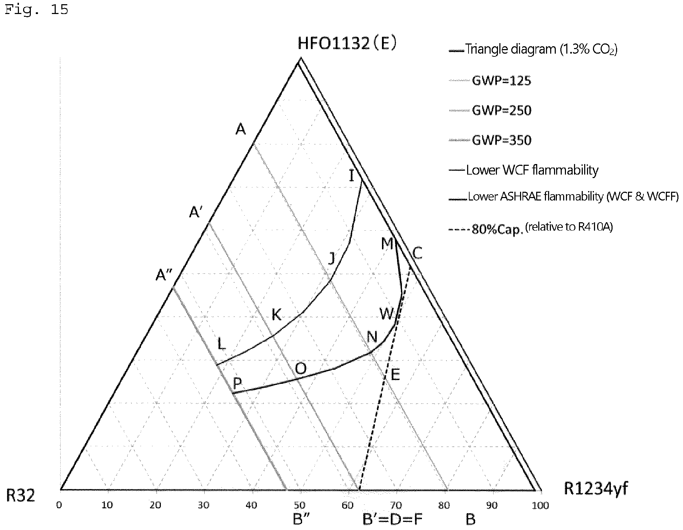

Fig. 15 is a diagram showing points and line segments that define the refrigerant according to the present disclosure in a ternary composition diagram in which the sum of R32, HFO-1132(E), and R1234yf is 98.7 mass% ($CO_2$ is present in an amount of 1.3 mass%).

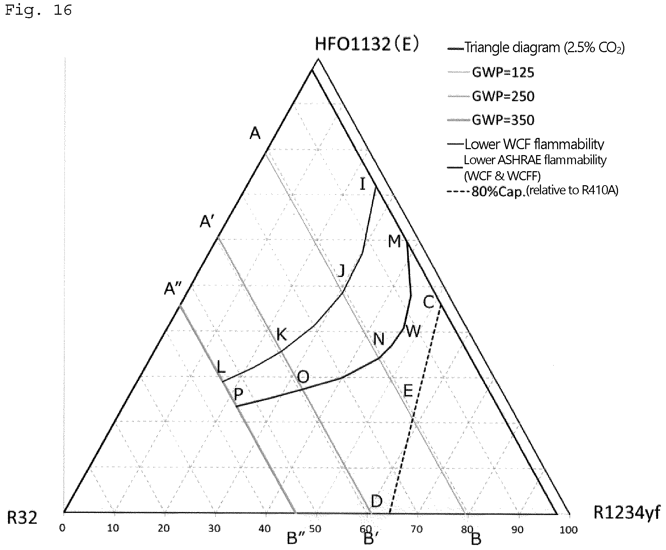

Fig. 16 is a diagram showing points and line segments that define the refrigerant according to the present disclosure in a ternary composition diagram in which the sum of R32, HFO-1132(E), and R1234yf is 97.5 mass% ($CO_2$ is present in an amount of 2.5 mass%).

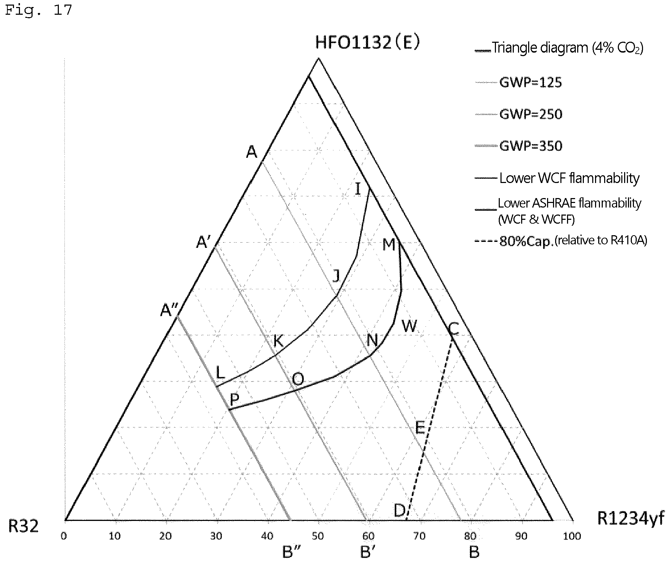

Fig. 17 is a diagram showing points and line segments that define the refrigerant according to the present disclosure in a ternary composition diagram in which the sum of R32, HFO-1132(E), and R1234yf is 96 mass% ($CO_2$ is present in an amount of 4 mass%).

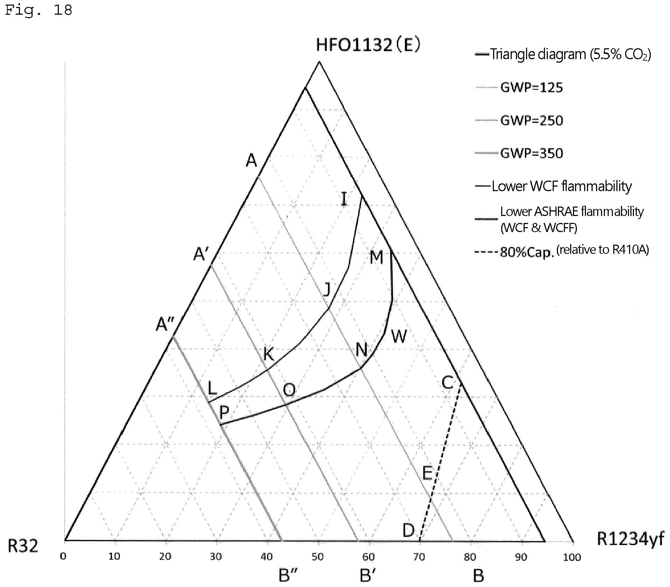

Fig. 18 is a diagram showing points and line segments that define the refrigerant according to the present disclosure in a ternary composition diagram in which the sum of R32, HFO-1132(E), and R1234yf is 94.5 mass% ($CO_2$ is present in an amount of 5.5 mass%).

Fig. 19 is a diagram showing points and line segments that define the refrigerant according to the present disclosure in a ternary composition diagram in which the sum of R32, HFO-1132(E), and R1234yf is 93 mass% ($CO_2$ is present in an amount of 7 mass%).

Fig. 20 is a schematic view of an experimental apparatus for examining flammability (flammable or non-flammable) .

Description of Embodiments

The present inventors conducted intensive studies to solve the above problem, and consequently found that the problem can be solved by a method for allowing a refrigerant comprising HFO-1132 and oxygen to coexist in a gas phase, the concentration of oxygen in the gas phase at a temperature of 25°C being 1000 volume ppm or less.

The present disclosure has been completed as a result of further research based on this finding. The present disclosure includes the following embodiments.

Definition of Terms

In the present specification, when only a compound name (e.g., "HFO-1132") is described for a compound that has isomers, without specifying the isomers, the sum of the isomers of the compound contained in the refrigerant used in the present disclosure is intended, unless there are special circumstances. For example, when the refrigerant used in the present disclosure contains HFO-1132(E) alone, "HFO-1132" refers to HFO-1132(E), and when the refrigerant used in the present disclosure contains both HFO-1132(E) and HFO-1132(Z), "HFO-1132" refers to the sum of HFO-1132(E) and HFO-1132(Z).

In the present specification, the term "refrigerant" includes at least compounds that are specified in ISO 817 (International Organization for Standardization), and that are given a refrigerant number (ASHRAE number) representing the type of refrigerant with "R" at the beginning; and further includes refrigerants that have properties equivalent to those of such refrigerants, even though a refrigerant number is not yet given. Refrigerants are broadly divided into fluorocarbon compounds and non-fluorocarbon compounds in terms of the structure of the compounds. Fluorocarbon compounds include chlorofluorocarbons (CFC), hydrochlorofluorocarbons (HCFC), and hydrofluorocarbons (HFC). Non-fluorocarbon compounds include propane (R290), propylene (R1270), butane (R600), isobutane (R600a), carbon dioxide (R744), ammonia (R717), and the like.

In the present specification, the term "refrigerating machine" refers to machines in general that draw heat from an object or space to make its temperature lower than the temperature of ambient air, and maintain a low temperature. In other words, refrigerating machines refer to conversion machines that gain energy from the outside to do work, and that perform energy conversion, in order to transfer heat from where the temperature is lower to where the temperature is higher.

1. Refrigerant Comprising HFO-1132

The refrigerant used in the present disclosure may be a refrigerant comprising HFO-1132. The refrigerant used in the present disclosure may be a refrigerant consisting of HFO-1132.

HFO-1132 may be trans-1,2-difluoroethylene (HFO-1132(E)) or cis-1,2-difluoroethylene (HFO-1132(Z)), or may be a mixture thereof.

The refrigerant used in the present disclosure may be a mixed refrigerant comprising HFO-1132 and further comprising an additional refrigerant.

Examples of additional refrigerants include trifluoroethylene (HFO-1123), difluoromethane (R32), 2,3,3,3-tetrafluoro-1-propene (R1234yf), 1,3,3,3-tetrafluoro-1-propene (R1234ze), $CO_2$, and the like. R1234ze may be trans-1,3,3,3-tetrafluoro-1-propene (R1234ze(E)), cis-1,3,3,3-tetrafluoro-1-propene (R1234ze(Z)), or a mixture thereof.

The refrigerant used in the present disclosure may further comprise other additional refrigerants in addition to the above specific additional refrigerants. The refrigerant according to the present disclosure may comprise HFO-1132 and the above specific additional refrigerants in a total amount of 99.5 mass% or more, 99.75 mass% or more, or 99.9 mass% or more, based on the entire refrigerant. The refrigerant according to the present disclosure may comprise HFO-1132 in an amount of 99.5 mass% or more, 99.75 mass% or more, or 99.9 mass% or more, based on the entire refrigerant.

Examples of the mixed refrigerant include those containing at least one member selected from the group consisting of the above additional refrigerants. Examples of the mixed refrigerant include a mixed refrigerant of HFO-1132 and HFO-1123, a mixed refrigerant of HFO-1132, HFO-1123, and R32, a mixed refrigerant of HFO-1132, HFO-1123, and R1234yf, a mixed refrigerant of HFO-1132, R32, and R1234yf, a mixed refrigerant of HFO-1132, HFO-1123, R32, and R1234yf, a mixed refrigerant of HFO-1132, HFO-1123, and R1234ze, a mixed refrigerant of HFO-1132, R32, and R1234ze, a mixed refrigerant of HFO-1132, HFO-1123, R32, and R1234ze, a mixed refrigerant of HFO-1132, HFO-1123, R1234yf, and R1234ze, a mixed refrigerant of HFO-1132, R32, R1234yf, and R1234ze, a mixed refrigerant of HFO-1132, HFO-1123, R32, R1234yf, and R1234ze, a mixed refrigerant of HFO-1132, R32, R1234yf, and $CO_2$, a mixed refrigerant of HFO-1132, R32, R1234ze, and $CO_2$, a mixed refrigerant of HFO-1132, R32, R1234yf, R1234ze, and $CO_2$, a mixed refrigerant of HFO-1132 and R1234yf, and the like. More specific examples include Refrigerants A to E and Refrigerants 1 to 5 described below.

According to the present disclosure, the stability of HFO-1132 can be improved by setting the concentration of oxygen coexisting with HFO-1132 in the gas phase to a specific concentration or lower. It will be understood that in order to obtain this effect, it is important to set the oxygen concentration of the gas phase containing HFO-1132 to a specific concentration or less. Therefore, it will be understood that the same effect would be obtained regardless of the type of additional refrigerant contained in the refrigerant.

2. Method for Allowing Refrigerant Comprising HFO-1132 and Oxygen to Coexist in Gas Phase

The present disclosure relates to a method for allowing a refrigerant comprising HFO-1132 and oxygen to coexist in a gas phase, the concentration of oxygen in the gas phase at a temperature of 25°C being 1000 volume ppm or less.

According to the above method, the stability of HFO-1132 can be improved by setting the concentration of oxygen coexisting with HFO-1132 in the gas phase to a specific concentration or lower. Improvement of the stability of HFO-1132 means that the polymerization reaction and/or self-decomposition reaction is suppressed.

In terms of easily obtaining this effect, the temperature of the gas phase is preferably less than 80°C, more preferably less than 60°C, and even more preferably less than 40°C.

In the present disclosure, a refrigerant comprising HFO-1132 and oxygen can be allowed to coexist in the gas phase in a closed container. In this case, the refrigerant comprising HFO-1132 can be stored in a closed container while improving the stability of HFO-1132. When the refrigerant comprising HFO-1132 is stored in a closed container in this way, the polymerization reaction that may occur during storage can be suppressed. In terms of suppressing the polymerization reaction, the concentration of oxygen in the gas phase at a temperature of 25°C is preferably 1000 volume ppm or less, more preferably 400 volume ppm or less, and even more preferably 200 volume ppm or less.

When the refrigerant comprising HFO-1132 is stored in a closed container in this way, the gas phase preferably coexists with a liquid phase containing the refrigerant; that is, the refrigerant is preferably present in a gas-liquid state. In this case, the refrigerant comprising HFO-1132 has a saturation vapor pressure in the gas phase.

When the refrigerant comprising HFO-1132 is stored in a closed container, the storage period can be, for example, 1 day or more. The storage period is preferably 10 days or more, more preferably 100 days or more, and even more preferably 1000 days or more. The storage temperature can be set so that the temperature of the gas phase is -50°C to 200°C, for example. As the storage temperature, the temperature of the gas phase is preferably -20°C to 180°C etc., more preferably 0°C to 180°C etc., and even more preferably 15°C to 180°C etc.

As the closed container, a wide range of closed containers generally used for storing refrigerants can be used. Such a closed container is generally a closed container capable of enclosing a gas-liquid mixture under internal pressure. The closed container may be a storage tank used after being fixed, or a filling cylinder used for transportation. Filling cylinders include secondary filling cylinders. The material of the part of the closed container that comes into contact with the refrigerant is not limited, and examples include carbon steels, manganese steels, stainless steels, low alloy steels, aluminum alloys, and the like. Examples of low alloy steels include chromium molybdenum steels.

In the present disclosure, the refrigerant comprising HFO-1132 and oxygen may be allowed to coexist in the gas phase in a refrigerating machine. In this case, the refrigerating machine can be operated using the refrigerant as a working fluid. When the refrigerating machine is operated in this way using the refrigerant comprising HFO-1132 as a working fluid, the self-decomposition reaction that may occur during operation can be suppressed. The self-decomposition reaction is facilitated by the decomposition of the refrigerant by a dehydrofluorination reaction in the presence of oxygen at a high temperature in the refrigerating machine, resulting in the formation of radicals. In terms of suppressing the self-decomposition reaction, the concentration of oxygen in the gas phase at a temperature of 25°C is preferably 1000 volume ppm or less, more preferably 400 volume ppm or less, and even more preferably 200 volume ppm or less.

When the refrigerating machine is operated in this way using the refrigerant comprising HFO-1132 as a working fluid, the gas phase preferably coexists with a liquid phase containing the refrigerant in at least part of the refrigerating machine; that is, the refrigerant is preferably present in a gas-liquid state. In this case, the refrigerant comprising HFO-1132 has a saturation vapor pressure in the gas phase.

As the refrigerating machine, general refrigerating machines can be widely used.

3. Storage Container

The storage container according to the present disclosure is a storage container of a refrigerant comprising HFO-1132, in which the refrigerant and oxygen coexist in the gas phase, the concentration of oxygen in the gas phase at a temperature of 25°C being 1000 volume ppm or less.

As described above, the stability of HFO-1132 is improved in the storage container.

4. Refrigerants A to E

Refrigerants A to E used in the present disclosure are described in detail below.

The disclosures of Refrigerant A, Refrigerant B, Refrigerant C, Refrigerant D, and Refrigerant E are independent from each other. Thus, the alphabetical letters used for points and line segments, as well as the numbers used for Examples and Comparative Examples, are all independent in each of Refrigerant A, Refrigerant B, Refrigerant C, Refrigerant D, and Refrigerant E. For example, Example 1 of Refrigerant A and Example 1 of Refrigerant B each represent an example according to a different embodiment.

4.1. Refrigerant A

Refrigerant A according to the present disclosure is a mixed refrigerant comprising trans-1,2-difluoroethylene (HFO-1132(E)), trifluoroethylene (HFO-1123), and 2,3,3,3-tetrafluoro-1-propene (R1234yf).

Refrigerant A according to the present disclosure has various properties that are desirable as an R410A-alternative refrigerant, i.e., a refrigerating capacity and a coefficient of performance that are equivalent to those of R410A, and a sufficiently low GWP.

Refrigerant A according to the present disclosure is a composition comprising HFO-1132(E) and R1234yf, and optionally further comprising HFO-1123, and may further satisfy the following requirements. This Refrigerant A also has various properties that are desirable as an R410A-alternative refrigerant, i.e., a refrigerating capacity and a coefficient of performance that are equivalent to those of R410A, and a sufficiently low GWP.

Requirements

When the mass% of HFO-1132(E), HFO-1123, and R1234yf based on their sum is respectively represented by x, y, and z,

coordinates (x,y,z) in a ternary composition diagram in which the sum of HFO-1132(E), HFO-1123, and R1234yf is 100 mass% are within the range of a figure surrounded by line segments OD, DG, GH, and HO that connect the following 4 points:

point D (87.6, 0.0, 12.4),
point G (18.2, 55.1, 26.7),
point H (56.7, 43.3, 0.0), and
point O (100.0, 0.0, 0.0),
or on the line segments OD, DG, and GH (excluding the points O and H) ;

the line segment DG is represented by coordinates $(0.0047y^2-1.5177y+87.598, y, -0.0047y^2+0.5177y+12.402)$,
the line segment GH is represented by coordinates $(-0.0134z^2-1.0825z+56.692, 0.0134z^2+0.0825z+43.308, z)$, and
the lines HO and OD are straight lines. When the requirements above are satisfied, Refrigerant A according to the present disclosure has a refrigerating capacity ratio of 92.5% or more relative to that of R410A, and a COP ratio of 92.5% or more relative to that of R410A.

Refrigerant A according to the present disclosure is preferably a refrigerant wherein

when the mass% of HFO-1132(E), HFO-1123, and R1234yf based on their sum is respectively represented by x, y, and z,
coordinates (x,y,z) in a ternary composition diagram in which the sum of HFO-1132(E), HFO-1123, and R1234yf is 100 mass% are within the range of a figure surrounded by line segments LG, GH, HI, and IL that connect the following 4 points:

point L (72.5, 10.2, 17.3),
point G (18.2, 55.1, 26.7),
point H (56.7, 43.3, 0.0), and
point I (72.5, 27.5, 0.0),
or on the line segments LG, GH, and IL (excluding the points H and I);

the line segment LG is represented by coordinates $(0.0047y^2-1.5177y+87.598, y, -0.0047y^2+0.5177y+12.402)$,
the line segment GH is represented by coordinates $(-0.0134z^2-1.0825z+56.692, 0.0134z^2+0.0825z+43.308, z)$, and
the line segments HI and IL are straight lines. When the requirements above are satisfied, Refrigerant A according to the present disclosure has a refrigerating capacity ratio of 92.5% or more relative to that of R410A, and a COP ratio of 92.5% or more relative to that of R410A; furthermore, the refrigerant has a slight flammability (Class 2L) according to the ASHRAE standard.

Refrigerant A according to the present disclosure is preferably a refrigerant wherein

when the mass% of HFO-1132(E), HFO-1123, and R1234yf based on their sum is respectively represented by x, y, and z, coordinates (x,y,z) in a ternary composition diagram in which the sum of HFO-1132(E), HFO-1123, and R1234yf is 100 mass% are within the range of a figure surrounded by line segments OD, DE, EF, and FO that connect the following 4 points:

point D (87.6, 0.0, 12.4),
point E (31.1, 42.9, 26.0),
point F (65.5, 34.5, 0.0), and
point O (100.0, 0.0, 0.0),
or on the line segments OD, DE, and EF (excluding the points O and F) ;

the line segment DE is represented by coordinates $(0.0047y^2-1.5177y+87.598, y, -0.0047y^2+0.5177y+12.402)$,
the line segment EF is represented by coordinates $(-0.0064z^2-1.1565z+65.501, 0.0064z^2+0.1565z+34.499, z)$, and
the line segments FO and OD are straight lines. When the requirements above are satisfied, Refrigerant A according to the present disclosure has a refrigerating capacity ratio of 93.5% or more relative to that of R410A, and a COP ratio of 93.5% or more relative to that of R410A.

Refrigerant A according to the present disclosure is preferably a refrigerant wherein

when the mass% of HFO-1132(E), HFO-1123, and R1234yf based on their sum is respectively represented by x, y, and z,
coordinates (x,y,z) in a ternary composition diagram in which the sum of HFO-1132(E), HFO-1123, and R1234yf is 100 mass% are within the range of a figure surrounded by line segments LE, EF, FI, and IL that connect the following 4 points:

point L (72.5, 10.2, 17.3),
point E (31.1, 42.9, 26.0),
point F (65.5, 34.5, 0.0), and
point I (72.5, 27.5, 0.0),
or on the line segments LE, EF, and IL (excluding the points F and I);

the line segment LE is represented by coordinates $(0.0047y^2-1.5177y+87.598, y, -0.0047y^2+0.5177y+12.402)$,
the line segment EF is represented by coordinates $(-0.0134z^2-1.0825z+56.692, 0.0134z^2+0.0825z+43.308, z)$, and
the line segments FI and IL are straight lines. When the requirements above are satisfied, Refrigerant A according to the present disclosure has a refrigerating capacity ratio of 93.5% or more relative to that of R410A, and a COP ratio of 93.5% or more relative to that of R410A; furthermore, the refrigerant has a slight flammability (Class 2L) according to the ASHRAE standard.

Refrigerant A according to the present disclosure is preferably a refrigerant wherein

when the mass% of HFO-1132(E), HFO-1123, and R1234yf based on their sum is respectively represented by x, y, and z,
coordinates (x,y,z) in a ternary composition diagram in which the sum of HFO-1132(E), HFO-1123, and R1234yf is 100 mass% are within the range of a figure surrounded by line segments OA, AB, BC, and CO that connect the following 4 points:

point A (93.4, 0.0, 6.6),
point B (55.6, 26.6, 17.8),
point C (77.6, 22.4, 0.0), and
point O (100.0, 0.0, 0.0),
or on the line segments OA, AB, and BC (excluding the points O and C);

the line segment AB is represented by coordinates $(0.0052y^2-1.5588y+93.385, y, -0.0052y^2+0.5588y+6.615)$,
the line segment BC is represented by coordinates $(-0.0032z^2-1.1791z+77.593, 0.0032z^2+0.1791z+22.407, z)$, and
the line segments CO and OA are straight lines. When the requirements above are satisfied, Refrigerant A according to the present disclosure has a refrigerating capacity ratio of 95% or more relative to that of R410A, and a COP ratio of 95% or more relative to that of R410A.

Refrigerant A according to the present disclosure is preferably a refrigerant wherein

when the mass% of HFO-1132(E), HFO-1123, and R1234yf based on their sum is respectively represented by x, y, and z,

coordinates (x,y,z) in a ternary composition diagram in which the sum of HFO-1132(E), HFO-1123, and R1234yf is 100 mass% are within the range of a figure surrounded by line segments KB, BJ, and JK that connect the following 3 points:

point K (72.5, 14.1, 13.4),
point B (55.6, 26.6, 17.8), and
point J (72.5, 23.2, 4.3),
or on the line segments KB, BJ, and JK;

the line segment KB is represented by coordinates $(0.0052y^2-1.5588y+93.385, y, \text{ and } -0.0052y^2+0.5588y+6.615)$,
the line segment BJ is represented by coordinates $(-0.0032z^2-1.1791z+77.593, 0.0032z^2+0.1791z+22.407, z)$, and
the line segment JK is a straight line. When the requirements above are satisfied, Refrigerant A according to the present disclosure has a refrigerating capacity ratio of 95% or more relative to that of R410A, and a COP ratio of 95% or more relative to that of R410A; furthermore, the refrigerant has a slight flammability (Class 2L) according to the ASHRAE standard.

Refrigerant A according to the present disclosure may further comprise difluoromethane (R32) in addition to HFO-1132(E), HFO-1123, and R1234yf as long as the above properties and effects are not impaired. The content of R32 based on the entire Refrigerant A according to the present disclosure is not particularly limited and can be selected from a wide range. For example, when the R32 content of Refrigerant A according to the present disclosure is 21.8 mass%, the mixed refrigerant has a GWP of 150. Therefore, the R32 content can be 21.8 mass% or less. The R32 content of Refrigerant A according to the present disclosure may be, for example, 5 mass% or more, based on the entire refrigerant.

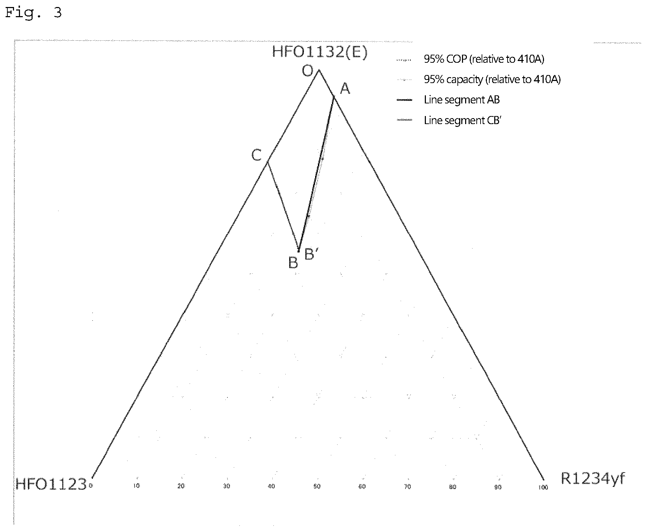

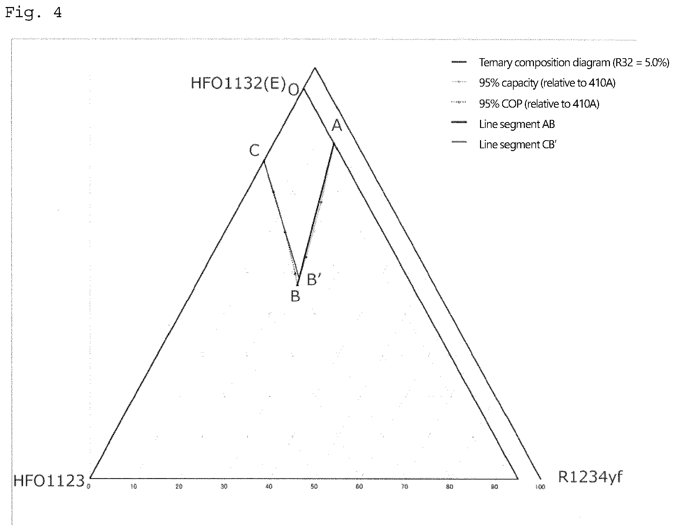

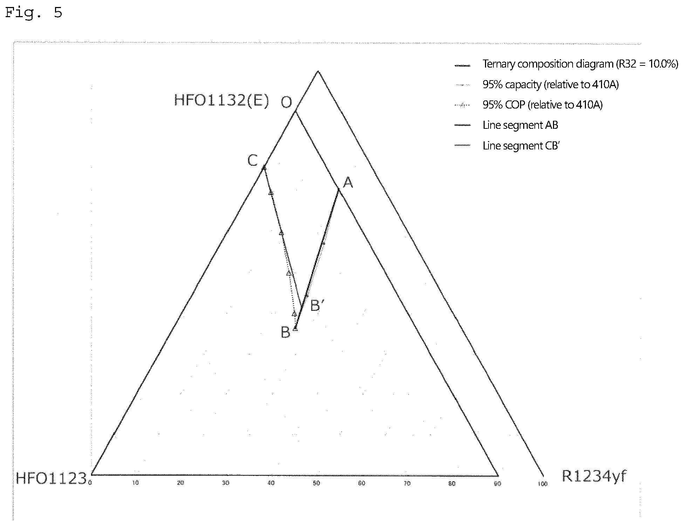

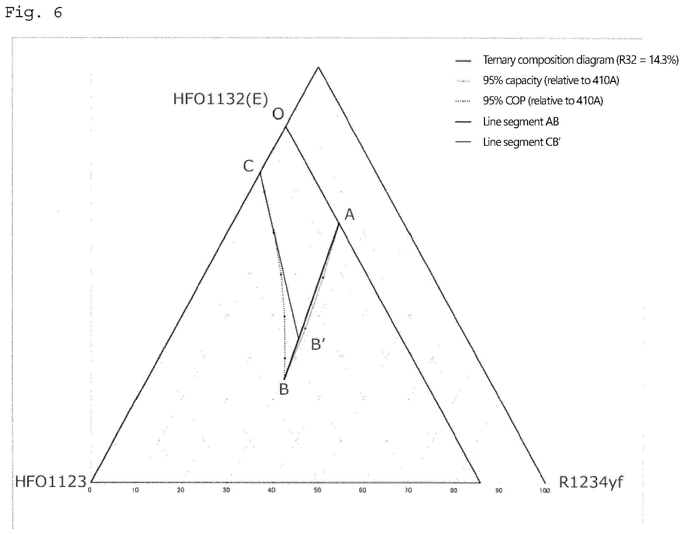

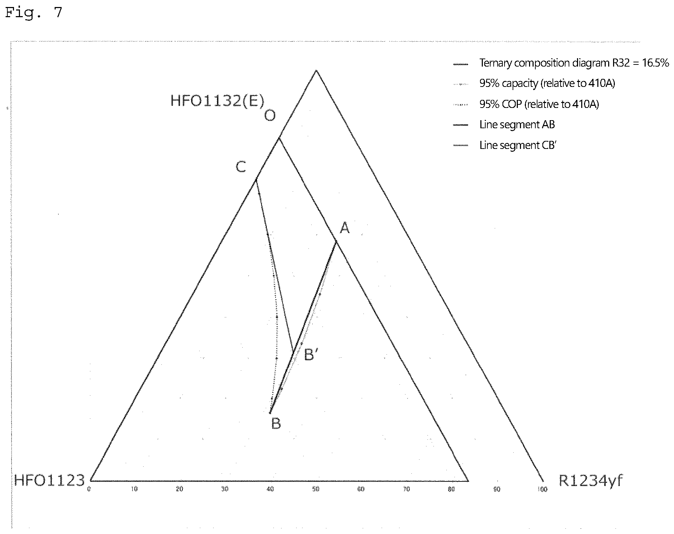

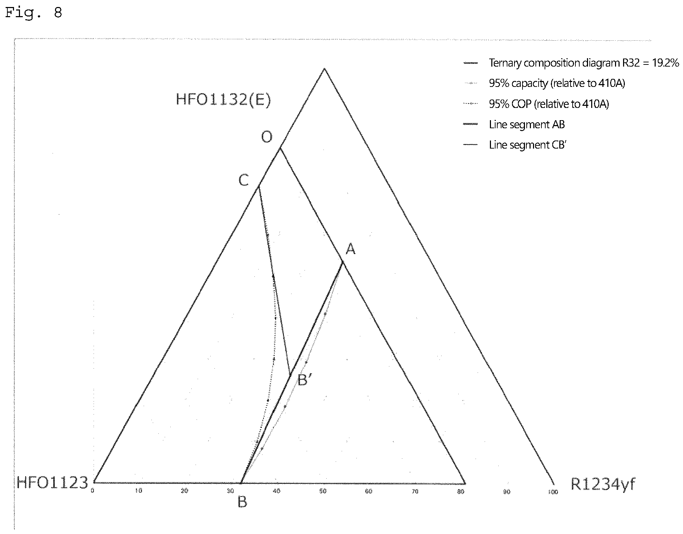

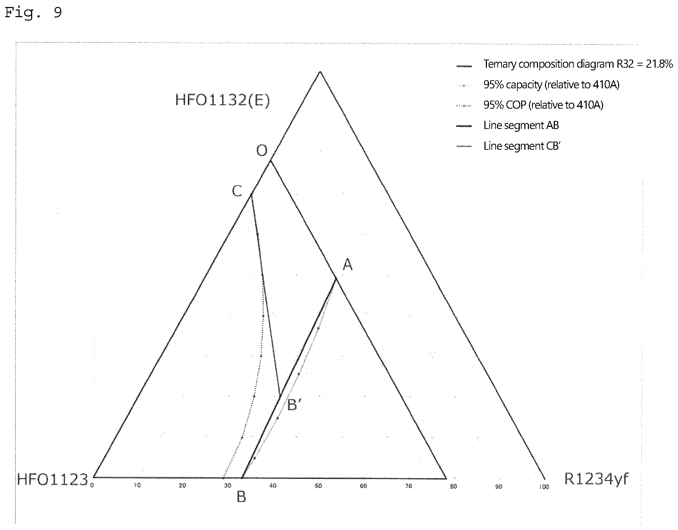

When Refrigerant A according to the present disclosure further contains R32 in addition to HFO-1132(E), HFO-1123, and R1234yf, the refrigerant may be a refrigerant wherein

when the mass% of HFO-1132(E), HFO-1123, R1234yf, and R32 based on their sum is respectively represented by x, y, z, and a,

if $0<a\leq10.0$, coordinates (x,y,z) in a ternary composition diagram (Figs. 3 to 9) in which the sum of HFO-1132(E), HFO-1123, and R1234yf is 100 mass% are within the range of a figure surrounded by straight lines that connect the following 4 points:

point A $(0.02a^2-2.46a+93.4, 0, -0.02a^2+2.46a+6.6)$,
point B' $(-0.008a^2-1.38a+56, 0.018a^2-0.53a+26.3, -0.01a^2+1.91a+17.7)$,
point C $(-0.016a^2+1.02a+77.6, 0.016a^2-1.02a+22.4, 0)$, and
point O (100.0, 0.0, 0.0),
or on the straight lines OA, AB', and B'C (excluding the points O and C);

if $10.0<a\leq16.5$, coordinates (x,y,z) in the ternary composition diagram are within the range of a figure surrounded by straight lines that connect the following 4 points:

point A $(0.0244a^2-2.5695a+94.056, 0, -0.0244a^2+2.5695a+5.944)$,
point B' $(0.1161a^2-1.9959a+59.749, 0.014a^2-0.3399a+24.8, -0.1301a^2+2.3358a+15.451)$,
point C $(-0.0161a^2+1.02a+77.6, 0.0161a^2-1.02a+22.4, 0)$, and
point O (100.0, 0.0, 0.0),
or on the straight lines OA, AB', and B'C (excluding the points O and C); or

if $16.5<a\leq21.8$, coordinates (x,y,z) in the ternary composition diagram are within the range of a figure surrounded by straight lines that connect the following 4 points:

point A $(0.0161a^2-2.3535a+92.742, 0, -0.0161a^2+2.3535a+7.258)$,
point B' $(-0.0435a^2-0.0435a+50.406, -0.0304a^2+1.8991a-0.0661, 0.0739a^2-1.8556a+49.6601)$,
point C $(-0.0161a^2+0.9959a+77.851, 0.0161a^2-0.9959a+22.149, 0)$, and
point O (100.0, 0.0, 0.0),
or on the straight lines OA, AB', and B'C (excluding the points O and C).

Note that when point B in the ternary composition diagram is defined as a point where a refrigerating capacity ratio of 95% relative to that of R410A and a COP ratio of 95% relative to that of R410A are both achieved, point B' is the intersection of straight line AB and an approximate line formed by connecting the points where the COP ratio relative to that of R410A is 95%. When the requirements above are satisfied, Refrigerant A according to the present disclosure has a refrigerating capacity ratio of 95% or more relative to that of R410A, and a COP ratio of 95% or more relative to that of R410A.

Refrigerant A according to the present disclosure may further comprise other additional refrigerants in addition to HFO-1132(E), HFO-1123, R1234yf, and R32 as long as the above properties and effects are not impaired. In this respect, Refrigerant A according to the present disclosure preferably comprises HFO-1132(E), HFO-1123, R1234yf, and R32 in a total amount of 99.5 mass% or more, more preferably 99.75 mass% or more, and still more preferably 99.9 mass% or more, based on the entire Refrigerant A.

Refrigerant A according to the present disclosure may comprise HFO-1132(E), HFO-1123, and R1234yf in a total amount of 99.5 mass% or more, 99.75 mass% or more, or 99.9 mass% or more, based on the entire Refrigerant A.

Refrigerant A according to the present disclosure may comprise HFO-1132(E), HFO-1123, R1234yf, and R32 in a total amount of 99.5 mass% or more, 99.75 mass% or more, or 99.9 mass% or more, based on the entire Refrigerant A.

Additional refrigerants are not particularly limited and can be widely selected. The mixed refrigerant may contain one additional refrigerant, or two or more additional refrigerants.

Refrigerant A according to the present disclosure is suitable for use as an alternative refrigerant for R410A.

Examples of Refrigerant A

The present disclosure is described in more detail below with reference to Examples of Refrigerant A. However, Refrigerant A according to the present disclosure is not limited to the Examples.

Mixed refrigerants were prepared by mixing HFO-1132(E), HFO-1123, and R1234yf at mass% based on their sum shown in Tables 1 to 5.

The COP ratio and the refrigerating capacity ratio of the mixed refrigerants relative to those of R410 were determined. The calculation conditions were as follows.

Evaporating temperature: 5°C
Condensation temperature: 45°C
Degree of superheating: 1 K
Degree of subcooling: 5 K
$E_{comp}$ (compressive modulus): 0.7 kWh

Tables 1 to 5 show these values together with the GWP of each mixed refrigerant.

Table 1

| Item | Unit | Comp. Ex. 1 | Example 1 | Example 2 | Example 3 | Example 4 | Example 5 | Example 6 |
|---|---|---|---|---|---|---|---|---|
| | | | A | | | | | B |
| HFO-1132(E) | mass% | R410A | 93.4 | 85.7 | 78.3 | 71.2 | 64.3 | 55.6 |
| HFO-1123 | mass% | | 0.0 | 5.0 | 10.0 | 15.0 | 20.0 | 26.6 |
| R1234yf | mass% | | 6.6 | 9.3 | 11.7 | 13.8 | 15.7 | 17.8 |
| GWP | - | 2088 | 1 | 1 | 1 | 1 | 1 | 2 |
| COP ratio | % (relative to R410A) | 100 | 98.0 | 97.5 | 96.9 | 96.3 | 95.8 | 95.0 |
| Refrigerating capacity ratio | % (relative to R410A) | 100 | 95.0 | 95.0 | 95.0 | 95.0 | 95.0 | 95.0 |

Table 2

| Item | Unit | Comp. Ex. 2 C | Example 7 | Example 8 | Example 9 |
|---|---|---|---|---|---|
| HFO-1132(E) | mass% | 77.6 | 71..6 | 65.5 | 59.2 |
| HFO-1123 | mass% | 22.4 | 23.4 | 24.5 | 25.8 |
| R1234yf | mass% | 0.0 | 5.0 | 10.0 | 15.0 |
| GWP | - | 1 | 1 | 1 | 1 |
| COP ratio | % (relative to R410A) | 95.0 | 95.0 | 95.0 | 95.0 |
| Refrigerating capacity ratio | % (relative to R410A) | 102.5 | 100.5 | 98.4 | 96.3 |

Table 3

| Item | Unit | Example 10 D | Example 11 | Example 12 | Example 13 | Example 14 | Example 15 | Example 16 G |
|---|---|---|---|---|---|---|---|---|
| HFO-1132(E) | mass% | 87.6 | 72.9 | 59.1 | 46.3 | 34.4 | 23.5 | 18.2 |
| HFO-1123 | mass% | 0.0 | 10.0 | 20.0 | 30.0 | 40.0 | 50.0 | 55.1 |
| R1234yf | mass% | 12.4 | 17.1 | 20.9 | 23.7 | 25.6 | 26.5 | 26.7 |
| GWP | - | 1 | 2 | 2 | 2 | 2 | 2 | 2 |
| COP ratio | % (relative to R410A) | 98.2 | 97.1 | 95.9 | 94.8 | 93.8 | 92.9 | 92.5 |
| Refrigerating capacity ratio | % (relative to R410A) | 92.5 | 92.5 | 92.5 | 92.5 | 92.5 | 92.5 | 92.5 |

Table 4

| Item | Unit | Comp. Ex. 3 H | Example 17 | Example 18 | Comp. Ex. 4 F | Example 19 | Example 20 | Example 21 E |
|---|---|---|---|---|---|---|---|---|
| HFO-1132(E) | mass% | 56.7 | 44.5 | 29.7 | 65.5 | 53.3 | 39.8 | 31.1 |
| HFO-1123 | mass% | 43.3 | 45.5 | 50.3 | 34.5 | 36.7 | 40.2 | 42.9 |
| R1234yf | mass% | 0.0 | 10.0 | 20.0 | 0.0 | 10.0 | 20.0 | 26.0 |
| GWP | - | 1 | 1 | 2 | 1 | 1 | 2 | 2 |
| COP ratio | % (relative to R410A) | 92.5 | 92.5 | 92.5 | 93.5 | 93.5 | 93.5 | 93.5 |
| Refrigerating capacity ratio | % (relative to R410A) | 105.8 | 101.2 | 96.2 | 104.5 | 100.2 | 95.5 | 92.5 |

Table 5

| Item | Unit | Comp. Ex. 5 | Example 22 | Example 23 | Example 24 | Comp. Ex. 6 |
|---|---|---|---|---|---|---|
| | | I | J | K | L | M |
| HFO-1132(E) | mass% | 72.5 | 72.5 | 72.5 | 72.5 | 72.5 |
| HFO-1123 | mass% | 27.5 | 23.2 | 14.1 | 10.2 | 0.0 |
| R1234yf | mass% | 0.0 | 4.3 | 13.4 | 17.3 | 27.5 |
| GWP | - | 1 | 1 | 1 | 2 | 2 |
| COP ratio | % (relative to R410A) | 94.4 | 95.0 | 96.4 | 97.1 | 98.8 |
| Refrigerating | % (relative | 103.5. | 100.8 | 95.0 | 92.5 | 85.7 |
| capacity ratio | to R410A) | | | | | |

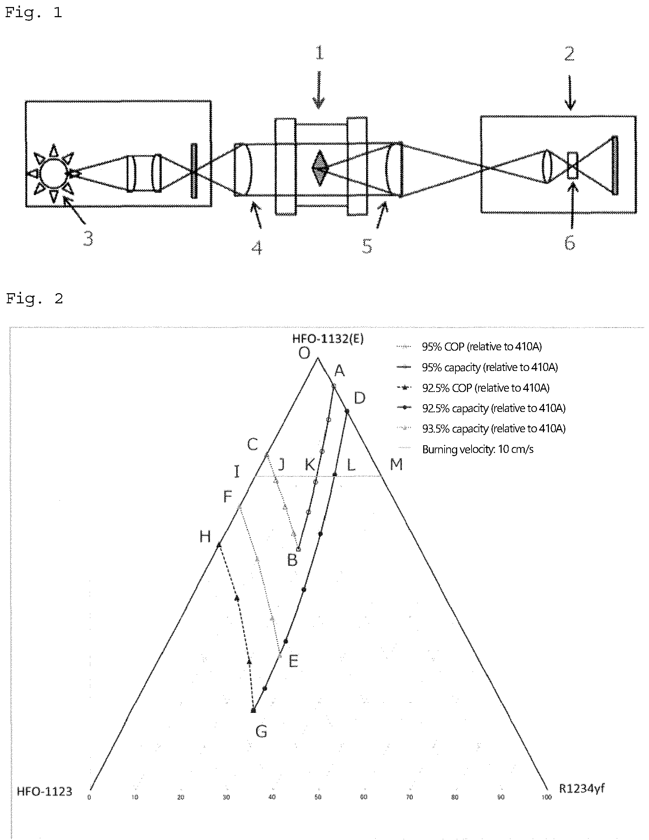

These results indicate that under the condition that the mass% of HFO-1132(E), HFO-1123, and R1234yf based on their sum is respectively represented by x, y, and z, when coordinates (x,y,z) in a ternary composition diagram in which the sum of HFO-1132(E), HFO-1123, and R1234yf is 100 mass% are within the range of a figure (Fig. 2) surrounded by line segments OD, DG, GH, and HO that connect the following 4 points:

point D (87.6, 0.0, 12.4),
point G (18.2, 55.1, 26.7),
point H (56.7, 43.3, 0.0), and
point O (100.0, 0.0, 0.0),
or on the line segments OD, DG, and GH (excluding the points O and H), the refrigerant has a refrigerating capacity ratio of 92.5% or more relative to that of R410A, and a COP ratio of 92.5% or more relative to that of R410A.

Likewise, the results indicate that when coordinates (x,y,z) are within the range of a figure (Fig. 2) surrounded by line segments OD, DE, EF, and FO that connect the following 4 points:

point D (87.6, 0.0, 12.4),
point E (31.1, 42.9, 26.0),
point F (65.5, 34.5, 0.0), and
point O (100.0, 0.0, 0.0),
or on the line segments OD, DE, and EF (excluding the points O and F), the refrigerant has a refrigerating capacity ratio of 93.5% or more relative to that of R410A, and a COP ratio of 93.5% or more relative to that of R410A.

Likewise, the results indicate that when coordinates (x,y,z) are within the range of a figure (Fig. 2) surrounded by line segments OA, AB, BC, and CO that connect the following 4 points:

point A (93.4, 0.0, 6.6),
point B (55.6, 26.6, 17.8),
point C (77.6, 22.4, 0.0), and
point O (100.0, 0.0, 0.0),
or on the line segments OA, AB, and BC (excluding the points O and C), the refrigerant has a refrigerating capacity ratio of 95% or more relative to that of R410A, and a COP ratio of 95% or more relative to that of R410A.

R1234yf contributes to reduction of flammability and deterioration of polymerization etc. in these compositions. Therefore, the composition according to the present disclosure preferably contains R1234yf.

Further, the burning velocity of these mixed refrigerants was measured according to ANSI/ASHRAE Standard 34-2013. Compositions that showed a burning velocity of 10 cm/s or less were determined to be Class 2L (slight flammability). These results clearly indicate that when the content of HFO-1132(E) in a mixed refrigerant of HFO-1132(E), HFO-1123, and R1234yf is 72.5 mass% or less based on their sum, the refrigerant can be determined to be Class 2L (slight flammability).

A burning velocity test was performed using the apparatus shown in Fig. 1 in the following manner. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps and stored on a PC. Mixed refrigerants were prepared by mixing HFO-1132(E), HFO-1123, R1234yf, and R32 in amounts shown in Tables 6 to 12, in terms of mass%, based on their sum.

The COP ratio and the refrigerating capacity ratio of these mixed refrigerants relative to those of R410A were determined. The calculation conditions were the same as described above. Tables 6 to 12 show these values together with the GWP of each mixed refrigerant.

Table 6

| Item | Unit | Comp. Ex. 1 | Comp. Ex. 7 | Comp. Ex. 8 | Comp. Ex. 9 | Example 25 | Comp. Ex. 10 | Example 26 | Example 27 | Comp. Ex. 11 |
| --- | --- | --- | --- | --- | --- | --- | --- | --- | --- | --- |
| | | | A | | | B' | B | | | C |
| HFO-1132(E) | mass% | R410A | 93.4 | 78.3 | 64.3 | 56.0 | 55.6 | 60.0 | 70.0 | 77.6 |
| HFO-1123 | mass% | | 0.0 | 10.0 | 20.0 | 26.3 | 26.6 | 25.6 | 23.7 | 22.4 |
| R1234yf | mass% | | 6.6 | 11.7 | 15.7 | 17.7 | 17.8 | 14.4 | 6.3 | 0.0 |
| R32 | mass% | | 0.0 | 0.0 | 0.0 | 0.0 | 0.0 | 0.0 | 0.0 | 0.0 |
| GWP | - | 2088 | 1 | 1.4 | 1.5 | 1.5 | 1.5 | 1.4 | 1.2 | 1.0 |
| COP ratio | % (relative to R410A) | 100 | 98.0 | 96.9 | 95.8 | 95.0 | 95.0 | 95.0 | 95.0 | 95.0 |
| Refrigerating capacity ratio | % (relative to R410A) | 100 | 95.0 | 95.0 | 95.0 | 95.0 | 95.0 | 96.5 | 100.0 | 102.5 |

EP 3 988 522 A1

23

Table 7

| Item | Unit | Comp. Ex. 12 A | Comp. Ex. 13 | Comp. Ex. 14 | Example 28 B' | Comp. Ex. 15 B | Example 29 | Example 30 | Comp. Ex. 16 C |
|---|---|---|---|---|---|---|---|---|---|
| HFO-1132(E) | mass% | 81.6 | 67.3 | 53.9 | 48.9 | 47.2 | 60.0 | 70.0 | 77.3 |
| HFO-1123 | mass% | 0.0 | 10.0 | 20.0 | 24.1 | 25.3 | 21.6 | 19.2 | 17.7 |
| R1234yf | mass% | 13.4 | 17.7 | 21.1 | 22.0 | 22.5 | 13.4 | 5.8 | 0.0 |
| R32 | mass% | 5.0 | 5.0 | 5.0 | 5.0 | 5.0 | 5.0 | 5.0 | 5.0 |
| GWP | - | 35 | 35 | 35 | 35 | 35 | 35 | 35 | 35 |
| COP ratio | % (relative to R410A) | 97.6 | 96.6 | 95.5 | 95.0 | 95.0 | 95.0 | 95.0 | 95.0 |
| Refrigerating capacity ratio | % (relative to R410A) | 95.0 | 95.0 | 95.0 | 104.4 | 95.0 | 99.0 | 102.1 | 104.4 |

Table 8

| Item | Unit | Comp. Ex. 17 | Comp. Ex. 18 | Comp. Ex. 19 | Example 31 | Comp. Ex. 20 | Example 32 | Example 33 | Com p. Ex. 21 |
|---|---|---|---|---|---|---|---|---|---|
| | | A | | | B' | B | | | C |
| HFO-1132(E) | mass% | 70.8 | 57.2 | 44.5 | 41.4 | 36.4 | 60.0 | 70.0 | 76.2 |
| HFO-1123 | mass% | 0.0 | 10.0 | 20.0 | 22.8 | 26.7 | 18.0 | 15.3 | 13.8 |
| R1234yf | mass% | 19.2 | 22.8 | 25.5 | 25.8 | 26.9 | 12.0 | 4.7 | 0.0 |
| R32 | mass% | 10.0 | 10.0 | 10.0 | 10.0 | 10.0 | 10.0 | 10.0 | 10.0 |
| GWP | - | 69 | 69 | 69 | 69 | 69 | 69 | 69 | 68 |
| COP ratio | % (relative to R410A) | 97.4 | 96.5 | 95.6 | 95.0 | 95.0 | 95.0 | 95.0 | 95.0 |
| Refrigerating capacity ratio | % (relative to R410A) | 95.0 | 95.0 | 95.0 | 106.2 | 95.0 | 101.5 | 104.4 | 106.2 |

Table 9

| Item | Unit | Comp. Ex. 22 | Comp. Ex. 23 | Comp. Ex. 24 | Example 34 | Comp. Ex. 25 | Example 35 | Example 36 | Com p. Ex. 26 |
|---|---|---|---|---|---|---|---|---|---|
| | | A | | | B' | B | | | C |
| HFO-1132(E) | mass% | 62.3 | 49.3 | 37.1 | 34.5 | 24.9 | 60.0 | 70.0 | 74.5 |
| HFO-1123 | mass% | 0.0 | 10.0 | 20.0 | 22.8 | 30.7 | 15.4 | 12.4 | 11.2 |
| R1234yf | mass% | 23.4 | 26.4 | 28.6 | 28.4 | 30.1 | 10.3 | 3.3 | 0.0 |
| R32 | mass% | 14.3 | 14.3 | 14.3 | 14.3 | 14.3 | 14.3 | 14.3 | 14.3 |
| GWP | - | 98 | 98 | 98 | 98 | 98 | 98 | 97 | 97 |
| COP ratio | % (relative to R410A) | 97.3 | 96.5 | 95.7 | 95.5 | 95.0 | 95.0 | 95.0 | 95.0 |
| Refrigerating capacity ratio | % (relative to R410A) | 95.0 | 95.0 | 95.0 | 95.4 | 95.0 | 103.7 | 106.5 | 107.7 |

Table 10

| Item | Unit | Comp. Ex. 27 | Comp. Ex. 28 | Comp. Ex. 29 | Example 37 | Comp. Ex. 30 | Example 38 | Example 39 | Com p. Ex. 31 |
|---|---|---|---|---|---|---|---|---|---|
| | | A | | | B' | B | | | C |
| HFO-1132(E) | mass% | 58.3 | 45.5 | 33.5 | 31.2 | 16.5 | 60.0 | 70.0 | 73.4 |

(continued)

| Item | Unit | Comp. Ex. 27 | Comp. Ex. 28 | Comp. Ex. 29 | Example 37 | Comp. Ex. 30 | Example 38 | Example 39 | Comp. Ex. 31 |
|---|---|---|---|---|---|---|---|---|---|
| | | A | | | B' | B | | | C |
| HFO-1123 | mass% | 0.0 | 10.0 | 20.0 | 23.0 | 35.5 | 14.2 | 11.1 | 10.1 |
| R1234yf | mass% | 25.2 | 28.0 | 30.0 | 29.3 | 31.5 | 9.3 | 2.4 | 0.0 |
| R32 | mass% | 16.5 | 16.5 | 16.5 | 16.5 | 16.5 | 16.5 | 16.5 | 16.5 |
| GWP | - | 113.0 | 113.1 | 113.1 | 113.1 | 113.2 | 112.5 | 112.3 | 112.2 |
| COP ratio | % (relative to R410A) | 97.4 | 96.6 | 95.9 | 95.6 | 95.0 | 95.0 | 95.0 | 95.0 |
| Refrigerating capacity ratio | % (relative to R410A) | 95.0 | 95.0 | 95.0 | 95.7 | 95.0 | 104.9 | 107.6 | 108.5 |

Table 11

| Item | Unit | Comp. Ex. 32 | Comp. Ex. 33 | Comp. Ex. 34 | Example 40 | Comp. Ex. 35 | Example 41 | Example 42 | Comp. Ex. 36 |
|---|---|---|---|---|---|---|---|---|---|
| | | A | | | B' | B | | | C |
| HFO-1132(E) | mass% | 53.5 | 41.0 | 29.3 | 25.8 | 0.0 | 50.0 | 60.0 | 71.7 |
| HFO-1123 | mass% | 0.0 | 10.0 | 20.0 | 25.2 | 48.8 | 16.8 | 12.9 | 9.1 |
| R1234yf | mass% | 27.3 | 29.8 | 31.5 | 29.8 | 32.0 | 14.0 | 7.9 | 0.0 |
| R32 | mass% | 19.2 | 19.2 | 19.2 | 19.2 | 19.2 | 19.2 | 19.2 | 19.2 |
| GWP | - | 131.2 | 131.3 | 131.4 | 131.3 | 131.4 | 130.8 | 130.6 | 130.4 |
| COP ratio | % (relative to R410A) | 97.4 | 96.7 | 96.1 | 97.8 | 95.0 | 95.0 | 95.0 | 95.0 |
| Refrigerating capacity ratio | % (relative to R410A) | 95.0 | 95.0 | 95.0 | 96.3 | 95.0 | 104.0 | 106.4 | 109.4 |

Table 12

| Item | Unit | Comp. Ex. 37 | Comp. Ex. 38 | Comp. Ex. 39 | Example 43 | Comp. Ex. 40 | Example 44 | Example 45 | Comp. Ex. 41 |
|---|---|---|---|---|---|---|---|---|---|
| | | A | | | B' | B | | | C |
| HFO-1132(E) | mass% | 49.1 | 36.9 | 25.5 | 20.0 | 0.0 | 50.0 | 60.0 | 69.7 |
| HFO-1123 | mass% | 0.0 | 10.0 | 20.0 | 26.9 | 45.3 | 15.8 | 11.9 | 8.5 |

(continued)

| Item | Unit | Comp. Ex. 37 | Comp. Ex. 38 | Comp. Ex. 39 | Example 43 | Comp. Ex. 40 | Example 44 | Example 45 | Comp. Ex. 41 |
|---|---|---|---|---|---|---|---|---|---|
| | | A | | | B' | B | | | C |
| R1234yf | mass% | 29.1 | 31.3 | 20.0 | 31.3 | 32.9 | 12.4 | 6.3 | 0.0 |
| R32 | mass% | 21.8 | 21.8 | 21.8 | 21.8 | 21.8 | 21.8 | 21.8 | 21.8 |
| GWP | - | 148.8 | 148.9 | 148.9 | 148.9 | 148.9 | 148.3 | 148.1 | 147.9 |
| COP ratio | % (relative to R410A) | 97.6 | 96.9 | 96.4 | 95.9 | 95.5 | 95.0 | 95.0 | 95.0 |
| Refrigerating capacity ratio | % (relative to R410A) | 95.0 | 95.0 | 95.0 | 98.4 | 95.0 | 105.6 | 108.0 | 110.3 |

These results indicate that the refrigerants according to the present disclosure that satisfy the following conditions have a refrigerating capacity ratio of 95% or more relative to that of R410A, and a COP ratio of 95% or more relative to that of R410A:

when the mass% of HFO-1132(E), HFO-1123, R1234yf, and R32 based on their sum is respectively represented by x, y, z, and a,

if $0 < a \leq 10.0$, coordinates (x,y,z) in a ternary composition diagram (Figs. 3 to 9) in which the sum of HFO-1132(E), HFO-1123, and R1234yf is (100-a) mass% are within the range of a figure surrounded by straight lines that connect the following 4 points:

point A ($0.02a^2$-2.46a+93.4, 0, $-0.02a^2$+2.46a+6.6),
point B' ($-0.008a^2$-1.38a+56, $0.018a^2$-0.53a+26.3,$-0.01a^2$+1.91a+17.7),
point C ($-0.016a^2$+1.02a+77.6, $0.016a^2$-1.02a+22.4, 0), and
point O (100.0, 0.0, 0.0),
or on the straight lines OA, AB', and B'C (excluding the points O and C);

if $10.0 < a \leq 16.5$, coordinates (x,y,z) in the ternary composition diagram are within the range of a figure surrounded by straight lines that connect the following 4 points:

point A ($0.0244a^2$-2.5695a+94.056, 0, $-0.0244a^2$+2.5695a+5.944),
point B' ($0.1161a^2$-1.9959a+59.749, $0.014a^2$-0.3399a+24.8,$-0.1301a^2$+2.3358a+15.451),
point C ($-0.0161a^2$+1.02a+77.6, $0.0161a^2$-1.02a+22.4, 0), and
point O (100.0, 0.0, 0.0),
or on the straight lines OA, AB', and B'C (excluding the points O and C); or

if $16.5 < a \leq 21.8$, coordinates (x,y,z) in the ternary composition diagram are within the range of a figure surrounded by straight lines that connect the following 4 points:

point A ($0.0161a^2$-2.3535a+92.742, 0, $-0.0161a^2$+2.3535a+7.258),
point B' ($-0.0435a^2$-0.0435a+50.406, $-0.0304a^2$+1.8991a-0.0661, $0.0739a^2$-1.8556a+49.6601),
point C ($-0.0161a^2$+0.9959a+77.851, $0.0161a^2$-0.9959a+22.149, 0), and
point O (100.0, 0.0, 0.0),
or on the straight lines OA, AB', and B'C (excluding the points O and C).

Figs. 3 to 9 show compositions whose R32 content a (mass%) is 0 mass%, 5 mass%, 10 mass%, 14.3 mass%, 16.5 mass%, 19.2 mass%, and 21.8 mass%, respectively.

Note that when point B in the ternary composition diagram is defined as a point where a refrigerating capacity ratio of 95% relative to that of R410A and a COP ratio of 95% relative to that of R410A are both achieved, point B' is the intersection of straight line AB and an approximate line formed by connecting three points, including point C, where the COP ratio relative to that of R410A is 95%.

Points A, B', and C were individually obtained by approximate calculation in the following manner.

Point A is a point where the HFO-1123 content is 0 mass% and a refrigerating capacity ratio of 95% relative to that of R410A is achieved. Three points corresponding to point A were obtained in each of the following three ranges by calculation, and their approximate expressions were obtained.

Table 13

| Item | $10.0 \geq R32 \geq 0$ | | | $16.5 \geq R32 \geq 10.0$ | | | $21.8 \geq R32 \geq 16.5$ | | |
|---|---|---|---|---|---|---|---|---|---|
| R32 | 0.0 | 5.0 | 10.0 | 10.0 | 14.3 | 16.5 | 16.5 | 19.2 | 21.8 |
| HFO-1132(E) | 93.4 | 81.6 | 70.8 | 70.8 | 62.3 | 58.3 | 58.3 | 53.5 | 49.1 |
| HFO-1123 | 0.0 | 0.0 | 0.0 | 0.0 | 0.0 | 0.0 | 0.0 | 0.0 | 0.0 |
| R1234yf | 6.6 | 13.4 | 19.2 | 19.2 | 23.4 | 25.2 | 25.2 | 27.3 | 29.1 |
| R32 | x | | | x | | | x | | |
| HFO-1132(E) approximate expression | $0.02x^2-2.46x+93.4$ | | | $0.0244x^2-2.5695x+94.056$ | | | $0.0161x^2-2.3535x+92.742$ | | |
| HFO-1123 approximate expression | 0 | | | 0 | | | 0 | | |
| R1234yf approximate expression | 100-R32-HF0-1132(E) | | | 100-R32-HF0-1132(E) | | | 100-R32-HFO-1132(E) | | |

Point C is a point where the R1234yf content is 0 mass% and a COP ratio of 95% relative to that of R410A is achieved. Three points corresponding to point C were obtained in each of the following three ranges by calculation, and their approximate expressions were obtained.

Table 14

| Item | 10.0≥R32≥0 | | | 16.5≥R32≥10.0 | | | 21.8≥R32≥16.5 | | |
|---|---|---|---|---|---|---|---|---|---|
| R32 | 0 | 5 | 10 | 10 | 14.3 | 16.5 | 16.5 | 19.2 | 21.8 |
| HFO-1132(E) | 77.6 | 77.3 | 76.2 | 76.2 | 74.5 | 73.4 | 73.4 | 71.7 | 69.7 |
| HFO-1123 | 22.4 | 17.7 | 13.8 | 13.8 | 11.2 | 10.1 | 10.1 | 9.1 | 8.5 |
| R1234yf | 0 | 0 | 0 | 0 | 0 | 0 | 0 | 0 | 0 |
| R32 | x | | | x | | | x | | |
| HFO-1132(E) approximate expression | 100-R32HF0-1123 | | | 100-R32HF0-1123 | | | 100-R32HF0-1123 | | |
| HFO-1123 approximate expression | 0.016x2-1.02x+22.4 | | | 0.0161x2-0.9959x+22.149 | | | 0.0161*2-0.9959*+22.149 | | |
| R1234yf approximate expression | 100-R32-HF0-1132(E) | | | 100-R32-HFO-1132(E) | | | 100-R32-HFO-1132(E) | | |

Three points corresponding to point B' were obtained in each of the following three ranges by calculation, and their approximate expressions were obtained.

Table 15

| Item | 10.0≥R32≥0 | | | 16.5≥R32≥10.0 | | | 21.8≥R32≥16.5 | | |
|---|---|---|---|---|---|---|---|---|---|
| R32 | 0 | 5 | 10 | 10 | 14.3 | 16.5 | 16.5 | 19.2 | 21.8 |
| HFO-1132(E) | 56 | 48.9 | 41.4 | 41.4 | 34.5 | 31.2 | 31.2 | 25.8 | 20 |
| HFO-1123 | 26.3 | 24.1 | 22.8 | 22.8 | 22.8 | 23 | 23 | 25.2 | 26.9 |
| R1234yf | 17.7 | 22 | 25.8 | 25.8 | 28.4 | 29.3 | 29.3 | 29.8 | 31.3 |
| R32 | x | | | x | | | x | | |
| HFO-1132(E) approximate expression | -0.008*2-1.38*56 | | | 0.0161x2-1.9959x+59.749 | | | -0.0435x2-0.4456x+50.406 | | |
| HFO-1123 approximate expression | 0.018x2-0.53x+26.3 | | | 0.014x2-0.3399x+24.8 | | | -0.0304*2+1.8991*-0.0661 | | |
| R1234yf approximate expression | 100-R32-HF0-1132(E) | | | 100-R32-HFO-1132(E) | | | 100-R32-HF0-1132(E) | | |

4.2. Refrigerant B

Refrigerant B according to the present disclosure is a mixed refrigerant comprising HFO-1132(E) and HFO-1123 in a total amount of 99.5 mass% or more based on the entire Refrigerant B, and comprising HFO-1132(E) in an amount of 62.5 mass% to 72.5 mass% based on the entire Refrigerant B. Refrigerant B according to the present disclosure has various properties that are desirable as an R410A-alternative refrigerant, i.e., (1) a coefficient of performance that is equivalent to that of R410A, (2) a refrigerating capacity that is equivalent to that of R410A, (3) a sufficiently low GWP, and (4) a slight flammability (Class 2L) according to the ASHRAE standard. Refrigerant B according to the present disclosure is particularly preferably a mixed refrigerant comprising 72.5 mass% or less of HFO-1132(E), because it has a slight flammability (Class 2L) according to the ASHRAE standard.

Refrigerant B according to the present disclosure is more preferably a mixed refrigerant comprising 62.5 mass% or more of HFO-1132(E). In this case, Refrigerant B according to the present disclosure has a higher ratio of coefficient of performance relative to that of R410A, further suppresses the polymerization reaction of HFO-1132(E) and/or HFO-1123, and has more excellent stability.

Refrigerant B according to the present disclosure may further comprise other additional refrigerants in addition to HFO-1132(E) and HFO-1123 as long as the above properties and effects are not impaired. In this respect, Refrigerant B according to the present disclosure more preferably comprises HFO-1132(E) and HFO-1123 in a total amount of 99.75 mass% or more, and even more preferably 99.9 mass% or more, based on the entire Refrigerant B.

Additional refrigerants are not particularly limited and can be widely selected. The mixed refrigerant may contain one additional refrigerant, or two or more additional refrigerants.

Refrigerant B according to the present disclosure is suitable for use as an alternative refrigerant for HFC refrigerants such as R410A, R407C, and R404A, as well as HCFC refrigerants such as R22.

Examples of Refrigerant B

The present disclosure is described in more detail below with reference to Examples of Refrigerant B. However, Refrigerant B according to the present disclosure is not limited to the Examples.

Mixed refrigerants were prepared by mixing HFO-1132(E) and HFO-1123 at mass% based on their sum shown in Tables 16 and 17.

The GWP of compositions each comprising a mixture of R410A (R32 = 50%/R125 = 50%) was evaluated based on the values stated in the Intergovernmental Panel on Climate Change (IPCC), fourth assessment report. The GWP of HFO-1132(E), which was not stated in the report, was assumed to be 1 from HFO-1132a (GWP = 1 or less) and HFO-1123 (GWP = 0.3, described in PTL 1). The refrigerating capacity of compositions each comprising R410A and a mixture of HFO-1132(E) and HFO-1123 was determined by performing theoretical refrigeration cycle calculations for the mixed refrigerants using the National Institute of Science and Technology (NIST) and Reference Fluid Thermodynamic and Transport Properties Database (Refprop 9.0) under the following conditions.

Evaporating temperature: 5°C
Condensation temperature: 45°C
Superheating temperature: 1 K
Subcooling temperature: 5 K
Compressor efficiency: 70%

Tables 1 and 2 shows GWP, COP, and refrigerating capacity, which were calculated based on these results. The COP and refrigerating capacity are ratios relative to R410A.

The coefficient of performance (COP) was determined by the following formula.

```
COP = (refrigerating capacity or heating capacity)/power

consumption
```

Further, as for flammability, the burning velocity of these mixed refrigerants was measured according to ANSI/ASHRAE Standard 34-2013. Compositions that showed a burning velocity of 10 cm/s or less were determined to be Class 2L (slight flammability).

A burning velocity test was performed using the apparatus shown in Fig. 1 in the following manner. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps and stored on a PC.

Table 16

| Item | Unit | Comp. Ex. 1 | Comp. Ex. 2 | Comp. Ex. 3 | Example 1 | Example 2 | Example 3 |
|---|---|---|---|---|---|---|---|
| | | R410A | HFO-1132E | | | | |
| HFO-1132E | mass% | 0 | 100 | 80 | 72.5 | 70 | 67.5 |
| HFO-1123 | mass% | 0 | 0 | 20 | 27.5 | 30 | 32.5 |
| GWP | - | 2088 | 1 | 1 | 1 | 1 | 1 |
| COP ratio | % (relative to R410A) | 100 | 98 | 95.3 | 94.4 | 94.1 | 93.8 |
| Refrigerating capacity ratio | % (relative to R410A) | 100 | 98 | 102.1 | 103.5 | 103.9 | 104.3 |
| Discharge pressure | MPa | 2.7 | 2.7 | 2.9 | 3.0 | 3.0 | 3.1 |
| Burning velocity | cm/sec | Non-flammable | 20 | 13 | 10 | 9 | 9 or less |

Table 17

| Item | Unit | Example 4 | Example 5 | Comp. Ex. 4 | Comp. Ex. 5 | Comp. Ex. 6 | Comp. Ex. 7 |
|---|---|---|---|---|---|---|---|
| | | | | | | | HFO-1123 |
| HFO-1132E | mass% | 65 | 62.5 | 60 | 50 | 25 | 0 |
| HFO-1123 | mass% | 35 | 37.5 | 40 | 50 | 75 | 100 |
| GWP | - | 1 | 1 | 1 | 1 | 1 | 1 |
| COP ratio | % (relative to R410A) | 93.5 | 93.2 | 92.9 | 91.8 | 89.9 | 89.9 |
| Refrigerating capacity ratio | % (relative to R410A) | 104.7 | 105.0 | 105.4 | 106.6 | 108.1 | 107.0 |
| Discharge pressure | MPa | 3.1 | 3.1 | 3.1 | 3.2 | 3.4 | 3.4 |
| Burning velocity | cm/sec | 9 or less | 9 or less | 9 or less | 9 or less | 9 or less | 5 |

Compositions comprising HFO-1132(E) in an amount of 62.5 mass% to 72.5 mass% based on the entire composition are stable while having a low GWP (GWP=1), and ensures ASHRAE flammability 2L. Furthermore, surprisingly, the compositions can ensure performance equivalent to that of R410A.

4.3. Refrigerant C

Refrigerant C according to the present disclosure is a mixed refrigerant comprising HFO-1132(E), R32, and 2,3,3,3-tetrafluoro-1-propene (R1234yf).

Refrigerant C according to the present disclosure has various properties that are desirable as an R410A-alternative refrigerant, i.e., a cooling capacity that is equivalent to that of R410A, a sufficiently low GWP, and a slight flammability (Class 2L) according to the ASHRAE standard.

Refrigerant C according to the present disclosure is preferably a refrigerant wherein

when the mass% of HFO-1132(E), R32, and R1234yf based on their sum is respectively represented by x, y, and z, coordinates (x,y,z) in a ternary composition diagram in which the sum of HFO-1132(E), R32, and R1234yf is 100 mass% are within the range of a figure surrounded by line segments AC, CF, FD, and DA that connect the following 4 points:

point A (71.1, 0.0, 28.9),
point C (36.5, 18.2, 45.3),
point F (47.6, 18.3, 34.1), and
point D (72.0, 0.0, 28.0),
or on the line segments AC, CF, FD, and DA;

the line segment AC is represented by coordinates $(0.0181y^2-2.2288y+71.096, y, -0.0181y^2+1.2288y+28.904)$,
the line segment FD is represented by coordinates $(0.02y^2-1.7y+72, y, -0.02y^2+0.7y+28)$, and
the line segments CF and DA are straight lines. When the requirements above are satisfied, Refrigerant C according to the present disclosure has a refrigerating capacity ratio of 85% or more relative to that of R410A, a GWP of 125 or less, and a slight flammability (Class 2L) according to the ASHRAE standard.

Refrigerant C according to the present disclosure is preferably a refrigerant wherein

when the mass% of HFO-1132(E), R32, and R1234yf based on their sum is respectively represented by x, y, and z, coordinates (x,y,z) in a ternary composition diagram in which the sum of HFO-1132(E), R32, and R1234yf is 100 mass% are within the range of a figure surrounded by line segments AB, BE, ED, and DA that connect the following 4 points:

point A (71.1, 0.0, 28.9),
point B (42.6, 14.5, 42.9),
point E (51.4, 14.6, 34.0), and
point D (72.0, 0.0, 28.0),
or on the line segments AB, BE, ED, and DA;

the line segment AB is represented by coordinates $(0.0181y^2-2.2288y+71.096, y, -0.0181y^2+1.2288y+28.904)$,
the line segment ED is represented by coordinates $(0.02y^2-1.7y+72, y, -0.02y^2+0.7y+28)$, and
the line segments BE and DA are straight lines. When the requirements above are satisfied, Refrigerant C according to the present disclosure has a refrigerating capacity ratio of 85% or more relative to that of R410A, a GWP of 100 or less, and a slight flammability (Class 2L) according to the ASHRAE standard.

Refrigerant C according to the present disclosure is preferably a refrigerant wherein

when the mass% of HFO-1132(E), R32, and R1234yf based on their sum is respectively represented by x, y, and z, coordinates (x,y,z) in a ternary composition diagram in which the sum of HFO-1132(E), R32, and R1234yf is 100 mass% are within the range of a figure surrounded by line segments GI, IJ, and JK that connect the following 3 points:

point G (77.5, 6.9, 15.6),
point I (55.1, 18.3, 26.6), and
point J (77.5. 18.4, 4.1),
or on the line segments GI, IJ, and JK;

the line segment GI is represented by coordinates $(0.02y^2-2.4583y+93.396, y, -0.02y^2+1.4583y+6.604)$, and
the line segments IJ and JK are straight lines. When the requirements above are satisfied, Refrigerant C according to the present disclosure has a refrigerating capacity ratio of 95% or more relative to that of R410A and a GWP of 100 or less, is less likely to undergo changes such as polymerization and degradation, and has excellent stability.

Refrigerant C according to the present disclosure is preferably a refrigerant wherein

when the mass% of HFO-1132(E), R32, and R1234yf based on their sum is respectively represented by x, y, and z, coordinates (x,y,z) in a ternary composition diagram in which the sum of HFO-1132(E), R32, and R1234yf is 100 mass% are within the range of a figure surrounded by line segments GH, HK, and KG that connect the following 3 points:

   point G (77.5, 6.9, 15.6),
   point H (61.8, 14.6, 23.6), and
   point K (77.5, 14.6, 7.9),
   or on the line segments GH, HK, and KG;

the line segment GH is represented by coordinates ($0.02y^2$-2.4583y+93.396, y, $-0.02y^2$+1.4583y+6.604), and
the line segments HK and KG are straight lines. When the requirements above are satisfied, Refrigerant C according to the present disclosure has a refrigerating capacity ratio of 95% or more relative to that of R410A and a GWP of 100 or less, is less likely to undergo changes such as polymerization and degradation, and has excellent stability.

Refrigerant C according to the present disclosure may further comprise other additional refrigerants in addition to HFO-1132(E), R32, and R1234yf as long as the above properties and effects are not impaired. In this respect, Refrigerant C according to the present disclosure preferably comprises HFO-1132(E), R32, and R1234yf in a total amount of 99.5 mass% or more, more preferably 99.75 mass% or more, and even more preferably 99.9 mass% or more, based on the entire Refrigerant C.

Additional refrigerants are not particularly limited and can be widely selected. The mixed refrigerant may contain one additional refrigerant, or two or more additional refrigerants.

Refrigerant C according to the present disclosure is suitable for use as an alternative refrigerant for R410A.

Examples of Refrigerant C

The present disclosure is described in more detail below with reference to Examples of Refrigerant C. However, Refrigerant C according to the present disclosure is not limited to the Examples.

The burning velocity of the mixed refrigerants of HFO-1132(E), R32, and R1234yf was measured according to ANSI/ASHRAE Standard 34-2013. While changing the concentration of R32 by 5 mass%, compositions showing a burning velocity of 10 cm/s were found. Table 18 shows the found compositions.

A burning velocity test was performed using the apparatus shown in Fig. 1 in the following manner. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps and stored on a PC.

Table 18

| Item | Unit | Point D | R32=5 mass% | R32=10 mass% | R32=15 mass% | R32=20 mass% |
|---|---|---|---|---|---|---|
| HFO-1132E | Mass% | 72 | 64 | 57 | 51 | 46 |
| R32 | Mass% | 0 | 5 | 10 | 15 | 20 |
| R1234yf | Mass% | 28 | 31 | 33 | 34 | 34 |
| Burning Velocity | cm/s | 10 | 10 | 10 | 10 | 10 |

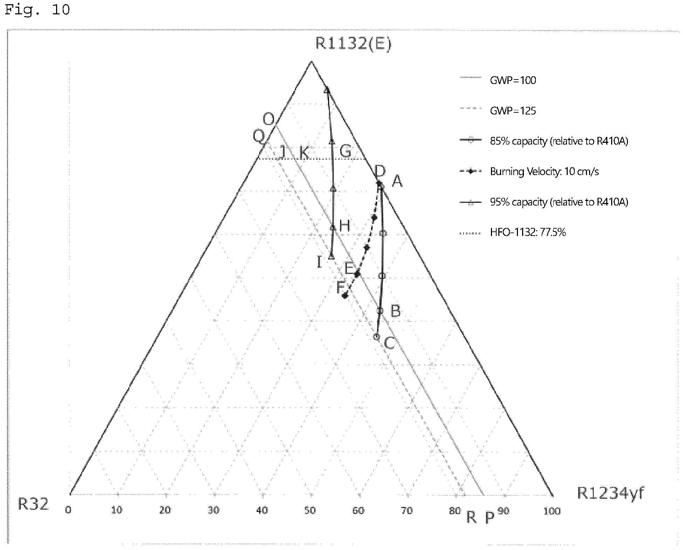

These results indicate that under the condition that the mass% of HFO-1132(E), R32, and R1234yf based on their sum is respectively represented by x, y, and z, when coordinates (x,y,z) in a ternary composition diagram (Fig. 10) in which the sum of HFO-1132(E), R32, and R1234yf is 100 mass% are on or on the right side of the line segments that connect 5 points shown in Table 18, the refrigerant has a slight flammability (Class 2L) according to the ASHRAE standard. This is because it has been known that the burning velocity of R1234yf is lower than that of both HFO-1132(E) and R32.

Mixed refrigerants were prepared by mixing HFO-1132(E), R32, and R1234yf at mass% based on their sum shown in Tables 19 to 23. The coefficient of performance (COP) ratio and the refrigerating capacity ratio of the mixed refrigerants of Tables 19 to 23 relative to those of R410 were determined. The calculation conditions were as follows.

Evaporating temperature: 5°C
Condensation temperature: 45°C
Degree of superheating: 1 K
Degree of subcooling: 5 K
$E_{comp}$ (compressive modulus): 0.7 kWh

Tables 19 to 23 show these values together with the GWP of each mixed refrigerant.

Table 19

| Item | Unit | Comp. Ex. 1 | Comp. Ex. 2 | Example 1 | Example 2 | Example 3 | Example 4 |
|---|---|---|---|---|---|---|---|
| | | | A | | | B | C |
| HFO-1132E | Mass% | R410A | 71.1 | 60.4 | 50.6 | 42.6 | 36.5 |
| R32 | Mass% | | 0.0 | 5.0 | 10.0 | 14.5 | 18.2 |
| R1234yf | Mass% | | 28.9 | 34.6 | 39.4 | 42.9 | 45.3 |
| GWP | - | 2088 | 2 | 36 | 70 | 100 | 125 |
| COP Ratio | % (relative to R410A) | 100 | 98.9 | 98.7 | 98.7 | 98.9 | 99.1 |
| Refrigerating Capacity Ratio | % (relative to R410A) | 100 | 85.0 | 85.0 | 85.0 | 85.0 | 85.0 |

Table 20

| Item | Unit | Comp. Ex. 3 | Comp. Ex. 4 | Comp. Ex. 5 | Comp. Ex. 6 |
|---|---|---|---|---|---|
| | | O | P | Q | R |
| HFO-1132E | Mass% | 85.3 | 0.0 | 81.6 | 0.0 |
| R32 | Mass% | 14.7 | 14.3 | 18.4 | 18.1 |
| R1234yf | Mass% | 0 | 85.7 | 0.0 | 81.9 |
| GWP | - | 100 | 100 | 125 | 125 |
| COP Ratio | % (relative to R410A) | 96.2 | 103.4 | 95.9 | 103.4 |
| Refrigerating Capacity Ratio | % (relative to R410A) | 105.7 | 57.3 | 107.4 | 60.9 |

Table 21

| Item | Unit | Comp. Ex. 7 | Example 5 | Example 6 | Example 7 | Example 8 | Example 9 | Comp. Ex. 8 |
|---|---|---|---|---|---|---|---|---|
| | | D | | | E | | F | |
| HFO-1132E | Mass% | 72.0 | 64.0 | 57.0 | 51.4 | 51.0 | 47.6 | 46.0 |
| R32 | Mass% | 0.0 | 5.0 | 10.0 | 14.6 | 15.0 | 18.3 | 20.0 |
| R1234yf | Mass% | 28.0 | 31.0 | 33.0 | 34.0 | 34.0 | 34.1 | 34.0 |
| GWP | - | 1.84 | 36 | 69 | 100 | 103 | 125 | 137 |
| COP Ratio | % (relative to R410A) | 98.8 | 98.5 | 98.2 | 98.1 | 98.1 | 98.0 | 98.0 |

(continued)

| Item | Unit | Comp. Ex. 7 | Example 5 | Example 6 | Example 7 | Example 8 | Example 9 | Comp. Ex. 8 |
|---|---|---|---|---|---|---|---|---|
| | | D | | | E | | F | |
| Refrigerating Capacity Ratio | % (relative to R410A) | 85.4 | 86.8 | 88.3 | 89.8 | 90.0 | 91.2 | 91.8 |

Table 22

| Item | Unit | Comp. Ex. 7 | Example 5 | Example 6 | Example 7 | Example 8 | Example 9 | Comp. Ex. 8 |
|---|---|---|---|---|---|---|---|---|
| | | D | | | E | | F | |
| HFO-1132E | Mass% | 72.0 | 64.0 | 57.0 | 51.4 | 51.0 | 47.6 | 46.0 |
| R32 | Mass% | 0.0 | 5.0 | 10.0 | 14.6 | 15.0 | 18.3 | 20.0 |
| R1234yf | Mass% | 28.0 | 31.0 | 33.0 | 34.0 | 34.0 | 34.1 | 34.0 |
| GWP | - | 1.84 | 36 | 69 | 100 | 103 | 125 | 137 |
| COP Ratio | % (relative to R410A) | 98.8 | 98.5 | 98.2 | 98.1 | 98.1 | 98.0 | 98.0 |
| Refrigerating Capacity Ratio | % (relative to R410A) | 85.4 | 86.8 | 88.3 | 89.8 | 90.0 | 91.2 | 91.8 |

Table 23

| Item | Unit | Comp. Ex. 11 | Example 13 | Example 14 | Example 15 | Comp. Ex. 12 |
|---|---|---|---|---|---|---|
| | | | J | K | G | |
| HFO-1132E | Mass% | 77.5 | 77.5 | 77.5 | 77.5 | 77.5 |
| R32 | Mass% | 22.5 | 18.4 | 14.6 | 6.9 | 0.0 |
| R1234yf | Mass% | 0.0 | 4.1 | 7.9 | 15.6 | 22.5 |
| GWP | - | 153 | 125 | 100 | 48.0 | 2 |
| COP Ratio | % (relative to R410A) | 95.8 | 96.1 | 96.5 | 97.5 | 98.6 |
| Refrigerating Capacity Ratio | % (relative to R410A) | 109.1 | 105.6 | 102.3 | 95.0 | 88.0 |

These results indicate that under the condition that the mass% of HFO-1132(E), R32, and R1234yf based on their sum is respectively represented by x, y, and z, when coordinates (x,y,z) in a ternary composition diagram in which the sum of HFO-1132(E), R32, and R1234yf is 100 mass% are within the range of a figure (Fig. 10) surrounded by line segments AC, CF, FD, and DA that connect the following 4 points:

point A (71.1, 0.0, 28.9),
point C (36.5, 18.2, 45.3),
point F (47.6, 18.3, 34.1), and
point D (72.0, 0.0, 28.0),
or on the line segments AC, CF, FD, and DA, the refrigerant has a refrigerating capacity ratio of 85% or more relative to that of R410A, a GWP of 125 or less, and a slight flammability (Class 2L) according to the ASHRAE standard.

Likewise, the results indicate that when coordinates (x,y,z) are within the range of a figure (Fig. 10) surrounded by line segments AB, BE, ED, and DA that connect the following 4 points:

point A (71.1, 0.0, 28.9),
point B (42.6, 14.5, 42.9),
point E (51.4, 14.6, 34.0), and
point D (72.0, 0.0, 28.0),
or on the line segments AB, BE, ED, and DA, the refrigerant has a refrigerating capacity ratio of 85% or more relative to that of R410A, a GWP of 100 or less, and a slight flammability (Class 2L) according to the ASHRAE standard.

Likewise, the results indicate that when coordinates (x,y,z) are within the range of a figure (Fig. 10) surrounded by line segments GI, IJ, and JK that connect the following 3 points: point G (77.5, 6.9, 15.6),

point I (55.1, 18.3, 26.6), and
point J (77.5. 18.4, 4.1),
or on the line segments GI, IJ, and JK, the refrigerant has a refrigerating capacity ratio of 95% or more ratio relative to that of R410A and a GWP of 125 or less, is less likely to undergo changes such as polymerization and degradation, and has excellent stability.

Likewise, the results indicate that when coordinates (x,y,z) are within the range of a figure (Fig. 10) surrounded by line segments GH, HK, and KG that connect the following 3 points: point G (77.5, 6.9, 15.6),

point H (61.8, 14.6, 23.6), and
point K (77.5, 14.6, 7.9),
or on the line segments GH, HK, and KG, the refrigerant has a refrigerating capacity ratio of 95% or more relative to that of R410A and a GWP of 100 or less, is less likely to undergo changes such as polymerization and degradation, and has excellent stability.

4.4 Refrigerant D

Refrigerant D according to the present disclosure is a mixed refrigerant comprising HFO-1132(E), HFO-1123, and R32. Refrigerant D according to the present disclosure has various properties that are desirable as an R410A-alternative refrigerant, i.e., a coefficient of performance that is equivalent to that of R410A, and a sufficiently low GWP.
Refrigerant D according to the present disclosure is preferably a refrigerant wherein

when the mass% of HFO-1132(E), HFO-1123, and R32 based on their sum is respectively represented by x, y, and z, coordinates (x,y,z) in a ternary composition diagram in which the sum of HFO-1132(E), HFO-1123, and R32 is 100 mass% are within the range of a figure surrounded by line segments OC', C'D', D'E', E'A', and A'O that connect the following 5 points: point O (100.0, 0.0, 0.0),

point C' (56.7, 43.3, 0.0),
point D' (52.2, 38.3, 9.5),
point E' (41.8, 39.8, 18.4), and
point A' (81.6, 0.0, 18.4),
or on the line segments C'D', D'E', and E'A' (excluding the points C' and A');

the line segment C'D' is represented by coordinates $(-0.0297z^2-0.1915z+56.7, 0.0297z^2+1.1915z+43.3, z)$,
the line segment D'E' is represented by coordinates $(-0.0535z^2+0.3229z+53.957, 0.0535z^2+0.6771z+46.043, z)$, and
the line segments OC', E'A', and A'O are straight lines.

When the requirements above are satisfied, Refrigerant D according to the present disclosure has a COP ratio of 92.5% or more relative to that of R410A, and a GWP of 125 or less.
Refrigerant D according to the present disclosure is preferably a refrigerant wherein

when the mass% of HFO-1132(E), HFO-1123, and R32 based on their sum is respectively represented by x, y, and z, coordinates (x,y,z) in a ternary composition diagram in which the sum of HFO-1132(E), HFO-1123, and R32 is 100 mass% are within the range of a figure surrounded by line segments OC, CD, DE, EA', and A'O that connect the following 5 points:

point O (100.0, 0.0, 0.0),
point C (77.7, 22.3, 0.0),
point D (76.3, 14.2, 9.5),
point E (72.2, 9.4, 18.4), and
point A' (81.6, 0.0, 18.4),
or on the line segments CD, DE, and EA' (excluding the points C and A');

the line segment CDE is represented by coordinates $(-0.017z^2+0.0148z+77.684, 0.017z^2+0.9852z+22.316, z)$, and the line segments OC, EA' and A'O are straight lines. When the requirements above are satisfied, Refrigerant D according to the present disclosure has a COP ratio of 95% or more relative to that of R410A, and a GWP of 125 or less.

Refrigerant D according to the present disclosure is preferably a refrigerant wherein

when the mass% of HFO-1132(E), HFO-1123, and R32 based on their sum is respectively represented by x, y, and z, coordinates (x,y,z) in a ternary composition diagram in which the sum of HFO-1132(E), HFO-1123, and R32 is 100 mass% are within the range of a figure surrounded by line segments OC', C'D', D'A, and AO that connect the following 5 points:

point O (100.0, 0.0, 0.0),
point C' (56.7, 43.3, 0.0),
point D' (52.2, 38.3, 9.5), and
point A (90.5, 0.0, 9.5),
or on the line segments C'D' and D'A (excluding the points C' and A) ;

the line segment C'D' is represented by coordinates $(-0.0297z^2-0.1915z+56.7, 0.0297z^2+1.1915z+43.3, z)$, and the line segments OC', D'A, and AO are straight lines. When the requirements above are satisfied, Refrigerant D according to the present disclosure has a COP ratio of 93.5% or more relative to that of R410A, and a GWP of 65 or less.

Refrigerant D according to the present disclosure is preferably a refrigerant wherein

when the mass% of HFO-1132(E), HFO-1123, and R32 based on their sum is respectively represented by x, y, and z, coordinates (x,y,z) in a ternary composition diagram in which the sum of HFO-1132(E), HFO-1123, and R32 is 100 mass% are within the range of a figure surrounded by line segments OC, CD, DA, and AO that connect the following 5 points:

point O (100.0, 0.0, 0.0),
point C (77.7, 22.3, 0.0),
point D (76.3, 14.2, 9.5), and
point A (90.5, 0.0, 9.5),
or on the line segments CD and DA (excluding the points C and A);

the line segment CD is represented by coordinates $(-0.017z^2+0.0148z+77.684, 0.017z^2+0.9852z+22.316, z)$, and the line segments OC, DA, and AO are straight lines. When the requirements above are satisfied, Refrigerant D according to the present disclosure has a COP ratio of 95% or more relative to that of R410A, and a GWP of 65 or less.

Refrigerant D according to the present disclosure may further comprise other additional refrigerants in addition to HFO-1132(E), HFO-1123, and R32 as long as the above properties and effects are not impaired. In this respect, Refrigerant D according to the present disclosure preferably comprises HFO-1132(E), HFO-1123, and R32 in a total amount of 99.5 mass% or more, more preferably 99.75 mass% or more, and even more preferably 99.9 mass% or more, based on the entire Refrigerant D.

Additional refrigerants are not particularly limited and can be widely selected. The mixed refrigerant may contain one additional refrigerant, or two or more additional refrigerants.

Refrigerant D according to the present disclosure is suitable for use as an alternative refrigerant for R410A.

Examples of Refrigerant D

The present disclosure is described in more detail below with reference to Examples of Refrigerant D. However, Refrigerant D according to the present disclosure is not limited to the Examples.

Mixed refrigerants were prepared by mixing HFO-1132(E), HFO-1123, and R32 at mass% based on their sum shown in Tables 24 to 26.

The COP ratio and the refrigerating capacity (also referred to as "cooling capacity" or "capacity") ratio of the mixed refrigerants relative to those of R410 were determined. The calculation conditions were as follows.

Evaporating temperature: 5°C
Condensation temperature: 45°C
Degree of superheating: 1 K
Degree of subcooling: 5 K
$E_{comp}$ (compressive modulus): 0.7 kWh

Tables 24 to 26 show these values together with the GWP of each mixed refrigerant.

Table 24

| Item | Unit | Comp. Ex. 1 | Comp. Ex. 2 C | Example 1 | Example 2 D | Example 3 | Example 4 E | Comp. Ex. 3 O |
|---|---|---|---|---|---|---|---|---|
| HFO-1132(E) | mass% | | 77.7 | 77.3 | 76.3 | 74.6 | 72.2 | 100.0 |
| HFO-1123 | mass% | R410A | 22.3 | 17.7 | 14.2 | 11.4 | 9.4 | 0.0 |
| R32 | mass% | | 0.0 | 5.0 | 9.5 | 14.0 | 18.4 | 0.0 |
| GWP | - | 2088 | 1 | 35 | 65 | 95 | 125 | 1 |
| COP ratio | % (relative to R410A) | 100.0 | 95.0 | 95.0 | 95.0 | 95.0 | 95.0 | 97.8 |
| Refrigerating capacity ratio | % (relative to R410A) | 100.0 | 102.5 | 104.4 | 106.0 | 107.6 | 109.1 | 97.8 |

Table 25

| Item | Unit | Comp. Ex.4 C' | Example 5 | Example 6 D' | Example 7 | Example 8 E' | Comp. Ex. 5 A | Comp. Ex. 6 B |
|---|---|---|---|---|---|---|---|---|
| HFO-1132(E) | mass% | 56.7 | 55.0 | 52.2 | 48.0 | 41.8 | 90.5 | 0.0 |
| HFO-1123 | mass% | 43.3 | 40.0 | 38.3 | 38.0 | 39.8 | 0.0 | 90.5 |
| R32 | mass% | 0.0 | 5.0 | 9.5 | 14.0 | 18.4 | 9.5 | 9.5 |
| GWP | - | 1 | 35 | 65 | 95 | 125 | 65 | 65 |
| COP ratio | % (relative to R410A) | 92.5 | 92.5 | 92.5 | 925 | 925 | 96.6 | 90.8 |
| Refrigerating capacity ratio | % (relative to R410A) | 105.8 | 107.9 | 109.7 | 111.5 | 113.2 | 103.2 | 111.0 |

Table 26]

| Item | Unit | Comp. Ex. 7 | Comp. Ex. 8 | Example 9 | Example 10 | Example 11 | Comp. Ex. 9 | Comp. Ex. 10 |
|---|---|---|---|---|---|---|---|---|
| | | A' | B' | | | | | |
| HFO-1132(E) | mass% | 81.6 | 0.0 | 85.0 | 65.0 | 70.0 | 50.0 | 20.0 |
| HFO-1123 | mass% | 0.0 | 81.6 | 10.0 | 30.0 | 15.0 | 20.0 | 20.0 |
| R32 | mass% | 18.4 | 18.4 | 5.0 | 5.0 | 15.0 | 30.0 | 60.0 |
| GWP | - | 125 | 125 | 35 | 35 | 102 | 203 | 405 |
| COP ratio | % (relative to R410A) | 95.9 | 91.9 | 95.9 | 93.6 | 94.6 | 94.3 | 97.6 |
| Refrigerating capacity ratio | % (relative to R410A) | 107.4 | 113.8 | 102.9 | 106.5 | 108.7 | 114.6 | 117.6 |

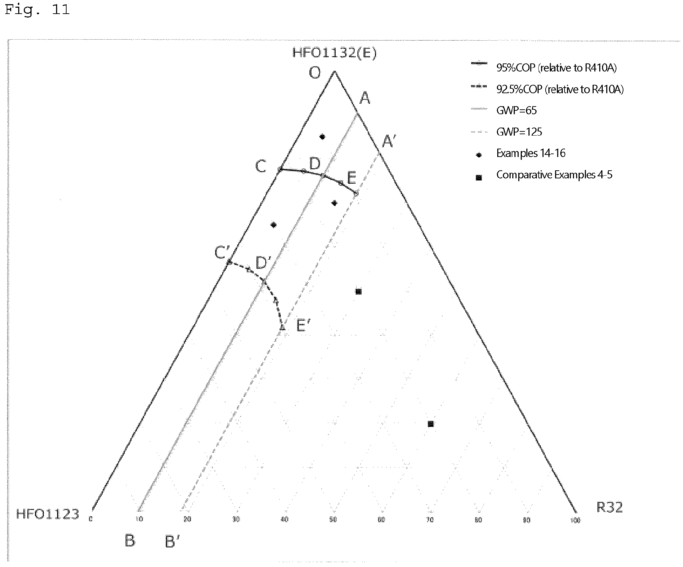

These results indicate that under the condition that the mass% of HFO-1132(E), HFO-1123, and R32 based on their sum is respectively represented by x, y, and z, when coordinates (x,y,z) in a ternary composition diagram in which the sum of HFO-1132(E), HFO-1123, and R1234yf is 100 mass% are within the range of a figure (Fig. 11) surrounded by line segments OC', C'D', D'E', E'A', and A'O that connect the following 5 points:

point O (100.0, 0.0, 0.0),
point C' (56.7, 43.3, 0.0),
point D' (52.2, 38.3, 9.5),
point E' (41.8, 39.8, 18.4), and
point A' (81.6, 0.0, 18.4),
or on the line segments C'D', D'E', and E'A' (excluding the points C' and A'), the refrigerant has a COP ratio of 92.5% or more relative to that of R410A, and a GWP of 125 or less.

Likewise, the results indicate that when coordinates (x,y,z) are within the range of a figure (Fig. 11) surrounded by line segments OC, CD, DE, EA', and A'O that connect the following 5 points:

point O (100.0, 0.0, 0.0),
point C (77.7, 22.3, 0.0),
point D (76.3, 14.2, 9.5),
point E (72.2, 9.4, 18.4), and
point A' (81.6, 0.0, 18.4),
or on the line segments CD, DE, and EA' (excluding the points C and A'), the refrigerant has a COP ratio of 95% or more relative to that of R410A, and a GWP of 125 or less.

Likewise, the results indicate that when coordinates (x,y,z) are within the range of a figure (Fig. 11) surrounded by line segments OC', C'D', D'A, and AO that connect the following 5 points:

point O (100.0, 0.0, 0.0),
point C' (56.7, 43.3, 0.0),
point D' (52.2, 38.3, 9.5), and
point A (90.5, 0.0, 9.5),
or on the line segments C'D' and D'A (excluding the points C' and A), the refrigerant has a COP ratio of 92.5% or more relative to that of R410A, and a GWP of 65 or less.

Likewise, the results indicate that when coordinates (x,y,z) are within the range of a figure (Fig. 11) surrounded by line segments OC, CD, DA, and AO that connect the following 5 points:

point O (100.0, 0.0, 0.0),
point C (77.7, 22.3, 0.0),
point D (76.3, 14.2, 9.5), and
point A (90.5, 0.0, 9.5),

or on the line segments CD and DA (excluding the points C and A), the refrigerant has a COP ratio of 95% or more relative to that of R410A, and a GWP of 65 or less.

In contrast, as shown in Comparative Examples 2, 3, and 4, when R32 is not contained, the concentrations of HFO-1132(E) and HFO-1123, which have a double bond, become relatively high; this undesirably leads to deterioration, such as decomposition, or polymerization in the refrigerant compound.

Moreover, as shown in Comparative Examples 3, 5, and 7, when HFO-1123 is not contained, the combustion-inhibiting effect thereof cannot be obtained; thus, undesirably, a composition having slight flammability cannot be obtained.

4.5. Refrigerant E

Refrigerant E according to the present disclosure is a mixed refrigerant comprising $CO_2$, R32, HFO-1132(E), and R1234yf. Refrigerant E according to the present disclosure has various properties that are desirable as an R410A-alternative refrigerant, i.e., a cooling capacity that is equivalent to that of R410A, a sufficiently low GWP, and a slight flammability.

Refrigerant E according to the present disclosure is a refrigerant wherein

when the mass% of $CO_2$, R32, HFO-1132(E), and R1234yf based on their sum is respectively represented by w, x, y, and z,
coordinates (x,y,z) in a ternary composition diagram in which the sum of R32, HFO-1132(E), and R1234yf is (100-w) mass% are within the range of a figure surrounded by curves IJ, JK, and KL, as well as straight lines LB", B"D, DC, and CI that connect the following 7 points:

point I (0.0, 72.0, 28.0-w),
point J (18.3, 48.5, 33.2-w),
point K (36.8, 35.6, 27.6-w),
point L (51.7, 28.9, 19.4-w),
point B" ($-1.5278w^2+2.75w+50.5$, 0.0, $1.5278w^2-3.75w+49.5$),
point D ($-2.9167w+40.317$, 0.0, $1.9167w+59.683$), and
point C (0.0, $-4.9167w+58.317$, $3.9167w+41.683$),
or on the above line segments (excluding the points on the straight lines B"D and CI);

if $1.2<w\leq4.0$, coordinates (x,y,z) in the ternary composition diagram are within the range of a figure surrounded by curves IJ, JK, and KL, as well as straight lines LB", B"D, DC, and CI that connect the following 7 points:

point I (0.0, 72.0, 28.0-w),
point J (18.3, 48.5, 33.2-w),
point K (36.8, 35.6, 27.6-w),
point L (51.7, 28.9, 19.4-w),
point B" (51.6, 0.0, 48.4-w),
point D ($-2.8226w+40.211$, 0.0, $1.8226w+59.789$), and
point C (0.0, $0.1081w^2-5.169w+58.447$, $-0.1081w^2+4.169w+41.553$),
or on the above line segments (excluding the points on the straight lines B"D and CI); or

if $4.0<w\leq7.0$, coordinates (x,y,z) in the ternary composition diagram are within the range of a figure surrounded by curves IJ, JK, and KL, as well as straight lines LB", B"D, DC, and CI that connect the following 7 points:

point I (0.0, 72.0, 28.0-w),
point J (18.3, 48.5, 33.2-w),
point K (36.8, 35.6, 27.6-w),
point L (51.7, 28.9, 19.4-w),
point B" (51.6, 0.0, 48.4-w),
point D ($-2.8w+40.1$, 0.0, $1.8w+59.9$), and
point C (0.0, $0.0667w^2-4.9667w+58.3$, $-0.0667w^2+3.9667w+41.7$), or on the above line segments (excluding the points on the straight lines B"D and CI);

the curve IJ is represented by coordinates (x, $0.0236x^2-1.716x+72$, $-0.0236x^2+0.716x+28-w$),

the curve JK is represented by coordinates (x, $0.0095x^2$-1.2222x+67.676, $-0.0095x^2$+0.2222x+32.324-w), and the curve KL is represented by coordinates (x, $0.0049x^2$-0.8842x+61.488, $-0.0049x^2$-0.1158x+38.512).

Refrigerant E according to the present disclosure has a refrigerating capacity ratio of 80% or more relative to that of R410A and a GWP of 350 or less, and further ensures a WCF slight flammability.

Refrigerant E according to the present disclosure is preferably a refrigerant wherein

when the mass% of $CO_2$, R32, HFO-1132(E), and R1234yf based on their sum is respectively represented by w, x, y, and z,

if 0<w≤1.2, coordinates (x,y,z) in a ternary composition diagram in which the sum of R32, HFO-1132(E), and R1234yf is (100-w) mass% are within the range of a figure surrounded by curves IJ and JK, as well as straight lines KF, FC, and CI that connect the following 5 points:

point I (0.0, 72.0, 28.0-w),
point J (18.3, 48.5, 33.2-w),
point K (36.8, 35.6, 27.6-w),
point F (-0.0833w+36.717, -4.0833w+5.1833, 3.1666w+58.0997), and
point C (0.0, -4.9167w+58.317, 3.9167w+41.683),
or on the above line segments (excluding the points on the straight line CI);

if 1.2<w≤1.3, coordinates (x,y,z) in the ternary composition diagram are within the range of a figure surrounded by curves IJ and JK, as well as straight lines KF, FC, and CI that connect the following 5 points:

point I (0.0, 72.0, 28.0-w),
point J (18.3, 48.5, 33.2-w),
point K (36.8, 35.6, 27.6-w),
point F (36.6, -3w+3.9, 2w+59.5), and
point C (0.0, $0.1081w^2$-5.169w+58.447, $-0.1081w^2$+4.169w+41.553), or on the above line segments (excluding the points on the straight line CI);

if 1.3<w≤4.0, coordinates (x,y,z) in the ternary composition diagram are within the range of a figure surrounded by curves IJ and JK, as well as straight lines KB', B'D, DC, and CI that connect the following 6 points:

point I (0.0, 72.0, 28.0-w),
point J (18.3, 48.5, 33.2-w),
point K (36.8, 35.6, 27.6-w),
point B' (36.6, 0.0, -w+63.4),
point D (-2.8226w+40.211, 0.0, 1.8226w+59.789), and
point C (0.0, $0.1081w^2$-5.169w+58.447, $-0.1081w^2$+4.169w+41.553),
or on the above line segments (excluding the points on the straight line CI); or

if 4.0<w≤7.0, coordinates (x,y,z) in the ternary composition diagram are within the range of a figure surrounded by curves IJ and JK, as well as straight lines KB', B'D, DC, and CI that connect the following 6 points:

point I (0.0, 72.0, 28.0-w),
point J (18.3, 48.5, 33.2-w),
point K (36.8, 35.6, 27.6-w),
point B' (36.6, 0.0, -w+63.4),
point D (-2.8w+40.1, 0.0, 1.8w+59.9), and
point C (0.0, $0.0667w^2$-4.9667w+58.3, $-0.0667w^2$+3.9667w+41.7),
or on the above line segments (excluding the points on the straight line CI);

the curve IJ is represented by coordinates (x, $0.0236x^2$-1.716x+72, $-0.0236x^2$+0.716x+28-w), and the curve JK is represented by coordinates (x, $0.0095x^2$-1.2222x+67.676, $-0.0095x^2$+0.2222x+32.324-w) . When the requirements above are satisfied, Refrigerant E according to the present disclosure has a refrigerating capacity ratio of 80% or more relative to that of R410A and a GWP of 250 or less, and further ensures a WCF slight flammability.

Refrigerant E according to the present disclosure is preferably a refrigerant wherein

when the mass% of $CO_2$, R32, HFO-1132(E), and R1234yf based on their sum is respectively represented by w, x, y, and z,

if $0<w\leq1.2$, coordinates (x,y,z) in a ternary composition diagram in which the sum of R32, HFO-1132(E), and R1234yf is (100-w) mass% are within the range of a figure surrounded by curves IJ and JK, as well as straight lines KF, FC, and CI that connect the following 4 points:

point I (0.0, 72.0, 28.0-w),
point J (18.3, 48.5, 33.2-w),
point E (18.2, $-1.1111w^2-3.1667w+31.9$, $1.1111w^2+2.1667w+49.9$), and
point C (0.0, $-4.9167w+58.317$, $3.9167w+41.683$),
or on the above line segments (excluding the points on the straight line CI);

if $1.2<w\leq4.0$, coordinates (x,y,z) in the ternary composition diagram are within the range of a figure surrounded by curves IJ and JK, as well as straight lines KF, FC, and CI that connect the following 4 points:

point I (0.0, 72.0, 28.0-w),
point J (18.3, 48.5, 33.2-w),
point E ($-0.0365w+18.26$, $0.0623w^2-4.5381w+31.856$, $-0.0623w^2+3.5746w+49.884$), and
point C (0.0, $0.1081w^2-5.169w+58.447$, $-0.1081w^2+4.169w+41.553$),
or on the above line segments (excluding the points on the straight line CI); or

if $4.0<w\leq7.0$, coordinates (x,y,z) in the ternary composition diagram are within the range of a figure surrounded by curves IJ and JK, as well as straight lines KF, FC, and CI that connect the following 4 points:

point I (0.0, 72.0, 28.0-w),
point J (18.3, 48.5, 33.2-w),
point E (18.1, $0.0444w^2-4.3556w+31.411$, $-0.0444w^2+3.3556w+50.489$), and
point C (0.0, $0.0667w^2-4.9667w+58.3$, $-0.0667w^2+3.9667w+41.7$),
or on the above line segments (excluding the points on the straight line CI); and

the curve IJ is represented by coordinates (x, $0.0236x^2-1.716x+72$, $-0.0236x^2+0.716x+28-w$). When the requirements above are satisfied, Refrigerant E according to the present disclosure has a refrigerating capacity ratio of 80% or more relative to that of R410A and a GWP of 125 or less, and further ensures a WCF slight flammability.

Refrigerant E according to the present disclosure is preferably a refrigerant wherein

when the mass% of $CO_2$, R32, HFO-1132(E), and R1234yf based on their sum is respectively represented by w, x, y, and z,

if $0<w\leq0.6$, coordinates (x,y,z) in a ternary composition diagram in which the sum of R32, HFO-1132(E), and R1234yf is (100-w) mass% are within the range of a figure surrounded by curves GO and OP, as well as straight lines PB", B"D, and DG that connect the following 5 points:

point G ($-5.8333w^2-3.1667w+22.2$, $7.0833w^2+1.4167w+26.2$, $-1.25w^2+0.75w+51.6$),
point O (36.8, $0.8333w^2+1.8333w+22.6$, $-0.8333w^2-2.8333w+40.6$),
point P (51.7, $1.1111w^2+20.5$, $-1.1111w^2-w+27.8$),
point B" ($-1.5278w^2+2.75w+50.5$, 0.0, $1.5278w^2-3.75w+49.5$), and
point D ($-2.9167w+40.317$, 0.0, $1.9167w+59.683$),
or on the above line segments (excluding the points on the straight line B"D);

if $0.6<w\leq1.2$, coordinates (x,y,z) in the ternary composition diagram are within the range of a figure surrounded by curves GN, NO, and OP, as well as straight lines PB", B"D, and DG that connect the following 6 points:

point G ($-5.8333w^2-3.1667w+22.2$, $7.0833w^2+1.4167w+26.2$, $-1.25w^2+0.75w+51.6$),
point N (18.2, $0.2778w2+3w+27.7$, $-0.2778w2-4w+54.1$),
point O (36.8, $0.8333w^2+1.8333w+22.6$, $-0.8333w^2-2.8333w+40.6$),
point P (51.7, $1.1111w^2+20.5$, $-1.1111w^2-w+27.8$),
point B" ($-1.5278w^2+2.75w+50.5$, 0.0, $1.5278w^2-3.75w+49.5$), and
point D ($-2.9167w+40.317$, 0.0, $1.9167w+59.683$),

or on the above line segments (excluding the points on the straight line B"D),

if $0 < w \leq 0.6$, the curve GO is represented by coordinates (x, $(0.00487w^2-0.0059w+0.0072)x^2+(-0.279w^2+0.2844w-0.6701)x+3.7639w^2-0.2467w+37.512$, 100-w-x-y),
if $0.6 < w \leq 1.2$, the curve GN is represented by coordinates (x, $(0.0122w^2-0.0113w+0.0313)x^2+(-0.3582w^2+0.1624w-1.4551)x+2.7889w^2+3.7417w+43.824$, 100-w-x-y),
if $0.6 < w \leq 1.2$, the curve NO is represented by coordinates (x, $(0.00487w^2-0.0059w+0.0072)x^2+(-0.279w^2+0.2844w-0.6701)x+3.7639w^2-0.2467w+37.512$, 100-w-x-y), and
if $0 < w \leq 1.2$, the curve OP is represented by coordinates (x, $(0.0074w^2-0.0133w+0.0064)x^2+(-0.5839w^2+1.0268w-0.7103)x+11.472w^2-17.455w+40.07$, 100-w-x-y) ;
if $1.2 < w \leq 4.0$, coordinates (x,y,z) in the ternary composition diagram are within the range of a figure surrounded by curves MW, WN, NO, and OP, as well as straight lines PB", B"D, DC, and CM that connect the following 8 points:

point M (0.0, $-0.3004w^2+2.419w+55.53$, $0.3004w^2-3.419w+44.47$),
point W (10.0, $-0.3645w^2+3.5024w+44.422$, $0.3645w^2-4.5024w+55.57$),
point N (18.2, $-0.3773w^2+3.319w+28.26$, $0.3773w^2-4.319w+53.54$),
point O (36.8, $-0.1392w^2+1.4381w+24.475$, $0.1392w^2-2.4381w+38.725$),
point P (51.7, $-0.2381w^2+1.881w+20.186$, $0.2381w^2-2.881w+28.114$),
point B" (51.6, 0.0, -w+48.4),
point D ($-2.8226w+40.211$, 0.0, $1.8226w+59.789$), and
point C (0.0, $0.1081w^2-5.169w+58.447$, $-0.1081w^2+4.169w+41.553$),
or on the above line segments (excluding the points on the straight lines B"D and CM),

the curve MW is represented by coordinates (x, $(0.0043w^2-0.0359w+0.1509)x^2+(-0.0493w^2+0.4669w-3.6193)x-0.3004w^2+2.419w+55.53$, 100-w-x-y),
the curve WN is represented by coordinates (x, $(0.0055w^2-0.0326w+0.0665)x^2+(-0.1571w^2+0.8981w-2.6274)x+0.6555w^2-2.2153w+54.044$, 100-w-x-y),
the curve NO is represented by coordinates (x, $(-0.00062w^2+0.0036w+0.0037)x^2+(0.0375w^2-0.239w-0.4977)x-0.8575w^2+6.4941w+36.078$, 100-w-x-y), and
the curve OP is represented by coordinates (x, $(-0.000463w^2+0.0024w-0.0011)x^2+(0.0457w^2-0.2581w-0.075)x-1.355w^2+8.749w+27.096$, 100-w-x-y); or
if $4.0 < w \leq 7.0$, coordinates (x,y,z) in the ternary composition diagram are within the range of a figure surrounded by curves MW, WN, NO, and OP, as well as straight lines PB", B"D, DC, and CM that connect the following 8 points:

point M (0.0, $-0.0667w^2+0.8333w+58.133$, $0.0667w^2-1.8333w+41.867$),
point W (10.0, $-0.0667w^2+1.1w+39.267$, $0.0667w^2-2.1w+50.733$),
point N (18.2, $-0.0889w^2+1.3778w+31.411$, $0.0889w^2-2.3778w+50.389$),
point O (36.8, $-0.0444w^2+0.6889w+25.956$, $0.0444w^2-1.6889w+37.244$),
point P (51.7, $-0.0667w^2+0.8333w+21.633$, $0.0667w^2-1.8333w+26.667$),
point B" (51.6, 0.0, -w+48.4),
point D (-2.8w+40.1, 0.0, 1.8w+59.9), and
point C (0.0, $0.0667w^2-4.9667w+58.3$, $-0.0667w^2+3.9667w+41.7$),
or on the above line segments (excluding the points on the straight lines B"D and CM),

the curve MW is represented by coordinates (x, $(0.00357w^2-0.0391w+0.1756)x^2+(-0.0356w^2+0.4178w-3.6422)x-0.0667w^2+0.8333w+58.103$, 100-w-x-y),
the curve WN is represented by coordinates (x, $(-0.002061w^2+0.0218w-0.0301)x^2+(0.0556w^2-0.5821w-0.1108)x-0.4158w^2+4.7352w+43.383$, 100-w-x-y),
the curve NO is represented by coordinates (x, $0.0082x^2+(0.0022w^2-0.0345w-0.7521)x-0.1307w^2+2.0247w+42.327$, 100-w-x-y), and
the curve OP is represented by coordinates (x, $(-0.0006258w^2+0.0066w-0.0153)x^2+(0.0516w^2-0.5478w+0.9894)x-1.074w^2+11.651w+10.992$, 100-w-x-y).

When the requirements above are satisfied, Refrigerant E according to the present disclosure has a refrigerating capacity ratio of 80% or more relative to that of R410A, a GWP of 350 or less, and further ensures an ASHRAE slight flammability.

Refrigerant E according to the present disclosure is preferably a refrigerant wherein

when the mass% of $CO_2$, R32, HFO-1132(E), and R1234yf based on their sum is respectively represented by w, x, y, and z,

if $0<w\leq0.6$, coordinates (x,y,z) in a ternary composition diagram in which the sum of R32, HFO-1132(E), and R1234yf is (100-a) mass% are within the range of a figure surrounded by curve GO and straight lines OF and FG that connect the following 3 points:

point G ($-5.8333w^2-3.1667w+22.2$, $7.0833w^2-1.4167w+26.2$, $-1.25w^2+3.5834w+51.6$),
point O (36.8, $0.8333w^2+1.8333w+22.6$, $-0.8333w^2-2.8333w+40.6$), and
point F ($-0.0833w+36.717$, $-4.0833w+5.1833$, $3.1666w+58.0997$),
or on the above line segments, and

the curve GO is represented by coordinates (x, $(0.00487w^2-0.0059w+0.0072)x^2+(-0.279w^2+0.2844w-0.6701)x+3.7639w^2-0.2467w+37.512$, 100-w-x-y);
if $0.6<w\leq1.2$, coordinates (x,y,z) in the ternary composition diagram are within the range of a figure surrounded by curves GN and NO, as well as straight lines OF and FG that connect the following 4 points:

point G ($-5.8333w^2-3.1667w+22.2$, $7.0833w^2-1.4167w+26.2$, $-1.25w^2+3.5834w+51.6$),
point N (18.2, $0.2778w^2+3.0w+27.7$, $-0.2778w^2-4.0w+54.1$),
point O (36.8, $0.8333w^2+1.8333w+22.6$, $-0.8333w^2-2.8333w+40.6$), and
point F ($-0.0833w+36.717$, $-4.0833w+5.1833$, $3.1666w+58.0997$),
or on the above line segments,

if $0.6<w\leq1.2$, the curve GN is represented by coordinates (x, $(0.0122w^2-0.0113w+0.0313)x^2+(-0.3582w^2+0.1624w-1.4551)x+2.7889w^2+3.7417w+43.824$, 100-w-x-y), and
if $0.6<w\leq1.2$, the curve NO is represented by coordinates (x, $(0.00487w^2-0.0059w+0.0072)x^2+(-0.279w^2+0.2844w-0.6701)x+3.7639w^2-0.2467w+37.512$, 100-w-x-y);
if $1.2<w\leq1.3$, coordinates (x,y,z) in the ternary composition diagram are within the range of a figure surrounded by curves MW, WN, and NO, as well as straight lines OF, FC, and CM that connect the following 6 points:

point M (0.0, $-0.3004w^2+2.419w+55.53$, $0.3004w^2-3.419w+44.47$),
point W (10.0, $-0.3645w^2+3.5024w34.422$, $0.3645w^2-4.5024w+55.578$),
point N (18.2, $-0.3773w^2+3.319w+28.26$, $0.3773w^2-4.319w+53.54$),
point O (36.8, $-0.1392w^2+1.4381w+24.475$, $0.1392w^2-2.4381w+38.725$),
point F (36.6, $-3w+3.9$, $2w+59.5$), and
point C ($0.1081w^2-5.169w+58.447$, 0.0, $-0.1081w^2+4.169w+41.553$),
or on the above line segments (excluding the points on straight line CM),

the curve MW is represented by coordinates (x, $(0.0043w^2-0.0359w+0.1509)x^2+(-0.0493w^2+0.4669w-3.6193)x-0.3004w^2+2.419w+55.53$, 100-w-x-y),
the curve WN is represented by coordinates (x, $(0.0055w^2-0.0326w+0.0665)x^2+(-0.1571w^2+0.8981w-2.6274)x+0.6555w^2-2.2153w+54.044$, 100-w-x-y), and
the curve NO is represented by coordinates (x, $(-0.00062w^2+0.0036w+0.0037)x^2+(0.0375w^2-0.239w-0.4977)x-0.8575w^2+6.4941w+36.078$, 100-w-x-y);
if $1.3<w\leq4.0$, coordinates (x,y,z) in the ternary composition diagram are within the range of a figure surrounded by curves MW, WN, and NO, as well as straight lines OB', B'D, DC, and CM that connect the following 7 points:

point M (0.0, $-0.3004w^2+2.419w+55.53$, $0.3004w^2-3.419w+44.47$),
point W (10.0, $-0.3645w^2+3.5024w+34.422$, $0.3645w^2-4.5024w+55.578$),
point N (18.2, $-0.3773w^2+3.319w+28.26$, $0.3773w^2-4.319w+53.54$),
point O (36.8, $-0.1392w^2+1.4381w+24.475$, $0.1392w^2-2.4381w+38.725$),
point B' (36.6, 0.0, $-w+63.4$),
point D ($-2.8226w+40.211$, 0.0, $1.8226w+59.789$), and
point C (0.0, $0.1081w^2-5.169w+58.447$, $-0.1081w^2+4.169w+41.553$), or on the above line segments (excluding the points on the straight line CM),

the curve MW is represented by coordinates (x, $(0.0043w^2-0.0359w+0.1509)x^2+(-0.0493w^2+0.4669w-3.6193)x-0.3004w^2+2.419w+55.53$, 100-w-x-y),
the curve WN is represented by coordinates (x, $(0.0055w^2-0.0326w+0.0665)x^2+(-0.1571w^2+0.8981w-2.6274)x+0.6555w^2-2.2153w+54.044$, 100-w-x-y), and

the curve NO is represented by coordinates (x, $(-0.00062w^2+0.0036w+0.0037)x^2+(0.0457w^2-0.2581w-0.075)x-1.355w^2+8.749w+27.096$, 100-w-x-y); or

if $4.0<w\leq7.0$, coordinates (x,y,z) in the ternary composition diagram are within the range of a figure surrounded by curves MW, WN, and NO, as well as straight lines OB', B'D, DC, and CM that connect the following 7 points:

point M (0.0, $-0.0667w^2+0.8333w58.133$, $0.0667w^2-1.8333w+41.867$),
point W (10.0, $-0.0667w^2+1.1w+39.267$, $0.0667w^2-2.1w+50.733$),
point N (18. 2, $-0.0889w^2+1.3778w+31.411$, $0.0889w^2-2.3778w+50.389$),
point O (36.8, $-0.0444w^2+0.6889w+25.956$, $0.0444w^2-1.6889w+37.244$), point B' (36.6, 0.0, -w+63.4),
point D (-2.8w+40. 1, 0.0, 1.8w+59.9), and
point C (0.0, $0.0667w^2-4.9667w+58.3$, $-0.0667w^2+3.9667w+41.7$),
or on the above line segments (excluding the points on the straight line CM),

the curve MW is represented by coordinates (x, $(0.00357w^2-0.0391w+0.1756)x^2+(-0.0356w^2+0.4178w-3.6422)x-0.0667w^2+0.8333w+58.103$, 100-w-x-y),
the curve WN is represented by coordinates (x, $(-0.002061w^2+0.0218w-0.0301)x^2+(0.0556w^2-0.5821w-0.1108)x-0.4158w^2+4.7352w+43.383$, 100-w-x-y), and
the curve NO is represented by coordinates (x, $(0.0082x^2+(0.0022w^2-0.0345w-0.7521)x-0.1307w^2+2.0247w+42.327$, 100-w-x-y).

When the requirements above are satisfied, Refrigerant E according to the present disclosure has a refrigerating capacity ratio of 80% or more relative to that of R410A and a GWP of 250 or less, and further ensures an ASHRAE slight flammability.

Refrigerant E according to the present disclosure is preferably a refrigerant wherein

when the mass% of $CO_2$, R32, HFO-1132(E), and R1234yf based on their sum is respectively represented by w, x, y, and z,

if $1.2<w\leq4.0$, coordinates (x,y,z) in a ternary composition diagram in which the sum of R32, HFO-1132(E), and R1234yf is (100-a) mass% are within the range of a figure surrounded by curves MW and WN, as well as straight lines NE, EC, and CM that connect the following 5 points:

point M (0.0, $-0.3004w^2+2.419w+55.53$, $0.3004w^2-3.419w+44.47$),
point W (10.0, $-0.3645w^2+3.5024w+34.422$, $0.3645w^2-4.5024w+55.578$),
point N (18.2, $-0.3773w^2+3.319w+28.26$, $0.3773w^2-4.319w+53.54$),
point E (-0.0365w+18.26, $0.0623w^2-4.5381w+31.856$, $-0.0623w^2+3.5746w+49.884$), and
point C (0.0, $0.1081w^2-5.169w+58.447$, $-0.1081w^2+4.169w+41.553$),
or on the above line segments (excluding the points on the straight line CM),

the curve MW is represented by coordinates (x, $(0.0043w^2-0.0359w+0.1509)x^2+(-0.0493w^2+0.4669w-3.6193)x-0.3004w^2+2.419w+55.53$, 100-w-x-y), and
the curve WN is represented by coordinates (x, $(0.0055w^2-0.0326w+0.0665)x^2+(-0.1571w^2+0.8981w-2.6274)x+0.6555w^2-2.2153w+54.044$, 100-w-x-y); or

if $4.0<w\leq7.0$, coordinates (x,y,z) in the ternary composition diagram are within the range of a figure surrounded by curves MW and WN, as well as straight lines NE, EC, and CM that connect the following 5 points:

point M (0.0, $-0.0667w^2+0.8333w+58.133$, $0.0667w^2-1.8333w+41.867$),
point W (10.0, $-0.0667w^2+1.1w+39.267$, $0.0667w^2-2.1w+50.733$),
point N (18.2, $-0.0889w^2+1.3778w+31.411$, $0.0889w^2-2.3778w+50.389$),
point E (18.1, $0.0444w^2-4.3556w+31.411$, $-0.0444w^2+3.3556w+50.489$), and
point C (0.0, $0.0667w^2-4.9667w+58.3$, $-0.0667w^2+3.9667w+41.7$),
or on the above line segments (excluding the points on the straight line CM),

the curve MW is represented by coordinates (x, $(0.00357w^2-0.0391w+0.1756)x^2+(-0.0356w^2+0.4178w-3.6422)x-0.0667w^2+0.8333w+58.103$, 100-w-x-y), and
the curve WN is represented by coordinates (x, $(-0.002061w^2+0.0218w-0.0301)x^2+(0.0556w^2-0.5821w-0.1108)x-0.4158w^2+4.7352w+43.383$, 100-w-x-y).

When the requirements above are satisfied, Refrigerant E according to the present disclosure has a refrigerating capacity ratio of 80% or more relative to that of R410A and a GWP of 125 or less, and further ensures an ASHRAE slight flammability.

Refrigerant E according to the present disclosure may further comprise other additional refrigerants in addition to $CO_2$, R32, HFO-1132(E), and R1234yf as long as the above properties and effects are not impaired. In this respect, Refrigerant E according to the present disclosure preferably comprises $CO_2$, R32, HFO-1132(E), and R1234yf in a total amount of 99.5 mass% or more, more preferably 99.75 mass% or more, and even more preferably 99.9 mass% or more, based on the entire refrigerant.

Additional refrigerants are not particularly limited and can be widely selected. The mixed refrigerant may contain one additional refrigerant, or two or more additional refrigerants.

Refrigerant E according to the present disclosure can be preferably used as a working fluid in a refrigerating machine.

The composition according to the present disclosure is suitable for use as an alternative refrigerant for R410A.

Examples of Refrigerant E

The present disclosure is described in more detail below with reference to Examples of Refrigerant E. However, Refrigerant E according to the present disclosure is not limited to the Examples.

The burning velocity of the mixed refrigerants of $CO_2$, R32, HFO-1132(E), and R1234yf was measured according to ANSI/ASHRAE Standard 34-2013. While changing the concentration of $CO_2$, compositions showing a burning velocity of 10 cm/s were found. Tables 27 to 29 shows the found compositions.

A burning velocity test was performed using the apparatus shown in Fig. 1 in the following manner. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps and stored on a PC. Each WCFF concentration was obtained by using the WCF concentration as the initial concentration and performing a leak simulation using NIST Standard Reference Database REFLEAK Version 4.0.

Table 27

| 0% $CO_2$ | | | | | | | | |
|---|---|---|---|---|---|---|---|---|
| Item | Unit | Comp. Ex. 13 | Comp. Ex. 14 | Comp. Ex. 15 | Comp. Ex. 16 | Comp. Ex. 17 | Comp. Ex. 18 | Comp. Ex. 19 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |
| R1234yf | mass% | 28.0 | 32.8 | 33.2 | 31.2 | 27.6 | 23.8 | 19.4 |
| $CO_2$ | mass% | 0.0 | 0.0 | 0.0 | 0.0 | 0.0 | 0.0 | 0.0 |
| Burning velocity (WCF) | cm/s | 10 | 10 | 10 | 10 | 10 | 10 | 10 |
| 0.6% $CO_2$ | | | | | | | | |
| Item | Unit | Example 3 | Example 4 | Example 5 | Example 6 | Example 7 | Example 8 | Example 9 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |

(continued)

| 0.6% CO₂ | | | | | | | | |
|---|---|---|---|---|---|---|---|---|
| Item | Unit | Example 3 | Example 4 | Example 5 | Example 6 | Example 7 | Example 8 | Example 9 |
| | | I | | J | | K | | L |
| R1234yf | mass% | 27.4 | 32.6 | 32.6 | 30.6 | 27.0 | 23.3 | 10.8 |
| $CO_2$ | mass% | 0.6 | 0.6 | 0.6 | 0.6 | 0.6 | 0.6 | 0.6 |
| Burning velocity (WCF) | cm/s | 10 | 10 | 10 | 10 | 10 | 10 | 10 |

| 1.2% CO₂ | | | | | | | | |
|---|---|---|---|---|---|---|---|---|
| Item | Unit | Comp.Ex. 48 | Example 17 | Example 18 | Example 19 | Example 20 | Example 21 | Example 22 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |
| R1234yf | mass% | 26.8 | 31.6 | 32.0 | 30.0 | 26.4 | 22.7 | 18.2 |
| $CO_2$ | mass% | 1.2 | 1.2 | 1.2 | 1.2 | 1.2 | 1.2 | 1.2 |
| Burning velocity (WCF) | cm/s | 10 | 10 | 10 | 10 | 10 | 10 | 10 |

| 1.3% CO₂ | | | | | | | | |
|---|---|---|---|---|---|---|---|---|
| Item | Unit | Comp.Ex. 59 | Example 29 | Example 30 | Example 31 | Example 32 | Example 33 | Example 34 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |
| R1234yf | mass% | 26.7 | 31.5 | 31.9 | 29.9 | 26.3 | 22.6 | 18.1 |
| $CO_2$ | mass% | 1.3 | 1.3 | 1.3 | 1.3 | 1.3 | 1.3 | 1.3 |
| Burning velocity (WCF) | cm/s | 10 | 10 | 10 | 10 | 10 | 10 | 10 |

| 2.5% CO₂ | | | | | | | | |
|---|---|---|---|---|---|---|---|---|
| Item | Unit | Comp.Ex. 69 | Example 44 | Example 45 | Example 46 | Example 47 | Example 48 | Example 49 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |
| R1234yf | mass% | 25.5 | 30.3 | 30.7 | 28.7 | 25.1 | 21.3 | 16.9 |
| $CO_2$ | mass% | 2.5 | 2.5 | 2.5 | 2.5 | 2.5 | 2.5 | 2.5 |

(continued)

| 2.5% $CO_2$ | | | | | | | | |
|---|---|---|---|---|---|---|---|---|
| Item | Unit | Comp.Ex. 69 | Example 44 | Example 45 | Example 46 | Example 47 | Example 48 | Example 49 |
| | | I | | J | | K | | L |
| Burning velocity (WCF) | cm/s | 10 | 10 | 10 | 10 | 10 | 10 | 10 |

| 4.0 $CO_2$ | | | | | | | | |
|---|---|---|---|---|---|---|---|---|
| Item | Unit | Comp.Ex. 79 | Example 59 | Example 60 | Example 61 | Example 62 | Example 63 | Example 64 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |
| R1234yf | mass% | 24.0 | 28.8 | 29.2 | 27.2 | 23.6 | 19.8 | 15.4 |
| $CO_2$ | mass% | 4.0 | 4.0 | 4.0 | 4.0 | 4.0 | 4.0 | 4.0 |
| Burning velocity (WCF) | cm/s | 10 | 10 | 10 | 10 | 10 | 10 | 10 |

| 5.5 $CO_2$ | | | | | | | | |
|---|---|---|---|---|---|---|---|---|
| Item | Unit | Comp.Ex. 89 | Example 74 | Example 75 | Example 76 | Example 77 | Example 78 | Example 79 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |
| R1234yf | mass% | 22.5 | 27.3 | 27.7 | 25.7 | 22.1 | 18.3 | 13.9 |
| $CO_2$ | mass% | 5.5 | 5.5 | 5.5 | 5.5 | 5.5 | 5.5 | 5.5 |
| Burning velocity (WCF) | cm/s | 10 | 10 | 10 | 10 | 10 | 10 | 10 |

| 7.0 $CO_2$ | | | | | | | | |
|---|---|---|---|---|---|---|---|---|
| Item | Unit | Comp.Ex. 99 | Example 89 | Example 90 | Example 91 | Example 92 | Example 93 | Example 94 |
| | | I | | J | | K | | L |
| HFO-1132(E) | mass% | 72.0 | 57.2 | 48.5 | 41.2 | 35.6 | 32.0 | 28.9 |
| R32 | mass% | 0.0 | 10.0 | 18.3 | 27.6 | 36.8 | 44.2 | 51.7 |
| R1234yf | mass% | 21.0 | 25.8 | 26.2 | 24.2 | 20.6 | 16.8 | 12.4 |
| $CO_2$ | mass% | 7.0 | 7.0 | 7.0 | 7.0 | 7.0 | 7.0 | 7.0 |
| Burning velocity (WCF) | cm/s | 10 | 10 | 10 | 10 | 10 | 10 | 10 |

Table 28

| 0% $CO_2$ | | | | | | | | | | | | |
|---|---|---|---|---|---|---|---|---|---|---|---|---|
| Item | | | Comp. Ex. 20 | Comp. Ex. 21 | Comp. Ex. 22 | Comp. Ex. 23 | Comp. Ex. 24 | Comp. Ex. 25 | Comp. Ex. 26 | Comp. Ex. 27 | Comp. Ex. 28 |
| | | | M | | W | | N | | O | | P |
| WCF | HFO-1132(E) | mass% | 52.6 | 39.2 | 32.4 | 29.3 | 27.7 | 24.5 | 22.6 | 21.2 | 20.5 |
| | R32 | mass% | 0.0 | 5.0 | 10.0 | 14.5 | 18.2 | 27.6 | 36.8 | 44.2 | 51.7 |
| | R1234yf | mass% | 47.4 | 55.8 | 57.6 | 56.2 | 54.1 | 47.9 | 40.6 | 34.6 | 27.8 |
| | $CO_2$ | mass% | 0.0 | 0.0 | 0.0 | 0.0 | 0.0 | 0.0 | 0.0 | 0.0 | 0.0 |
| Leak conditions to make WCFF | | | Storage / transport, -40°C, 0%, at release, gas phase side | Storage / transport, -40°C, 0%, at release, gas phase side | Storage / transport, -40°C, 0%, at release, gas phase side | Storage / transport, -40°C, 0%, at release, gas phase side | Storage / transport, -40°C, 0%, at release, gas phase side | Storage / transport, -40°C, 0%, at release, gas phase side | Storage / transport, -40°C, 0%, at release, gas phase side | Storage / transport, -40°C, 0%, at release, gas phase side | Storage / transport, -40°C, 0%, at release, gas phase side |
| WCFF | HFO-1132(E) | mass% | 72.0 | 57.8 | 48.7 | 43.6 | 40.6 | 34.9 | 31.4 | 29.2 | 27.1 |
| | R32 | mass% | 0.0 | 9.5 | 17.9 | 24.2 | 28.7 | 38.1 | 45.7 | 51.1 | 56.4 |
| | R1234yf | mass% | 28.0 | 32.7 | 33.4 | 32.2 | 30.7 | 27.0 | 23.0 | 19.7 | 16.5 |
| | $CO_2$ | mass% | 0.0 | 0.0 | 0.0 | 0.0 | 0.0 | 0.0 | 0.0 | 0.0 | 0.0 |
| Burning velocity (WCF) | | cm/s | ≤8 | ≤8 | ≤8 | ≤8 | ≤8 | ≤8 | ≤8 | ≤8 | ≤8 |
| Burning velocity (WCFF) | | cm/s | 10 | 10 | 10 | 10 | 10 | 10 | 10 | 10 | 10 |

(continued)

| 0.6% $CO_2$ | | | | | | | | | | | | |
|---|---|---|---|---|---|---|---|---|---|---|---|---|
| Item | | | Comp. Ex. 35 | Comp. Ex. 37 | Comp. Ex. 38 | Comp. Ex. 39 | Example 1 | Example 10 | Example 11 | Example 12 | Example 13 |
| | | | C=M | | W | | N(=E=G) | | O | | P |
| WCF | HFO-1132(E) | mass% | 55.4 | 42.4 | 35.1 | 31.6 | 29.6 | 26.3 | 24.0 | 22.4 | 20.9 |
| | R32 | mass% | 0.0 | 5.0 | 10.0 | 14.5 | 18.2 | 27.6 | 36.8 | 44.0 | 51.7 |
| | R1234yt | mass% | 44.0 | 52.0 | 54.3 | 53.3 | 51.6 | 45.5 | 38.6 | 33.0 | 26.8 |
| | $CO_2$ | mass% | 0.6 | 0.6 | 0.6 | 0.6 | 0.6 | 0.6 | 0.6 | 0.6 | 0.6 |
| Leak conditions to make WCFF | | | Storage / transport, -40°C, 0%, at release, gas phase side | Storage / transport, -40°C, 0%, at release, gas phase side | Storage / transport, -40°C, 0%, at release, liquid phase side | Storage / transport, -40°C, 0%, at release, liquid phase side | Storage / transport, -40°C, 0%, at release, gas phase side | Storage / transport, -40°C, 0%, at release, gas phase side | Storage / transport, -40°C, 0%, at release, liquid phase side | Storage / transport, -40°C, 0%, at release, liquid phase side | Storage / transport, -40°C, 0%, at release, liquid phase side |
| WCFF | HFO-1132(E) | mass% | 72.0 | 58.6 | 49.7 | 44.5 | 41.3 | 35.8 | 32.1 | 29.8 | 27.8 |
| | R32 | mass% | 0.0 | 8.9 | 16.9 | 23.0 | 27.4 | 36.6 | 44.1 | 49.4 | 54.7 |
| | R1234yf | mass% | 2.7 | 29.1 | 30.2 | 29.4 | 28.3 | 24.8 | 21.1 | 18.2 | 14.9 |
| | $CO_2$ | mass% | 3.3 | 3.4 | 3.2 | 3.1 | 3.0 | 2.8 | 2.7 | 2.6 | 2.6 |
| Burning velocity (WCF) | | cm/s | ≤8 | ≤8 | ≤8 | ≤8 | ≤8 | ≤8 | ≤8 | ≤8 | ≤8 |
| Burning velocity (WCFF) | | cm/s | 10 | 10 | 10 | 10 | 10 | 10 | 10 | 10 | 10 |

(continued)

| 1.2% CO$_2$ | | | | | | | | | | |
|---|---|---|---|---|---|---|---|---|---|---|
| Item | | | Comp. Ex. 49 | Comp. Ex. 50 | Example 16 | Example 23 | Example 24 | Example 25 | Example 26 | Example 27 | Example 28 |
| | | | M | | G=W | | N | | O | | P |
| WCF | HFO-1132(E) | mass% | 58.0 | 45.2 | 38.1 | 34.0 | 31.7 | 27.9 | 25.4 | 23.7 | 22.1 |
| | R32 | mass% | 0.0 | 5.0 | 10.0 | 14.4 | 18.2 | 27.6 | 36.8 | 44.0 | 51.7 |
| | R1234yf | mass% | 40.8 | 48.6 | 50.7 | 48.9 | 48.9 | 43.3 | 36.0 | 31.1 | 25.0 |
| | CO$_2$ | mass% | 1.2 | 1.2 | 1.2 | 1.2 | 1.2 | 1.2 | 1.2 | 1.2 | 1.2 |
| Leak conditions to make WCFF | | | Storage / transport, -40°C, 0%, at release, gas phase side | Storage / transport, -40°C, 6%, at release, gas phase side | Storage / transport, -40°C, 6% at release, liquid phase side | Storage / transport, -40°C, 6% at release, liquid phase side | Storage / transport, -40°C, 4% at release, liquid phase side | Storage / transport, -40°C, 4% at release, liquid phase side | Storage / transport, -40°C, 4% at release, liquid phase side | Storage / transport, -40°C, 4% at release, liquid phase side | Storage / transport, -40°C, 4% at release, liquid phase side |
| WCFF | HFO-1132(E) | mass% | 72.0 | 59.3 | 50.9 | 45.6 | 42.2 | 36.4 | 32.7 | 30.3 | 28.3 |
| | R32 | mass% | 0.0 | 8.3 | 15.8 | 21.7 | 26.2 | 35.3 | 42.8 | 48.1 | 53.4 |
| | R1234yf | mass% | 24.8 | 28.0 | 28.5 | 27.7 | 26.7 | 23.6 | 20.0 | 17.1 | 13.9 |
| | CO$_2$ | mass% | 3.2 | 4.4 | 4.8 | 5.0 | 4.9 | 4.7 | 4.5 | 4.5 | 4.4 |
| Burning velocity (WCF) | | cm/s | ≤8 | ≤8 | ≤8 | ≤8 | ≤8 | ≤8 | ≤8 | ≤8 | ≤8 |
| Burning velocity (WCFF) | | cm/s | 10 | 10 | 10 | 10 | 10 | 10 | 10 | 10 | 10 |

(continued)

| 1.3% $CO_2$ | | | | | | | | | | | |
|---|---|---|---|---|---|---|---|---|---|---|---|
| Item | | | Comp. Ex. 60 | Example 35 | Example 36 | Example 37 | Example 38 | Example 39 | Example 40 | Example 41 | Example 42 |
| | | | M | | W | | N | | O | | P |
| WCF | HFO-1132(E) | mass% | 58.2 | 45.5 | 38.4 | 34.3 | 31.9 | 28.1 | 25.6 | 23.9 | 22.3 |
| | R32 | mass% | 0.0 | 5.0 | 10.0 | 14.4 | 18.2 | 27.6 | 36.8 | 44.0 | 51.7 |
| | R1234yf | mass% | 40.5 | 48.2 | 50.3 | 50.0 | 48.6 | 43.0 | 36.3 | 30.8 | 24.7 |
| | $CO_2$ | mass% | 1.3 | 1.3 | 1.3 | 1.3 | 1.3 | 1.3 | 1.3 | 1.3 | 1.3 |
| Leak conditions to make WCFF | | | Storage / transport, -40°C, 0%, at release, gas phase side | Storage / transport, -40°C, 8%, at release, gas phase side | Storage / transport, -40°C, 6% at release, liquid phase side | Storage / transport, -40°C, 6% at release, liquid phase side | Storage / transport, -40°C, 6% at release, liquid phase side | Storage / transport, -40°C, 4% at release, liquid phase side | Storage / transport, -40°C, 4% at release, liquid phase side | Storage / transport, -40°C, 4% at release, liquid phase side | Storage / transport, -40°C, 4% at release, liquid phase side |
| WCFF | HFO-1132(E) | mass% | 72.0 | 59.4 | 51.0 | 45.7 | 42.2 | 36.5 | 32.8 | 30.4 | 28.4 |
| | R32 | mass% | 0.0 | 8.2 | 15.8 | 21.5 | 26.0 | 35.1 | 42.6 | 47.9 | 53.2 |
| | R1234yf | mass% | 25.0 | 27.6 | 28.1 | 27.8 | 26.9 | 26.3 | 19.7 | 16.9 | 13.6 |
| | $CO_2$ | mass% | 3.0 | 4.8 | 5.1 | 5.0 | 4.9 | 5.1 | 4.9 | 4.8 | 4.8 |
| Burning velocity (WCF) | | cm/s | ≤8 | ≤8 | ≤8 | ≤8 | ≤8 | ≤8 | ≤8 | ≤8 | ≤8 |
| Burning velocity (WCFF) | | cm/s | 10 | 10 | 10 | 10 | 10 | 10 | 10 | 10 | 10 |

Table 29

| 2.5% $CO_2$ | | | | | | | | | | | | |
|---|---|---|---|---|---|---|---|---|---|---|---|---|
| Item | | | Comp. Ex. 70 | Example 50 | Example 51 | Example 52 | Example 53 | Example 54 | Example 55 | Example 56 | Example 57 |
| | | | M | | W | | N | | O | | P |
| WCF | HFO-1132(E) | mass % | 59.7 | 48.1 | 40.9 | 36.9 | 34.2 | 29.9 | 27.2 | 25.2 | 23.4 |
| | R32 | mass % | 0.0 | 5.0 | 10.0 | 14.4 | 18.2 | 27.6 | 36.8 | 44.0 | 51.7 |
| | R1234yf | mass % | 37.8 | 44.4 | 46.6 | 46.2 | 45.1 | 40.0 | 33.5 | 28.1 | 22.4 |
| | $CO_2$ | mass % | 2.5 | 2.5 | 2.5 | 2.5 | 2.5 | 2.5 | 2.5 | 2.5 | 2.5 |
| Leak conditions to make WCFF | | | Storage / transport, -40°C, 26%, at release, gas phase side | Storage / transport, -40°C, 20%, at release, gas phase side | Storage / transport, -40°C, 20%, at release, gas phase side | Storage / transport, -40°C, 20%, at release, gas phase side | Storage / transport, -40°C, 18% at release, liquid phase side | Storage / transport, -40°C, 18% at release, liquid phase side | Storage / transport, -40°C, 18% at release, liquid phase side | Storage / transport, -40°C, 20%, at release, gas phase side | Storage / transport, -40°C, 22%, at release, gas phase side |
| WCFF | HFO-1132(E) | mass % | 72.0 | 60.3 | 52.1 | 46.9 | 43.2 | 37.1 | 33.2 | 30.6 | 28.3 |
| | R32 | mass % | 0.0 | 7.5 | 14.6 | 20.2 | 24.7 | 34.1 | 41.8 | 47.6 | 53.4 |
| | R1234yf | mass % | 24.9 | 27.4 | 28.4 | 28.0 | 26.7 | 23.4 | 19.7 | 16.9 | 13.8 |
| | $CO_2$ | mass % | 3.1 | 4.8 | 4.9 | 4.9 | 5.4 | 5.4 | 5.4 | 4.9 | 4.5 |
| Burning velocity (WCF) | | cm/s | ≤8 | ≤8 | ≤8 | ≤8 | ≤8 | ≤8 | ≤8 | ≤8 | ≤8 |

| Burning velocity (WCFF) | cm/s | 10 | 10 | 10 | 10 | 10 | 10 | 10 | 10 | 10 |
|---|---|---|---|---|---|---|---|---|---|---|
| 4.0% $CO_2$ | | | | | | | | | | |
| Item | | | Comp. Ex. 80 | Example 65 | Example 66 | Example 67 | Example 68 | Example 69 | Example 70 | Example 71 | Example 72 |
| | | | M | | W | | N | | O | | P |
| WCF | HFO-1132 (E) | mass% | 60.4 | 49.6 | 42.6 | 38.3 | 35.5 | 31.0 | 28.0 | 25.9 | 23.9 |
| | R32 | mass% | 0.0 | 5.0 | 10.0 | 14.4 | 18.2 | 27.6 | 36.8 | 44.0 | 51.7 |
| | R1234yf | mass% | 35.6 | 41.4 | 43.4 | 43.3 | 42.3 | 37.4 | 31.2 | 26.1 | 20.4 |
| | $CO_2$ | mass% | 4.0 | 4.0 | 4.0 | 4.0 | 4.0 | 4.0 | 4.0 | 4.0 | 4.0 |
| Leak conditions to make WCFF | | | Storage / transport, -40°C, 32%, at release, gas phase side | Storage / transport, -40°C, 28%, at release, gas phase side | Storage / transport, -40°C, 28%, at release, gas phase side | Storage / transport, -40°C, 28%, at release, gas phase side | Storage / transport, -40°C, 28%, at release, gas phase side | Storage / transport, -40°C, 28%, at release, gas phase side | Storage / transport, -40°C, 32%, at release, gas phase side | Storage / transport, -40°C, 32%, at release, gas phase side | Storage / transport, -40°C, 32%, at release, gas phase side |
| WCFF | HFO-1132 (E) | mass% | 72.0 | 60.9 | 52.9 | 47.5 | 43.8 | 37.4 | 33.1 | 30.5 | 28.1 |
| | R32 | mass% | 0.0 | 7.1 | 13.9 | 19.4 | 23.9 | 33.5 | 41.7 | 47.6 | 53.6 |
| | R1234yf | mass% | 24.5 | 27.0 | 28.0 | 27.8 | 26.9 | 23.6 | 20.5 | 17.2 | 13.5 |
| | $CO_2$ | mass% | 3.5 | 5.0 | 5.2 | 5.3 | 5.4 | 5.5 | 4.7 | 4.7 | 4.8 |
| Burning velocity (WCF) | cm/s | ≤8 | ≤8 | ≤8 | ≤8 | ≤8 | ≤8 | ≤8 | ≤8 | ≤8 |
| Burning velocity (WCFF) | cm/s | 10 | 10 | 10 | 10 | 10 | 10 | 10 | 10 | 10 |

56

(continued)

EP 3 988 522 A1

| 5.5% $CO_2$ | | | | | | | | | | | |
|---|---|---|---|---|---|---|---|---|---|---|---|
| Item | | | Comp. Ex. 90 | Example 80 | Example 81 | Example 82 | Example 83 | Example 84 | Example 85 | Example 86 | Example 87 |
| | | | M | | W | | N | | O | | P |
| WCF | HFO-1132 (E) | mass% | 60.7 | 50.3 | 43.3 | 39.0 | 36.3 | 31.6 | 28.4 | 26.2 | 24.2 |
| | R32 | mass% | 0.0 | 5.0 | 10.0 | 14.4 | 18.2 | 27.6 | 36.8 | 44.0 | 51.7 |
| | R1234yf | mass% | 33.8 | 39.2 | 41.2 | 41.1 | 40.0 | 35.3 | 29.3 | 24.3 | 18.6 |
| | $CO_2$ | mass% | 5.5 | 5.5 | 5.5 | 5.5 | 5.5 | 5.5 | 5.5 | 5.5 | 5.5 |
| Leak conditions to make WCFF | | | Storage / transport, -40°C, 36%, at release, gas phase side | Storage / transport, -40°C, 34%, at release, gas phase side | Storage / transport, -40°C, 34%, at release, gas phase side | Storage / transport, -40°C, 32%, at release, gas phase side | Storage / transport, -40°C, 34%, at release, gas phase side | Storage / transport, -40°C, 36%, at release, gas phase side | Storage / transport, -40°C, 38%, at release, gas phase side | Storage / transport, -40°C, 40%, at release, gas phase side | Storage / transport, -40°C, 40%, at release, gas phase side |
| WCFF | HFO-1132 (E) | mass% | 72.0 | 61.2 | 53.2 | 47.8 | 44.2 | 37.6 | 33.2 | 30.3 | 27.9 |
| | R32 | mass% | 0.0 | 6.8 | 13.5 | 19.0 | 23.4 | 33.2 | 41.7 | 47.9 | 54.2 |
| | R1234yf | mass% | 24.5 | 27.0 | 28.1 | 27.7 | 26.8 | 23.9 | 20.2 | 17.3 | 13.3 |
| | $CO_2$ | mass% | 3.5 | 5.0 | 5.2 | 5.5 | 5.6 | 5.3 | 4.9 | 4.5 | 4.6 |
| Burning velocity (WCF) | | cm/s | ≤8 | ≤8 | ≤8 | ≤8 | ≤8 | ≤8 | ≤8 | ≤8 | ≤8 |
| Burning velocity (WCFF) | | cm/s | 10 | 10 | 10 | 10 | 10 | 10 | 10 | 10 | 10 |

(continued)

| 7.0% CO$_2$ | | | | | | | | | | | |
|---|---|---|---|---|---|---|---|---|---|---|---|
| Item | | | Comp. Ex. 100 | Example 95 | Example 96 | Example 97 | Example 98 | Example 99 | Example 100 | Example 101 | Example 102 |
| | | | M | | W | | N | | O | | P |
| WCF | HFO-1132(E) | mass% | 60.7 | 50.3 | 43.7 | 39.5 | 36.7 | 31.9 | 28.6 | 26.4 | 24.2 |
| | R32 | mass% | 0.0 | 5.0 | 10.0 | 14.4 | 18.2 | 27.6 | 36.8 | 44.0 | 51.7 |
| | R1234yf | mass% | 32.3 | 37.7 | 39.3 | 39.1 | 38.1 | 33.5 | 27.6 | 22.6 | 17.1 |
| | CO$_2$ | mass% | 7.0 | 7.0 | 7.0 | 7.0 | 7.0 | 7.0 | 7.0 | 7.0 | 7.0 |
| Leak conditions to make WCFF | | | Storage / transport, -40°C, 42%, at release, gas phase side | Storage / transport, -40°C, 34%, at release, gas phase side | Storage / transport, -40°C, 38%, at release, gas phase side | Storage / transport, -40°C, 40%, at release, gas phase side | Storage / transport, -40°C, 40%, at release, gas phase side | Storage / transport, -40°C, 42%, at release, gas phase side | Storage / transport, -40°C, 42%, at release, gas phase side | Storage / transport, -40°C, 42%, at release, gas phase side | Storage / transport, -40°C, 44%, at release, gas phase side |
| WCFF | HFO-1132(E) | mass% | 72.0 | 61.2 | 53.4 | 48.1 | 44.4 | 37.7 | 33.2 | 30.4 | 27.8 |
| | R32 | mass% | 0.0 | 6.8 | 13.3 | 18.7 | 23.2 | 33.1 | 41.7 | 47.9 | 54.6 |
| | R1234yf | mass% | 24.4 | 27.0 | 27.8 | 28.1 | 27.1 | 24.1 | 19.8 | 16.3 | 12.7 |
| | CO$_2$ | mass% | 3.6 | 5.0 | 5.5 | 5.1 | 5.3 | 5.1 | 5.3 | 5.4 | 4.9 |
| Burning velocity (WCF) | | cm/s | ≤8 | ≤8 | ≤8 | ≤8 | ≤8 | ≤8 | ≤8 | ≤8 | ≤8 |
| Burning velocity (WCFF) | | cm/s | 10 | 10 | 10 | 10 | 10 | 10 | 10 | 10 | 10 |

These results indicate that under the condition that the mass% of $CO_2$, R32, HFO-1132 (E), and R1234yf based on their sum is respectively represented by w, x, y, and z, when coordinates (x,y,z) in a ternary composition diagram (Figs. 2 to 9) in which the sum of R32, HFO-1132(E), and R1234yf is (100-w) mass% are on or below the line segments that connect points I, J, K, and L, the refrigerant has a WCF slight flammability. Further, the results indicate that when coordinates (x,y,z) in the ternary composition diagram of Fig. 2 are on or below line segments that connect points M, N, O, and P, the refrigerant has an ASHRAE slight flammability. Mixed refrigerants were prepared by mixing R32, HFO-1132(E), and R1234yf at mass% based on their sum shown in Tables 30 to 40. The coefficient of performance (COP) ratio and the refrigerating capacity ratio of the mixed refrigerants in Tables 30 to 37 relative to those of R410 were determined.

The GWP of compositions each comprising a mixture of R1234yf and R410A (R32 = 50%/R125 = 50%) was evaluated based on the values stated in the Intergovernmental Panel on Climate Change (IPCC), fourth assessment report. The GWP of HFO-1132(E), which was not stated in the report, was assumed to be 1 from HFO-1132a (GWP = 1 or less) and HFO-1123 (GWP = 0.3, described in PTL 1). The refrigerating capacity of compositions each comprising R410A and a mixture of HFO-1132(E), HFO-1123, and R1234yf was determined by performing theoretical refrigeration cycle calculations for the mixed refrigerants using the National Institute of Science and Technology (NIST) and Reference Fluid Thermodynamic and Transport Properties Database (Refprop 9.0) under the following conditions.

Evaporating temperature: 5°C
Condensation temperature: 45°C
Degree of superheating: 5 K
Degree of subcooling: 5 K
$E_{comp}$ (compressive modulus): 0.7 kWh

Tables 30 to 37 show these values together with the GWP of each mixed refrigerant. Tables 30 to 37 respectively show the cases in which the $CO_2$ concentrations are 0 mass%, 0.6 mass%, 1.2 mass%, 1.3 mass%, 2.5 mass%, 4 mass%, 5.5 mass%, and 7 mass%.

Table 30

| 0% $CO_2$ | | | | | | | | | | |
|---|---|---|---|---|---|---|---|---|---|---|
| Item | Unit | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 | Comp. Ex. 9 |
| | | | A | B | A' | B' | A" | B" | C | D |
| HFO-1132(E) | mass% | R410A | 81.6 | 0.0 | 63.1 | 0.0 | 48.2 | 0.0 | 58.3 | 0.0 |
| R32 | mass% | | 18.4 | 18.1 | 36.9 | 36.7 | 51.8 | 51.5 | 0.0 | 40.3 |
| R1234yf | mass% | | 0.0 | 81.9 | 0.0 | 63.3 | 0.0 | 49.5 | 41.7 | 59.7 |
| $CO_2$ | mass% | | 0.0 | 0.0 | 0.0 | 0.0 | 0.0 | 0.0 | 0.0 | 0.0 |
| GWP | - | 2088 | 125 | 125 | 250 | 250 | 350 | 350 | 2 | 274 |
| COP ratio | % (relative to R410A) | 100 | 98.7 | 103.6 | 98.7 | 102.3 | 99.2 | 102.1 | 100.3 | 102.2 |
| Refrigerating capacity ratio | % (relative to R410A) | 100 | 105.3 | 62.5 | 109.9 | 77.5 | 112.1 | 87.0 | 80.0 | 80.0 |
| Condensation glide | °C | 0.1 | 0.3 | 6.8 | 0.1 | 4.5 | 0.0 | 2.7 | 2.9 | 4.0 |
| Item | Unit | Comp. Ex. 10 | Comp. Ex. 11 | Comp. Ex. 12 | Comp. Ex. 13 | Comp. Ex. 14 | Comp. Ex. 15 | Comp. Ex. 16 | Comp. Ex. 17 | Comp. Ex. 18 |
| | | E | F | G | I | | J | | K | |
| HFO-1132(E) | mass% | 31.9 | 5.2 | 26.2 | 72.0 | 57.2 | 48.5 | 41.2 | 35.6 | 32.0 |
| R32 | mass% | 18.2 | 36.7 | 22.2 | 0.0 | 10.0 | 18.3 | 27.6 | 36.8 | 44.2 |
| R1234yf | mass% | 49.9 | 58.1 | 51.6 | 28.0 | 32.8 | 33.2 | 31.2 | 27.6 | 23.8 |
| $CO_2$ | mass% | 0.0 | 0.0 | 0.0 | 0.0 | 0.0 | 0.0 | 0.0 | 0.0 | 0.0 |
| GWP | - | 125 | 250 | 152 | 2 | 69 | 125 | 188 | 250 | 300 |
| COP ratio | % (relative to R410A) | 100.3 | 101.8 | 100.5 | 99.9 | 99.5 | 99.4 | 99.5 | 99.6 | 99.8 |
| Refrigerati ng capacity ratio | % (relative to R410A) | 82.3 | 80.8 | 82.4 | 86.6 | 88.4 | 90.9 | 94.2 | 97.7 | 100.5 |
| Condensation glide | °C | 4.4 | 4.3 | 4.5 | 1.7 | 2.6 | 2.7 | 2.4 | 1.9 | 1.6 |

| Item | Unit | Comp. Ex. 19 | Comp. Ex. 20 | Comp. Ex. 21 | Comp. Ex. 22 | Comp. Ex. 23 | Comp. Ex. 24 | Comp. Ex. 25 | Comp. Ex. 26 | Comp. Ex. 27 | Comp. Ex. 28 |
|---|---|---|---|---|---|---|---|---|---|---|---|
| | | L | M | | W | | N | | O | | P |
| HFO-1132(E) | mass% | 28.9 | 52.6 | 39.2 | 32.4 | 29.3 | 27.7 | 24.5 | 22.6 | 21.2 | 20.5 |
| R32 | mass% | 51.7 | 0.0 | 5.0 | 10.0 | 14.5 | 18.2 | 27.6 | 36.8 | 44.2 | 51.7 |
| R1234yf | mass% | 19.4 | 47.4 | 55.8 | 57.6 | 56.2 | 54.1 | 47.9 | 40.6 | 34.6 | 27.8 |
| $CO_2$ | mass% | 0.0 | 0.0 | 0.0 | 0.0 | 0.0 | 0.0 | 0.0 | 0.0 | 0.0 | 0.0 |
| GWP | - | 350 | 2 | 36 | 70 | 100 | 125 | 188 | 250 | 300 | 350 |
| COP ratio | % (relative to R410A) | 100.1 | 100.5 | 100.9 | 100.9 | 100.8 | 100.7 | 100.4 | 100.4 | 100.5 | 100.6 |
| Refrigerati ng capacity ratio | % (relative to R410A) | 103.3 | 77.1 | 74.8 | 75.6 | 77.8 | 80.0 | 85.5 | 91.0 | 95.0 | 99.1 |
| Condensation glide | °C | 1.2 | 3.4 | 4.7 | 5.2 | 5.1 | 4.9 | 4.0 | 3.0 | 2.3 | 1.7 |

Table 31

| Item | Unit | Comp. Ex. 29 | Comp. Ex. 30 | Comp. Ex. 31 | Comp. Ex. 32 | Comp. Ex. 33 | Comp. Ex. 34 | Comp. Ex. 35 | Comp. Ex. 36 | Example 1 |
|---|---|---|---|---|---|---|---|---|---|---|
| 0.6% $CO_2$ | | | | | | | | | | |
| | | A | B | A' | B' | A" | B" | C=M | D | E=G=N |
| HFO-1132(E) | mass% | 81.0 | 0.0 | 62.5 | 0.0 | 47.6 | 0.0 | 55.4 | 0.0 | 29.6 |
| R32 | mass% | 18.4 | 18.1 | 36.9 | 36.7 | 51.8 | 51.6 | 0.0 | 38.6 | 18.2 |
| R1234yf | mass% | 0.0 | 81.3 | 0.0 | 62.7 | 0.0 | 47.8 | 44.0 | 60.8 | 51.6 |
| $CO_2$ | mass% | 0.6 | 0.6 | 0.6 | 0.6 | 0.6 | 0.6 | 0.6 | 0.6 | 0.6 |
| GWP | - | 125 | 125 | 250 | 250 | 350 | 350 | 2 | 263 | 125 |
| COP ratio | % (relative to R410A) | 98.4 | 103.4 | 98.4 | 102.1 | 99.0 | 102.0 | 100.1 | 102.1 | 100.2 |
| Refrigerating capacity ratio | % (relative to R410A) | 106.5 | 63.7 | 111.1 | 78.7 | 113.1 | 88.6 | 80.0 | 80.0 | 82.4 |
| Condensation glide | °C | 0.7 | 7.5 | 0.4 | 4.9 | 0.3 | 3.0 | 3.9 | 4.7 | 5.2 |

| Item | Unit | Example 2 | Example 3 | Example 4 | Example 5 | Example 6 | Example 7 | Example 8 | Example 9 | Comp. Ex. 37 |
|---|---|---|---|---|---|---|---|---|---|---|
| | | F | I | | J | | K | | L | |
| HFO-1132(E) | mass% | 2.7 | 72.0 | 57.2 | 48.5 | 41.2 | 35.6 | 32.0 | 28.9 | 42.4 |
| R32 | mass% | 36.7 | 0.0 | 10.0 | 18.3 | 27.6 | 36.8 | 44.2 | 51.7 | 5.0 |
| R1234yf | mass% | 60.0 | 27.4 | 32.6 | 32.6 | 30.6 | 27.0 | 23.3 | 10.8 | 52.0 |
| $CO_2$ | mass% | 0.6 | 0.6 | 0.6 | 0.6 | 0.6 | 0.6 | 0.6 | 0.6 | 0.6 |
| GWP | - | 250 | 2 | 69 | 125 | 188 | 250 | 300 | 350 | 36 |
| COP ratio | % (relative to R410A) | 101.8 | 99.5 | 99.2 | 99.1 | 99.2 | 99.4 | 99.6 | 99.7 | 100.3 |
| Refrigerating capacity ratio | % (relative to R410A) | 80.4 | 88.1 | 89.7 | 92.3 | 95.5 | 99.0 | 101.7 | 108.2 | 77.9 |
| Condensation glide | °C | 4.8 | 5.2 | 2.4 | 3.2 | 3.1 | 2.8 | 2.3 | 1.9 | 3.9 |

(continued)

| Item | Unit | Comp. Ex. 38 | Comp. Ex. 39 | Example 10 | Example 11 | Example 12 | Example 13 |
|---|---|---|---|---|---|---|---|
| | | W | | | O | | P |
| HFO-1132(E) | mass% | 35.1 | 31.6 | 26.3 | 24.0 | 22.4 | 20.9 |
| R32 | mass% | 10.0 | 14.5 | 27.6 | 36.8 | 44.0 | 51.7 |
| R1234yf | mass% | 54.3 | 53.3 | 45.5 | 38.6 | 33.0 | 26.8 |
| $CO_2$ | mass% | 0.6 | 0.6 | 0.6 | 0.6 | 0.6 | 0.6 |
| GWP | - | 70 | 100 | 188 | 250 | 299 | 350 |
| COP ratio | % (relative to R410A) | 100.4 | 100.3 | 100.1 | 100.1 | 100.2 | 100.4 |
| Refrigerati ng capacity ratio | % (relative to R410A) | 78.5 | 80.4 | 87.8 | 93.0 | 96.8 | 100.5 |
| Condensation glide | °C | 5.1 | 5.5 | 5.4 | 5.1 | 4.2 | 3.2 |

Table 6

1.2% $CO_2$

| Item | Unit | Comp. Ex. 40 | Comp. Ex. 41 | Comp. Ex. 42 | Comp. Ex. 43 | Comp. Ex. 44 | Comp. Ex. 45 | Comp. Ex. 46 | Comp. Ex. 47 | Example 14 |
|---|---|---|---|---|---|---|---|---|---|---|
| | | A | B | A' | B' | A'' | B'' | C | D | E |
| HFO-1132(E) | mass% | 80.4 | 0.0 | 61.9 | 0.0 | 47.0 | 0.0 | 52.4 | 0.0 | 26.5 |
| R32 | mass% | 18.4 | 18.1 | 36.9 | 36.6 | 51.8 | 51.6 | 0.0 | 36.8 | 18.2 |
| R1234yf | mass% | 0.0 | 80.7 | 0.0 | 62.2 | 0.0 | 46.9 | 46.4 | 62.0 | 54.1 |
| $CO_2$ | mass% | 1.2 | 1.2 | 1.2 | 1.2 | 1.2 | 1.2 | 1.2 | 1.2 | 1.2 |
| GWP | - | 125 | 125 | 250 | 250 | 350 | 350 | 2 | 251 | 125 |
| COP ratio | % (relative to R410A) | 98.1 | 103.2 | 98.2 | 101.9 | 98.7 | 101.7 | 99.9 | 101.9 | 100.2 |
| Refrigerating capacity ratio | % (relative to R410A) | 107.7 | 65.0 | 112.2 | 79.8 | 114.2 | 89.9 | 80.0 | 80.0 | 82.0 |
| Condensation glide | ℃ | 1.2 | 8.1 | 0.8 | 5.4 | 0.6 | 3.4 | 4.9 | 5.3 | 6.0 |

| Item | Unit | Example 15 | Example 16 | Comp. Ex. 48 | Example 17 | Example 18 | Example 19 | Example 20 | Example 21 | Example 22 |
|---|---|---|---|---|---|---|---|---|---|---|
| | | F | G=W | I | | J | | K | | L |
| HFO-1132(E) | mass% | 0.3 | 38.1 | 72.0 | 57.2 | 48.5 | 41.2 | 35.6 | 32.0 | 28.9 |
| R32 | mass% | 36.6 | 10.0 | 0.0 | 10.0 | 18.3 | 27.6 | 36.8 | 44.2 | 51.7 |
| R1234yf | mass% | 61.9 | 50.7 | 26.8 | 31.6 | 32.0 | 30.0 | 26.4 | 22.7 | 18.2 |
| $CO_2$ | mass% | 1.2 | 1.2 | 1.2 | 1.2 | 1.2 | 1.2 | 1.2 | 1.2 | 1.2 |
| GWP | - | 250 | 70 | 2 | 69 | 125 | 188 | 250 | 300 | 350 |
| COP ratio | % (relative to R410A) | 101.9 | 99.9 | 99.2 | 98.9 | 98.8 | 98.9 | 99.1 | 99.4 | 99.6 |
| Refrigerating capacity ratio | % (relative to R410A) | 80.0 | 81.6 | 89.7 | 91.3 | 93.7 | 96.9 | 100.3 | 103.0 | 105.8 |
| Condensation glide | ℃ | 5.4 | 5.7 | 3.1 | 3.6 | 3.6 | 3.2 | 2.6 | 2.2 | 1.8 |

| Item | Unit | Comp. Ex. 49 | Comp. Ex. 50 | Example 23 | Example 24 | Example 25 | Example 26 | Example 27 | Example 28 |
|---|---|---|---|---|---|---|---|---|---|
| | | M | | | N | | O | | P |
| HFO-1132 (E) | mass% | 58.0 | 45.2 | 34.0 | 31.7 | 27.9 | 25.4 | 23.7 | 22.1 |
| R32 | mass% | 0.0 | 5.0 | 14.4 | 18.2 | 27.6 | 36.8 | 44.0 | 51.7 |
| R1234yf | mass% | 40.8 | 48.6 | 48.9 | 48.9 | 43.3 | 36.0 | 31.1 | 25.0 |
| $CO_2$ | mass% | 1.2 | 1.2 | 1.2 | 1.2 | 1.2 | 1.2 | 1.2 | 1.2 |
| GWP | - | 2 | 36 | 100 | 125 | 188 | 250 | 298 | 350 |
| COP ratio | % (relative to R410A) | 99.6 | 99.8 | 99.8 | 99.8 | 99.7 | 99.7 | 99.9 | 100.0 |
| Refrigerating capacity ratio | % (relative to R410A) | 82.9 | 80.9 | 83.6 | 84.9 | 90.0 | 95.3 | 98.7 | 102.4 |
| Condensation glide | ℃ | 4.3 | 5.4 | 5.6 | 5.4 | 4.4 | 3.4 | 2.8 | 2.2 |

Table 7

1.3% $CO_2$

| Item | Unit | Comp. Ex. 51 | Comp. Ex. 52 | Comp. Ex. 53 | Comp. Ex. 54 | Comp. Ex. 55 | Comp. Ex. 56 | Comp. Ex. 57 | Comp. Ex. 58 | Comp. Ex. 59 |
|---|---|---|---|---|---|---|---|---|---|---|
| | | A | B | A' | B'=D=F | A'' | B'' | C | E | I |
| HFO-1132(E) | mass% | 80.3 | 0.0 | 61.8 | 0.0 | 46.9 | 0.0 | 51.9 | 26.1 | 72.0 |
| R32 | mass% | 18.4 | 18.1 | 36.9 | 36.6 | 51.8 | 51.6 | 0.0 | 18.2 | 0.0 |
| R1234yf | mass% | 0.0 | 80.6 | 0.0 | 62.1 | 0.0 | 47.1 | 46.8 | 54.4 | 26.7 |
| $CO_2$ | mass% | 1.3 | 1.3 | 1.3 | 1.3 | 1.3 | 1.3 | 1.3 | 1.3 | 1.3 |
| GWP | - | 125 | 125 | 250 | 250 | 350 | 350 | 2 | 125 | 2 |
| COP ratio | % (relative to R410A) | 98.0 | 103.2 | 98.1 | 101.9 | 98.7 | 101.7 | 99.8 | 100.2 | 99.1 |
| Refrigerating capacity ratio | % (relative to R410A) | 107.9 | 65.2 | 112.3 | 80.0 | 114.3 | 90.0 | 80.0 | 82.0 | 89.9 |
| Condensation glide | °C | 1.2 | 8.2 | 0.8 | 5.4 | 0.7 | 3.4 | 5.1 | 6.1 | 3.2 |

| Item | Unit | Example 29 | Example 30 | Example 31 | Example 32 | Example 33 | Example 34 | Comp. Ex. 60 | Example 35 | Example 36 |
|---|---|---|---|---|---|---|---|---|---|---|
| | | | J | | K | | L | M | | W |
| HFO-1132 (E) | mass% | 57.2 | 48.5 | 41.2 | 35.6 | 32.0 | 28.9 | 58.2 | 45.5 | 38.4 |
| R32 | mass% | 10.0 | 18.3 | 27.6 | 36.8 | 44.2 | 51.7 | 0.0 | 5.0 | 10.0 |
| R1234yf | mass% | 31.5 | 31.9 | 29.9 | 26.3 | 22.6 | 18.1 | 40.5 | 48.2 | 50.3 |
| $CO_2$ | mass% | 1.3 | 1.3 | 1.3 | 1.3 | 1.3 | 1.3 | 1.3 | 1.3 | 1.3 |
| GWP | - | 69 | 125 | 188 | 250 | 300 | 350 | 2 | 36 | 70 |
| COP ratio | % (relative to R410A) | 98.9 | 98.8 | 98.9 | 99.1 | 99.3 | 99.6 | 99.5 | 99.8 | 99.8 |
| Refrigerating capacity ratio | % (relative to R410A) | 91.5 | 93.9 | 97.1 | 100.5 | 103.2 | 106.0 | 83.3 | 81.3 | 82.0 |
| Condensation glide | °C | 3.7 | 3.6 | 3.2 | 2.7 | 2.3 | 1.8 | 4.4 | 5.4 | 5.8 |

| Item | Unit | Example 37 | Example 38 | Example 39 | Example 40 | Example 41 | Example 42 |
|---|---|---|---|---|---|---|---|
| | | | N | | O | | P |
| HFO-1132 (E) | mass% | 34.3 | 31.9 | 28.1 | 25.6 | 23.9 | 22.3 |
| R32 | mass% | 14.4 | 18.2 | 27.6 | 36.8 | 44.0 | 51.7 |
| R1234yf | mass% | 50.0 | 48.6 | 43.0 | 36.3 | 30.8 | 24.7 |
| $CO_2$ | mass% | 1.3 | 1.3 | 1.3 | 1.3 | 1.3 | 1.3 |
| GWP | - | 100 | 125 | 188 | 250 | 298 | 350 |
| COP ratio | % (relative to R410A) | 99.8 | 99.8 | 99.6 | 99.7 | 99.8 | 100.0 |
| Refrigerating capacity ratio | % (relative to R410A) | 83.5 | 85.2 | 90.3 | 95.4 | 99.0 | 102.7 |
| Condensation glide | °C | 6 | 5.4 | 4.5 | 3.5 | 2.9 | 2.3 |

Table 34

2.5% $CO_2$

| Item | Unit | Comp. Ex. 61 | Comp. Ex. 62 | Comp. Ex. 63 | Comp. Ex. 64 | Comp. Ex. 65 | Comp. Ex. 66 | Comp. Ex. 67 | Comp. Ex. 68 | Example 43 |
|---|---|---|---|---|---|---|---|---|---|---|
| | | A | B | A′ | B′ | A″ | B″ | C | D | E |
| HFO-1132(E) | mass% | 79.1 | 0.0 | 60.6 | 0.0 | 45.7 | 0.0 | 46.2 | 0.0 | 20.9 |
| R32 | mass% | 18.4 | 18.1 | 36.9 | 36.6 | 51.8 | 51.6 | 0.0 | 33.2 | 18.2 |
| R1234yf | mass% | 0.0 | 79.4 | 0.0 | 60.9 | 0.0 | 45.9 | 51.3 | 64.3 | 58.4 |
| $CO_2$ | mass% | 2.5 | 2.5 | 2.5 | 2.5 | 2.5 | 2.5 | 2.5 | 2.5 | 2.5 |
| GWP | - | 125 | 125 | 250 | 250 | 350 | 350 | 3 | 227 | 125 |
| COP ratio | % (relative to R410A) | 97.4 | 102.7 | 97.6 | 101.5 | 98.3 | 101.3 | 99.6 | 101.6 | 100.2 |
| Refrigerating capacity ratio | % (relative to R410A) | 110.3 | 67.8 | 114.5 | 82.5 | 116.4 | 92.5 | 80.0 | 80.0 | 81.7 |
| Condensation glide | °C | 2.0 | 9.5 | 1.5 | 6.3 | 1.3 | 4.1 | 7.1 | 6.9 | 7.6 |

| Item | Unit | Comp. Ex. 69 | Example 44 | Example 45 | Example 46 | Example 47 | Example 48 | Example 49 | Comp. Ex. 70 | Example 50 |
|---|---|---|---|---|---|---|---|---|---|---|
| | | I | | J | | K | | L | M | |
| HFO-1132(E) | mass% | 72.0 | 57.2 | 48.5 | 41.2 | 35.6 | 32.0 | 28.9 | 59.7 | 48.1 |
| R32 | mass% | 0.0 | 10.0 | 18.3 | 27.6 | 36.8 | 44.2 | 51.7 | 0.0 | 5.0 |
| R1234yf | mass% | 25.5 | 30.3 | 30.7 | 28.7 | 25.1 | 21.3 | 16.9 | 37.8 | 44.4 |
| $CO_2$ | mass% | 2.5 | 2.5 | 2.5 | 2.5 | 2.5 | 2.5 | 2.5 | 2.5 | 2.5 |
| GWP | - | 2 | 69 | 125 | 188 | 250 | 300 | 350 | 2 | 36 |
| COP ratio | % (relative to R410A) | 98.4 | 98.2 | 98.2 | 98.4 | 98.6 | 98.9 | 99.1 | 98.8 | 99.0 |
| Refrigerating capacity ratio | % (relative to R410A) | 93.1 | 94.5 | 96.7 | 99.8 | 103.1 | 105.9 | 108.6 | 87.1 | 85.7 |
| Condensation glide | °C | 4.4 | 4.7 | 4.5 | 3.9 | 3.3 | 2.8 | 2.4 | 5.6 | 6.3 |

| Item | Unit | Example 51 | Example 52 | Example 53 | Example 54 | Example 55 | Example 56 | Example 57 |
|---|---|---|---|---|---|---|---|---|
| | | W | | N | | O | | P |
| HFO-1132(E) | mass% | 40.9 | 36.9 | 34.2 | 29.9 | 27.2 | 25.2 | 23.4 |
| R32 | mass% | 10.0 | 14.4 | 18.2 | 27.6 | 36.8 | 44.0 | 51.7 |
| R1234yf | mass% | 46.6 | 46.2 | 45.1 | 40.0 | 33.5 | 28.1 | 22.4 |
| $CO_2$ | mass% | 2.5 | 2.5 | 2.5 | 2.5 | 2.5 | 2.5 | 2.5 |
| GWP | - | 70 | 99 | 125 | 188 | 250 | 298 | 350 |
| COP ratio | % (relative to R410A) | 99.1 | 99.1 | 99.1 | 99.0 | 99.1 | 99.3 | 99.5 |
| Refrigerating capacity ratio | % (relative to R410A) | 86.2 | 87.7 | 89.2 | 94.0 | 98.8 | 102.4 | 105.8 |
| Condensation glide | °C | 6 | 6.3 | 6.0 | 5.0 | 4.0 | 3.4 | 2.8 |

Table 35

4% $CO_2$

| Item | Unit | Comp. Ex. 71 | Comp. Ex. 72 | Comp. Ex. 73 | Comp. Ex. 74 | Comp. Ex. 75 | Comp. Ex. 76 | Comp. Ex. 77 | Comp. Ex. 78 | Example 58 |
|---|---|---|---|---|---|---|---|---|---|---|
| | | A | B | A′ | B′ | A″ | B″ | C | D | E |
| HFO-1132(E) | mass% | 77.6 | 0.0 | 59.1 | 0.0 | 44.2 | 0.0 | 39.5 | 0.0 | 14.7 |
| R32 | mass% | 18.4 | 18.1 | 36.9 | 36.6 | 51.8 | 51.6 | 0.0 | 28.9 | 18.1 |
| R1234yf | mass% | 0.0 | 77.9 | 0.0 | 59.4 | 0.0 | 44.4 | 56.5 | 67.1 | 63.2 |
| $CO_2$ | mass% | 4.0 | 4.0 | 4.0 | 4.0 | 4.0 | 4.0 | 4.0 | 4.0 | 4.0 |
| GWP | - | 125 | 125 | 250 | 249 | 350 | 350 | 3 | 198 | 125 |
| COP ratio | % (relative to R410A) | 96.7 | 102.2 | 97.0 | 101.0 | 97.7 | 100.8 | 99.4 | 101.3 | 100.4 |
| Refrigerating capacity ratio | % (relative to R410A) | 113.3 | 71.2 | 117.3 | 85.7 | 118.9 | 95.6 | 80.0 | 80.0 | 81.2 |
| Condensation glide | °C | 3.0 | 10.9 | 2.2 | 7.2 | 2.0 | 5.0 | 9.6 | 8.7 | 9.6 |

| Item | Unit | Comp. Ex. 79 | Example 59 | Example 60 | Example 61 | Example 62 | Example 63 | Example 64 | Comp. Ex. 80 | Example 65 |
|---|---|---|---|---|---|---|---|---|---|---|
| | | I | | J | | K | | L | M | |
| HFO-1132(E) | mass% | 72.0 | 57.2 | 48.5 | 41.2 | 35.6 | 32.0 | 28.9 | 60.4 | 49.6 |
| R32 | mass% | 0.0 | 10.0 | 18.3 | 27.6 | 36.8 | 44.2 | 51.7 | 0.0 | 5.0 |
| R1234yf | mass% | 24.0 | 28.8 | 29.2 | 27.2 | 23.6 | 19.8 | 15.4 | 35.6 | 41.4 |
| $CO_2$ | mass% | 4.0 | 4.0 | 4.0 | 4.0 | 4.0 | 4.0 | 4.0 | 4.0 | 4.0 |
| GWP | - | 2 | 69 | 125 | 188 | 250 | 300 | 350 | 2 | 36 |
| COP ratio | % (relative to R410A) | 97.6 | 97.5 | 97.5 | 97.7 | 98.0 | 98.3 | 98.6 | 98.0 | 98.2 |
| Refrigerating capacity ratio | % (relative to R410A) | 97.0 | 98.1 | 100.2 | 103.2 | 106.5 | 109.1 | 111.8 | 91.3 | 90.2 |
| Condensation glide | °C | 5.8 | 5.8 | 5.4 | 4.7 | 4.0 | 3.5 | 3.1 | 6.9 | 7.4 |

| Item | Unit | Example 66 | Example 67 | Example 68 | Example 69 | Example 70 | Example 71 | Example 72 |
|---|---|---|---|---|---|---|---|---|
| | | W | | N | | O | | P |
| HFO-1132(E) | mass% | 42.6 | 38.3 | 35.5 | 31.0 | 28.0 | 25.9 | 23.9 |
| R32 | mass% | 10.0 | 14.4 | 18.2 | 27.6 | 36.8 | 44.0 | 51.7 |
| R1234yf | mass% | 43.4 | 43.3 | 42.3 | 37.4 | 31.2 | 26.1 | 20.4 |
| $CO_2$ | mass% | 4.0 | 4.0 | 4.0 | 4.0 | 4.0 | 4.0 | 4.0 |
| GWP | - | 70 | 99 | 125 | 188 | 250 | 298 | 350 |
| COP ratio | % (relative to R410A) | 98.3 | 98.3 | 98.3 | 98.3 | 98.5 | 98.7 | 98.9 |
| Refrigerating capacity ratio | % (relative to R410A) | 90.7 | 92.0 | 93.4 | 97.9 | 102.5 | 105.9 | 109.3 |
| Condensation glide | °C | 7 | 7.2 | 6.9 | 5.8 | 4.7 | 4.0 | 3.4 |

Table 36

5.5% CO₂

| Item | Unit | Comp. Ex. 81 | Comp. Ex. 82 | Comp. Ex. 83 | Comp. Ex. 84 | Comp. Ex. 85 | Comp. Ex. 86 | Comp. Ex. 87 | Comp. Ex. 88 | Example 73 |
|---|---|---|---|---|---|---|---|---|---|---|
| | | A | B | A′ | B′ | A″ | B″ | C | D | E |
| HFO-1132(E) | mass% | 76.1 | 0.0 | 57.6 | 0.0 | 42.7 | 0.0 | 33.0 | 0.0 | 8.8 |
| R32 | mass% | 18.4 | 18.1 | 36.9 | 36.6 | 51.8 | 51.6 | 0.0 | 24.7 | 18.1 |
| R1234yf | mass% | 0.0 | 76.4 | 0.0 | 57.9 | 0.0 | 42.9 | 61.5 | 69.8 | 67.6 |
| CO₂ | mass% | 5.5 | 5.5 | 5.5 | 5.5 | 5.5 | 5.5 | 5.5 | 5.5 | 5.5 |
| GWP | - | 125 | 125 | 250 | 249 | 350 | 350 | 3 | 170 | 125 |
| COP ratio | % (relative to R410A) | 96.0 | 101.8 | 96.4 | 100.5 | 97.2 | 100.3 | 99.4 | 101.2 | 100.6 |
| Refrigerating capacity ratio | % (relative to R410A) | 116.2 | 74.6 | 119.9 | 88.9 | 121.5 | 98.7 | 80.0 | 80.0 | 80.8 |
| Condensation glide | °C | 3.7 | 12.3 | 2.9 | 8.2 | 2.6 | 5.8 | 12.1 | 10.8 | 11.5 |

| Item | Unit | Comp. Ex. 89 | Example 74 | Example 75 | Example 76 | Example 77 | Example 78 | Example 79 | Comp. Ex. 90 | Example 80 |
|---|---|---|---|---|---|---|---|---|---|---|
| | | I | | J | | K | | L | M | |
| HFO-1132(E) | mass% | 72.0 | 57.2 | 48.5 | 41.2 | 35.6 | 32.0 | 28.9 | 60.7 | 50.3 |
| R32 | mass% | 0.0 | 10.0 | 18.3 | 27.6 | 36.8 | 44.2 | 51.7 | 0.0 | 5.0 |
| R1234yf | mass% | 22.5 | 27.3 | 27.7 | 25.7 | 22.1 | 18.3 | 13.9 | 33.8 | 39.2 |
| CO₂ | mass% | 5.5 | 5.5 | 5.5 | 5.5 | 5.5 | 5.5 | 5.5 | 5.5 | 5.5 |
| GWP | - | 2 | 69 | 125 | 188 | 250 | 299 | 350 | 2 | 36 |
| COP ratio | % (relative to R410A) | 96.8 | 96.8 | 96.9 | 97.1 | 97.4 | 97.7 | 98.0 | 97.2 | 97.4 |
| Refrigerating capacity ratio | % (relative to R410A) | 100.9 | 101.8 | 103.8 | 106.6 | 109.8 | 112.4 | 115.0 | 95.4 | 94.3 |
| Condensation glide | °C | 6.9 | 6.7 | 6.2 | 5.4 | 4.7 | 4.1 | 3.7 | 8.1 | 8.5 |

| Item | Unit | Example 81 | Example 82 | Example 83 | Example 84 | Example 85 | Example 86 | Example 87 |
|---|---|---|---|---|---|---|---|---|
| | | W | | N | | O | | P |
| HFO-1132(E) | mass% | 43.3 | 39.0 | 36.3 | 31.6 | 28.4 | 26.2 | 24.2 |
| R32 | mass% | 10.0 | 14.4 | 18.2 | 27.6 | 36.8 | 44.0 | 51.7 |
| R1234yf | mass% | 41.2 | 41.1 | 40.0 | 35.3 | 29.3 | 24.3 | 18.6 |
| CO₂ | mass% | 5.5 | 5.5 | 5.5 | 5.5 | 5.5 | 5.5 | 5.5 |
| GWP | - | 70 | 99 | 125 | 188 | 250 | 298 | 350 |
| COP ratio | % (relative to R410A) | 97.5 | 97.6 | 97.6 | 97.7 | 97.9 | 98.1 | 98.3 |
| Refrigerating capacity ratio | % (relative to R410A) | 94.7 | 95.9 | 97.4 | 101.6 | 106.1 | 109.3 | 112.6 |
| Condensation glide | °C | 8 | 8.1 | 7.6 | 6.5 | 5.4 | 4.7 | 4.0 |

Table 37

7% $CO_2$

| Item | Unit | Comp. Ex. 91 | Comp. Ex. 92 | Comp. Ex. 93 | Comp. Ex. 94 | Comp. Ex. 95 | Comp. Ex. 96 | Comp. Ex. 97 | Comp. Ex. 98 | Example 88 |
|---|---|---|---|---|---|---|---|---|---|---|
| | | A | B | A′ | B′ | A″ | B″ | C | D | E |
| HFO-1132(E) | mass% | 74.6 | 0.0 | 56.1 | 0.0 | 41.2 | 0.0 | 26.8 | 0.0 | 3.1 |
| R32 | mass% | 18.4 | 18.1 | 36.9 | 36.6 | 51.8 | 51.6 | 0.0 | 20.5 | 18.1 |
| R1234yf | mass% | 0.0 | 74.9 | 0.0 | 56.4 | 0.0 | 41.4 | 66.2 | 72.5 | 71.8 |
| $CO_2$ | mass% | 7.0 | 7.0 | 7.0 | 7.0 | 7.0 | 7.0 | 7.0 | 7.0 | 7.0 |
| GWP | - | 125 | 125 | 250 | 249 | 350 | 350 | 3 | 141 | 125 |
| COP ratio | % (relative to R410A) | 95.3 | 101.3 | 95.8 | 100.0 | 96.7 | 99.8 | 99.5 | 101.1 | 100.9 |
| Refrigerating capacity ratio | % (relative to R410A) | 119.0 | 78.0 | 122.6 | 92.2 | 124.0 | 101.9 | 80.0 | 80.0 | 80.3 |
| Condensation glide | °C | 4.4 | 13.6 | 3.4 | 9.0 | 3.1 | 6.5 | 14.6 | 13.0 | 13.3 |

| Item | Unit | Comp. Ex. 99 | Example 89 | Example 90 | Example 91 | Example 92 | Example 93 | Example 94 | Comp. Ex.100 | Example 95 |
|---|---|---|---|---|---|---|---|---|---|---|
| | | I | | J | | K | | L | M | |
| HFO-1132(E) | mass% | 72.0 | 57.2 | 48.5 | 41.2 | 35.6 | 32.0 | 28.9 | 60.7 | 50.3 |
| R32 | mass% | 0.0 | 10.0 | 18.3 | 27.6 | 36.8 | 44.2 | 51.7 | 0.0 | 5.0 |
| R1234yf | mass% | 21.0 | 25.8 | 26.2 | 24.2 | 20.6 | 16.8 | 12.4 | 32.3 | 37.7 |
| $CO_2$ | mass% | 7.0 | 7.0 | 7.0 | 7.0 | 7.0 | 7.0 | 7.0 | 7.0 | 7.0 |
| GWP | - | 2 | 69 | 125 | 188 | 250 | 299 | 350 | 2 | 36 |
| COP ratio | % (relative to R410A) | 96.0 | 96.1 | 96.2 | 96.5 | 96.8 | 97.1 | 97.5 | 96.5 | 96.7 |
| Refrigerating capacity ratio | % (relative to R410A) | 104.7 | 105.5 | 107.3 | 110.0 | 113.1 | 115.6 | 118.2 | 99.2 | 98.0 |
| Condensation glide | °C | 7.9 | 7.5 | 6.9 | 6.0 | 5.3 | 4.7 | 4.2 | 9.2 | 9.4 |

| Item | Unit | Example 96 | Example 97 | Example 98 | Example 99 | Example 100 | Example 101 | Example 102 |
|---|---|---|---|---|---|---|---|---|
| | | W | | N | | O | | P |
| HFO-1132(E) | mass% | 43.7 | 39.5 | 36.7 | 31.9 | 28.6 | 26.4 | 24.2 |
| R32 | mass% | 10.0 | 14.4 | 18.2 | 27.6 | 36.8 | 44.0 | 51.7 |
| R1234yf | mass% | 39.3 | 39.1 | 38.1 | 33.5 | 27.6 | 22.6 | 17.1 |
| $CO_2$ | mass% | 7.0 | 7.0 | 7.0 | 7.0 | 7.0 | 7.0 | 7.0 |
| GWP | - | 70 | 99 | 125 | 188 | 250 | 298 | 350 |
| COP ratio | % (relative to R410A) | 96.9 | 96.9 | 97.0 | 97.1 | 97.3 | 97.5 | 97.8 |
| Refrigerating capacity ratio | % (relative to R410A) | 98.6 | 99.7 | 101.1 | 105.2 | 109.5 | 112.7 | 115.8 |
| Condensation glide | °C | 9 | 8.8 | 8.4 | 7.1 | 6.0 | 5.2 | 4.6 |

Table 38

| Item | Unit | Comp. Ex. 101 | Comp. Ex. 102 | Comp. Ex. 103 | Example 103 | Example 104 | Comp. Ex. 104 | Comp. Ex. 105 | Comp. Ex. 106 |
|---|---|---|---|---|---|---|---|---|---|
| HFO-1132(E) | mass% | 10.0 | 10.0 | 10.0 | 10.0 | 10.0 | 10.0 | 10.0 | 10.0 |
| R32 | mass% | 78.8 | 68.8 | 58.8 | 48.8 | 38.8 | 28.8 | 18.8 | 8.8 |
| R1234yf | mass% | 10.0 | 20.0 | 30.0 | 40.0 | 50.0 | 60.0 | 70.0 | 80.0 |
| $CO_2$ | mass% | 1.2 | 1.2 | 1.2 | 1.2 | 1.2 | 1.2 | 1.2 | 1.2 |
| GWP | - | 532 | 465 | 398 | 331 | 264 | 197 | 130 | 63 |
| COP ratio | % (relative to R410A) | 101.3 | 101.2 | 101.1 | 101.0 | 101.0 | 101.3 | 102.0 | 102.8 |
| Refrigerating capacity ratio | % (relative to R410A) | 108.5 | 104.1 | 99.2 | 93.6 | 87.2 | 80.1 | 72.2 | 63.1 |
| Condensation glide | °C | 1.1 | 1.6 | 2.2 | 3.1 | 4.3 | 5.8 | 7.4 | 8.4 |

| Item | Unit | Comp. Ex. 107 | Comp. Ex. 108 | Example 105 | Example 106 | Example 107 | Comp. Ex. 109 | Comp. Ex. 110 | Comp. Ex. 111 |
|---|---|---|---|---|---|---|---|---|---|
| HFO-1132(E) | mass% | 20.0 | 20.0 | 20.0 | 20.0 | 20.0 | 20.0 | 20.0 | 30.0 |
| R32 | mass% | 68.8 | 58.8 | 48.8 | 38.8 | 28.8 | 18.8 | 8.8 | 58.8 |
| R1234yf | mass% | 10.0 | 20.0 | 30.0 | 40.0 | 50.0 | 60.0 | 70.0 | 10.0 |
| $CO_2$ | mass% | 1.2 | 1.2 | 1.2 | 1.2 | 1.2 | 1.2 | 1.2 | 1.2 |
| GWP | - | 465 | 398 | 331 | 264 | 197 | 130 | 62 | 398 |
| COP ratio | % (relative to R410A) | 100.6 | 100.5 | 100.4 | 100.3 | 100.4 | 100.9 | 101.8 | 100.0 |
| Refrigerating capacity ratio | % (relative to R410A) | 108.6 | 103.9 | 98.6 | 92.6 | 85.8 | 78.2 | 69.6 | 108.3 |
| Condensation glide | °C | 1.1 | 1.7 | 2.5 | 3.5 | 4.8 | 6.4 | 7.7 | 1.2 |

| Item | Unit | Example 108 | Example 109 | Example 110 | Example 111 | Comp. Ex. 112 | Comp. Ex. 113 | Comp. Ex. 114 | Example 112 |
|---|---|---|---|---|---|---|---|---|---|
| HFO-1132(E) | mass% | 30.0 | 30.0 | 30.0 | 30.0 | 30.0 | 40.0 | 40.0 | 40.0 |
| R32 | mass% | 48.8 | 38.8 | 28.8 | 18.8 | 8.8 | 48.8 | 38.8 | 28.8 |
| R1234yf | mass% | 20.0 | 30.0 | 40.0 | 50.0 | 60.0 | 10.0 | 20.0 | 30.0 |
| $CO_2$ | mass% | 1.2 | 1.2 | 1.2 | 1.2 | 1.2 | 1.2 | 1.2 | 1.2 |
| GWP | – | 331 | 263 | 196 | 129 | 62 | 330 | 263 | 196 |
| COP ratio | % (relative to R410A) | 99.9 | 99.8 | 99.8 | 100.1 | 100.8 | 99.4 | 99.3 | 99.3 |
| Refrigerating capacity ratio | % (relative to R410A) | 103.2 | 97.5 | 91.0 | 83.7 | 75.6 | 107.5 | 102.0 | 95.8 |
| Condensation glide | °C | 1.8 | 2.7 | 3.8 | 5.2 | 6.6 | 1.3 | 2.0 | 2.9 |

| Item | Unit | Example 113 | Example 114 | Comp. Ex. 115 | Comp. Ex. 116 | Comp. Ex. 117 | Example 115 | Comp. Ex. 118 | Comp. Ex. 119 |
|---|---|---|---|---|---|---|---|---|---|
| HFO-1132(E) | mass% | 40.0 | 40.0 | 50.0 | 50.0 | 50.0 | 50.0 | 60.0 | 60.0 |
| R32 | mass% | 18.8 | 8.8 | 38.8 | 28.8 | 18.8 | 8.8 | 28.8 | 18.8 |
| R1234yf | mass% | 40.0 | 50.0 | 10.0 | 20.0 | 30.0 | 40.0 | 10.0 | 20.0 |
| $CO_2$ | mass% | 1.2 | 1.2 | 1.2 | 1.2 | 1.2 | 1.2 | 1.2 | 1.2 |
| GWP | – | 129 | 62 | 263 | 196 | 129 | 62 | 195 | 128 |
| COP ratio | % (relative to R410A) | 99.5 | 100.0 | 99.0 | 98.9 | 99.0 | 99.4 | 98.7 | 98.7 |
| Refrigerating capacity ratio | % (relative to R410A) | 88.9 | 81.1 | 106.2 | 100.3 | 93.7 | 86.2 | 104.5 | 98.2 |
| Condensation glide | °C | 4.1 | 5.4 | 1.4 | 2.2 | 3.2 | 4.3 | 1.5 | 2.4 |

| Item | Unit | Comp. Ex. 120 | Comp. Ex. 121 | Comp. Ex. 122 | Comp. Ex. 123 | Example 116 | Example 117 | Example 118 | Example 119 |
|---|---|---|---|---|---|---|---|---|---|
| HFO-1132(E) | mass% | 60.0 | 70.0 | 70.0 | 80.0 | 15.0 | 15.0 | 15.0 | 15.0 |
| R32 | mass% | 8.8 | 18.8 | 8.8 | 8.8 | 48.8 | 46.3 | 43.8 | 41.3 |
| R1234yf | mass% | 30.0 | 10.0 | 20.0 | 10.0 | 35.0 | 37.5 | 40.0 | 42.5 |
| $CO_2$ | mass% | 1.2 | 1.2 | 1.2 | 1.2 | 1.2 | 1.2 | 1.2 | 1.2 |
| GWP | – | 61 | 128 | 61 | 61 | 331 | 314 | 297 | 281 |
| COP ratio | % (relative to R410A) | 99.0 | 98.5 | 98.8 | 98.6 | 100.7 | 100.7 | 100.6 | 100.6 |
| Refrigerating capacity ratio | % (relative to R410A) | 91.0 | 102.4 | 95.5 | 99.7 | 96.1 | 94.7 | 93.1 | 91.6 |
| Condensation glide | °C | 3.3 | 1.7 | 2.5 | 1.9 | 2.8 | 3.0 | 3.3 | 3.6 |

| Item | Unit | Example 120 | Example 121 | Example 122 | Example 123 | Example 124 | Example 125 | Example 126 | Example 127 |
|---|---|---|---|---|---|---|---|---|---|
| HFO-1132(E) | mass% | 15.0 | 15.0 | 15.0 | 15.0 | 15.0 | 17.5 | 17.5 | 17.5 |
| R32 | mass% | 38.8 | 36.3 | 33.8 | 31.3 | 28.8 | 48.8 | 46.3 | 43.8 |
| R1234yf | mass% | 45.0 | 47.5 | 50.0 | 52.5 | 55.0 | 32.5 | 35.0 | 37.5 |
| $CO_2$ | mass% | 1.2 | 1.2 | 1.2 | 1.2 | 1.2 | 1.2 | 1.2 | 1.2 |
| GWP | – | 264 | 247 | 230 | 214 | 197 | 331 | 314 | 297 |
| COP ratio | % (relative to R410A) | 100.6 | 100.7 | 100.7 | 100.7 | 100.8 | 100.5 | 100.5 | 100.5 |
| Refrigerating capacity ratio | % (relative to R410A) | 89.9 | 88.3 | 86.6 | 84.8 | 83.0 | 97.4 | 95.9 | 94.4 |
| Condensation glide | °C | 3.9 | 4.2 | 4.6 | 4.9 | 5.3 | 2.6 | 2.9 | 3.1 |

Table 39

| Item | Unit | Example 128 | Example 129 | Example 130 | Example 131 | Example 132 | Example 133 | Example 134 | Example 135 |
|---|---|---|---|---|---|---|---|---|---|
| HFO-1132(E) | mass% | 17.5 | 17.5 | 17.5 | 17.5 | 17.5 | 17.5 | 17.5 | 20.0 |
| R32 | mass% | 41.3 | 38.8 | 36.3 | 33.8 | 31.3 | 28.8 | 26.3 | 46.3 |
| R1234yf | mass% | 40.0 | 42.5 | 45.0 | 47.5 | 50.0 | 52.5 | 55.0 | 32.5 |
| $CO_2$ | mass% | 1.2 | 1.2 | 1.2 | 1.2 | 1.2 | 1.2 | 1.2 | 1.2 |
| GWP | – | 281 | 264 | 247 | 230 | 213 | 197 | 180 | 314 |
| COP ratio | % (relative to R410A) | 100.5 | 100.5 | 100.5 | 100.5 | 100.6 | 100.6 | 100.7 | 100.4 |
| Refrigerating capacity ratio | % (relative to R410A) | 92.9 | 91.3 | 89.6 | 87.9 | 86.2 | 84.4 | 82.6 | 97.1 |
| Condensation glide | °C | 3.4 | 3.7 | 4.0 | 4.3 | 4.7 | 5.1 | 5.4 | 2.7 |

| Item | Unit | Example 136 | Example 137 | Example 138 | Example 139 | Example 140 | Example 141 | Example 142 | Example 143 |
|---|---|---|---|---|---|---|---|---|---|
| HFO-1132(E) | mass% | 20.0 | 20.0 | 20.0 | 20.0 | 20.0 | 20.0 | 22.5 | 22.5 |
| R32 | mass% | 43.8 | 41.3 | 36.3 | 33.8 | 31.3 | 26.3 | 46.3 | 43.8 |
| R1234yf | mass% | 35.0 | 37.5 | 42.5 | 45.0 | 47.5 | 52.5 | 30.0 | 32.5 |
| $CO_2$ | mass% | 1.2 | 1.2 | 1.2 | 1.2 | 1.2 | 1.2 | 1.2 | 1.2 |
| GWP | – | 297 | 280 | 247 | 230 | 213 | 180 | 314 | 297 |
| COP ratio | % (relative to R410A) | 100.3 | 100.3 | 100.3 | 100.3 | 100.4 | 100.5 | 100.2 | 100.2 |
| Refrigerating capacity ratio | % (relative to R410A) | 95.7 | 94.1 | 90.9 | 89.3 | 87.5 | 84.0 | 98.4 | 96.9 |
| Condensation glide | °C | 2.9 | 3.2 | 3.8 | 4.1 | 4.4 | 5.2 | 2.5 | 2.7 |

| Item | Unit | Example 144 | Example 145 | Example 146 | Example 147 | Example 148 | Example 149 | Example 150 | Example 151 |
|---|---|---|---|---|---|---|---|---|---|
| HFO-1132(E) | mass% | 22.5 | 22.5 | 22.5 | 22.5 | 22.5 | 22.5 | 22.5 | 22.5 |
| R32 | mass% | 41.3 | 38.8 | 36.3 | 33.8 | 31.3 | 28.8 | 26.3 | 23.8 |
| R1234yf | mass% | 35.0 | 37.5 | 40.0 | 42.5 | 45.0 | 47.5 | 50.0 | 52.5 |
| $CO_2$ | mass% | 1.2 | 1.2 | 1.2 | 1.2 | 1.2 | 1.2 | 1.2 | 1.2 |
| GWP | – | 280 | 264 | 247 | 230 | 213 | 197 | 180 | 163 |
| COP ratio | % (relative to R410A) | 100.2 | 100.2 | 100.2 | 100.2 | 100.2 | 100.3 | 100.3 | 100.4 |
| Refrigerating capacity ratio | % (relative to R410A) | 95.4 | 93.8 | 92.2 | 90.6 | 88.9 | 87.1 | 85.3 | 83.5 |
| Condensation glide | °C | 3.0 | 3.3 | 3.6 | 3.9 | 4.2 | 4.5 | 4.9 | 5.3 |

| Item | Unit | Example 152 | Example 153 | Example 154 | Example 155 | Example 156 | Example 157 | Example 158 | Example 159 |
|---|---|---|---|---|---|---|---|---|---|
| HFO-1132(E) | mass% | 25.0 | 25.0 | 25.0 | 25.0 | 25.0 | 25.0 | 27.5 | 27.5 |
| R32 | mass% | 33.8 | 31.3 | 28.8 | 26.3 | 23.8 | 21.3 | 21.9 | 21.9 |
| R1234yf | mass% | 40.0 | 42.5 | 45.0 | 47.5 | 50.0 | 52.5 | 45.0 | 47.5 |
| $CO_2$ | mass% | 1.2 | 1.2 | 1.2 | 1.2 | 1.2 | 1.2 | 1.2 | 1.2 |
| GWP | – | 230 | 213 | 196 | 180 | 163 | 146 | 150 | 150 |
| COP ratio | % (relative to 410A) | 100.0 | 100.0 | 100.1 | 100.1 | 100.2 | 100.3 | 100.0 | 100.1 |
| Refrigerating capacity ratio | % (relative to 410A) | 91.8 | 90.2 | 88.4 | 86.7 | 84.8 | 83.0 | 86.3 | 85.4 |
| Condensation glide | °C | 3.6 | 4.0 | 4.3 | 4.7 | 5.0 | 5.4 | 4.8 | 4.9 |

| Item | Unit | Example 160 | Example 161 | Example 162 | Example 163 | Example 164 |
|---|---|---|---|---|---|---|
| HFO-1132(E) | mass% | 27.5 | 27.5 | 30.0 | 32.0 | 34.0 |
| R32 | mass% | 21.9 | 21.9 | 21.9 | 21.9 | 13.8 |
| R1234yf | mass% | 50.0 | 52.5 | 52.5 | 51.0 | 51.0 |
| $CO_2$ | mass% | 1.2 | 1.2 | 1.2 | 1.2 | 1.2 |
| GWP | – | 150 | 150 | 150 | 150 | 96 |
| COP ratio | % (relative to R410A) | 100.1 | 100.2 | 100.1 | 100.0 | 100.1 |
| Refrigerating capacity ratio | % (relative to R410A) | 84.5 | 83.7 | 84.2 | 85.1 | 82.0 |
| Condensation glide | °C | 5.1 | 5.2 | 5.0 | 4.9 | 5.5 |

Table 40

| Item | Unit | Comp. Ex. 125 | Comp. Ex. 126 | Comp. Ex. 127 | Example 166 | Example 167 | Example 168 | Comp. Ex. 128 | Comp. Ex. 129 |
|---|---|---|---|---|---|---|---|---|---|
| HFO-1132(E) | mass% | 10.0 | 10.0 | 10.0 | 10.0 | 10.0 | 10.0 | 10.0 | 10.0 |
| R32 | mass% | 77.5 | 67.5 | 57.5 | 47.5 | 37.5 | 27.5 | 17.5 | 7.5 |
| R1234yf | mass% | 10.0 | 20.0 | 30.0 | 40.0 | 50.0 | 60.0 | 70.0 | 80.0 |
| $CO_2$ | mass% | 2.5 | 2.5 | 2.5 | 2.5 | 2.5 | 2.5 | 2.5 | 2.5 |
| GWP | - | 524 | 457 | 389 | 322 | 255 | 188 | 121 | 54 |
| COP ratio | % (relative to R410A) | 100.9 | 100.8 | 100.6 | 100.5 | 100.5 | 100.9 | 101.6 | 102.4 |
| Refrigerating capacity ratio | % (relative to R410A) | 110.6 | 106.2 | 101.2 | 95.5 | 89.1 | 81.9 | 74.0 | 64.8 |
| Condensation glide | °C | 1.8 | 2.3 | 3.0 | 4.0 | 5.3 | 7.0 | 8.8 | 10.1 |

| Item | Unit | Comp. Ex. 130 | Comp. Ex. 131 | Example 169 | Example 170 | Example 171 | Comp. Ex. 132 | Comp. Ex. 133 | Comp. Ex. 134 |
|---|---|---|---|---|---|---|---|---|---|
| HFO-1132(E) | mass% | 20.0 | 20.0 | 20.0 | 20.0 | 20.0 | 20.0 | 20.0 | 30.0 |
| R32 | mass% | 67.5 | 57.5 | 47.5 | 37.5 | 27.5 | 17.5 | 7.5 | 57.5 |
| R1234yf | mass% | 10.0 | 20.0 | 30.0 | 40.0 | 50.0 | 60.0 | 70.0 | 10.0 |
| $CO_2$ | mass% | 2.5 | 2.5 | 2.5 | 2.5 | 2.5 | 2.5 | 2.5 | 2.5 |
| GWP | - | 456 | 389 | 322 | 255 | 188 | 121 | 54 | 389 |
| COP ratio | % (relative to R410A) | 100.1 | 100.0 | 99.9 | 99.8 | 100.0 | 100.5 | 101.3 | 99.5 |
| Refrigerating capacity ratio | % (relative to R410A) | 110.7 | 106.0 | 100.6 | 94.5 | 87.7 | 80.1 | 71.5 | 110.4 |
| Condensation glide | °C | 1.8 | 2.5 | 3.3 | 4.4 | 5.9 | 7.7 | 9.3 | 1.9 |

| Item | Unit | Example 172 | Example 173 | Example 174 | Example 175 | Comp. Ex. 135 | Comp. Ex. 136 | Comp. Ex. 137 | Example 176 |
|---|---|---|---|---|---|---|---|---|---|
| HFO-1132(E) | mass% | 30.0 | 30.0 | 30.0 | 30.0 | 30.0 | 40.0 | 40.0 | 40.0 |
| R32 | mass% | 47.5 | 37.5 | 27.5 | 17.5 | 7.5 | 47.5 | 37.5 | 27.5 |
| R1234yf | mass% | 20.0 | 30.0 | 40.0 | 50.0 | 60.0 | 10.0 | 20.0 | 30.0 |
| $CO_2$ | mass% | 2.5 | 2.5 | 2.5 | 2.5 | 2.5 | 2.5 | 2.5 | 2.5 |
| GWP | - | 322 | 255 | 188 | 120 | 53 | 321 | 254 | 187 |
| COP ratio | % (relative to R410A) | 99.3 | 99.2 | 99.3 | 99.6 | 100.3 | 98.9 | 98.8 | 98.7 |
| Refrigerating capacity ratio | % (relative to R410A) | 105.3 | 99.5 | 93.0 | 85.7 | 77.5 | 109.6 | 104.1 | 97.9 |
| Condensation glide | °C | 2.6 | 3.6 | 4.8 | 6.4 | 8.1 | 2.0 | 2.8 | 3.9 |

| Item | Unit | Example 177 | Example 178 | Comp. Ex. 138 | Comp. Ex.139 | Comp. Ex. 140 | Example 179 | Comp. Ex. 141 | Comp. Ex. 142 |
|---|---|---|---|---|---|---|---|---|---|
| HFO-1132(E) | mass% | 40.0 | 40.0 | 50.0 | 50.0 | 50.0 | 50.0 | 60.0 | 60.0 |
| R32 | mass% | 17.5 | 7.5 | 37.5 | 27.5 | 17.5 | 7.5 | 27.5 | 17.5 |
| R1234yf | mass% | 40.0 | 50.0 | 10.0 | 20.0 | 30.0 | 40.0 | 10.0 | 20.0 |
| $CO_2$ | mass% | 2.5 | 2.5 | 2.5 | 2.5 | 2.5 | 2.5 | 2.5 | 2.5 |
| GWP | - | 120 | 53 | 254 | 187 | 120 | 53 | 187 | 120 |
| COP ratio | % (relative to R410A) | 98.9 | 99.4 | 98.4 | 98.3 | 98.4 | 98.8 | 98.0 | 98.1 |
| Refrigerating capacity ratio | % (relative to R410A) | 91.0 | 83.1 | 108.4 | 102.5 | 95.9 | 88.4 | 106.8 | 100.4 |
| Condensation glide | °C | 5.3 | 6.8 | 2.2 | 3.1 | 4.3 | 5.6 | 2.4 | 3.4 |

| Item | Unit | Example 180 | Comp. Ex. 143 | Comp. Ex. 144 | Comp. Ex. 145 | Example 181 | Example 182 | Example 183 | Example 184 |
|---|---|---|---|---|---|---|---|---|---|
| HFO-1132 (E) | mass% | 60.0 | 70.0 | 70.0 | 80.0 | 15.0 | 15.0 | 15.0 | 15.0 |
| R32 | mass% | 7.5 | 17.5 | 7.5 | 7.5 | 50.0 | 47.5 | 45.0 | 42.5 |
| R1234yf | mass% | 30.0 | 10.0 | 20.0 | 10.0 | 32.5 | 35.0 | 37.5 | 40.0 |
| $CO_2$ | mass% | 2.5 | 2.5 | 2.5 | 2.5 | 2.5 | 2.5 | 2.5 | 2.5 |
| GWP | - | 52 | 119 | 52 | 52 | 339 | 322 | 305 | 289 |
| COP ratio | % (relative to R410A) | 98.4 | 97.9 | 98.1 | 98.0 | 100.2 | 100.2 | 100.2 | 100.2 |
| Refrigerating capacity ratio | % (relative to R410A) | 93.3 | 104.7 | 97.8 | 102.1 | 99.6 | 98.1 | 96.6 | 95.1 |
| Condensation glide | °C | 4.6 | 2.7 | 3.8 | 3.0 | 3.4 | 3.6 | 3.9 | 4.2 |

| Item | Unit | Example 185 | Example 186 | Example 187 | Example 188 | Example 189 | Example 190 | Example 191 | Example 192 |
|---|---|---|---|---|---|---|---|---|---|
| HFO-1132(E) | mass% | 15.0 | 15.0 | 15.0 | 15.0 | 15.0 | 15.0 | 15.0 | 17.5 |
| R32 | mass% | 40.0 | 37.5 | 35.0 | 32.5 | 30.0 | 27.5 | 25.0 | 50.0 |
| R1234yf | mass% | 42.5 | 45.0 | 47.5 | 50.0 | 52.5 | 55.0 | 57.5 | 30.0 |
| $CO_2$ | mass% | 2.5 | 2.5 | 2.5 | 2.5 | 2.5 | 2.5 | 2.5 | 2.5 |
| GWP | - | 272 | 255 | 238 | 222 | 205 | 188 | 171 | 339 |
| COP ratio | % (relative to R410A) | 100.2 | 100.2 | 100.2 | 100.2 | 100.3 | 100.4 | 100.5 | 100.1 |
| Refrigerating capacity ratio | % (relative to R410A) | 93.5 | 91.9 | 90.2 | 88.5 | 86.7 | 84.9 | 83.0 | 100.8 |
| Condensation glide | °C | 4.5 | 4.8 | 5.2 | 5.6 | 6.0 | 6.4 | 6.9 | 3.2 |

Table 41

| Item | Unit | Example 193 | Example 194 | Example 195 | Example 196 | Example 197 | Example 198 | Example 199 | Example 200 |
|---|---|---|---|---|---|---|---|---|---|
| HFO-1132(E) | mass% | 17.5 | 17.5 | 17.5 | 17.5 | 17.5 | 17.5 | 17.5 | 17.5 |
| R32 | mass% | 47.5 | 45.0 | 42.5 | 40.0 | 37.5 | 35.0 | 32.5 | 30.0 |
| R1234yf | mass% | 32.5 | 35.0 | 37.5 | 40.0 | 42.5 | 45.0 | 47.5 | 50.0 |
| $CO_2$ | mass% | 2.5 | 2.5 | 2.5 | 2.5 | 2.5 | 2.5 | 2.5 | 2.5 |
| GWP | – | 322 | 305 | 289 | 272 | 255 | 238 | 221 | 205 |
| COP ratio | % (relative to R410A) | 100.0 | 100.0 | 100.0 | 100.0 | 100.0 | 100.0 | 100.0 | 100.1 |
| Refrigerating capacity ratio | % (relative to R410A) | 99.4 | 97.9 | 96.4 | 94.8 | 93.2 | 91.5 | 89.8 | 88.1 |
| Condensation glide | °C | 3.5 | 3.7 | 4.0 | 4.3 | 4.6 | 5.0 | 5.3 | 5.7 |

| Item | Unit | Example 201 | Example 202 | Example 203 | Example 204 | Example 205 | Example 206 | Example 207 | Example 208 |
|---|---|---|---|---|---|---|---|---|---|
| HFO-1132(E) | mass% | 17.5 | 17.5 | 17.5 | 20.0 | 20.0 | 20.0 | 20.0 | 20.0 |
| R32 | mass% | 27.5 | 25.0 | 22.5 | 50.0 | 45.0 | 42.5 | 40.0 | 35.0 |
| R1234yf | mass% | 52.5 | 55.0 | 57.5 | 27.5 | 32.5 | 35.0 | 37.5 | 42.5 |
| $CO_2$ | mass% | 2.5 | 2.5 | 2.5 | 2.5 | 2.5 | 2.5 | 2.5 | 2.5 |
| GWP | – | 188 | 171 | 154 | 339 | 305 | 289 | 272 | 238 |
| COP ratio | % (relative to R410A) | 100.2 | 100.3 | 100.4 | 99.9 | 99.9 | 99.8 | 99.8 | 99.8 |
| Refrigerating capacity ratio | % (relative to R410A) | 86.3 | 84.4 | 82.6 | 102.0 | 99.2 | 97.7 | 96.1 | 92.9 |
| Condensation glide | °C | 6.2 | 6.6 | 7.0 | 3.1 | 3.5 | 3.8 | 4.1 | 4.7 |

| Item | Unit | Example 209 | Example 210 | Example 211 | Example 212 | Example 213 | Example 214 | Example 215 | Example 216 |
|---|---|---|---|---|---|---|---|---|---|
| HFO-1132(E) | mass% | 20.0 | 20.0 | 20.0 | 20.0 | 20.0 | 22.5 | 22.5 | 22.5 |
| R32 | mass% | 32.5 | 30.0 | 25.0 | 22.5 | 20.0 | 50.0 | 47.5 | 45.0 |
| R1234yf | mass% | 45.0 | 47.5 | 52.5 | 55.0 | 57.5 | 25.0 | 27.5 | 30.0 |
| $CO_2$ | mass% | 2.5 | 2.5 | 2.5 | 2.5 | 2.5 | 2.5 | 2.5 | 2.5 |
| GWP | – | 221 | 205 | 171 | 154 | 138 | 339 | 322 | 305 |
| COP ratio | % (relative to R410A) | 99.8 | 99.9 | 100.0 | 100.2 | 100.3 | 99.8 | 99.7 | 99.7 |
| Refrigerating capacity ratio | % (relative to R410A) | 91.2 | 89.5 | 85.9 | 84.0 | 82.1 | 103.2 | 101.8 | 100.4 |
| Condensation glide | °C | 5.1 | 5.5 | 6.3 | 6.7 | 7.2 | 2.9 | 3.1 | 3.4 |

| Item | Unit | Example 217 | Example 218 | Example 219 | Example 220 | Example 221 | Example 222 | Example 223 | Example 224 |
|---|---|---|---|---|---|---|---|---|---|
| HFO-1132(E) | mass% | 22.5 | 22.5 | 22.5 | 22.5 | 22.5 | 22.5 | 22.5 | 22.5 |
| R32 | mass% | 42.5 | 40.0 | 37.5 | 35.0 | 32.5 | 30.0 | 27.5 | 25.0 |
| R1234yf | mass% | 32.5 | 35.0 | 37.5 | 40.0 | 42.5 | 45.0 | 47.5 | 50.0 |
| $CO_2$ | mass% | 2.5 | 2.5 | 2.5 | 2.5 | 2.5 | 2.5 | 2.5 | 2.5 |
| GWP | - | 288 | 272 | 255 | 238 | 221 | 205 | 188 | 171 |
| COP ratio | % (relative to R410A) | 99.7 | 99.7 | 99.7 | 99.7 | 99.7 | 99.7 | 99.8 | 99.8 |
| Refrigerating capacity ratio | % (relative to R410A) | 98.9 | 97.4 | 95.8 | 94.2 | 92.5 | 90.8 | 89.0 | 87.2 |
| Condensation glide | °C | 3.6 | 3.9 | 4.2 | 4.5 | 4.9 | 5.2 | 5.6 | 6.0 |

| Item | Unit | Example 225 | Example 226 | Example 227 | Example 228 | Example 229 | Example 230 | Example 231 | Example 232 |
|---|---|---|---|---|---|---|---|---|---|
| HFO-1132(E) | mass% | 22.5 | 22.5 | 22.5 | 25.0 | 25.0 | 25.0 | 25.0 | 25.0 |
| R32 | mass% | 22.5 | 20.0 | 17.5 | 40.0 | 37.5 | 35.0 | 32.5 | 30.0 |
| R1234yf | mass% | 52.5 | 55.0 | 57.5 | 32.5 | 35.0 | 37.5 | 40.0 | 42.5 |
| $CO_2$ | mass% | 2.5 | 2.5 | 2.5 | 2.5 | 2.5 | 2.5 | 2.5 | 2.5 |
| GWP | - | 154 | 137 | 121 | 272 | 255 | 238 | 221 | 204 |
| COP ratio | % (relative to R410A) | 99.9 | 100.1 | 100.2 | 99.5 | 99.5 | 99.5 | 99.5 | 99.5 |
| Refrigerating capacity ratio | % (relative to R410A) | 85.4 | 83.5 | 81.5 | 98.6 | 97.1 | 95.5 | 93.8 | 92.1 |
| Condensation glide | °C | 6.5 | 6.9 | 7.3 | 3.7 | 4.0 | 4.3 | 4.6 | 5.0 |

| Item | Unit | Example 233 | Example 234 | Example 235 | Example 236 | Example 237 | Example 238 | Example 239 | Example 240 |
|---|---|---|---|---|---|---|---|---|---|
| HFO-1132(E) | mass% | 25.0 | 25.0 | 25.0 | 25.0 | 25.0 | 27.5 | 27.5 | 27.5 |
| R32 | mass% | 27.5 | 25.0 | 22.5 | 20.0 | 17.5 | 32.5 | 30.0 | 27.5 |
| R1234yf | mass% | 45.0 | 47.5 | 50.0 | 52.5 | 55.0 | 37.5 | 40.0 | 42.5 |
| $CO_2$ | mass% | 2.5 | 2.5 | 2.5 | 2.5 | 2.5 | 2.5 | 2.5 | 2.5 |
| GWP | - | 188 | 171 | 154 | 137 | 121 | 221 | 204 | 188 |
| COP ratio | % (relative to R410A) | 99.6 | 99.6 | 99.7 | 99.9 | 100.0 | 99.4 | 99.4 | 99.4 |
| Refrigerating capacity ratio | % (relative to R410A) | 90.4 | 88.6 | 86.8 | 84.9 | 83.0 | 95.1 | 93.4 | 91.7 |
| Condensation glide | °C | 5.4 | 5.7 | 6.2 | 6.6 | 7.0 | 4.4 | 4.7 | 5.1 |

Table 42

| Item | Unit | Example 241 | Example 242 | Example 243 | Example 244 | Example 245 | Example 246 | Example 247 | Example 248 |
|---|---|---|---|---|---|---|---|---|---|
| HFO-1132(E) | mass% | 27.5 | 27.5 | 27.5 | 27.5 | 27.5 | 30.0 | 30.0 | 30.0 |
| R32 | mass% | 25.0 | 22.5 | 20.0 | 17.5 | 15.0 | 25.0 | 22.5 | 20.0 |
| R1234yf | mass% | 45.0 | 47.5 | 50.0 | 52.5 | 55.0 | 42.5 | 45.0 | 47.5 |
| $CO_2$ | mass% | 2.5 | 2.5 | 2.5 | 2.5 | 2.5 | 2.5 | 2.5 | 2.5 |
| GWP | – | 171 | 154 | 137 | 121 | 104 | 171 | 154 | 137 |
| COP ratio | % (relative to R410A) | 99.5 | 99.5 | 99.6 | 99.8 | 99.9 | 99.3 | 99.4 | 99.5 |
| Refrigerating capacity ratio | % (relative to R410A) | 89.9 | 88.1 | 86.3 | 84.3 | 82.4 | 91.3 | 89.5 | 87.6 |
| Condensation glide | °C | 5.5 | 5.9 | 6.3 | 6.7 | 7.2 | 5.2 | 5.6 | 6.0 |

| Item | Unit | Example 249 | Example 250 | Example 251 | Example 252 | Example 253 | Example 254 | Example 255 | Example 256 |
|---|---|---|---|---|---|---|---|---|---|
| HFO-1132(E) | mass% | 30.0 | 30.0 | 32.5 | 32.5 | 32.5 | 32.5 | 35.0 | 35.0 |
| R32 | mass% | 15.0 | 12.5 | 20.0 | 17.5 | 15.0 | 12.5 | 15.0 | 12.5 |
| R1234yf | mass% | 52.5 | 55.0 | 45.0 | 47.5 | 50.0 | 52.5 | 47.5 | 50.0 |
| $CO_2$ | mass% | 2.5 | 2.5 | 2.5 | 2.5 | 2.5 | 2.5 | 2.5 | 2.5 |
| GWP | – | 104 | 87 | 137 | 120 | 104 | 87 | 104 | 87 |
| COP ratio | % (relative to R410A) | 99.7 | 99.9 | 99.3 | 99.4 | 99.5 | 99.7 | 99.3 | 99.5 |
| Refrigerating capacity ratio | % (relative to R410A) | 83.8 | 81.8 | 88.9 | 87.1 | 85.1 | 83.1 | 86.5 | 84.5 |
| Condensation glide | °C | 6.8 | 7.3 | 5.7 | 6.1 | 6.5 | 7.0 | 6.2 | 6.6 |

| Item | Unit | Example 257 | Example 258 | Example 259 | Example 260 | Example 261 | Example 262 | Example 263 | Example 264 |
|---|---|---|---|---|---|---|---|---|---|
| HFO-1132(E) | mass% | 35.0 | 37.5 | 37.5 | 37.5 | 40.0 | 40.0 | 42.5 | 42.5 |
| R32 | mass% | 10.0 | 12.5 | 10.0 | 7.5 | 10.0 | 5.0 | 7.5 | 5.0 |
| R1234yf | mass% | 52.5 | 47.5 | 50.0 | 52.5 | 47.5 | 52.5 | 47.5 | 50.0 |
| $CO_2$ | mass% | 2.5 | 2.5 | 2.5 | 2.5 | 2.5 | 2.5 | 2.5 | 2.5 |
| GWP | – | 70 | 87 | 70 | 53 | 70 | 36 | 53 | 36 |
| COP ratio | % (relative to R410A) | 99.6 | 99.3 | 99.4 | 99.6 | 99.3 | 99.6 | 99.3 | 99.4 |
| Refrigerating capacity ratio | % (relative to R410A) | 82.5 | 85.8 | 83.8 | 81.8 | 85.2 | 81.0 | 84.5 | 82.4 |
| Condensation glide | °C | 7.1 | 6.3 | 6.7 | 7.1 | 6.4 | 7.2 | 6.5 | 6.9 |

EP 3 988 522 A1

| Item | Unit | Example 265 | Example 266 | Example 267 | Example 268 | Example 269 | Example 270 | Example 271 |
|---|---|---|---|---|---|---|---|---|
| HFO-1132(E) | mass% | 45.0 | 45.0 | 47.5 | 47.5 | 50.0 | 52.5 | 55.0 |
| R32 | mass% | 5.0 | 2.5 | 4.0 | 1.5 | 2.5 | 1.5 | 1.0 |
| R1234yf | mass% | 47.5 | 50.0 | 46.0 | 48.5 | 45.0 | 43.5 | 41.5 |
| CO2 | mass% | 2.5 | 2.5 | 2.5 | 2.5 | 2.5 | 2.5 | 2.5 |
| GWP | - | 36 | 19 | 29 | 13 | 19 | 12 | 9 |
| COP ratio | % (relative to R410A) | 99.3 | 99.4 | 99.2 | 99.3 | 99.1 | 99.1 | 99.0 |
| Refrigerating capacity ratio | % (relative to R410A) | 83.7 | 81.6 | 84.2 | 82.0 | 84.2 | 84.7 | 85.6 |
| Condensation glide | °C | 6.6 | 6.9 | 6.4 | 6.7 | 6.3 | 6.2 | 5.9 |

Table 43

| Item | Unit | Comp. Ex. 146 | Comp. Ex. 147 | Comp. Ex. 148 | Example 272 | Example 273 | Example 274 | Comp. Ex. 149 | Comp. Ex. 150 |
|---|---|---|---|---|---|---|---|---|---|
| HFO-1132(E) | mass% | 10.0 | 10.0 | 10.0 | 10.0 | 10.0 | 10.0 | 10.0 | 10.0 |
| R32 | mass% | 76.0 | 66.0 | 56.0 | 46.0 | 36.0 | 26.0 | 16.0 | 6.0 |
| R1234yf | mass% | 10.0 | 20.0 | 30.0 | 40.0 | 50.0 | 60.0 | 70.0 | 80.0 |
| $CO_2$ | mass% | 4.0 | 4.0 | 4.0 | 4.0 | 4.0 | 4.0 | 4.0 | 4.0 |
| GWP | - | 514 | 446 | 379 | 312 | 245 | 178 | 111 | 44 |
| COP ratio | % (relative to R410A) | 100.3 | 100.2 | 100.1 | 100.0 | 100.0 | 100.4 | 101.2 | 102.0 |
| Refrigerating capacity ratio | % (relative to R410A) | 113.0 | 108.6 | 103.5 | 97.8 | 91.3 | 84.1 | 76.1 | 66.8 |
| Condensation glide | °C | 2.5 | 3.1 | 3.9 | 5.0 | 6.4 | 8.3 | 10.4 | 12.2 |

| Item | Unit | Comp. Ex. 146 | Comp. Ex. 147 | Example 275 | Example 276 | Example 277 | Example 278 | Comp. Ex. 153 | Comp. Ex. 154 |
|---|---|---|---|---|---|---|---|---|---|
| HFO-1132(E) | mass% | 20.0 | 20.0 | 20.0 | 20.0 | 20.0 | 20.0 | 20.0 | 30.0 |
| R32 | mass% | 66.0 | 56.0 | 46.0 | 36.0 | 26.0 | 16.0 | 6.0 | 56.0 |
| R1234yf | mass% | 10.0 | 20.0 | 30.0 | 40.0 | 50.0 | 60.0 | 70.0 | 10.0 |
| $CO_2$ | mass% | 4.0 | 4.0 | 4.0 | 4.0 | 4.0 | 4.0 | 4.0 | 4.0 |
| GWP | - | 446 | 379 | 312 | 245 | 178 | 111 | 44 | 379 |
| COP ratio | % (relative to R410A) | 99.6 | 99.5 | 99.3 | 99.2 | 99.4 | 100.0 | 100.9 | 98.9 |
| Refrigerating capacity ratio | % (relative to R410A) | 113.1 | 108.4 | 103.0 | 96.8 | 89.9 | 82.3 | 73.7 | 112.9 |
| Condensation glide | °C | 2.6 | 3.3 | 4.2 | 5.5 | 7.1 | 9.2 | 11.2 | 2.7 |

79

| Item | Unit | Example 279 | Example 280 | Example 281 | Example 282 | Comp. Ex.155 | Comp. Ex.156 | Comp. Ex.157 | Example 283 |
|---|---|---|---|---|---|---|---|---|---|
| HFO-1132(E) | mass% | 30.0 | 30.0 | 30.0 | 30.0 | 30.0 | 40.0 | 40.0 | 40.0 |
| R32 | mass% | 46.0 | 36.0 | 26.0 | 16.0 | 6.0 | 46.0 | 36.0 | 26.0 |
| R1234yf | mass% | 20.0 | 30.0 | 40.0 | 50.0 | 60.0 | 10.0 | 20.0 | 30.0 |
| $CO_2$ | mass% | 4.0 | 4.0 | 4.0 | 4.0 | 4.0 | 4.0 | 4.0 | 4.0 |
| GWP | - | 312 | 245 | 177 | 110 | 43 | 311 | 244 | 177 |
| COP ratio | % (relative to R410A) | 98.7 | 98.6 | 98.7 | 99.0 | 99.8 | 98.3 | 98.1 | 98.1 |
| Refrigerating capacity ratio | % (relative to R410A) | 107.7 | 101.9 | 95.4 | 88.0 | 79.9 | 112.1 | 106.6 | 100.4 |
| Condensation glide | °C | 3.5 | 4.6 | 6.0 | 7.8 | 9.8 | 2.8 | 3.8 | 5.0 |

| Item | Unit | Example 284 | Example 285 | Comp. Ex. 158 | Comp. Ex. 159 | Example 286 | Example 287 | Comp. Ex. 160 | Comp. Ex. 161 |
|---|---|---|---|---|---|---|---|---|---|
| HFO-1132(E) | mass% | 40.0 | 40.0 | 50.0 | 50.0 | 50.0 | 50.0 | 60.0 | 60.0 |
| R32 | mass% | 16.0 | 6.0 | 36.0 | 26.0 | 16.0 | 6.0 | 26.0 | 16.0 |
| R1234yf | mass% | 40.0 | 50.0 | 10.0 | 20.0 | 30.0 | 40.0 | 10.0 | 20.0 |
| $CO_2$ | mass% | 4.0 | 4.0 | 4.0 | 4.0 | 4.0 | 4.0 | 4.0 | 4.0 |
| GWP | - | 110 | 43 | 244 | 177 | 110 | 43 | 177 | 109 |
| COP ratio | % (relative to R410A) | 98.3 | 98.8 | 97.7 | 97.7 | 97.8 | 98.2 | 97.3 | 97.4 |
| Refrigerating capacity ratio | % (relative to R410A) | 93.4 | 85.6 | 110.9 | 105.0 | 98.4 | 90.9 | 109.3 | 103.0 |
| Condensation glide | °C | 6.6 | 8.4 | 3.1 | 4.1 | 5.5 | 7.1 | 3.4 | 4.6 |

| Item | Unit | Example 288 | Comp. Ex. 162 | Comp. Ex. 163 | Comp. Ex. 164 | Example 289 | Example 290 | Example 291 | Example 292 |
|---|---|---|---|---|---|---|---|---|---|
| HFO-1132(E) | mass% | 60.0 | 70.0 | 70.0 | 80.0 | 15.0 | 15.0 | 15.0 | 15.0 |
| R32 | mass% | 6.0 | 16.0 | 6.0 | 6.0 | 48.5 | 46.0 | 43.5 | 41.0 |
| R1234yf | mass% | 30.0 | 10.0 | 20.0 | 10.0 | 32.5 | 35.0 | 37.5 | 40.0 |
| $CO_2$ | mass% | 4.0 | 4.0 | 4.0 | 4.0 | 4.0 | 4.0 | 4.0 | 4.0 |
| GWP | - | 42 | 109 | 42 | 42 | 329 | 312 | 295 | 279 |
| COP ratio | % (relative to R410A) | 97.7 | 97.2 | 97.4 | 97.2 | 99.7 | 99.6 | 99.6 | 99.6 |
| Refrigerating capacity ratio | % (relative to R410A) | 95.9 | 107.3 | 100.5 | 104.9 | 101.9 | 100.4 | 98.9 | 97.4 |
| Condensation glide | °C | 6.0 | 3.8 | 5.1 | 4.3 | 4.3 | 4.6 | 4.9 | 5.2 |

| Item | Unit | Example 293 | Example 294 | Example 295 | Example 296 | Example 297 | Example 298 | Example 299 | Example 300 |
|---|---|---|---|---|---|---|---|---|---|
| HFO-1132(E) | mass% | 15.0 | 15.0 | 15.0 | 15.0 | 15.0 | 15.0 | 15.0 | 15.0 |
| R32 | mass% | 38.5 | 36.0 | 33.5 | 31.0 | 28.5 | 26.0 | 23.5 | 21.0 |
| R1234yf | mass% | 42.5 | 45.0 | 47.5 | 50.0 | 52.5 | 55.0 | 57.5 | 60.0 |
| $CO_2$ | mass% | 4.0 | 4.0 | 4.0 | 4.0 | 4.0 | 4.0 | 4.0 | 4.0 |
| GWP | - | 262 | 245 | 228 | 211 | 195 | 178 | 161 | 144 |
| COP ratio | % (relative to R410A) | 99.6 | 99.6 | 99.6 | 99.7 | 99.8 | 99.9 | 100.0 | 100.2 |
| Refrigerating capacity ratio | % (relative to R410A) | 95.8 | 94.1 | 92.4 | 90.7 | 88.9 | 87.1 | 85.2 | 83.3 |
| Condensation glide | °C | 5.6 | 5.9 | 6.3 | 6.8 | 7.2 | 7.7 | 8.2 | 8.7 |

Table 44

| Item | Unit | Example 301 | Example 302 | Example 303 | Example 304 | Example 305 | Example 306 | Example 307 | Example 308 |
|---|---|---|---|---|---|---|---|---|---|
| HFO-1132(E) | mass% | 15.0 | 17.5 | 17.5 | 17.5 | 17.5 | 17.5 | 17.5 | 17.5 |
| R32 | mass% | 18.5 | 48.5 | 46.0 | 43.5 | 41.0 | 38.5 | 36.0 | 33.5 |
| R1234yf | mass% | 62.5 | 30.0 | 32.5 | 35.0 | 37.5 | 40.0 | 42.5 | 45.0 |
| $CO_2$ | mass% | 4.0 | 4.0 | 4.0 | 4.0 | 4.0 | 4.0 | 4.0 | 4.0 |
| GWP | - | 128 | 329 | 312 | 295 | 278 | 262 | 245 | 228 |
| COP ratio | % (relative to R410A) | 100.4 | 99.5 | 99.5 | 99.4 | 99.4 | 99.4 | 99.4 | 99.4 |
| Refrigerating capacity ratio | % (relative to R410A) | 81.3 | 103.1 | 101.7 | 100.2 | 98.7 | 97.1 | 95.5 | 93.8 |
| Condensation glide | °C | 9.3 | 4.1 | 4.4 | 4.7 | 5.0 | 5.3 | 5.7 | 6.1 |

| Item | Unit | Example 309 | Example 310 | Example 311 | Example 312 | Example 313 | Example 314 | Example 315 | Example 316 |
|---|---|---|---|---|---|---|---|---|---|
| HFO-1132(E) | mass% | 17.5 | 17.5 | 17.5 | 17.5 | 17.5 | 17.5 | 20.0 | 20.0 |
| R32 | mass% | 31.0 | 28.5 | 26.0 | 23.5 | 21.0 | 18.5 | 48.5 | 43.5 |
| R1234yf | mass% | 47.5 | 50.0 | 52.5 | 55.0 | 57.5 | 60.0 | 27.5 | 32.5 |
| $CO_2$ | mass% | 4.0 | 4.0 | 4.0 | 4.0 | 4.0 | 4.0 | 4.0 | 4.0 |
| GWP | - | 211 | 195 | 178 | 161 | 144 | 127 | 329 | 295 |
| COP ratio | % (relative to R410A) | 99.5 | 99.5 | 99.6 | 99.8 | 99.9 | 100.1 | 99.3 | 99.3 |
| Refrigerating capacity ratio | % (relative to R410A) | 92.1 | 90.3 | 88.5 | 86.7 | 84.8 | 82.8 | 104.4 | 101.5 |
| Condensation glide | °C | 6.5 | 7.0 | 7.4 | 7.9 | 8.4 | 9.0 | 4.0 | 4.5 |

| Item | Unit | Example 317 | Example 318 | Example 319 | Example 320 | Example 321 | Example 322 | Example 323 | Example 324 |
|---|---|---|---|---|---|---|---|---|---|
| HFO-1132(E) | mass% | 20.0 | 20.0 | 20.0 | 20.0 | 20.0 | 20.0 | 20.0 | 20.0 |
| R32 | mass% | 41.0 | 38.5 | 33.5 | 31.0 | 28.5 | 23.5 | 21.0 | 18.5 |
| R1234yf | mass% | 35.0 | 37.5 | 42.5 | 45.0 | 47.5 | 52.5 | 55.0 | 57.5 |
| $CO_2$ | mass% | 4.0 | 4.0 | 4.0 | 4.0 | 4.0 | 4.0 | 4.0 | 4.0 |
| GWP | – | 278 | 262 | 228 | 211 | 195 | 161 | 144 | 127 |
| COP ratio | % (relative to R410A) | 99.3 | 99.2 | 99.3 | 99.3 | 99.3 | 99.5 | 99.6 | 99.8 |
| Refrigerating capacity ratio | % (relative to R410A) | 100.0 | 98.4 | 95.2 | 93.5 | 91.7 | 88.1 | 86.2 | 84.3 |
| Condensation glide | °C | 4.8 | 5.1 | 5.8 | 6.2 | 6.7 | 7.6 | 8.1 | 8.6 |

| Item | Unit | Example 325 | Example 326 | Example 327 | Example 328 | Example 329 | Example 330 | Example 331 | Example 332 |
|---|---|---|---|---|---|---|---|---|---|
| HFO-1132(E) | mass% | 22.5 | 22.5 | 22.5 | 22.5 | 22.5 | 22.5 | 22.5 | 22.5 |
| R32 | mass% | 48.5 | 46.0 | 43.5 | 41.0 | 38.5 | 36.0 | 33.5 | 31.0 |
| R1234yf | mass% | 25.0 | 27.5 | 30.0 | 32.5 | 35.0 | 37.5 | 40.0 | 42.5 |
| $CO_2$ | mass% | 4.0 | 4.0 | 4.0 | 4.0 | 4.0 | 4.0 | 4.0 | 4.0 |
| GWP | – | 329 | 312 | 295 | 278 | 262 | 245 | 228 | 211 |
| COP ratio | % (relative to R410A) | 99.2 | 99.2 | 99.1 | 99.1 | 99.1 | 99.1 | 99.1 | 99.1 |
| Refrigerating capacity ratio | % (relative to R410A) | 105.6 | 104.2 | 102.7 | 101.3 | 99.7 | 98.1 | 96.5 | 94.8 |
| Condensation glide | °C | 3.8 | 4.0 | 4.3 | 4.6 | 4.9 | 5.2 | 5.6 | 6.0 |

| Item | Unit | Example 333 | Example 334 | Example 335 | Example 336 | Example 337 | Example 338 | Example 339 | Example 340 |
|---|---|---|---|---|---|---|---|---|---|
| HFO-1132(E) | mass% | 22.5 | 22.5 | 22.5 | 22.5 | 22.5 | 22.5 | 22.5 | 25.0 |
| R32 | mass% | 28.5 | 26.0 | 23.5 | 21.0 | 18.5 | 16.0 | 13.5 | 43.5 |
| R1234yf | mass% | 45.0 | 47.5 | 50.0 | 52.5 | 55.0 | 57.5 | 60.0 | 27.5 |
| $CO_2$ | mass% | 4.0 | 4.0 | 4.0 | 4.0 | 4.0 | 4.0 | 4.0 | 4.0 |
| GWP | – | 194 | 178 | 161 | 144 | 127 | 111 | 94 | 295 |
| COP ratio | % (relative to R410A) | 99.1 | 99.2 | 99.3 | 99.4 | 99.5 | 99.7 | 99.9 | 99.0 |
| Refrigerating capacity ratio | % (relative to R410A) | 93.1 | 91.3 | 89.5 | 87.7 | 85.8 | 83.8 | 81.8 | 104.0 |
| Condensation glide | °C | 6.4 | 6.8 | 7.3 | 7.8 | 8.3 | 8.8 | 9.3 | 4.1 |

| Item | Unit | Example 341 | Example 342 | Example 343 | Example 344 | Example 345 | Example 346 | Example 347 | Example 348 |
|---|---|---|---|---|---|---|---|---|---|
| HFO-1132(E) | mass% | 25.0 | 25.0 | 25.0 | 25.0 | 25.0 | 25.0 | 25.0 | 25.0 |
| R32 | mass% | 41.0 | 38.5 | 36.0 | 33.5 | 31.0 | 28.5 | 26.0 | 23.5 |
| R1234yf | mass% | 30.0 | 32.5 | 35.0 | 37.5 | 40.0 | 42.5 | 45.0 | 47.5 |
| $CO_2$ | mass% | 4.0 | 4.0 | 4.0 | 4.0 | 4.0 | 4.0 | 4.0 | 4.0 |
| GWP | - | 278 | 261 | 245 | 228 | 211 | 194 | 178 | 161 |
| COP ratio | % (relative to R410A) | 98.9 | 98.9 | 98.9 | 98.9 | 98.9 | 99.0 | 99.0 | 99.1 |
| Refrigerating capacity ratio | % (relative to R410A) | 102.5 | 101.0 | 99.4 | 97.8 | 96.1 | 94.4 | 92.7 | 90.9 |
| Condensation glide | °C | 4.4 | 4.7 | 5.0 | 5.4 | 5.7 | 6.1 | 6.5 | 7.0 |

Table 45

| Item | Unit | Example 349 | Example 350 | Example 351 | Example 352 | Example 353 | Example 354 | Example 355 | Example 356 |
|---|---|---|---|---|---|---|---|---|---|
| HFO-1132(E) | mass% | 25.0 | 25.0 | 25.0 | 25.0 | 27.5 | 27.5 | 27.5 | 27.5 |
| R32 | mass% | 21.0 | 18.5 | 16.0 | 13.5 | 35.0 | 31.0 | 28.5 | 26.0 |
| R1234yf | mass% | 50.0 | 52.5 | 55.0 | 57.5 | 35.0 | 37.5 | 40.0 | 42.5 |
| $CO_2$ | mass% | 4.0 | 4.0 | 4.0 | 4.0 | 4.0 | 4.0 | 4.0 | 4.0 |
| GWP | - | 144 | 127 | 110 | 94 | 238 | 211 | 194 | 178 |
| COP ratio | % (relative to R410A) | 99.2 | 99.3 | 99.5 | 99.7 | 98.8 | 98.8 | 98.8 | 98.8 |
| Refrigerati ng capacity ratio | % (relative to R410A) | 89.1 | 87.2 | 85.2 | 83.2 | 99.4 | 97.4 | 95.8 | 94.0 |
| Condensation glide | °C | 7.5 | 8.0 | 8.5 | 9.0 | 5.0 | 5.5 | 5.9 | 6.3 |
| Item | Unit | Example 357 | Example 358 | Example 359 | Example 360 | Example 361 | Example 362 | Example 363 | Example 364 |
| HFO-1132(E) | mass% | 27.5 | 27.5 | 27.5 | 27.5 | 27.5 | 27.5 | 30.0 | 30.0 |
| R32 | mass% | 23.5 | 21.0 | 18.5 | 16.0 | 13.5 | 11.0 | 23.5 | 21.0 |
| R1234yf | mass% | 45.0 | 47.5 | 50.0 | 52.5 | 55.0 | 57.5 | 42.5 | 45.0 |
| $CO_2$ | mass% | 4.0 | 4.0 | 4.0 | 4.0 | 4.0 | 4.0 | 4.0 | 4.0 |
| GWP | - | 161 | 144 | 127 | 110 | 94 | 77 | 161 | 144 |
| COP ratio | % (relative to R410A) | 98.9 | 99.0 | 99.1 | 99.2 | 99.4 | 99.6 | 98.7 | 98.8 |
| Refrigerati ng capacity ratio | % (relative to R410A) | 92.3 | 90.4 | 88.6 | 86.7 | 84.7 | 82.6 | 93.6 | 91.8 |
| Condensation glide | °C | 6.7 | 7.2 | 7.6 | 8.1 | 8.7 | 9.2 | 6.4 | 6.9 |
| Item | Unit | Example 365 | Example 366 | Example 367 | Example 368 | Example 369 | Example 400 | Example 401 | Example 402 |
| HFO-1132(E) | mass% | 30.0 | 30.0 | 30.0 | 30.0 | 32.5 | 32.5 | 32.5 | 32.5 |
| R32 | mass% | 18.5 | 13.5 | 11.0 | 8.5 | 21.0 | 18.5 | 16.0 | 35.0 |
| R1234yf | mass% | 47.5 | 52.5 | 55.0 | 57.5 | 42.5 | 45.0 | 47.5 | 50.0 |
| $CO_2$ | mass% | 4.0 | 4.0 | 4.0 | 4.0 | 4.0 | 4.0 | 4.0 | 4.0 |

EP 3 988 522 A1

(continued)

| Item | Unit | Example 365 | Example 366 | Example 367 | Example 368 | Example 369 | Example 400 | Example 401 | Example 402 |
|---|---|---|---|---|---|---|---|---|---|
| GWP | - | 127 | 94 | 77 | 60 | 144 | 127 | 110 | 239 |
| COP ratio | % (relative to R410A) | 98.9 | 99.2 | 99.3 | 99.5 | 98.6 | 98.7 | 98.8 | 99.1 |
| Refrigerati ng capacity ratio | % (relative to R410A) | 89.9 | 86.1 | 84.1 | 82.0 | 93.1 | 91.3 | 89.4 | 94.0 |
| Condensation glide | °C | 7.3 | 8.3 | 8.8 | 9.3 | 6.6 | 7.0 | 7.5 | 5.5 |
| Item | Unit | Example 403 | Example 404 | Example 405 | Example 406 | Example 407 | Example 408 | Example 409 | Example 410 |
| HFO-1132(E) | mass% | 32.5 | 32.5 | 32.5 | 35.0 | 35.0 | 35.0 | 35.0 | 35.0 |
| R32 | mass% | 11.0 | 8.5 | 6.0 | 16.0 | 13.5 | 11.0 | 8.5 | 6.0 |
| R1234yf | mass% | 52.5 | 55.0 | 57.5 | 45.0 | 47.5 | 50.0 | 52.5 | 55.0 |
| $CO_2$ | mass% | 4.0 | 4.0 | 4.0 | 4.0 | 4.0 | 4.0 | 4.0 | 4.0 |
| GWP | - | 77 | 60 | 43 | 110 | 93 | 77 | 60 | 43 |
| COP ratio | % (relative to R410A) | 99.1 | 99.3 | 99.5 | 98.6 | 98.7 | 98.9 | 99.1 | 99.3 |
| Refrigerati ng capacity ratio | % (relative to R410A) | 85.5 | 83.4 | 81.3 | 90.8 | 88.8 | 86.9 | 84.8 | 82.8 |
| Condensation glide | °C | 8.5 | 9.0 | 9.5 | 7.2 | 7.6 | 8.1 | 8.6 | 9.1 |
| Item | Unit | Example 411 | Example 412 | Example 413 | Example 414 | Example 415 | Example 416 | Example 417 | Example 418 |
| HFO-1132(E) | mass% | 37.5 | 37.5 | 37.5 | 37.5 | 37.5 | 40.0 | 40.0 | 40.0 |
| R32 | mass% | 13.5 | 11.0 | 8.5 | 6.0 | 3.5 | 11.0 | 8.5 | 3.5 |
| R1234yf | mass% | 45.0 | 47.5 | 50.0 | 52.5 | 55.0 | 45.0 | 47.5 | 52.5 |
| $CO_2$ | mass% | 4.0 | 4.0 | 4.0 | 4.0 | 4.0 | 4.0 | 4.0 | 4.0 |
| GWP | - | 93 | 77 | 60 | 43 | 26 | 76 | 60 | 26 |
| COP ratio | % (relative to R410A) | 98.6 | 98.7 | 98.9 | 99.0 | 99.2 | 98.5 | 98.7 | 99.0 |
| Refrigerati ng capacity ratio | % (relative to R410A) | 90.2 | 88.2 | 86.2 | 84.2 | 82.0 | 89.6 | 87.6 | 83.4 |

(continued)

| Item | Unit | Example 411 | Example 412 | Example 413 | Example 414 | Example 415 | Example 416 | Example 417 | Example 418 |
|---|---|---|---|---|---|---|---|---|---|
| Condensation glide | °C | 7.3 | 7.8 | 8.3 | 8.8 | 9.2 | 7.5 | 7.9 | 8.9 |
| Item | Unit | Example 419 | Example 420 | Example 421 | Example 422 | Example 423 | Example 424 | Example 425 | Example 426 |
| HFO-1132(E) | mass% | 40.0 | 42.5 | 42.5 | 42.5 | 42.5 | 45.0 | 45.0 | 45.0 |
| R32 | mass% | 1.0 | 8.5 | 35.0 | 3.5 | 1.0 | 6.0 | 3.5 | 1.0 |
| R1234yf | mass% | 55.0 | 45.0 | 47.5 | 50.0 | 52.5 | 45.0 | 47.5 | 50.0 |
| $CO_2$ | mass% | 4.0 | 4.0 | 4.0 | 4.0 | 4.0 | 4.0 | 4.0 | 4.0 |
| GWP | - | 9 | 60 | 239 | 26 | 9 | 43 | 26 | 9 |
| COP ratio | % (relative to R410A) | 99.2 | 98.5 | 98.8 | 98.8 | 99.0 | 98.5 | 98.6 | 98.8 |
| Refrigerati ng capacity ratio | % (relative to R410A) | 81.2 | 88.9 | 95.6 | 84.8 | 82.6 | 88.3 | 86.2 | 84.0 |
| Condensation glide | °C | 9.3 | 7.6 | 5.0 | 8.5 | 9.0 | 7.8 | 8.2 | 8.7 |

EP 3 988 522 A1

Table 46

| Item | Unit | Example 427 | Example 428 | Example 429 | Example 430 | Example 431 | Example 432 |
|---|---|---|---|---|---|---|---|
| HFO-1132(E) | mass% | 47.5 | 47.5 | 50.0 | 50.0 | 52.5 | 55.0 |
| R32 | mass% | 4.5 | 2.0 | 3.5 | 1.0 | 2.0 | 1.0 |
| R1234yf | mass% | 44.0 | 46.5 | 42.5 | 45.0 | 41.5 | 40.0 |
| $CO_2$ | mass% | 4.0 | 4.0 | 4.0 | 4.0 | 4.0 | 4.0 |
| GWP | - | 33 | 16 | 26 | 9 | 16 | 9 |
| COP ratio | % (relative to R410A) | 98.4 | 98.6 | 98.3 | 98.5 | 98.3 | 98.2 |
| Refrigerating capacity ratio | % (relative to R410A) | 88.4 | 86.3 | 88.9 | 86.8 | 88.9 | 89.4 |
| Condensation glide | °C | 7.7 | 8.1 | 7.6 | 8.0 | 7.5 | 7.4 |

These results indicate that under the condition that the mass% of $CO_2$, R32, HFO-1132 (E), and R1234yf based on their sum is respectively represented by w, x, y, and z, when coordinates (x,y,z) in a ternary composition diagram (Figs. 2 to 9) in which the sum of R32, HFO-1132(E), and R1234yf is (100-w) mass% are on straight line A''B'', the mixed refrigerant has a GWP of 350, and when coordinates (x,y,z) in the ternary composition diagram are positioned on the right side of the line, the mixed refrigerant has a GWP of less than 350. The results also indicate that when coordinates (x,y,z) in the ternary composition diagram of Figs. 2 to 9 are on straight line A'B', the mixed refrigerant has a GWP of 250, and that when coordinates (x,y,z) in the ternary composition diagram are positioned on the right side of the line, the mixed refrigerant has a GWP of less than 250. Further, the results also indicate that when coordinates (x,y,z) in the ternary composition diagram of Figs. 2 to 9 are on straight line AB, the mixed refrigerant has a GWP of 125, and that when coordinates (x,y,z) in the ternary composition diagram are positioned on the right side of the line, the mixed refrigerant has a GWP of less than 125.

It is found that the straight line connecting points D and C is positioned on the slightly left side of the curve connecting points at which the refrigerating capacity ratio relative to R410A is 80%. Accordingly, it is indicated that when the coordinates (x, y, z) are on the left side of the straight line connecting points D and C, the refrigerating capacity ratio of the mixed refrigerant is 80% or more relative to R410A.

Coordinates of points A and B, A' and B', and A" and B" were determined by obtaining the approximate expression based on each of the points shown in the above tables. Specifically, calculation was performed according to Table 47 (points A and B), Table 48 (points A' and B'), and Table 49 (points A" and B'').

Table 47

| Point A | | | | | | | | | |
|---|---|---|---|---|---|---|---|---|---|
| Item | $1.2{\geq}CO_2{>}O$ | | | $4.0{\geq}CO_2{\geq}1.2$ | | | $7.0{\geq}CO_2{\geq}4.0$ | | |
| $CO_2$ | 0.0 | 0.6 | 1.2 | 1.2 | 2.5 | 4.0 | 4.0 | 5.5 | 7.0 |
| E-HFO-1132 | 81.6 | 81.0 | 80.4 | 80.4 | 79.1 | 77.6 | 77.6 | 76.1 | 74.6 |
| R32 | 18.4 | 18.4 | 18.4 | 18.4 | 18.4 | 18.4 | 18.4 | 18.4 | 18.4 |
| R1234yf | 0.0 | 0.0 | 0.0 | 0.0 | 0.0 | 0.0 | 0.0 | 0.0 | 0.0 |
| $CO_2$ | W | | | w | | | w | | |
| Approximate formula of HFO-1132 (E) | -w+81.6 | | | -w+81.6 | | | -w+81.6 | | |
| Approximate formula of R32 | 18.4 | | | 18.4 | | | 18.4 | | |
| Approximate formula of R1234yf | 0.0 | | | 0.0 | | | 0.0 | | |

87

(continued)

| Point B | | | | | | | | | |
|---|---|---|---|---|---|---|---|---|---|
| Item | $1.2 \geq CO_2 > 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 0.0 | 0.0 | 0.0 | 0.0 | 0.0 | 0.0 | 0.0 | 0.0 | 0.0 |
| R32 | 18.1 | 18.1 | 18.1 | 18.1 | 18.1 | 18.1 | 18.1 | 18.1 | 18.1 |
| R1234yf | 81.9 | 81.3 | 80.7 | 80.7 | 79.4 | 77.9 | 77.9 | 76.4 | 74.9 |
| $CO_2$ | w | | | w | | | W | | |
| Approximate formula of HFO-1132 (E) | 0.0 | | | 0.0 | | | 0.0 | | |
| Approximate formula of R32 | 18.1 | | | 18.1 | | | 18.1 | | |
| Approximate formula of R1234yf | -w+81.9 | | | -w+81.9 | | | -w+81.9 | | |

Table 48

| Point A' | | | | | | | | | |
|---|---|---|---|---|---|---|---|---|---|
| Item | $1.2 \geq CO_2 > 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 63.1 | 62.5 | 61.9 | 61.9 | 60.6 | 59.1 | 59.1 | 57.6 | 56.1 |
| R32 | 36.9 | 36.9 | 36.9 | 36.9 | 36.9 | 36.9 | 36.9 | 36.9 | 36.9 |
| R1234yf | 0.0 | 0.0 | 0.0 | 0.0 | 0.0 | 0.0 | 0.0 | 0.0 | 0.0 |
| $CO_2$ | w | | | w | | | w | | |
| Approximate formula of HFO-1132 (E) | -w+63.1 | | | -w+63.1 | | | -w+63.1 | | |
| Approximate formula of R32 | 36.9 | | | 36.9 | | | 36.9 | | |
| Approximate formula of R1234yf | 0.0 | | | 0.0 | | | 0.0 | | |
| Point B' | | | | | | | | | |
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 0.0 | 0.0 | 0.0 | 0.0 | 0.0 | 0.0 | 0.0 | 0.0 | 0.0 |
| R32 | 36.7 | 36.7 | 36.6 | 36.6 | 36.6 | 36.6 | 36.6 | 36.6 | 36.6 |
| R1234yf | 63.3 | 62.7 | 62.2 | 62.2 | 60.9 | 59.4 | 59.4 | 57.9 | 56.4 |
| $CO_2$ | w | | | w | | | w | | |
| Approximate formula of HFO-1132 (E) | 0 | | | 0.0 | | | 0.0 | | |
| Approximate formula of R32 | 100-R1234yf-$CO_2$ | | | 36.6 | | | 36.6 | | |
| Approximate formula of R1234yf | -0.9167w+63.283 | | | -w+63.4 | | | -w+63.4 | | |

Table 49

| Point A" | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |

(continued)

| Point A" | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| E-HFO-1132 | 48.2 | 47.6 | 47.0 | 47.0 | 45.7 | 44.2 | 44.2 | 42.7 | 41.2 |
| R32 | 51.8 | 51.8 | 51.8 | 51.8 | 51.8 | 51.8 | 51.8 | 51.8 | 51.8 |
| R1234yf | 0.0 | 0.0 | 0.0 | 0.0 | 0.0 | 0.0 | 0.0 | 0.0 | 0.0 |
| $CO_2$ | W | | | w | | | w | | |
| Approximate formula of HFO-1132 (E) | -w+48.2 | | | -w+48.2 | | | -w+48.2 | | |
| Approximate formula of R32 | 51.8 | | | 51.8 | | | 51.8 | | |
| Approximate formula of R1234yf | 0.0 | | | 0.0 | | | 0.0 | | |
| Point B" | | | | | | | | | |
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 0.0 | 0.0 | 0.0 | 0.0 | 0.0 | 0.0 | 0.0 | 0.0 | 0.0 |
| R32 | 51.5 | 51.6 | 51.6 | 51.6 | 51.6 | 51.6 | 51.6 | 51.6 | 51.6 |
| R1234yf | 49.5 | 47.8 | 47.2 | 47.2 | 45.9 | 44.4 | 44.4 | 42.9 | 41.4 |
| $CO_2$ | W | | | w | | | w | | |
| Approximate formula of HFO-1132 (E) | 0.0 | | | 0.0 | | | 0.0 | | |
| Approximate formula of R32 | $100-R1234yf-CO_2$ | | | 51.6 | | | 51.6 | | |
| Approximate formula of R1234yf | $1.5278W^2-3.75w+49.5$ | | | -w+48.4 | | | -w+48.4 | | |

Coordinates C to G were determined by obtaining the approximate expression based on each of the points shown in the above tables. Specifically, calculation was performed according to Tables 50 and 51.

Table 50

| Point C | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 58.3 | 55.4 | 52.4 | 52.4 | 46.2 | 39.5 | 39.5 | 33.0 | 26.8 |
| R32 | 0.0 | 0.0 | 0.0 | 0.0 | 0.0 | 0.0 | 0.0 | 0.0 | 0.0 |
| R1234yf | 41.7 | 44.0 | 46.4 | 46.4 | 51.3 | 56.5 | 56.5 | 61.5 | 66.2 |
| $CO_2$ | w | | | w | | | w | | |
| Approximate formula of HFO-1132 (E) | -4.9167w+58.317 | | | $0.1081w^2-5.169w+58.447$ | | | $0.0667w^2-4.9667w+58.3$ | | |
| Approximate formula of R32 | 0.0 | | | 0.0 | | | 0.0 | | |
| Approximate formula of R1234yf | $100-E-HFO-1132-CO_2$ | | | $100-E-HFO-1132-CO_2$ | | | $100-E-HFO-1132-CO_2$ | | |
| Point D | | | | | | | | | |
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |

(continued)

| Point D | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| E-HFO-1132 | 0.0 | 0.0 | 0.0 | 0.0 | 0.0 | 0.0 | 0.0 | 0.0 | 0.0 |
| R32 | 40.3 | 38.6 | 36.8 | 36.8 | 33.2 | 28.9 | 28.9 | 24.7 | 20.5 |
| R1234yf | 59.7 | 60.8 | 62.0 | 62.0 | 64.3 | 67.1 | 67.1 | 69.8 | 72.5 |
| $CO_2$ | w | | | W | | | w | | |
| Approximate formula of HFO-1132 (E) | 0.0 | | | 0.0 | | | 0.0 | | |
| Approximate formula of R32 | $-2.9167w+40.317$ | | | $-2.8226w+40.211$ | | | $-2.8w+40.1$ | | |
| Approximate formula of R1234yf | $100-R32-CO_2$ | | | $100-R32-CO_2$ | | | $100-R32-CO_2$ | | |

| Point E | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 31.9 | 29.6 | 26.5 | 26.5 | 20.9 | 14.7 | 14.7 | 8.8 | 3.1 |
| R32 | 18.2 | 18.2 | 18.2 | 18.2 | 18.2 | 18.1 | 18.1 | 18.1 | 18.1 |
| R1234yf | 49.9 | 51.6 | 54.1 | 54.1 | 58.4 | 63.2 | 63.2 | 67.6 | 71.8 |
| $CO_2$ | w | | | W | | | W | | |
| Approximate formula of HFO-1132 (E) | $-1.1111w^2-3.1667w+31.9$ | | | $0.0623w^2-4.5381w+31.856$ | | | $0.0444w^2-4.3556w+31.411$ | | |
| Approximate formula of R32 | 18.2 | | | $-0.0365w+18.26$ | | | 18.1 | | |
| Approximate formula of R1234yf | $100-E\text{-}HFO\text{-}1132\text{-}R32\text{-}CO_2$ | | | $100-E\text{-}HFO\text{-}1132\text{-}R32\text{-}CO_2$ | | | $100-E\text{-}HFO\text{-}1132\text{-}R32\text{-}CO_2$ | | |

| Point F | | | | | |
|---|---|---|---|---|---|
| Item | $1.2 \geq CO_2 \geq 0$ | | | $1.3 \geq CO_2 \geq 1.2$ | |
| $CO_2$ | 0.0 | 0.6 | 1.2 | 1.2 | 1.3 |
| E-HFO-1132 | 5.2 | 2.7 | 0.3 | 0.3 | 0 |
| R32 | 36.7 | 36.7 | 36.6 | 36.6 | 36.6 |
| R1234yf | 58.1 | 60.0 | 61.9 | 61.9 | 62.1 |
| $CO_2$ | W | | | w | |
| Approximate formula of HFO-1132(E) | $-4.0833w+5.1833$ | | | $-3w+3.9$ | |
| Approximate formula of R32 | $-0.0833w+36.717$ | | | 36.6 | |
| Approximate formula of R1234yf | $100-E\text{-}HFO\text{-}1132\text{-}R32\text{-}CO_2$ | | | $100-E\text{-}HFO\text{-}1132\text{-}R32\text{-}CO_2$ | |

(continued)

| Point G | | | | |
|---|---|---|---|---|
| Item | $1.2 \geq CO_2 \geq 0$ | | | |
| $CO_2$ | 0.0 | 0.6 | 1.2 | |
| E-HFO-1132 | 26.2 | 29.6 | 38.1 | |
| R32 | 22.2 | 18.2 | 10.0 | |
| R1234yf | 51.6 | 51.6 | 50.7 | |
| $CO_2$ | w | | | |
| Approximate formula of HFO-1132(E) | $7.0833w^2+1.4167w+26.2$ | | | |
| Approximate formula of R32 | $-5.8333w^2-3.1667w+22.2$ | | | |
| Approximate formula of R1234yf | $100-E-HFO-1132-R32-CO_2$ | | | |

Table 51

| Point M | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 52.6 | 55.4 | 58.0 | 58.0 | 59.7 | 60.4 | 0.0 | 33.0 | 26.8 |
| R32 | 0.0 | 0.0 | 0.0 | 0.0 | 0.0 | 0.0 | 0.0 | 0.0 | 0.0 |
| R1234yf | 47.4 | 44.0 | 40.8 | 40.8 | 37.8 | 35.6 | 56.5 | 61.5 | 66.2 |
| $CO_2$ | w | | | w | | | w | | |
| Approximate formula of HFO-1132 (E) | $100-E-HFO-1132-R1234yf-CO_2$ | | | $100-E-HFO-1132-R1234yf-CO_2$ | | | $100-E-HFO-1132-R1234yf-CO_2$ | | |
| Approximate formula of R32 | 0.0 | | | 0.0 | | | 0.0 | | |
| Approximate formula of R1234yf | $0.2778w^2-5.8333w+47.4$ | | | $0,3004w^2-3.419w+44.47$ | | | $0.0667w^2-1.8333w+41.867$ | | |
| Point W | | | | | | | | | |
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 32.4 | 35.1 | 38.1 | 38.1 | 40.9 | 42.6 | 42.6 | 43.3 | 43.7 |
| R32 | 10.0 | 10.0 | 10.0 | 10.0 | 10.0 | 10.0 | 10.0 | 10.0 | 10.0 |
| R1234yf | 57.6 | 54.3 | 50.7 | 50.7 | 46.6 | 43.4 | 43.4 | 41.2 | 39.3 |
| $CO_2$ | W | | | w | | | w | | |
| Approximate formula of HFO-1132 (E) | $100-R32-R1234yf-CO_2$ | | | $100-R32-R1234yf-CO_2$ | | | $100-R32-R1234yf-CO_2$ | | |
| Approximate formula of R32 | 10.0 | | | 10.0 | | | 10.0 | | |

| Point W | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| Approximate formula of R1234yf | $-0.167w^2-5.25w+57.6$ | | | $0.3645w^2-4.5024w+55.578$ | | | $0.0667w^2-2.1w+50.733$ | | |

| Point N | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 27.7 | 29.6 | 31.7 | 31.7 | 34.2 | 35.5 | 35.5 | 36.3 | 36.7 |
| R32 | 18.2 | 18.2 | 18.2 | 18.2 | 18.2 | 18.2 | 18.2 | 18.2 | 18.2 |
| R1234yf | 54.1 | 51.6 | 48.9 | 48.9 | 45.1 | 42.3 | 42.3 | 40.0 | 38.1 |
| $CO_2$ | w | | | w | | | w | | |
| Approximate formula of HFO-1132 (E) | $100-R32-R1234yf -CO_2$ | | | $100-R32-R1234yf -CO_2$ | | | $100-R32-R1234yf -CO_2$ | | |
| Approximate formula of R32 | 18.2 | | | 18.2 | | | 18.2 | | |
| Approximate formula of R1234yf | $-0.2778w^2-4w+54.1$ | | | $0.3773w^2-4.319w+53.54$ | | | $0.0889w^2-2.3778w+50.389$ | | |

| Point O | | | | | | | | | |
|---|---|---|---|---|---|---|---|---|---|
| Item | $1.2 \geq CO_2 \geq 0$ | | | $4.0 \geq CO2_{\geq} 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 22.6 | 24.0 | 25.4 | 25.4 | 27.2 | 28.0 | 28.0 | 28.4 | 28.6 |
| R32 | 36.8 | 36.8 | 36.8 | 36.8 | 36.8 | 36.8 | 36.8 | 36.8 | 36.8 |
| R1234yf | 40.6 | 38.6 | 36.0 | 36.0 | 33.5 | 31.2 | 31.2 | 29.3 | 27.6 |
| $CO_2$ | w | | | w | | | w | | |
| Approximate formula of HFO-1132 (E) | $100-R32-R1234yf -CO_2$ | | | $100-R32-R1234yf -CO_2$ | | | $100-R32-R1234yf -CO_2$ | | |
| Approximate formula of R32 | 36.8 | | | 36.8 | | | 36.8 | | |
| Approximate formula of R1234yf | $-0.8333w^2-2.8333w+40.6$ | | | $0.1392w^2-2.4381w+38.725$ | | | $0.0444w^2-1.6889w+37.244$ | | |

| Point P | | | | | | | | | |
|---|---|---|---|---|---|---|---|---|---|
| Item | $1.2 \geq CO_2 \geq 0$ | | | $4.0 \geq CO_2 \geq 1.2$ | | | $7.0 \geq CO_2 \geq 4.0$ | | |
| $CO_2$ | 0.0 | 0.6 | 1.2 | 1.2 | 2.5 | 4.0 | 4.0 | 5.5 | 7.0 |
| E-HFO-1132 | 20.5 | 20.9 | 22.1 | 22.1 | 23.4 | 23.9 | 23.9 | 24.2 | 24.2 |
| R32 | 51.7 | 51.7 | 51.7 | 51.7 | 51.7 | 51.7 | 51.7 | 51.7 | 51.7 |
| R1234yf | 27.8 | 26.8 | 25.0 | 25.0 | 22.4 | 20.4 | 20.4 | 18.6 | 17.1 |
| $CO_2$ | W | | | w | | | w | | |

(continued)

| Point P | | | |
|---|---|---|---|
| Item | $1.2 \geq CO_2 \geq 0$ | $4.0 \geq CO_2 \geq 1.2$ | $7.0 \geq CO_2 \geq 4.0$ |
| Approximate formula of HFO-1132 (E) | 100-R32-R1234yf -$CO_2$ | 100-R32-R1234yf -$CO_2$ | 100-R32-R1234yf -$CO_2$ |
| Approximate formula of R32 | 51.7 | 51.7 | 51.7 |
| Approximate formula of R1234yf | $-1.1111w^2-w+27.8$ | $0.2381w^2-2.881w+28.114$ | $0.0667w^2-1.8333w+26.667$ |

Coordinates of the points on the curves IJ, JK, and KL were determined by obtaining the approximate expression based on each of the points shown in the above tables. Specifically, calculation was performed according to Table 52.

Table 52

| Refrigerant type | | I | Example | J | J | Example | K | K | Example | L |
|---|---|---|---|---|---|---|---|---|---|---|
| $CO_2$ | R32 | 0.0 | 10.0 | 18.3 | 18.3 | 27.6 | 36.8 | 36.8 | 44.2 | 51.7 |
| 0.0 | E-HFO-1132 | 72.0 | 57.2 | 48.5 | 48.5 | 41.2 | 35.6 | 35.6 | 32.0 | 28.9 |
| | R1234yf | 28.0 | 32.8 | 33.2 | 33.2 | 31.2 | 27.6 | 27.6 | 23.8 | 19.4 |
| 0.6 | E-HFO-1132 | 72.0 | 57.2 | 48.5 | 48.5 | 41.2 | 35.6 | 35.6 | 32.0 | 28.9 |
| | R1234yf | 27.4 | 32.2 | 32.6 | 32.6 | 30.6 | 27.0 | 27.0 | 23.2 | 18.8 |
| 1.2 | E-HFO-1132 | 72.0 | 57.2 | 48.5 | 48.5 | 41.2 | 35.6 | 35.6 | 32.0 | 28.9 |
| | R1234yf | 26.8 | 31.6 | 32.0 | 32.0 | 30.0 | 26.4 | 26.4 | 22.6 | 18.2 |
| 2.5 | E-HFO-1132 | 72.0 | 57.2 | 48.5 | 48.5 | 41.2 | 35.6 | 35.6 | 32.0 | 28.9 |
| | R1234yf | 25.5 | 30.3 | 30.7 | 30.7 | 28.7 | 25.1 | 25.1 | 21.3 | 16.9 |
| 4.0 | E-HFO-1132 | 72.0 | 57.2 | 48.5 | 48.5 | 41.2 | 35.6 | 35.6 | 32.0 | 28.9 |
| | R1234yf | 24.0 | 28.8 | 29.2 | 29.2 | 27.2 | 23.6 | 23.6 | 19.8 | 15.4 |
| 5.5 | E-HFO-1132 | 72.0 | 57.2 | 48.5 | 48.5 | 41.2 | 35.6 | 35.6 | 32.0 | 28.9 |
| | R1234yf | 22.5 | 27.3 | 27.7 | 27.7 | 25.7 | 22.1 | 22.1 | 18.3 | 13.9 |
| 7.0 | E-HFO-1132 | 72.0 | 57.2 | 48.5 | 48.5 | 41.2 | 35.6 | 35.6 | 32.0 | 28.9 |
| | R1234yf | 21.0 | 25.8 | 26.2 | 26.2 | 24.2 | 20.6 | 20.6 | 16.8 | 12.4 |
| w = $CO_2$ | Approximate formula of E-HFO-1132 when x=R32 | $0.0236x^2-1.716x+72$ | | | $0.0095x^2-1.2222x+67.676$ | | | $0.0049x^2-0.8842x+61.488$ | | |
| | R1234yf | 100-E-HFO-1132-x-w | | | 100-E-HFO-1132-x-w | | | 100-E-HFO-1132-x-w | | |
| E-HFO-1132 approximate expression when x=R32 | | | | | | | | | | |

Coordinates of the points on the curves MW and WM were determined by obtaining the approximate expression based on each of the points shown in the above tables. Specifically, calculation was performed according to Table 53 (when 0 mass% < $CO_2$ concentration ≤ 1.2 mass%), Table 54 (when 1.2 mass% < $CO_2$ concentration ≤ 4.0 mass%), and Table 55 (when 4.0 mass% < $CO_2$ concentration ≤ 7.0 mass%).

Table 53

| 1.2≥$CO_2$≥0 | | | | | | |
|---|---|---|---|---|---|---|
| Item | M | Example | W | W | Example | N |
| | 0.0 | 5.0 | 10.0 | 10.0 | 14.5 | 18.2 |
| $CO_2$=0 mass% | 52.6 | 39.2 | 32.4 | 32.4 | 29.3 | 27.7 |
| Approximate formula of E-HFO-1132 when x=R32 | $0.132x^2-3.34x+52.6$ | | | $0.0313x^2-1.4551x+43.824$ | | |
| $CO_2$=0.6 mass% | 55.4 | 42.4 | 35.1 | 35.1 | 31.6 | 29.6 |
| Approximate formula of E-HFO-1132 when x=R32 | $0.114x^2-3.17x+55.4$ | | | $0.0289x^2-1.4866x+47.073$ | | |
| $CO_2$=1.2 mass% | 58.0 | 45.2 | 38.1 | 38.1 | 34.0 | 31.7 |
| Approximate formula of E-HFO-1132 when x=R32 | $0.114x^2-3.13x+58.0$ | | | $0.0353x^2-1.776x+52.330$ | | |
| In $ax^2+bx+c$, which is the approximate formula of E-HFO-1132, approximate formulas of coefficients a, b, and c when w=$CO_2$ concentration | | | | | | |
| Approximate formula of coefficient a | $0.025w^2-0.045w+0.132$ | | | $0.0122w^2-0.0113w+0.0313$ | | |
| Approximate formula of coefficient b | $-0.1806w^2+0.3917w-3.34$ | | | $-0.3582w^2+0.1624w-1.4551$ | | |
| Approximate formula of coefficient c | $-0.2778w^2+4.8333w+52.6$ | | | $2.7889w^2+3.7417w+43.824$ | | |
| Approximate formula of E-HFO-1132 when x=R32, w=$CO_2$, and 1.2≥w≥0 | $(0.025w^2-0.045w+0.132)x^2 +(-0.1806w^2+0.3917w-3.34)x+(-0.2778w^2+4.833 3w+52.6)$ | | | $(0.0122w^2-0.0113w+0.0313)x^2+(-0.3582w^2+0.1624w-1.4551)x+ (2.7889w^2+3.7417w +43.824)$ | | |
| R1234yf | 100-E-HFO-1132-R32-$CO_2$ | | | 100-E-HFO-1132-R32-$CO_2$ | | |

Table 54

| 4 .0≥$CO_2$≥1.2 | | | | | | |
|---|---|---|---|---|---|---|
| Item | M | Example | W | W | Example | N |
| | 0.0 | 5.0 | 10.0 | 10.0 | 14.5 | 18.2 |
| $CO_2$=1.2 mass% | 58 | 45.2 | 38.1 | 38.1 | 34 | 31.7 |
| Approximate formula of E-HFO-1132 when x=R32 | $0.114x^2-3.13x+58.0$ | | | $0.0353x^2-1.776x+52.330$ | | |
| $CO_2$=2.5 mass% | 59.7 | 48.1 | 40.9 | 40.9 | 36.9 | 34.2 |

(continued)

| 4 .0≥$CO_2$≥1.2 | | | | | | |
|---|---|---|---|---|---|---|
| Item | M | Example | W | W | Example | N |
| | 0.0 | 5.0 | 10.0 | 10.0 | 14.5 | 18.2 |
| Approximate formula of E-HFO-1132 when x=R32 | $0.088x^2$-2.76x+59.7 | | | $0.0194x^2$-1.3644x+52.603 | | |
| $CO_2$=4.0 mass% | 60.4 | 49.6 | 42.6 | 42.6 | 38.3 | 35.5 |
| Approximate formula of E-HFO-1132 when x=R32 | $0.076x^2$-2.54x+60.4 | | | $0.0242x^2$-1.5495x+55.671 | | |
| In the approximate formula of E-HFO-1132 $ax^2$+bx+c, approximate formulas of coefficients a, b, and c when w=$CO_2$ concentration | | | | | | |
| Approximate formula of coefficient a | $0.0043w^2$-0.0359w0.1509 | | | $0.0055w^2$-0.0326w+0.0665 | | |
| Approximate formula of coefficient b | $-0.0493w^2$+0.4669w-3.6193 | | | $-0.1571w^2$+0.8981w-2.6274 | | |
| Approximate formula of coefficient c | $-0.3004w^2$+2.419w+55.53 | | | $0.6555w^2$-2.2153w+54.044 | | |
| Approximate formula of E-HFO-1132 when x=R32, w=$CO_2$, and 4.0≥w≥1.2 | $(0.0043w^2$-0.0359w+0.1509)x2 +(-0.0493$w^2$+0.4669w-3.6193)x+(-0.3004w2+2.4 19w+55.53) | | | $(0.0055w^2$-0.0326w+0.0665)x2 +(-0.1571$w^2$+0.8981w-2.6274)x+ (0.6555w2-2.2153w+54.044) | | |
| R1234yf | 100-E-HFO-1132-R32-$CO_2$ | | | 100-E-HFO-1132-R32-$CO_2$ | | |

Table 55

| 7 .0≥$CO_2$≥4.0 | | | | | | | |
|---|---|---|---|---|---|---|---|
| Item | M | Example | W | W | Example | N |
| | 0.0 | 5.0 | 10.0 | 10.0 | 14.5 | 18.2 |
| $CO_2$=4.0 mass% | 60.4 | 49.6 | 42.6 | 42.6 | 38.3 | 35.5 |
| Approximate formula of E-HFO-1132 when x=R32 | $0.076x^2$-2.54x+60.4 | | | | $0.0242x^2$-1.5495x+55.671 | |
| $CO_2$=5.5 mass% | 60.7 | 50.3 | 43.3 | 43.3 | 39 | 36.3 |
| Approximate formula of E-HFO-1132 when x=R32 | $0.068x^2$-2.42x+60.7 | | | | $0.0275x^2$-1.6303x+56.849 | |
| $CO_2$=7.0 mass % | 60.7 | 50.3 | 43.7 | 43.7 | 39.5 | 36.7 |
| Approximate formula of E-HFO-1132 when x=R32 | $0.076x^2$-2.46x+60.7 | $0.0215x^2$-1.4609x+56.156 | | | | |
| In $ax^2$+bx+c, which is the approximate formula of E-HFO-1132, approximate formulas of coefficients a, b, and c when w=$CO_2$ concentration | | | | | | |
| Approximate formula of coefficient a | $0.00357w^2$-0.0391w+0.1756 | $-0.002061w^2$+0.0218w-0.0301 | | | | |
| Approximate formula of coefficient b | $-0.0356w^2$+0.4178w-3.6422 | $0.0556w^2$-0.5821w-0.1108 | | | | |
| Approximate formula of coefficient c | $-0.0667w^2$+0.8333w+58.103 | $-0.4158w^2$+4.7352w+43.383 | | | | |
| Approximate formula of E-HFO-1132 when x=R32, w=$CO_2$, and 7.0≥w≥4.0 | $(0.00357w^2$-0.0391w+0.1756)$x^2$+(-$0.0356w^2$+0.4178w-3.6422)x+(-$0.0667w^2$+0.8333w +58.103) | $(-0.002061w^2$+0.0218w-0.0301)$x^2$+($0.0556w^2$-0.5821w-0.1108)x+(-$0.4158w^2$+4.7352w +43.383) | | | | |
| R1234yf | 100-E-HFO-1132-R32-$CO_2$ | 100-E-HFO-1132-R32-$CO_2$ | | | | |

Coordinates of the points on the curves NO and OP were determined by obtaining the approximate expression based on each of the points shown in the above tables. Specifically, calculation was performed according to Table 56 (when 0 mass% < $CO_2$ concentration $\leq$ 1.2 mass%), Table 57 (when 1.2 mass% < $CO_2$ concentration $\leq$ 4.0 mass%), and Table 58 (when 4.0 mass% < $CO_2$ concentration $\leq$ 7.0 mass%).

Table 56

| $1.2 \geq CO_2 \geq 0$ | | | | | | |
|---|---|---|---|---|---|---|
| Item | N | Example | O | O | Example | P |
| | 18.2 | 27.6 | 36.8 | 36.8 | 44.2 | 51.7 |
| $CO_2$=0 mass% | 27.7 | 24.5 | 22.6 | 22.6 | 21.2 | 20.5 |
| Approximate formula of E-HFO-1132 when x=R32 | $0.0072x^2-0.6701x+37.512$ | | | $0.0064x^2-0.7103x+40.07$ | | |
| $CO_2$=0.6 mass% | 29.6 | 26.3 | 24 | 24 | 22.4 | 20.9 |
| Approximate formula of E-HFO-1132 when x=R32 | $0.0054x^2-0.5999x+38.719$ | | | $0.0011x^2-0.3044x+33.727$ | | |
| $CO_2$=1.2 mass% | 31.7 | 27.9 | 25.4 | 25.4 | 23.7 | 22.1 |
| Approximate formula of E-HFO-1132 when x=R32 | $0.0071x^2-0.7306x+42.636$ | | | $0.0011x^2-0.3189x+35.644$ | | |
| In $ax^2+bx+c$, which is the approximate formula of E-HFO-1132, approximate formulas of coefficients a, b, and c when w=$CO_2$ concentration | | | | | | |
| Approximate formula of coefficient a | $0.00487w^2-0.0059w+0.0072$ | | | $0.0074w^2-0.0133w+0.0064$ | | |
| Approximate formula of coefficient b | $-0.279w^2+0.2844w-0.6701$ | | | $-0.5839w^2+1.0268w-0.7103$ | | |
| Approximate formula of coefficient c | $3.7639w^2-0.2467w+37.512$ | | | $11.472w^2-17.455w+40.07$ | | |
| Approximate formula of E-HFO-1132 when x=R32, w=$CO_2$, and $1.2 \geq w > 0$ | $(0.00487w^2-0.0059w+0.0072)x^2 +(-0.279w^2+0.2844w-0.6701)x +(3.7639w^2-0.2467w+37.512)$ | | | $(0.0074w^2-0.0133w+0.0064)x^2 +(-0.5839w^2+1.0268w-0.7103)x + (11.472w^2-17.455w+40.07)$ | | |
| R1234yf | 100-E-HFO-1132-R32-$CO_2$ | | | 100-E-HFO-1132-R32-$CO_2$ | | |

Table 57

| 4 .0≥$CO_2$≥1.2 | | | | | | |
|---|---|---|---|---|---|---|
| Item | N | Example | O | O | Example | P |
| | 18.2 | 27.6 | 36.8 | 36.8 | 44.2 | 51.7 |
| $CO_2$=1.2 mass% | 31.7 | 27.9 | 25.4 | 25.4 | 23.7 | 22.1 |
| Approximate formula of E-HFO-1132 when x=R32 | $0.0071x^2$-0.7306x+42.636 | | | $0.0011x^2$-0.3189x+35.644 | | |
| $CO_2$=2.5 mass % | 34.2 | 29.9 | 27.2 | 27.2 | 25.2 | 23.4 |
| Approximate formula of E-HFO-1132 when x=R32 | $0.0088x^2$-0.8612x+46.954 | | | $0.002x^2$-0.4348x+40.5 | | |
| $CO_2$=4.0 mass% | 35.5 | 31 | 28 | 28 | 25.9 | 23.9 |
| Approximate formula of E-HFO-1132 when x=R32 | $0.0082x^2$-0.8546x+48.335 | | | $0.0011x^2$-0.3768x+40.412 | | |
| In $ax^2$+bx+c, which is the approximate formula of E-HFO-1132, approximate formulas of coefficients a, b, and c when w=$CO_2$ concentration | | | | | | |
| Approximate formula of coefficient a | $-0.00062w^2$+0.0036w+0.0037 | $-0.000463w^2$+0.0024w-0.0011 | | | | |
| Approximate formula of coefficient b | $0.0375w^2$-0.239w-0.4977 | $0.0457w^2$-0.2581w-0.075 | | | | |
| Approximate formula of coefficient c | $-0.8575w^2$+6.4941w+36.078 | $-1.355w^2$+8.749w+27.096 | | | | |
| Approximate formula of E-HFO-1132 when x=R32, w=$CO_2$, and 4.0≥w≥1.2 | $(-0.00062w^2+0.0036w+0.0037)x2+(0.0375w^2-0.239w-0.4977)x+(-0.8575w2+6.4941w+3\ 6.078)$ | $(-0.000463w^2+0.0024w-0.0011)x^2+(0.0457w2-0.2581w-0.075)x+(-1.355w^2+8.749w+\ 27.096)$ | | | | |
| R1234yf | 100-E-HFO-1132-R32-$CO_2$ | 100-E-HFO-1132-R32-$CO_2$ | | | | |

Table 58

| 7.0≥$CO_2$≥4.0 | | | | | | |
|---|---|---|---|---|---|---|
| Item | N | Example | O | O | Example | P |
| | 18.2 | 27.6 | 36.8 | 36.8 | 44.2 | 51.7 |
| $CO_2$=4.0 mass% | 35.5 | 31.0 | 28.0 | 28.0 | 25.9 | 23.9 |
| Approximate formula of E-HFO-1132 when x=R32 | $0.0082x^2$-0.8546x+48.335 | | | $0.0011x^2$-0.3768x+40.412 | | |
| $CO_2$=5.5 mass% | 36.3 | 31.6 | 28.4 | 28.4 | 26.2 | 24.2 |
| Approximate formula of E-HFO-1132 when x=R32 | $0.0082x^2$-0.8747x+49.51 | | | $0.0021x^2$-0.4638x+42.584 | | |
| $CO_2$=7.0 mass% | 36.7 | 31.9 | 28.6 | 28.6 | 26.4 | 24.2 |
| Approximate formula of E-HFO-1132 when x=R32 | $0.0082x^2$-0.8848x+50.097 | | | $0.0003x^2$-0.3188x+39.923 | | |
| In $ax^2$+bx+c, which is the approximate formula of E-HFO-1132, approximate formulas of coefficients a, b, and c when w=$CO_2$ concentration | | | | | | |
| Approximate formula of coefficient a | 0.0082 | -0.0006258w2+0.0066w-0.0153 | | | | |
| Approximate formula of coefficient b | $0.0022w^2$-0.0345w-0.7521 | $0.0516w^2$-0.5478w+0.9894 | | | | |
| Approximate formula of coefficient c | $-0.1307w^2$+2.0247w+42.327 | $-1.074w^2$+11.651w+10.992 | | | | |
| Approximate formula of E-HFO-1132 when x=R32, w=$CO_2$, and 7.0≥w≥4.0 | $0.0082x^2$+($0.0022w^2$-0.0345w-0.7521)x+(-$0.1307w^2$+2.0247w+42.327) | (-0.0006258w2+0.0066w-0.0153)x2+(0.0516w2-0.5478w+0.9894)x+(-1.074w2+11.651w+10.992) | | | | |
| R1234yf | 100-E-HFO-1132-R32-$CO_2$ | 100-E-HFO-1132-R32-$CO_2$ | | | | |

5. Refrigerants 1 to 5

Refrigerants 1 to 5 used in the present disclosure are described in detail below.

The disclosures of Refrigerant 1, Refrigerant 2, Refrigerant 3, Refrigerant 4, and Refrigerant 5 are independent from each other. Thus, the alphabetical letters used for points and line segments, as well as the numbers used for Examples and Comparative Examples, are all independent in each of Refrigerant 1, Refrigerant 2, Refrigerant 3, Refrigerant 4, and Refrigerant 5. For example, Example 1 of Refrigerant 1 and Example 1 of Refrigerant 2 each represent an example according to a different embodiment.

5.1. Refrigerant 1

In an embodiment, the refrigerant according to the present disclosure comprises HFO-1132 (E) and HFO-1234yf, wherein HFO-1132 (E) is present in an amount of 35.0 to 65.0 mass%, and HFO-1234yf is present in an amount of 65.0 to 35.0 mass%, based on the total mass of HFO-1132 (E) and HFO-1234yf. This refrigerant may be referred to as "Refrigerant 1."

In the present disclosure, Refrigerant 1 is for use in operating a refrigeration cycle in which the evaporating temperature is -75 to -5°C.

Refrigerant 1 has the above feature and thus has the following characteristics: (1) the GWP is sufficiently low (100 or less); (2) it has a COP equivalent to or higher than that of R404A; and (3) it has a refrigerating capacity equivalent to or higher than that of R404A.

Since Refrigerant 1 comprises HFO-1132 (E) in an amount of 35.0 mass% or more based on the total mass of HFO-1132 (E) and HFO-1234yf, Refrigerant 1 has a refrigerating capacity equivalent to or higher than that of R404A.

Moreover, since Refrigerant 1 comprises HFO-1132 (E) in an amount of 65.0 mass% or less based on the total mass of HFO-1132 (E) and HFO-1234yf, the saturation pressure of Refrigerant 1 at a saturation temperature of 40°C in the refrigeration cycle can be maintained within a suitable range (in particular 2.10 Mpa or less).

Refrigerant 1 may have a refrigerating capacity of 95% or more, preferably 98% or more, more preferably 100% or more, even more preferably 101% or more, and particularly preferably 102% or more, relative to that of R404A.

Since the GWP is 100 or less, Refrigerant 1 can notably reduce the burden on the environment from a global warming perspective, compared with other general-purpose refrigerants.

In Refrigerant 1, the ratio of refrigerating capacity to power consumed in a refrigeration cycle (coefficient of performance (COP)) relative to that of R404A is preferably high, from the viewpoint of energy consumption efficiency. Specifically, the COP relative to that of R404A is preferably 98% or more, more preferably 100% or more, and particularly preferably 102% or more.

In Refrigerant 1, it is preferred that HFO-1132 (E) be present in an amount of 40.5 to 59.0 mass%, and HFO-1234yf be present in an amount of 59.5 to 41.0 mass%, based on the total mass of HFO-1132 (E) and HFO-1234yf. In this case, Refrigerant 1 has a GWP of 100 or less, a COP of 101% or more relative to that of R404A, and a refrigerating capacity of 99% or more relative to that of R404A. Further, in this case, Refrigerant 1 has a saturation pressure of 1.75 MPa or more and 2.00 MPa or less at a saturation temperature of 40°C, and is thus applicable to commercially available refrigeration apparatuses for R404A without significant design change.

In Refrigerant 1, it is more preferred that HFO-1132 (E) be present in an amount of 41.3 to 59.0 mass%, and HFO-1234yf be present in an amount of 58.7 to 41.0 mass%, based on the total mass of HFO-1132 (E) and HFO-1234yf. In this case, Refrigerant 1 has a GWP of 100 or less, a COP of 101% or more relative to that of R404A, and a refrigerating capacity of 99.5% or more relative to that of R404A. Further, in this case, Refrigerant 1 has a saturation pressure of 1.76 MPa or more and 2.00 MPa or less at a saturation temperature of 40°C, and is thus applicable to commercially available refrigeration apparatuses for R404A without significant design change.

In Refrigerant 1, it is further preferred that HFO-1132 (E) be present in an amount of 41.3 to 55.0 mass%, and HFO-1234yf be present in an amount of 58.7 to 45.0 mass%, based on the total mass of HFO-1132 (E) and HFO-1234yf. In this case, Refrigerant 1 has a GWP of 100 or less, a COP of 101% or more relative to that of R404A, and a refrigerating capacity of 99.5% or more relative to that of R404A. Further, in this case, Refrigerant 1 has a saturation pressure of 1.76 MPa or more and 1.95 MPa or less at a saturation temperature of 40°C, and is thus applicable to commercially available refrigeration apparatuses for R404A without significant design change.

In Refrigerant 1, it is particularly preferred that HFO-1132 (E) be present in an amount of 41.3 to 53.5 mass%, and HFO-1234yf be present in an amount of 58.7 to 46.5 mass%, based on the total mass of HFO-1132 (E) and HFO-1234yf. In this case, Refrigerant 1 has characteristics of having a GWP of 100 or less, a COP of 102% or more relative to that of R404A, and a refrigerating capacity of 99.5% or more relative to that of R404A, as well as being slightly flammable according to ASHRAE Standards (Class 2L). Further, in this case, Refrigerant 1 has a saturation pressure of 1.76 MPa or more and 1.94 MPa or less at a saturation temperature of 40°C, and is thus applicable to commercially available refrigeration apparatuses for R404A without significant design change.

In Refrigerant 1, it is particularly preferred that HFO-1132 (E) be present in an amount of 41.3 to 51.0 mass%, and HFO-1234yf be present in an amount of 58.7 to 49.0 mass%, based on the total mass of HFO-1132 (E) and HFO-1234yf. In this case, Refrigerant 1 has characteristics of having a GWP of 100 or less, a COP of 102% or more relative to that of R404A, and a refrigerating capacity of 99% or more relative to that of R404A, as well as being slightly flammable according to ASHRAE Standards (Class 2L). Further, in this case, Refrigerant 1 has a saturation pressure of 1.76 MPa or more and 1.90 MPa or less at a saturation temperature of 40°C, and is thus applicable to commercially available refrigeration apparatuses for R404A without significant design change.

In Refrigerant 1, it is most preferred that HFO-1132 (E) be present in an amount of 41.3 to 49.2 mass%, and HFO-1234yf be present in an amount of 58.7 to 50.8 mass%, based on the total mass of HFO-1132 (E) and HFO-1234yf. In this case, Refrigerant 1 has characteristics of having a GWP of 100 or less, a COP of 102% or more relative to that of R404A, and a refrigerating capacity of 99.5% or more relative to that of R404A, as well as being slightly flammable according to ASHRAE Standards (Class 2L). Further, in this case, Refrigerant 1 has a saturation pressure of 1.76 MPa or more and 1.88 MPa or less at a saturation temperature of 40°C, and is thus applicable to commercially available refrigeration apparatuses for R404A without significant design change.

In Refrigerant 1, the saturation pressure at a saturation temperature of 40°C is usually 2.10 MPa or less, preferably 2.00 MPa or less, more preferably 1.95 MPa or less, even more preferably 1.90 MPa or less, and particularly preferably 1.88 MPa or less. If the saturation pressure at a saturation temperature of 40°C is within the above range, Refrigerant 1 is applicable to commercially available refrigeration apparatuses for R404A without significant design change.

In Refrigerant 1, the saturation pressure at a saturation temperature of 40°C is usually 1.70 MPa or more, preferably 1.73 MPa or more, more preferably 1.74 MPa or more, even more preferably 1.75 MPa or more, and particularly preferably 1.76 MPa or more. If the saturation pressure at a saturation temperature of 40°C is within the above range, Refrigerant 1 is applicable to commercially available refrigeration apparatuses for R404A without significant design change.

In the present disclosure, when Refrigerant 1 is used for operating a refrigeration cycle, the discharge temperature is preferably 150°C or lower, more preferably 140°C or lower, even more preferably 130°C or lower, and particularly preferably 120°C or lower, from the viewpoint of extending the life of the components of a commercially available refrigeration apparatus for R404A.

The use of Refrigerant 1 for operating a refrigeration cycle in which the evaporating temperature is -75 to -5°C is advantageous in terms of ensuring a refrigerating capacity equivalent to or higher than that of R404A.

In a refrigeration cycle in which Refrigerant 1 according to the present disclosure is used, when the evaporating temperature exceeds -5°C, the compression ratio becomes less than 2.5, which reduces the efficiency of the refrigeration cycle. In a refrigeration cycle in which Refrigerant 1 according to the present disclosure is used, when the evaporating temperature is less than -75°C, the evaporation pressure becomes less than 0.02 MPa, which makes suction of the refrigerant into a compressor difficult. The compression ratio is calculated with the following equation.

```
Compression ratio = condensation pressure (Mpa)/evaporation
pressure (Mpa).
```

In a refrigeration cycle in which Refrigerant 1 according to the present disclosure is used, the evaporating temperature is preferably -7.5°C or lower, more preferably -10°C or lower, and even more preferably -35°C or lower.

In a refrigeration cycle in which Refrigerant 1 according to the present disclosure is used, the evaporating temperature is preferably -65°C or more, more preferably -60°C or more, even more preferably -55°C or more, and particularly preferably -50°C or more.

In a refrigeration cycle in which Refrigerant 1 according to the present disclosure is used, the evaporating temperature is preferably -65°C or more and -5°C or lower, more preferably -60°C or more and -5°C or lower, even more preferably -55°C or more and -7.5°C or lower, and particularly preferably - 50°C or more and -10°C or lower.

In a refrigeration cycle in which Refrigerant 1 according to the present disclosure is used, the evaporation pressure is preferably 0.02 MPa or more, more preferably 0.03 MPa or more, even more preferably 0.04 MPa or more, and particularly preferably 0.05 MPa or more, from the viewpoint of improving the suction of the refrigerant into a compressor.

In a refrigeration cycle in which Refrigerant 1 according to the present disclosure is used, the compression ratio is preferably 2.5 or more, more preferably 3.0 or more, even more preferably 3.5 or more, and particularly preferably 4.0 or more, from the viewpoint of improving the efficiency of the refrigeration cycle. In a refrigeration cycle in which Refrigerant 1 according to the present disclosure is used, the compression ratio is preferably 200 or less, more preferably 150 or less, even more preferably 100 or less, and particularly preferably 50 or less, from the viewpoint of improving the efficiency of the refrigeration cycle.

Refrigerant 1 may comprise HFO-1132 (E) and HFO-1234yf in such amounts that the sum of their concentrations is usually 99.5 mass% or more. In the present disclosure, the total amount of HFO-1132 (E) and HFO-1234yf is preferably 99.7 mass% or more, more preferably 99.8 mass% or more, and even more preferably 99.9 mass% or more, of entire Refrigerant 1.

Refrigerant 1 may further comprise an additional refrigerant in addition to HFO-1132 (E) and HFO-1234yf as long as the above characteristics are not impaired. In this case, the content of the additional refrigerant is preferably 0.5 mass% or less, more preferably 0.3 mass% or less, even more preferably 0.2 mass% or less, and particularly preferably 0.1 mass% or less, of entire Refrigerant 1. The additional refrigerant is not limited and may be selected from a wide range of known refrigerants widely used in the field. Refrigerant 1 may comprise one additional refrigerant or two or more additional refrigerants.

It is particularly preferred that Refrigerant 1 consist of HFO-1132 (E) and HFO-1234yf. In other words, the total concentration of HFO-1132 (E) and HFO-1234yf in Refrigerant 1 is particularly preferably 100 mass% of entire Refrigerant 1.

When Refrigerant 1 consists of HFO-1132 (E) and HFO-1234yf, HFO-1132 (E) is usually present in an amount of 35.0 to 65.0 mass%, and HFO-1234yf is usually present in an amount of 65.0 to 35.0 mass%, based on the total mass of HFO-1132 (E) and HFO-1234yf. Refrigerant 1 has the above feature and thus has the following characteristics: (1) the GWP is sufficiently low (100 or less); (2) it has a COP equivalent to or higher than that of R404A; and (3) it has a refrigerating capacity equivalent to or higher than that of R404A.

When Refrigerant 1 consists of HFO-1132 (E) and HFO-1234yf, it is preferred that HFO-1132 (E) be present in an amount of 40.5 to 59.0 mass%, and HFO-1234yf be present in an amount of 59.5 to 41.0 mass%, based on the total mass of HFO-1132 (E) and HFO-1234yf. In this case, Refrigerant 1 has a GWP of 100 or less, a COP of 101% or more relative to that of R404A, and a refrigerating capacity of 99% or more relative to that of R404A. Further, in this case, Refrigerant 1 has a saturation pressure of 1.75 MPa or more and 2.00 MPa or less at a saturation temperature of 40°C, and is thus applicable to commercially available refrigeration apparatuses for R404A without significant design change.

When Refrigerant 1 consists of HFO-1132 (E) and HFO-1234yf, it is preferred that HFO-1132 (E) be present in an amount of 41.3 to 59.0 mass%, and HFO-1234yf be present in an amount of 58.7 to 41.0 mass%, based on the total mass of HFO-1132 (E) and HFO-1234yf. In this case, Refrigerant 1 has a GWP of 100 or less, a COP of 101% or more relative to that of R404A, and a refrigerating capacity of 99.5% or more relative to that of R404A. Further, in this case, Refrigerant 1 has a saturation pressure of 1.76 MPa or more and 2.00 MPa or less at a saturation temperature of 40°C, and is thus applicable to commercially available refrigeration apparatuses for R404A without significant design change.

When Refrigerant 1 consists of HFO-1132 (E) and HFO-1234yf, it is further preferred that HFO-1132 (E) be present in an amount of 41.3 to 55.0 mass%, and HFO-1234yf be present in an amount of 58.7 to 45.0 mass%, based on the total mass of HFO-1132 (E) and HFO-1234yf. In this case, Refrigerant 1 has a GWP of 100 or less, a COP of 101% or more relative to that of R404A, and a refrigerating capacity of 99.5% or more relative to that of R404A. Further, in this case, Refrigerant 1 has a saturation pressure of 1.76 MPa or more and 1.95 MPa or less at a saturation temperature of 40°C, and is thus applicable to commercially available refrigeration apparatuses for R404A without significant design change.

When Refrigerant 1 consists of HFO-1132 (E) and HFO-1234yf, it is particularly preferred that HFO-1132 (E) be present in an amount of 41.3 to 53.5 mass%, and HFO-1234yf be present in an amount of 58.7 to 46.5 mass%, based on the total mass of HFO-1132 (E) and HFO-1234yf. In this case, Refrigerant 1 has characteristics of having a GWP of 100 or less, a COP of 102% or more relative to that of R404A, and a refrigerating capacity of 99.5% or more relative to that of R404A, as well as being slightly flammable according to ASHRAE Standards (Class 2L). Further, in this case, Refrigerant 1 has a saturation pressure of 1.76 MPa or more and 1.94 MPa or less at a saturation temperature of 40°C, and is thus applicable to commercially available refrigeration apparatuses for R404A without significant design change.

When Refrigerant 1 consists of HFO-1132 (E) and HFO-1234yf, it is particularly preferred that HFO-1132 (E) be present in an amount of 41.3 to 51.0 mass%, and HFO-1234yf be present in an amount of 58.7 to 49.0 mass%, based on the total mass of HFO-1132 (E) and HFO-1234yf. In this case, Refrigerant 1 has characteristics of having a GWP of 100 or less, a COP of 102% or more relative to that of R404A, and a refrigerating capacity of 99% or more relative to that of R404A, as well as being slightly flammable according to ASHRAE Standards (Class 2L). Further, in this case, Refrigerant 1 has a saturation pressure of 1.76 MPa or more and 1.90 MPa or less at a saturation temperature of 40°C, and is thus applicable to commercially available refrigeration apparatuses for R404A without significant design change.

When Refrigerant 1 consists of HFO-1132 (E) and HFO-1234yf, it is most preferred that HFO-1132 (E) be present in an amount of 41.3 to 49.2 mass%, and HFO-1234yf be present in an amount of 58.7 to 50.8 mass%, based on the total mass of HFO-1132 (E) and HFO-1234yf. In this case, Refrigerant 1 has characteristics of having a GWP of 100 or less, a COP of 102% or more relative to that of R404A, and a refrigerating capacity of 99.5% or more relative to that of R404A, as well as being slightly flammable according to ASHRAE Standards (Class 2L). Further, in this case, Refrigerant 1 has a saturation pressure of 1.76 MPa or more and 1.88 MPa or less at a saturation temperature of 40°C, and is thus applicable to commercially available refrigeration apparatuses for R404A without significant design change.

5.2 Refrigerant 2

In an embodiment, the refrigerant according to the present disclosure comprises HFO-1132 (E) and HFO-1234yf, wherein HFO-1132 (E) is present in an amount of 40.5 to 49.2 mass%, and HFO-1234yf is present in an amount of 59.5 to 50.8 mass%, based on the total mass of HFO-1132 (E) and HFO-1234yf. This refrigerant may be referred to as "Refrigerant 2."

Refrigerant 2 has the above feature and thus has the following characteristics: (1) the GWP is sufficiently low (100 or less); (2) it has a COP equivalent to or higher than that of R404A; (3) it has a refrigerating capacity equivalent to or higher than that of R404A; and (4) it is slightly flammable according to ASHRAE Standards (Class 2L). Further, in this case, Refrigerant 2 has a saturation pressure of 1.75 MPa or more and 1.88 MPa or less at a saturation temperature of 40°C, and is thus applicable to commercially available refrigeration apparatuses for R404A without significant design change.

Since Refrigerant 2 comprises HFO-1132 (E) in an amount of 40.5 mass% or more based on the total mass of HFO-1132 (E) and HFO-1234yf, Refrigerant 2 has a refrigerating capacity equivalent to or higher than that of R404A. Moreover, since Refrigerant 2 comprises HFO-1132 (E) in an amount of 49.2 mass% or less based on the total mass of HFO-1132 (E) and HFO-1234yf, the saturation pressure of Refrigerant 2 at a saturation temperature of 40°C in the refrigeration cycle can be maintained within a suitable range (in particular 2.10 Mpa or less).

Refrigerant 2 may have a refrigerating capacity of 99% or more, preferably 100% or more, more preferably 101% or more, even more preferably 102% or more, and particularly preferably 103% or more, relative to that of R404A.

Since the GWP is 100 or less, Refrigerant 2 can notably reduce the burden on the environment from a global warming perspective, compared with other general-purpose refrigerants.

In Refrigerant 2, the ratio of refrigerating capacity to power consumed in a refrigeration cycle (coefficient of performance (COP)) relative to that of R404A is preferably high, from the viewpoint of energy consumption efficiency. Specifically, the COP relative to that of R404A is preferably 98% or more, more preferably 100% or more, even more preferably 101% or more, and particularly preferably 102% or more.

In Refrigerant 2, it is preferred that HFO-1132 (E) be present in an amount of 41.3 to 49.2 mass%, and HFO-1234yf be present in an amount of 58.7 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 99.5% or more relative to that of R404A, as well as being slightly flammable according to ASHRAE Standards (Class 2L). Further, in this case, Refrigerant 2 has a saturation pressure of 1.76 MPa or more and 1.88 MPa or less at a saturation temperature of 40°C, and is thus applicable to commercially available refrigeration apparatuses for R404A without significant design change.

In Refrigerant 2, it is preferred that HFO-1132 (E) be present in an amount of 43.0 to 49.2 mass%, and HFO-1234yf be present in an amount of 57.0 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 101% or more relative to that of R404A, as well as being slightly flammable according to ASHRAE Standards (Class 2L). Further, in this case, Refrigerant 2 has a saturation pressure of 1.78 MPa or more and 1.88 MPa or less at a saturation temperature of 40°C, and is thus applicable to commercially available refrigeration apparatuses for R404A without significant design change.

In Refrigerant 2, it is further preferred that HFO-1132 (E) be present in an amount of 44.0 to 49.2 mass%, and HFO-1234yf be present in an amount of 56.0 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 101% or more relative to that of R404A, as well as being slightly flammable according to ASHRAE Standards (Class 2L). Further, in this case, Refrigerant 2 has a saturation pressure of 1.80 MPa or more and 1.88 MPa or less at a saturation temperature of 40°C, and is thus applicable to commercially available refrigeration apparatuses for R404A without significant design change.

In Refrigerant 2, it is particularly preferred that HFO-1132 (E) be present in an amount of 45.0 to 49.2 mass%, and HFO-1234yf be present in an amount of 55.0 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 102% or more relative to that of R404A, as well as being slightly flammable according to ASHRAE Standards (Class 2L). Further, in this case, Refrigerant 2 has a saturation pressure of 1.81 MPa or more and 1.88 MPa or less at a saturation temperature of 40°C, and is thus applicable to commercially available refrigeration apparatuses for R404A without significant design change.

In Refrigerant 2, it is particularly preferred that HFO-1132 (E) be present in an amount of 45.0 to 48.0 mass%, and HFO-1234yf be present in an amount of 55.0 to 52.0 mass%, based on the total mass of HFO-1132 (E) and HFO-1234yf. In this case, Refrigerant 2 has characteristics of having a GWP of 100 or less, a COP of 102.5% or more relative to that of R404A, and a refrigerating capacity of 102.5% or more relative to that of R404A, as well as being slightly flammable according to ASHRAE Standards (Class 2L). Further, in this case, Refrigerant 2 has a saturation pressure of 1.81 MPa or more and 1.87 MPa or less at a saturation temperature of 40°C, and is thus applicable to commercially available refrigeration apparatuses for R404A without significant design change.

In Refrigerant 2, it is most preferred that HFO-1132 (E) be present in an amount of 45.0 to 47.0 mass%, and HFO-1234yf be present in an amount of 55.0 to 53.0 mass%, based on the total mass of HFO-1132 (E) and HFO-1234yf. In this case, Refrigerant 2 has characteristics of having a GWP of 100 or less, a COP of 102.5% or more relative to that of R404A, and a refrigerating capacity of 102.5% or more relative to that of R404A, as well as being slightly flammable according to ASHRAE Standards (Class 2L). Further, in this case, Refrigerant 2 has a saturation pressure of 1.81 MPa or more and 1.85 MPa or less at a saturation temperature of 40°C, and is thus applicable to commercially available refrigeration apparatuses for R404A without significant design change.

In Refrigerant 2, the saturation pressure at a saturation temperature of 40°C is usually 2.10 MPa or less, preferably 2.00 MPa or less, more preferably 1.95 MPa or less, even more preferably 1.90 MPa or less, and particularly preferably 1.88 MPa or less. If the saturation pressure at a saturation temperature of 40°C is within the above range, Refrigerant 2 is applicable to commercially available refrigeration apparatuses for R404A without significant design change.

In Refrigerant 2, the saturation pressure at a saturation temperature of 40°C is usually 1.70 MPa or more, preferably 1.73 MPa or more, more preferably 1.74 MPa or more, even more preferably 1.75 MPa or more, and particularly preferably 1.76 MPa or more. If the saturation pressure at a saturation temperature of 40°C is within the above range, Refrigerant 2 is applicable to commercially available refrigeration apparatuses for R404A without significant design change.

In the present disclosure, when Refrigerant 2 is used for operating a refrigeration cycle, the discharge temperature is preferably 150°C or lower, more preferably 140°C or lower, even more preferably 130°C or lower, and particularly preferably 120°C or lower, from the viewpoint of extending the life of the components of a commercially available refrigeration apparatus for R404A.

In the present disclosure, Refrigerant 2 is preferably used for operating a refrigeration cycle in which the evaporating temperature is -75 to 15°C from the viewpoint of obtaining a refrigerating capacity equivalent to or higher than that of R404A.

In a refrigeration cycle in which Refrigerant 2 according to the present disclosure is used, the evaporating temperature is preferably 15°C or lower, more preferably 5°C or lower, even more preferably 0°C or lower, and particularly preferably -5°C or lower.

In a refrigeration cycle in which Refrigerant 2 according to the present disclosure is used, the evaporating temperature is preferably -65°C or more, more preferably -60°C or more, even more preferably -55°C or more, and particularly preferably -50°C or more.

In a refrigeration cycle in which Refrigerant 2 according to the present disclosure is used, the evaporating temperature is preferably -65°C or more and 15°C or lower, more preferably -60°C or more and 5°C or lower, even more preferably -55°C or more and 0°C or lower, and particularly preferably -50°C or more and -5°C or lower.

In a refrigeration cycle in which Refrigerant 2 according to the present disclosure is used, the evaporation pressure is preferably 0.02 MPa or more, more preferably 0.03 MPa or more, even more preferably 0.04 MPa or more, and particularly preferably 0.05 MPa or more, from the viewpoint of improving the suction of the refrigerant into a compressor.

In a refrigeration cycle in which Refrigerant 2 according to the present disclosure is used, the compression ratio is preferably 2.5 or more, more preferably 3.0 or more, even more preferably 3.5 or more, and particularly preferably 4.0 or more, from the viewpoint of improving the efficiency of the refrigeration cycle.

Refrigerant 2 may comprise HFO-1132 (E) and HFO-1234yf in such amounts that the sum of their concentrations is usually 99.5 mass% or more. In the present disclosure, the total amount of HFO-1132 (E) and HFO-1234yf is preferably 99.7 mass% or more, more preferably 99.8 mass% or more, and even more preferably 99.9 mass% or more, of entire Refrigerant 2.

Refrigerant 2 may further comprise an additional refrigerant in addition to HFO-1132 (E) and HFO-1234yf as long as the above characteristics are not impaired. In this case, the content of the additional refrigerant is preferably 0.5 mass% or less, more preferably 0.3 mass% or less, even more preferably 0.2 mass% or less, and particularly preferably 0.1 mass% or less, of entire Refrigerant 2. The additional refrigerant is not limited and may be selected from a wide range of known refrigerants widely used in the field. Refrigerant 2 may comprise one additional refrigerant or two or more additional refrigerants.

It is particularly preferred that Refrigerant 2 consist of HFO-1132 (E) and HFO-1234yf. In other words, the total concentration of HFO-1132 (E) and HFO-1234yf in Refrigerant 2 is particularly preferably 100 mass% of entire Refrigerant 2.

When Refrigerant 2 consists of HFO-1132 (E) and HFO-1234yf, HFO-1132 (E) is usually present in an amount of 40.5 to 49.2 mass%, and HFO-1234yf is usually present in an amount of 59.5 to 50.8 mass%, based on the total mass of HFO-1132 (E) and HFO-1234yf. Refrigerant 2 has the above feature and thus has the following characteristics: (1) the GWP is sufficiently low (100 or less); (2) it has a COP equivalent to or higher than that of R404A; (3) it has a refrigerating capacity equivalent to or higher than that of R404A; and (4) it is slightly flammable according to ASHRAE Standards (Class 2L). Further, in this case, Refrigerant 2 has a saturation pressure of 1.75 MPa or more and 1.88 MPa or less at a saturation temperature of 40°C, and is thus applicable to commercially available refrigeration apparatuses for R404A without significant design change.

When Refrigerant 2 consists of HFO-1132 (E) and HFO-1234yf, it is preferred that HFO-1132 (E) be present in an amount of 41.3 to 49.2 mass%, and HFO-1234yf be present in an amount of 58.7 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 99.5% or more relative to that of R404A, as well as being slightly flammable according to ASHRAE Standards (Class 2L). Further, in this case, Refrigerant 2 has a saturation pressure of 1.76 MPa or more and 1.88 MPa or less at a saturation temperature of 40°C, and is thus applicable to commercially available refrigeration apparatuses for R404A without significant design change.

When Refrigerant 2 consists of HFO-1132 (E) and HFO-1234yf, it is preferred that HFO-1132 (E) be present in an amount of 43.0 to 49.2 mass%, and HFO-1234yf be present in an amount of 57.0 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 101% or more relative to that of R404A, as well as being slightly flammable according to ASHRAE Standards (Class 2L). Further, in this case, Refrigerant 2 has a saturation pressure of 1.78 MPa or more and 1.88 MPa or less at a saturation temperature of 40°C, and is thus applicable to commercially available refrigeration apparatuses for R404A without significant design change.

When Refrigerant 2 consists of HFO-1132 (E) and HFO-1234yf, it is further preferred that HFO-1132 (E) be present in an amount of 44.0 to 49.2 mass%, and HFO-1234yf be present in an amount of 56.0 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 101% or more relative to that of R404A, as well as being slightly flammable according to ASHRAE Standards (Class 2L). Further, in this case, Refrigerant 2 has a saturation pressure of 1.80 MPa or more and 1.88 MPa or less at a saturation temperature of 40°C, and is thus applicable to commercially available refrigeration apparatuses for R404A without significant design change.

When Refrigerant 2 consists of HFO-1132 (E) and HFO-1234yf, it is particularly preferred that HFO-1132 (E) be present in an amount of 45.0 to 49.2 mass%, and HFO-1234yf be present in an amount of 55.0 to 50.8 mass%, based on the total mass of HFO-1132 (E) and HFO-1234yf. In this case, Refrigerant 2 has characteristics of having a GWP of 100 or less, a COP of 102% or more relative to that of R404A, and a refrigerating capacity of 102% or more relative to that of R404A, as well as being slightly flammable according to ASHRAE Standards (Class 2L). Further, in this case, Refrigerant 2 has a saturation pressure of 1.81 MPa or more and 1.88 MPa or less at a saturation temperature of 40°C, and is thus applicable to commercially available refrigeration apparatuses for R404A without significant design change.

When Refrigerant 2 consists of HFO-1132 (E) and HFO-1234yf, it is particularly preferred that HFO-1132 (E) be present in an amount of 45.0 to 48.0 mass%, and HFO-1234yf be present in an amount of 55.0 to 52.0 mass%, based on the total mass of HFO-1132 (E) and HFO-1234yf. In this case, Refrigerant 2 has characteristics of having a GWP of 100 or less, a COP of 102.5% or more relative to that of R404A, and a refrigerating capacity of 102.5% or more relative to that of R404A, as well as being slightly flammable according to ASHRAE Standards (Class 2L). Further, in this case, Refrigerant 2 has a saturation pressure of 1.81 MPa or more and 1.87 MPa or less at a saturation temperature of 40°C, and is thus applicable to commercially available refrigeration apparatuses for R404A without significant design change.

5.3 Refrigerant 3

In an embodiment, the refrigerant according to the present disclosure comprises HFO-1132 (E) and HFO-1234yf, wherein HFO-1132 (E) is present in an amount of 31.1 to 39.8 mass%, and HFO-1234yf is present in an amount of 68.9 to 60.2 mass%, based on the total mass of HFO-1132 (E) and HFO-1234yf. This refrigerant may be referred to as "Refrigerant 3."

Refrigerant 3 has the above feature and thus has the following characteristics: (1) the GWP is sufficiently low (100 or less); (2) it has a COP almost equivalent to that of R134a; (3) it has a refrigerating capacity of 150% or more relative to that of R134a; and (4) the discharge temperature is 90°C or less.

Since Refrigerant 3 comprises HFO-1132 (E) in an amount of 31.1 mass% or more based on the total mass of HFO-1132 (E) and HFO-1234yf, Refrigerant 3 has a refrigerating capacity of 150% or more relative to that of R134a. Moreover, since Refrigerant 3 comprises HFO-1132 (E) in an amount of 39.8 mass% or less based on the total mass of HFO-1132 (E) and HFO-1234yf, the discharge temperature of Refrigerant 3 in a refrigeration cycle can be maintained at 90°C or less, and long life of the components of a refrigeration apparatus for R134a can be ensured.

Refrigerant 3 may have a refrigerating capacity of 150% or more, preferably 151% or more, more preferably 152% or more, even more preferably 153% or more, and particularly preferably 154% or more, relative to that of R134a.

Refrigerant 3 has a discharge temperature of preferably 90.0°C or less, more preferably 89.7°C or less, even more preferably 89.4°C or less, and particularly preferably 89.0°C or less, in a refrigeration cycle.

Since the GWP is 100 or less, Refrigerant 3 can notably reduce the burden on the environment from a global warming perspective, compared with other general-purpose refrigerants.

In Refrigerant 3, the ratio of refrigerating capacity to power consumed in a refrigeration cycle (coefficient of performance (COP)) relative to that of R134a is preferably high, from the viewpoint of energy consumption efficiency. Specifically, the COP relative to that of R134a is preferably 90% or more, more preferably 91% or more, even more preferably 91.5% or more, and particularly preferably 92% or more.

In Refrigerant 3, HFO-1132 (E) is usually present in an amount of 31.1 to 39.8 mass%, and HFO-1234yf is usually present in an amount of 68.9 to 60.2 mass%, based on the total mass of HFO-1132 (E) and HFO-1234yf. Refrigerant 3 has the above feature and thus has the following characteristics: (1) the GWP is sufficiently low (100 or less); (2) it has a COP almost equivalent to that of R134a; (3) it has a refrigerating capacity of 150% or more relative to that of R134a; and (4) the discharge temperature is 90.0°C or less.

In Refrigerant 3, it is preferred that HFO-1132 (E) be present in an amount of 31.1 to 37.9 mass%, and HFO-1234yf be present in an amount of 68.9 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has an COP of 92% or more relative to that of R134a; (3) it has a refrigerating capacity of 150% or more relative to that of R134a; (4) the discharge temperature is 90.0°C or less; and (5) the critical temperature is 81°C or more.

In Refrigerant 3, it is more preferred that HFO-1132 (E) be present in an amount of 32.0 to 37.9 mass%, and HFO-1234yf be present in an amount of 68.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 151% or more relative to that of R134a; (4) the discharge temperature is 90.0°C or less, and (5) the critical temperature is 81°C or more.

In Refrigerant 3, it is further preferred that HFO-1132 (E) be present in an amount of 33.0 to 37.9 mass%, and HFO-1234yf be present in an amount of 67.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 152% or more relative to that of R134a; (4) the discharge temperature is 90.0°C or less; and (5) the critical temperature is 81°C or more.

In Refrigerant 3, it is even more preferred that HFO-1132 (E) be present in an amount of 34.0 to 37.9 mass%, and HFO-1234yf be present in an amount of 66.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 153% or more relative to that of R134a; (4) the discharge temperature is 90.0°C or less; and (5) the critical temperature is 81°C or more.

In Refrigerant 3, it is particularly preferred that HFO-1132 (E) be present in an amount of 35.0 to 37.9 mass%, and HFO-1234yf be present in an amount of 65.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 155% or more relative to that of R134a; (4) the discharge temperature is 90.0°C or less; and (5) the critical temperature is 81°C or more.

In the present disclosure, when Refrigerant 3 is used for operating a refrigeration cycle, the discharge temperature is preferably 90.0°C or less, more preferably 89.7°C or less, even more preferably 89.4°C or less, and particularly preferably 89.0°C or less, from the viewpoint of extending the life of the components of a commercially available refrigeration apparatus for R134a.

In the present disclosure, when Refrigerant 3 is used for operating a refrigeration cycle, the refrigeration cycle requires the process of liquefying (condensing) the refrigerant; thus, the critical temperature needs to be notably higher than the temperature of cooling water or cooling air for liquefying the refrigerant. From this viewpoint, in a refrigeration cycle in which Refrigerant 3 according to the present disclosure is used, the critical temperature is preferably 80°C or more, more preferably 81°C or more, even more preferably 81.5°C or more, and particularly preferably 82°C or more.

In the present disclosure, Refrigerant 3 is usually used for operating a refrigeration cycle in which the evaporating temperature is -75 to 15°C, from the viewpoint of obtaining a refrigerating capacity of 150% or more relative to that of R134a.

In a refrigeration cycle in which Refrigerant 3 according to the present disclosure is used, the evaporating temperature is preferably 15°C or less, more preferably 5°C or less, even more preferably 0°C or less, and particularly preferably -5°C or less.

In a refrigeration cycle in which Refrigerant 3 according to the present disclosure is used, the evaporating temperature is preferably -65°C or more, more preferably -60°C or more, even more preferably -55°C or more, and particularly preferably -50°C or more.

In a refrigeration cycle in which Refrigerant 3 according to the present disclosure is used, the evaporating temperature is preferably -65°C or more and 15°C or less, more preferably -60°C or more and 5°C or less, even more preferably - 55°C or more and 0°C or less, and particularly preferably -50°C or more and -5°C or less.

In a refrigeration cycle in which Refrigerant 3 according to the present disclosure is used, the critical temperature of the refrigerant is preferably 80°C or more, more preferably 81°C or more, even more preferably 81.5°C or more, and particularly preferably 82°C or more, from the viewpoint of improving the performance.

Refrigerant 3 may comprise HFO-1132 (E) and HFO-1234yf in such amounts that the sum of their concentrations is usually 99.5 mass% or more. In the present disclosure, the total amount of HFO-1132 (E) and HFO-1234yf is preferably 99.7 mass% or more, more preferably 99.8 mass% or more, and even more preferably 99.9 mass% or more, of entire Refrigerant 3.

Refrigerant 3 may further comprise an additional refrigerant in addition to HFO-1132 (E) and HFO-1234yf as long as the above characteristics are not impaired. In this case, the content of the additional refrigerant is preferably 0.5 mass% or less, more preferably 0.3 mass% or less, even more preferably 0.2 mass% or less, and particularly preferably 0.1 mass% or less, of entire Refrigerant 3. The additional refrigerant is not limited and may be selected from a wide range of known refrigerants widely used in the field. Refrigerant 3 may comprise one additional refrigerant or two or more additional refrigerants.

It is particularly preferred that Refrigerant 3 consist of HFO-1132 (E) and HFO-1234yf. In other words, the total concentration of HFO-1132 (E) and HFO-1234yf in Refrigerant 3 is particularly preferably 100 mass% of entire Refrigerant 3.

When Refrigerant 3 consists of HFO-1132 (E) and HFO-1234yf, HFO-1132 (E) is usually present in an amount of 31.1 to 39.8 mass%, and HFO-1234yf is usually present in an amount of 68.9 to 60.2 mass%, based on the total mass of HFO-1132 (E) and HFO-1234yf. Refrigerant 3 has the above feature and thus has the following characteristics: (1) the GWP is sufficiently low (100 or less); (2) it has a COP almost equivalent to that of R134a; (3) it has a refrigerating capacity of 150% or more relative to that of R134a; and (4) the discharge temperature is 90°C or less.

When Refrigerant 3 consists of HFO-1132 (E) and HFO-1234yf, it is preferred that HFO-1132 (E) be present in an amount of 31.1 to 37.9 mass%, and HFO-1234yf be present in an amount of 68.9 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 150% or more relative to that of R134a; (4) the discharge temperature is 90.0°C or less; and (5) the critical temperature is 81°C or more.

When Refrigerant 3 consists of HFO-1132 (E) and HFO-1234yf, it is more preferred that HFO-1132 (E) be present in an amount of 32.0 to 37.9 mass%, and HFO-1234yf be present in an amount of 68.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 151% or more relative to that of R134a; (4) the discharge temperature is 90.0°C or less; and (5) the critical temperature is 81°C or more.

When Refrigerant 3 consists of HFO-1132 (E) and HFO-1234yf, it is even more preferred that HFO-1132 (E) be present in an amount of 33.0 to 37.9 mass%, and HFO-1234yf be present in an amount of 67.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 152% or more relative to that of R134a; (4) the discharge temperature is 90.0°C or less; and (5) the critical temperature is 81°C or more.

When Refrigerant 3 consists of HFO-1132 (E) and HFO-1234yf, it is further preferred that HFO-1132 (E) be present in an amount of 34.0 to 37.9 mass%, and HFO-1234yf be present in an amount of 66.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 153% or more relative to that of R134a; (4) the discharge temperature is 90.0°C or less; and (5) the critical temperature is 81°C or more.

When Refrigerant 3 consists of HFO-1132 (E) and HFO-1234yf, it is further preferred that HFO-1132(E) be present in an amount of 35.0 to 37.9 mass%, and HFO-1234yf be present in an amount of 65.0 to 62.1 mass%, based on the total mass of HFO-1132 (E) and HFO-1234yf. In this case, Refrigerant 3 has the above feature and thus has the following characteristics: (1) the GWP is sufficiently low (100 or less); (2) it has a COP of 92% or more relative to that of R134a; (3) it has a refrigerating capacity of 155% or more relative to that of R134a; (4) the discharge temperature is 90.0°C or less; and (5) the critical temperature is 81°C or more.

5.4 Refrigerant 4

In an embodiment, the refrigerant according to the present disclosure comprises HFO-1132 (E) and HFO-1234yf, wherein HFO-1132 (E) is present in an amount of 21.0 to 28.4 mass%, and HFO-1234yf is present in an amount of 79.0 to 71.6 mass%, based on the total mass of HFO-1132 (E) and HFO-1234yf. This refrigerant may be referred to as "Refrigerant 4."

Refrigerant 4 has the above feature and thus has the following characteristics: (1) the GWP is sufficiently low (100 or less); (2) it has a COP almost equivalent to that of R1234yf; (3) it has a refrigerating capacity of 140% or more relative to that of R1234yf; and (4) it is slightly flammable according to ASHRAE standards (Class 2L). Further, in this case, Refrigerant 4 has a saturation pressure of 0.380 MPa or more and 0.420 MPa or less at a saturation temperature of -10°C, and is thus applicable to commercially available refrigeration apparatuses for R1234yf without significant design change.

Since Refrigerant 4 comprises HFO-1132 (E) in an amount of 21.0 mass% or more based on the total mass of HFO-1132 (E) and HFO-1234yf, Refrigerant 4 has a refrigerating capacity of 140% or more relative to that of R1234yf. Moreover, Refrigerant 4 comprises HFO-1132 (E) in an amount of 28.4 mass% or less based on the total mass of HFO-1132 (E) and HFO-1234yf. This makes it easy to ensure a critical temperature of 83.5°C or more.

Refrigerant 4 may have a refrigerating capacity of 140% or more, preferably 142% or more, more preferably 143% or more, even more preferably 145% or more, and particularly preferably 146% or more, relative to that of R1234yf.

Since the GWP is 100 or less, Refrigerant 4 can notably reduce the burden on the environment from a global warming perspective, compared with other general-purpose refrigerants.

In Refrigerant 4, the ratio of refrigerating capacity to power consumed in a refrigeration cycle (coefficient of performance (COP)) relative to that of R1234yf is preferably high from the viewpoint of energy consumption efficiency. Specifically, the COP relative to that of R1234yf is preferably 95% or more, more preferably 96% or more, even more preferably 97% or more, and particularly preferably 98% or more.

In Refrigerant 4, it is preferred that HFO-1132 (E) be present in an amount of 21.5 to 28.0 mass%, and HFO-1234yf be present in an amount of 78.5 to 72.0 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 140% or more relative to that of R1234yf; it is slightly flammable according to ASHRAE standards (Class 2L); the discharge temperature is 65.0°C or less; and the critical temperature is 83.5°C or more. Further, in this case, Refrigerant 4 has a saturation pressure of 0.383 MPa or more and 0.418 MPa or less at a saturation temperature of -10°C, and is thus applicable to commercially available refrigeration apparatuses for R1234yf without significant design change.

In Refrigerant 4, it is more preferred that HFO-1132 (E) be present in an amount of 22.0 to 27.7 mass%, and HFO-1234yf be present in an amount of 78.0 to 72.3 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 140% or more relative to that of R1234yf; it is slightly flammable according to ASHRAE standards (Class 2L); the discharge temperature is 65.0°C or less; and the critical temperature is 83.5°C or more. Further, in this case, Refrigerant 4 has a saturation pressure of 0.385 MPa or more and 0.417 MPa or less at a saturation temperature of -10°C, and is thus applicable to commercially available refrigeration apparatuses for R1234yf without significant design change.

In Refrigerant 4, it is even more preferred that HFO-1132 (E) be present in an amount of 22.5 to 27.5 mass%, and HFO-1234yf be present in an amount of 77.5 to 72.5 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 140% or more relative to that of R1234yf; it is slightly flammable according to ASHRAE standards (Class 2L); the discharge temperature is 64.8°C or less; and the critical temperature is 83.8°C or more. Further, in this case, Refrigerant 4 has a saturation pressure of 0.388 MPa or more and 0.414 MPa or less at a saturation temperature of - 10°C, and is thus applicable to commercially available refrigeration apparatuses for R1234yf without significant design change.

In Refrigerant 4, it is particularly preferred that HFO-1132 (E) be present in an amount of 23.0 to 27.2 mass%, and HFO-1234yf be present in an amount of 77.0 to 72.8 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 141% or more relative to that of R1234yf; it is slightly flammable according to ASHRAE standards (Class 2L); the discharge temperature is 64.8°C or less; and the critical temperature is 83.8°C or more. Further, in this case, Refrigerant 4 has a saturation pressure of 0.390 MPa or more and 0.414 MPa or less at a saturation temperature of - 10°C, and is thus applicable to commercially available refrigeration apparatuses for R1234yf without significant design change.

In Refrigerant 4, it is further particularly preferred that HFO-1132 (E) be present in an amount of 23.5 to 27.0 mass%, and HFO-1234yf be present in an amount of 76.5 to 73.0 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 142% or more relative to that of R1234yf; it is slightly flammable according to ASHRAE standards (Class 2L); the discharge temperature is 64.8°C or less; and the critical temperature is 83.8°C or more. Further, in this case, Refrigerant 4 has a saturation pressure of 0.390 MPa or more and 0.414 MPa or less at a saturation temperature of - 10°C, and is thus applicable to commercially available refrigeration apparatuses for R1234yf without significant design change.

In Refrigerant 4, it is most preferred that HFO-1132 (E) be present in an amount of 24.0 to 26.7 mass%, and HFO-1234yf be present in an amount of 76.0 to 73.3 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 144% or more relative to that of R1234yf; it is slightly flammable according to ASHRAE standards (Class 2L); the discharge temperature is 64.6°C or less; and the critical temperature is 84.0°C or more. Further, in this case, Refrigerant 4 has a saturation pressure of 0.396 MPa or more and 0.411 MPa or less at a saturation temperature of -10°C, and is thus applicable to commercially available refrigeration apparatuses for R1234yf without significant design change.

In Refrigerant 4, the saturation pressure at a saturation temperature of -10°C is usually 0.420 MPa or less, preferably 0.418 MPa or less, more preferably 0.417 MPa or less, even more preferably 0.415 MPa or less, and particularly preferably 0.413 MPa or less. When the saturation pressure is within this range, Refrigerant 4 is applicable to commercially available refrigeration apparatuses for R1234yf without significant design change.

In Refrigerant 4, the saturation pressure at a saturation temperature of -10°C is usually 0.380 MPa or more, preferably 0.385 MPa or more, more preferably 0.390 MPa or more, even more preferably 0.400 MPa or more, and particularly preferably 0.410 MPa or more. In these cases, Refrigerant 4 is applicable to commercially available refrigeration apparatuses for R1234yf without significant design change.

In the present disclosure, when Refrigerant 4 is used for operating a refrigeration cycle, the discharge temperature is preferably 65°C or less, more preferably 64.8°C or less, even more preferably 64.7°C or less, and particularly preferably 64.5°C or less, from the viewpoint of extending the life of the components of a commercially available refrigeration apparatus for R1234yf.

In the present disclosure, Refrigerant 4 is preferably used for operating a refrigeration cycle in which the evaporating temperature is -75 to 5°C, from the viewpoint of obtaining a refrigerating capacity of 140% or more relative to that of R1234yf.

In a refrigeration cycle in which Refrigerant 4 according to the present disclosure is used, the evaporating temperature is preferably 5°C or less, more preferably 0°C or less, even more preferably -5°C or less, and particularly preferably -10°C or less, from the viewpoint of obtaining a refrigerating capacity of 140% or more relative to that of R1234yf.

In a refrigeration cycle in which Refrigerant 4 according to the present disclosure is used, the evaporating temperature is preferably -75°C or more, more preferably -60°C or more, even more preferably -55°C or more, and particularly preferably -50°C or more, from the viewpoint of obtaining a refrigerating capacity of 140% or more relative to that of R1234yf.

In a refrigeration cycle in which Refrigerant 4 according to the present disclosure is used, the evaporating temperature is preferably -65°C or more and 0°C or less, more preferably -60°C or more and -5°C or less, even more preferably - 55°C or more and -7.5°C or less, and particularly preferably - 50°C or more and -10°C or less, from the viewpoint of obtaining a refrigerating capacity of 140% or more relative to that of R1234yf.

In a refrigeration cycle in which Refrigerant 4 according to the present disclosure is used, the discharge temperature is preferably 65.0°C or less, more preferably 64.9°C or less, even more preferably 64.8°C or less, and particularly preferably 64.7°C or less, from the viewpoint of extending the life of the components of a commercially available refrigeration apparatus for R1234yf.

In the present disclosure, when Refrigerant 4 is used for operating a refrigeration cycle, the refrigeration cycle requires the process of liquefying (condensing) the refrigerant; thus, the critical temperature needs to be notably higher than the temperature of cooling water or cooling air for liquefying the refrigerant. From this viewpoint, in a refrigeration cycle in which Refrigerant 4 according to the present disclosure is used, the critical temperature is preferably 83.5°C or more, more preferably 83.8°C or more, even more preferably 84.0°C or more, and particularly preferably 84.5°C or more.

Refrigerant 4 may further comprise an additional refrigerant in addition to HFO-1132 (E) and HFO-1234yf as long as the above characteristics are not impaired. In this case, the content of the additional refrigerant is preferably 0.5 mass% or less, more preferably 0.3 mass% or less, even more preferably 0.2 mass% or less, and particularly preferably 0.1 mass% or less, of entire Refrigerant 4. The additional refrigerant is not limited and may be selected from a wide range of known refrigerants widely used in the field. Refrigerant 4 may comprise one additional refrigerant or two or more additional refrigerants.

It is particularly preferred that Refrigerant 4 consist of HFO-1132 (E) and HFO-1234yf. In other words, the total concentration of HFO-1132 (E) and HFO-1234yf in Refrigerant 4 is particularly preferably 100 mass% of entire Refrigerant 4.

When Refrigerant 4 consists of HFO-1132 (E) and HFO-1234yf, HFO-1132 (E) is usually present in an amount of 21.0 to 28.4 mass%, and HFO-1234yf is usually present in an amount of 79.0 to 71.6 mass%, based on the total mass of HFO-1132 (E) and HFO-1234yf. Refrigerant 4 has the above feature and thus has the following characteristics: (1) the GWP is sufficiently low (100 or less); (2) it has a COP almost equivalent to that of R1234yf; (3) it has a refrigerating capacity of 140% or more relative to that of R1234yf; and (4) it is slightly flammable according to ASHRAE standards (Class 2L). Further, in this case, Refrigerant 4 has a saturation pressure of 0.380 MPa or more and 0.420 MPa or less at a saturation temperature of -10°C and is thus applicable to commercially available refrigeration apparatuses for R1234yf without significant design change.

When Refrigerant 4 consists of HFO-1132 (E) and HFO-1234yf, it is preferred that HFO-1132 (E) be present in an amount of 21.5 to 28.0 mass%, and HFO-1234yf be present in an amount of 78.5 to 72.0 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 140% or more relative to that of R1234yf; it is slightly flammable according to ASHRAE standards (Class 2L); the discharge temperature is 65.0°C or less; and the critical temperature is 83.5°C or more. Further, in this case, Refrigerant 4 has a saturation pressure of 0.383 MPa or more and 0.418 MPa or less at a saturation temperature of -10°C and is thus applicable to commercially available refrigeration apparatuses for R1234yf without significant design change.

When Refrigerant 4 consists of HFO-1132 (E) and HFO-1234yf, it is more preferred that HFO-1132 (E) be present in an amount of 22.0 to 27.7 mass%, and HFO-1234yf be present in an amount of 78.0 to 72.3 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 140% or more relative to that of R1234yf; it is slightly flammable according to ASHRAE standards (Class 2L); the discharge temperature is 65.0°C or less; and the critical temperature is 83.5°C or more. Further, in this case, Refrigerant 4 has a saturation pressure of 0.385 MPa or more and 0.417 MPa or less at a saturation temperature of -10°C, and is thus applicable to commercially available refrigeration apparatuses for R1234yf without significant design change.

When Refrigerant 4 consists of HFO-1132 (E) and HFO-1234yf, it is even more preferred that HFO-1132 (E) be present in an amount of 22.5 to 27.5 mass%, and HFO-1234yf be present in an amount of 77.5 to 72.5 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 140% or more relative to that of R1234yf; it is slightly flammable according to ASHRAE standards (Class 2L); the discharge temperature is 64.8°C or less; and the critical temperature is 83.8°C or more. Further, in this case, Refrigerant 4 has a saturation pressure of 0.388 MPa or more and 0.414 MPa or less at a saturation temperature of -10°C, and is thus applicable to commercially available refrigeration apparatuses for R1234yf without significant design change.

When Refrigerant 4 consists of HFO-1132 (E) and HFO-1234yf, it is particularly preferred that HFO-1132 (E) be present in an amount of 23.0 to 27.2 mass%, and HFO-1234yf be present in an amount of 77.0 to 72.8 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 141% or more relative to that of R1234yf; it is slightly flammable according to ASHRAE standards (Class 2L); the discharge temperature is 64.8°C or less; and the critical temperature is 83.8°C or more. Further, in this case, Refrigerant 4 has a saturation pressure of 0.390 MPa or more and 0.414 MPa or less at a saturation temperature of -10°C, and is thus applicable to commercially available refrigeration apparatuses for R1234yf without significant design change.

When Refrigerant 4 consists of HFO-1132 (E) and HFO-1234yf, it is further particularly preferred that HFO-1132 (E) be present in an amount of 23.5 to 27.0 mass%, and HFO-1234yf be present in an amount of 76.5 to 73.0 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 142% or more relative to that of R1234yf; it is slightly flammable according to ASHRAE standards (Class 2L); the discharge temperature is 64.8°C or less; and the critical temperature is 83.8°C or more. Further, in this case, Refrigerant 4 has a saturation pressure of 0.390 MPa or more and 0.414 MPa or less at a saturation temperature of -10°C, and is thus applicable to commercially available refrigeration apparatuses for R1234yf without significant design change.

When Refrigerant 4 consists of HFO-1132 (E) and HFO-1234yf, it is most preferred that HFO-1132 (E) be present in an amount of 24.0 to 26.7 mass%, and HFO-1234yf be present in an amount of 76.0 to 73.3 mass%, based on the total mass of HFO-1132 (E) and HFO-1234yf. In this case, Refrigerant 4 has the following characteristics: the GWP is 100 or less; it has a COP of 98% or more relative to that of R1234yf; it has a refrigerating capacity of 144% or more relative to that of R1234yf; it is slightly flammable according to ASHRAE standards (Class 2L); the discharge temperature is 64.6°C or less; and the critical temperature is 84.0°C or more. Further, in this case, Refrigerant 4 has a saturation pressure of 0.396 MPa or more and 0.411 MPa or less at a saturation temperature of -10°C, and is thus applicable to commercially available refrigeration apparatuses for R1234yf without significant design change.

5.5 <u>Refrigerant 5</u>

In an embodiment, the refrigerant according to the present disclosure comprises HFO-1132 (E) and HFO-1234yf, wherein HFO-1132 (E) is present in an amount of 12.1 to 72.0 mass%, and HFO-1234yf is present in an amount of 87.9 to 28.0 mass%, based on the total mass of HFO-1132 (E) and HFO-1234yf. This refrigerant may be referred to as "Refrigerant 5."

In the present disclosure, Refrigerant 5 is used for an air-conditioning system for vehicles.

Refrigerant 5 has the above feature and thus has the following characteristics: (1) the GWP is sufficiently low (100 or less); (2) it has a COP almost equivalent to that of R1234yf; (3) it has a refrigerating capacity of 128% or more relative to that of R1234yf; and (4) the burning velocity is less than 10.0 cm/s.

Refrigerant 5 comprises HFO-1132 (E) in an amount of 12.1 mass% or more based on the total mass of HFO-1132 (E) and HFO-1234yf. This makes it possible to ensure a boiling point of - 40°C or less, which is advantageous when an electric vehicle is heated using a heat pump. A boiling point of -40°C or less means that the saturation pressure is equal to or higher than atmospheric pressure at -40°C. For the above application, a lower boiling point that is not higher than -40°C is preferred. Further, Refrigerant 5 comprises HFO-1132 (E) in an amount of 72.0 mass% or less based on the total mass of HFO-1132 (E) and HFO-1234yf. This makes it possible to ensure a burning velocity of less than 10.0 cm/s, which contributes to safety when used for an air-conditioning system for vehicles.

Refrigerant 5 may have a refrigerating capacity of 128% or more, preferably 130% or more, more preferably 140% or more, even more preferably 150% or more, and particularly preferably 160% or more, relative to that of R1234yf.

Since the GWP is 5 or more and 100 or less, Refrigerant 5 can notably reduce the burden on the environment from a global warming perspective, compared with other general-purpose refrigerants.

In Refrigerant 5, the ratio of refrigerating capacity to power consumed in a refrigeration cycle (coefficient of performance (COP)) relative to that of R1234yf may be 100% or more from the viewpoint of energy consumption efficiency.

The use of Refrigerant 5 for an air-conditioning system for vehicles enables heating with a heat pump, which consumes less power than electrical heaters.

The air-conditioning system for which Refrigerant 5 is used is preferably for gasoline vehicles, hybrid vehicles, electric vehicles, or hydrogen vehicles. From the viewpoint of improving the travel distance of a vehicle while the interior of the vehicle is heated with a heat pump, the air-conditioning system for which Refrigerant 5 is used is particularly preferably for electric vehicles among these. Specifically, in the present disclosure, Refrigerant 5 is particularly preferably used for electric vehicles.

In the present disclosure, Refrigerant 5 is used for air-conditioning systems for vehicles. In the present disclosure, Refrigerant 5 is preferably used for air-conditioning systems for gasoline vehicles, air-conditioning systems for hybrid vehicles, air-conditioning systems for electric vehicles, or air-conditioning systems for hydrogen vehicles. In the present disclosure, Refrigerant 5 is particularly preferably used for air-conditioning systems for electric vehicles.

In the present disclosure, Refrigerant 5 has a boiling point of preferably -51.2 to -40.0°C, more preferably -50.0 to - 42.0°C, and even more preferably -48.0 to -44.0°C, since a pressure equal to or higher than atmospheric pressure at -40°C is required when the interior of a vehicle is heated using a heat pump.

In Refrigerant 5, it is preferred that HFO-1132 (E) be present in an amount of 15.0 to 65.0 mass%, and HFO-1234yf be present in an amount of 85.0 to 35.0 mass%, based on the total mass of HFO-1132 (E) and HFO-1234yf.

In Refrigerant 5, it is more preferred that HFO-1132 (E) be present in an amount of 20.0 to 55.0 mass%, and HFO-1234yf be present in an amount of 80.0 to 45.0 mass%, based on the total mass of HFO-1132 (E) and HFO-1234yf.

In Refrigerant 5, it is even more preferred that HFO-1132 (E) be present in an amount of 25.0 to 50.0 mass%, and HFO-1234yf be present in an amount of 75.0 to 50.0 mass%, based on the total mass of HFO-1132 (E) and HFO-1234yf.

In Refrigerant 5, it is particularly preferred that HFO-1132 (E) be present in an amount of 30.0 to 45.0 mass%, and HFO-1234yf be present in an amount of 70.0 to 55.0 mass%, based on the total mass of HFO-1132 (E) and HFO-1234yf.

In Refrigerant 5, it is most preferred that HFO-1132 (E) be present in an amount of 35.0 to 40.0 mass%, and HFO-1234yf be present in an amount of 65.0 to 60.0 mass%, based on the total mass of HFO-1132 (E) and HFO-1234yf.

In the present disclosure, the burning velocity of Refrigerant 5 is preferably less than 10.0 cm/s, more preferably less than 5.0 cm/s, even more preferably less than 3.0 cm/s, and particularly preferably 2.0 cm/s.

In the present disclosure, Refrigerant 5 is preferably used for operating a refrigeration cycle in which the evaporating temperature is -40 to 10°C from the viewpoint of obtaining a refrigerating capacity equivalent to or higher than that of R1234yf.

In the present disclosure, when Refrigerant 5 is used for operating a refrigeration cycle, the discharge temperature is preferably 79°C or less, more preferably 75°C or less, even more preferably 70°C or less, and particularly preferably 67°C or less.

Refrigerant 5 may comprise HFO-1132 (E) and HFO-1234yf in such amounts that the sum of their concentrations is usually 99.5 mass% or more. In the present disclosure, the total amount of HFO-1132 (E) and HFO-1234yf is preferably 99.7 mass% or more, more preferably 99.8 mass% or more, and even more preferably 99.9 mass% or more, of entire Refrigerant 5.

Refrigerant 5 may further comprise an additional refrigerant in addition to HFO-1132 (E) and HFO-1234yf as long as the above characteristics are not impaired. In this case, the content of the additional refrigerant is preferably 0.5 mass% or less, more preferably 0.3 mass% or less, even more preferably 0.2 mass% or less, and particularly preferably 0.1 mass% or less, of entire Refrigerant 5. The additional refrigerant is not limited and may be selected from a wide range of known refrigerants widely used in the field. Refrigerant 5 may comprise one additional refrigerant or two or more additional refrigerants.

It is particularly preferred that Refrigerant 5 consist of HFO-1132 (E) and HFO-1234yf. In other words, the total concentration of HFO-1132 (E) and HFO-1234yf in Refrigerant 5 is particularly preferably 100 mass% of entire Refrigerant 5.

When Refrigerant 5 consists of HFO-1132 (E) and HFO-1234yf, HFO-1132 (E) is usually present in an amount of 12.1 to 72.0 mass%, and HFO-1234yf is usually present in an amount of 87.9 to 28.0 mass%, based on the total mass of HFO-1132 (E) and HFO-1234yf.

When Refrigerant 5 consists of HFO-1132 (E) and HFO-1234yf, it is preferred that HFO-1132 (E) be present in an amount of 15.0 to 65.0 mass%, and HFO-1234yf be present in an amount of 85.0 to 35.0 mass%, based on the total mass of HFO-1132 (E) and HFO-1234yf.

When Refrigerant 5 consists of HFO-1132 (E) and HFO-1234yf, it is more preferred that HFO-1132 (E) be present in an amount of 20.0 to 55.0 mass%, and HFO-1234yf be present in an amount of 80.0 to 45.0 mass%, based on the total mass of HFO-1132 (E) and HFO-1234yf.

When Refrigerant 5 consists of HFO-1132 (E) and HFO-1234yf, it is even more preferred that HFO-1132 (E) be present in an amount of 25.0 to 50.0 mass%, and HFO-1234yf be present in an amount of 75.0 to 50.0 mass%, based on the total mass of HFO-1132 (E) and HFO-1234yf.

When Refrigerant 5 consists of HFO-1132 (E) and HFO-1234yf, it is particularly preferred that HFO-1132 (E) be present in an amount of 30.0 to 45.0 mass%, and HFO-1234yf be present in an amount of 70.0 to 55.0 mass%, based on the total mass of HFO-1132 (E) and HFO-1234yf.

When Refrigerant 5 consists of HFO-1132 (E) and HFO-1234yf, it is most preferred that HFO-1132 (E) be present in an amount of 35.0 to 40.0 mass%, and HFO-1234yf be present in an amount of 65.0 to 60.0 mass%, based on the total mass of HFO-1132 (E) and HFO-1234yf.

### 1.6 Application

The composition containing the refrigerant according to the present disclosure can be widely used as a working fluid for known refrigerant applications in 1) a refrigeration method comprising operating a refrigeration cycle and 2) a method for operating a refrigeration apparatus that operates a refrigeration cycle.

The refrigeration cycle herein means performing energy conversion by circulating in the refrigeration apparatus the refrigerant (Refrigerant 1, 2, 3, 4, or 5 according to the present disclosure) in the state of the single refrigerant, or in the state of a refrigerant composition or a refrigerant oil-containing working fluid explained below, through a compressor.

The composition containing the refrigerant according to the present disclosure is not limited; however, it is suitably used in a vapor-compression refrigeration cycle. A vapor-compression refrigeration cycle comprises a series of cycles of (1) compressing a refrigerant in a gaseous state in a compressor, (2) cooling the refrigerant to convert it into a high-pressure liquid state in a condenser, (3) reducing the pressure with an expansion valve, and (4) evaporating the liquid refrigerant at a low temperature in an evaporator and removing heat by the heat of evaporation. Depending on the system of compressing gaseous refrigerants, vapor-compression refrigeration cycles can be classified into a turbo (centrifugal) cycle, a reciprocating cycle, a twin-screw cycle, a single-screw cycle, a scroll compressor cycle, etc., and can be selected according to heat capacity, compression ratio, and size.

The composition containing the refrigerant according to the present disclosure is not limited, and is suitable as a refrigerant used for large chiller refrigerating machines, and particularly turbo (centrifugal) compressors.

The present disclosure includes use of the refrigerant (or composition comprising the refrigerant) according to the present disclosure in a refrigeration method, use of the refrigerant (or composition comprising the refrigerant) according to the present disclosure in a method of operating a refrigeration apparatus etc., and a refrigeration apparatus or the like comprising the refrigerant (or composition comprising the refrigerant) according to the present disclosure.

The composition comprising Refrigerant 1 according to the present disclosure is used for operating a refrigeration cycle in which the evaporating temperature is -75 to -5°C.

By using the composition comprising Refrigerant 1 according to the present disclosure for operating a refrigeration cycle in which the evaporating temperature is -75 to -5°C, there is an advantage that a refrigerating capacity that is equivalent to or higher than that of R404A can be obtained.

In the refrigeration cycle in which the composition comprising Refrigerant 1 according to the present disclosure is used, the evaporating temperature is preferably -7.5°C or less, more preferably -10°C or less, even more preferably -35°C or less.

In the refrigeration cycle in which the composition comprising Refrigerant 1 according to the present disclosure is used, the evaporating temperature is preferably -65°C or more, more preferably -60°C or more, even more preferably -55°C or more, and particularly preferably -50°C or more.

From the viewpoint of obtaining a refrigerating capacity that is equivalent to or higher than R404A, the composition comprising Refrigerant 2 according to the present disclosure is preferably used for operating a refrigeration cycle in which the evaporating temperature is -75 to 5°C.

In the refrigeration cycle in which the composition comprising Refrigerant 2 according to the present disclosure is used, the evaporating temperature is preferably 0°C or less, more preferably -5°C or less, even more preferably -7.5°C or less, and particularly preferably -10°C or less.

In the refrigeration cycle in which the composition comprising Refrigerant 2 according to the present disclosure is used, the evaporating temperature is preferably -65°C or more, more preferably -60°C or more, even more preferably -55°C or more, and particularly preferably -50°C or more.

From the viewpoint of obtaining a refrigerating capacity that is equivalent to or higher than R134a, the composition comprising Refrigerant 3 according to the present disclosure is preferably used for operating a refrigeration cycle in which the evaporating temperature is -75 to 15°C.

In the refrigeration cycle in which the composition comprising Refrigerant 3 according to the present disclosure is used, the evaporating temperature is preferably 15°C or less, more preferably 5°C or less, even more preferably 0°C or less, and particularly preferably -5°C or less.

In the refrigeration cycle in which the composition comprising Refrigerant 3 according to the present disclosure is used, the evaporating temperature is preferably -65°C or more, more preferably -60°C or more, even more preferably -55°C or more, and particularly preferably -50°C or more.

In the refrigeration cycle in which the composition comprising Refrigerant 3 according to the present disclosure is used, the evaporating temperature is preferably -65°C or more to 15°C or less, more preferably -60°C or more to 5°C or less, even more preferably -55°C or more to 0°C or less, and particularly preferably -50°C or more to -5°C or less.

From the viewpoint of obtaining a refrigerating capacity of 140% or more relative to that of R1234yf, the composition comprising Refrigerant 4 according to the present disclosure is preferably used for operating a refrigeration cycle in which the evaporating temperature is -75 to 20°C.

From the viewpoint of obtaining a refrigerating capacity of 140% or more relative to that of R1234yf, in the refrigeration cycle in which the composition comprising Refrigerant 4 according to the present disclosure is used, the evaporating temperature is preferably 20°C or less, more preferably 10°C or less, even more preferably 0°C or less, and particularly preferably -10°C or less.

From the viewpoint of obtaining a refrigerating capacity of 140% or more relative to that of R1234yf, in the refrigeration cycle in which he composition comprising Refrigerant 4 according to the present disclosure is used, the evaporating temperature is preferably -75°C or more, more preferably -60°C or more, even more preferably -55°C or more, and particularly preferably -50°C or more.

Preferable examples of refrigeration apparatuses in which Refrigerant 1, 2, 3, or 4 (or a composition containing the refrigerant) according to the present disclosure can be used include air-conditioning systems, refrigerators, freezers, water coolers, ice makers, refrigerated showcases, freezing showcases, freezing and refrigerating units, refrigerating machines for freezing and refrigerating warehouses, air-conditioning systems for vehicles, turbo refrigerating machines, or screw refrigerating machines. Of these, air-conditioning systems for vehicles are preferred. Of the air-conditioning systems for vehicles, air-conditioning systems for gas vehicles, air-conditioning systems for hybrid vehicles, air-conditioning systems for electric vehicles, and air-conditioning systems for hydrogen vehicles are more preferred. Of the air-conditioning systems for vehicles, air-conditioning systems for electric vehicles are particularly preferred.

The composition comprising Refrigerant 1 or 2 according to the present disclosure is suitably used as an alternative refrigerant for R12, R22, R134a, R404A, R407A, R407C, R407F, R407H, R410A, R413A, R417A, R422A, R422B, R422C, R422D, R423A, R424A, R426A, R427A, R430A, R434A, R437A, R438A, R448A, R449A, R449B, R449C, R452A, R452B, R454A, R454B, R454C, R455A, R465A, R502, R507, or R513A. The composition comprising Refrigerant 1 or 2 according to the present disclosure is suitably used as an alternative refrigerant for R22, R404A, R407F, R407H, R448A, R449A, R454C, R455A, or R465A. Additionally, since the composition comprising Refrigerant 1 or 2 according to the present disclosure has a refrigerating capacity equivalent to R404A, which has been widely used, and a sufficiently low GWP, it is particularly suitable as an alternative refrigerant for R404A.

The composition comprising Refrigerant 3 according to the present disclosure is suitably used as an alternative refrigerant for R134a, R1234yf, or $CO_2$. The composition comprising Refrigerant 3 according to the present disclosure is suitably used as an alternative refrigerant for R134a. Additionally, since the composition comprising Refrigerant 3 according to the present disclosure has a refrigerating capacity of 150% or more relative to that of R134a, which has been widely used, and a sufficiently low GWP, it is particularly suitable as an alternative refrigerant for R134a.

The composition comprising Refrigerant 4 according to the present disclosure is suitably used as an alternative refrigerant for R12, R22, R134a, R404A, R407A, R407C, R407F, R407H, R410A, R413A, R417A, R422A, R422B, R422C, R422D, R423A, R424A, R426A, R427A, R430A, R434A, R437A, R438A, R448A, R449A, R449B, R449C, R452A, R452B, R454A, R454B, R454C, R455A, R465A, R502, R507, R513A, R1234yf, or R1234ze. The composition comprising Refrigerant 4 according to the present disclosure is suitably used as an alternative refrigerant for R12, R134a, R404A, R407C, R449C, R454C, R1234yf, or R1234ze. Additionally, since the composition comprising Refrigerant 4 according to the present disclosure has a refrigerating capacity of 140% or more relative to that of R1234yf, which has been widely used, and a sufficiently low GWP, it is particularly suitable as an alternative refrigerant for R1234yf.

The composition comprising Refrigerant 5 according to the present disclosure is suitably used as an alternative refrigerant for R12, R22, R134a, R404A, R407A, R407C, R407F, R407H, R410A, R413A, R417A, R422A, R422B, R422C, R422D, R423A, R424A, R426A, R427A, R430A, R434A, R437A, R438A, R448A, R449A, R449B, R449C, R452A, R452B, R454A, R454B, R454C, R455A, R465A, R502, R507, R513A, R1234yf, or R1234ze. The composition comprising Refrigerant 5 according to the present disclosure is suitably used as an alternative refrigerant for R12, R134a, or R1234yf. Additionally, since the composition comprising Refrigerant 5 according to the present disclosure has a refrigerating capacity of 140% or more relative to that of R1234yf, which has been widely used, and a sufficiently low GWP, it is particularly suitable as an alternative refrigerant for R1234yf.

The composition comprising Refrigerant 5 according to the present disclosure is preferably used in air-conditioning systems for vehicles. The air-conditioning systems for vehicles are preferably air-conditioning systems for gas vehicles, air-conditioning systems for hybrid vehicles, air-conditioning systems for electric vehicles, or air-conditioning systems for hydrogen vehicles. Of these, the air-conditioning systems for vehicles are particularly preferably air-conditioning systems for electric vehicles. That is, in the present disclosure, the composition comprising Refrigerant 5 is particularly preferably used for electric vehicles.

Examples of Refrigerants 1 to 5

The present disclosure is described in more detail below with reference to Examples of Refrigerants 1 to 5. However, Refrigerants 1 to 5 according to the present disclosure are not limited to the Examples.

Test Example 1-1

The GWP of each of the mixed refrigerants shown in Examples 1-1 to 1-13, Comparative Examples 1-1 to 1-2, and Reference Example 1-1 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using the National Institute of Science and Technology (NIST) and Reference Fluid Thermodynamic and Transport Properties Database (Refprop 9.0) under the following conditions.

Evaporating temperature: -50°C
Condensation temperature: 40°C
Superheating temperature: 20 K
Subcooling temperature: 0 K
Compressor efficiency: 70%

The "evaporating temperature of -50°C" means that the evaporating temperature of the mixed refrigerant in the evaporator provided in the refrigeration apparatus is -50°C. Further, the "condensation temperature of 40°C" means that the condensation temperature of the mixed refrigerant in the condenser provided in the refrigeration apparatus is 40°C.

Table 59 shows the results of Test Example 1-1. Table 59 shows Examples and Comparative Examples with regard to Refrigerant 1 according to the present disclosure. In Table 59, the "COP ratio" and the "refrigerating capacity ratio" refer to a ratio (%) relative to R404A.

In Table 59, the "saturation pressure (40°C)" refers to a saturation pressure at a saturation temperature of 40°C. In Table 1, the "discharge temperature (°C)" refers to a temperature at which the mixed refrigerant has the highest temperature in the refrigeration cycle according to the theoretical refrigeration cycle calculations of the mixed refrigerant.

The coefficient of performance (COP) was calculated according to the following formula.

$$COP = (refrigerating\ capacity\ or\ heating\ capacity)/power$$
$$consumption$$

The compression ratio was calculated according to the following formula.

$$Compression\ ratio = condensation\ pressure\ (Mpa)/evaporation$$
$$pressure\ (Mpa)$$

The flammability of the mixed refrigerants were determined by adjusting the mixed formulations of the mixed refrigerants to WCF concentrations and measuring the burning velocity according to ANSI/ASHRAE Standard 34-2013. The mixed refrigerant with a burning velocity of 0 cm/s to 10 cm/s was classified as Class 2L (slight flammability), the mixed refrigerant with a burning velocity of more than 10 cm/s was classified as Class 2 (weak flammability), and the mixed refrigerant with no flame propagation was classified as Class 1 (non-flammability). In Table 59, the ASHRAE flammability classification shows the results based on these criteria.

The burning velocity test was performed as follows. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps, and stored in a PC.

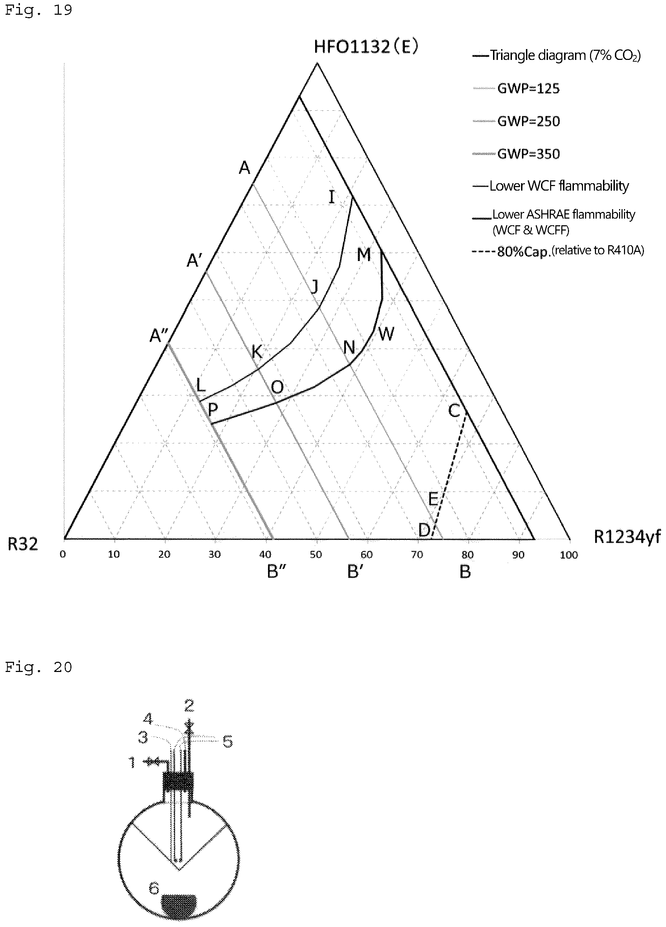

The flammable range of each of the mixed refrigerants was measured using a measurement device according to ASTM E681-09 (see Fig. 20).

More specifically, a 12-L spherical glass flask was used so that the combustion state could be visually observed and photographically recorded. When excessive pressure was generated by combustion in the glass flask, gas was allowed to escape from the upper lid. Ignition was achieved by electric discharge from electrodes disposed at one-third the distance from the bottom.

Test conditions

Test vessel: 280-mm φ spherical (internal volume: 12 liters)
Test temperature: 60°C +3°C
Pressure: 101.3 kPa +0.7 kPa
Water: 0.0088 g +0.0005 g (water content at a relative humidity of 50% at 23°C) per gram of dry air
Mixing ratio of refrigerant composition/air: 1 vol.% increments +0.2 vol.%
Mixture of refrigerant composition: +0.1 mass%
Ignition method: AC discharge, voltage: 15 kV, electric current: 30 mA, neon transformer
Electrode spacing: 6.4 mm (1/4 inch)
Spark: 0.4 seconds +0.05 seconds

Evaluation criteria:

When the flame spread at an angle of more than 90° from the ignition point, it was evaluated that flame propagation was present (flammable).
When the flame spread at an angle of 90° or less from the ignition point, it was evaluated that flame propagation was absent (non-flammable).

Table 59

| Item | | Unit | Reference Example 1-1 (R404A) | Comparative Example 1-1 | Example 1-1 | Example 1-2 | Example 1-3 | Example 1-4 | Example 1-5 | Example 1-6 | Example 1-7 | Example 1-8 | Example 1-9 | Example 1-10 | Example 1-11 | Example 1-12 | Example 1-13 | Comparative Example 1-2 |
|---|---|---|---|---|---|---|---|---|---|---|---|---|---|---|---|---|---|---|
| Composition ratio | HFO-1132 (E) | mass % | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass % | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass % | 4.0 | 0 | 0 | 0 | 0 | 0 | 0 | 0 | 0 | 0 | 0 | 0 | 0 | 0 | 0 | 0 |
| | HFC-143a | mass % | 52.0 | 0 | 0 | 0 | 0 | 0 | 0 | 0 | 0 | 0 | 0 | 0 | 0 | 0 | 0 | 0 |
| | HFC-125 | mass % | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Dischargetemperature | | oc | 100.6 | 108.6 | 114.7 | 115.0 | 115.5 | 116.5 | 117.6 | 118.8 | 120.0 | 121.0 | 122.4 | 123.3 | 124.4 | 125.5 | 126.0 | 131.7 |
| Saturation pressure (40°Q | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2.128 |
| Evaporation pressure | | MPa | 0.082 | 0.063 | 0.072 | 0.073 | 0.074 | 0.075 | 0.077 | 0.079 | 0.081 | 0.083 | 0.085 | 0.086 | 0.088 | 0.090 | 0.091 | 0.099 |
| Compression ratio | | - | 22.2 | 25.3 | 24.1 | 24.0 | 23.9 | 23.8 | 23.6 | 23.4 | 23.1 | 23.0 | 228 | 22.6 | 22.5 | 22.3 | 22.2 | 21.6 |
| COP ratio (relative to R404A) | | % | 100 | 106.2 | 106.2 | 106.2 | 106.2 | 106.2 | 106.2 | 106.2 | 106.2 | 106.3 | 106.3 | 106.3 | 106.3 | 106.4 | 106.4 | 106.7 |
| Refrigerating capacity ratio (relative to R404A) | | % | 100 | 86.2 | 98.5 | 99.1 | 100 | 1021 | 104.5 | 106.9 | 109.5 | 111.7 | 114.6 | 116.4 | 118.7 | 121 | 1222 | 133.3 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

EP 3 988 522 A1

116

Test Example 1-2

The GWP of each of the mixed refrigerants shown in Examples 1-14 to 1-26, Comparative Examples 1-3 to 1-4, and Reference Example 1-2 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: -35°C
Condensation temperature: 40°C
Superheating temperature: 20 K
Subcooling temperature: 0 K
Compressor efficiency: 70%

The definitions of the terms are the same as those in Test Example 1-1.

Table 60 shows the results of Test Example 1-2. Table 60 shows Examples and Comparative Examples with regard to Refrigerant 1 according to the present disclosure. In Table 60, the definitions of the terms are the same as those in Test Example 1-1.

The coefficient of performance (COP) and the compression ratio were determined as in Test Example 1-1.

The flammability of each of the mixed refrigerants was evaluated as in Test Example 1-1. The burning velocity test was performed as in Test Example 1-1.

The flammable range of each of the mixed refrigerants was measured in the same manner and under the same test conditions as in Test Example 1-1 by using a measurement device according to ASTM E681-09 (see Fig. 20).

Table 60

| Item | | Unit | Reference Example 1-2 (R404A) | Comparative Example 1-3 | Example 1-14 | Example 1-15 | Example 1-16 | Example 1-17 | Example 1-18 | Example 1-19 | Example 1-20 | Example 1-21 | Example 1-22 | Example 1-23 | Example 1-24 | Example 1-25 | Example 1-26 | Comparative Example 1-4 |
|---|---|---|---|---|---|---|---|---|---|---|---|---|---|---|---|---|---|---|
| Composition ratio | HFO-1132 (E) | mass % | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass % | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass % | 4.0 | 0 | 0 | 0 | 0 | 0 | 0 | 0 | 0 | 0 | 0 | 0 | 0 | 0 | 0 | 0 |
| | HFC-143a | mass % | 52.0 | 0 | 0 | 0 | 0 | 0 | 0 | 0 | 0 | 0 | 0 | 0 | 0 | 0 | 0 | 0 |
| | HFC-125 | mass % | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 89.1 | 95.8 | 100.6 | 100.8 | 101.2 | 102.0 | 1029 | 103.8 | 104.7 | 105.5 | 106.6 | 107.3 | 108.1 | 109.0 | 109.5 | 113.9 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2.128 |
| Evaporation pressure | | MPa | 0.165 | 0.131 | 0.148 | 0.149 | 0.151 | 0.154 | 0.157 | 0.160 | 0.164 | 0.167 | 0.171 | 0.174 | 0.177 | 0.180 | 0.181 | 0.196 |
| Compression ratio | | - | 11.0 | 12.2 | 11.8 | 11.7 | 11.7 | 11.6 | 11.6 | 11.5 | 11.4 | 11.4 | 11.3 | 11.2 | 11.2 | 11.1 | 11.1 | 10.8 |
| COP ratio (relative to R404A) | | % | 100 | 105.1 | 104.8 | 104.7 | 104.7 | 104.7 | 104.6 | 104.5 | 104.5 | 104.4 | 104.4 | 104.4 | 104.3 | 104.3 | 104.3 | 104.3 |
| Refrigerating capacity ratio (relative to R404A) | | % | 100 | 87.7 | 98.5 | 99.0 | 99.8 | 101.6 | 103.7 | 105.7 | 108.0 | 109.8 | 112.3 | 113.8 | 115.7 | 117.7 | 118.6 | 128.0 |

EP 3 988 522 A1

(continued)

| Item | Unit | Reference Example 1-2 (R404A) | Comparative Example 1-3 | Example 1-14 | Example 1-15 | Example 1-16 | Example 1-17 | Example 1-18 | Example 1-19 | Example 1-20 | Example 1-21 | Example 1-22 | Example 1-23 | Example 1-24 | Example 1-25 | Example 1-26 | Comparative Example 1-4 |
|---|---|---|---|---|---|---|---|---|---|---|---|---|---|---|---|---|---|
| ASHRAE flammability classification | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 1-3

The GWP of each of the mixed refrigerants shown in Examples 1-27 to 1-39, Comparative Examples 1-5 to 1-6, and Reference Example 1-3 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: -10°C
Condensation temperature: 40°C
Superheating temperature: 20 K
Subcooling temperature: 0 K
Compressor efficiency: 70%

The definitions of the terms are the same as those in Test Example 1-1.

Table 61 shows the results of Test Example 1-3. Table 61 shows Examples and Comparative Examples with regard to Refrigerant 1 according to the present disclosure. In Table 61, the definitions of the terms are the same as those in Test Example 1-1.

The coefficient of performance (COP) and the compression ratio were determined as in Test Example 1-1.

The flammability of each of the mixed refrigerants was evaluated as in Test Example 1-1. The burning velocity test was performed as in Test Example 1-1.

The flammable range of each of the mixed refrigerants was measured in the same manner and under the same test conditions as in Test Example 1-1 by using a measurement device according to ASTM E681-09 (see Fig. 20).

Table 61

| Item | | Unit | Reference Example 1-3 (R404A) | Comparative Example 1-5 | Example 1-27 | Example 1-28 | Example 1-29 | Example 1-30 | Example 1-31 | Example 1-32 | Example 1-33 | Example 1-34 | Example 1-35 | Example 1-36 | Example 1-37 | Example 1-38 | Example 1-39 | Comparative Example 1-6 |
|---|---|---|---|---|---|---|---|---|---|---|---|---|---|---|---|---|---|---|
| Composition ratio | HFO-1132(E) | mass % | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass % | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass % | 4.0 | 0 | 0 | 0 | 0 | 0 | 0 | 0 | 0 | 0 | 0 | 0 | 0 | 0 | 0 | 0 |
| | HFC-143a | mass % | 52.0 | 0 | 0 | 0 | 0 | 0 | 0 | 0 | 0 | 0 | 0 | 0 | 0 | 0 | 0 | 0 |
| | HFC-125 | mass % | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 75.8 | 80.8 | 83.7 | 83.9 | 84.1 | 84.5 | 85.1 | 85.6 | 86.2 | 86.6 | 87.3 | 87.7 | 88.2 | 88.7 | 88.9 | 91.5 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2.128 |
| Evaporation pressure | | MPa | 0.434 | 0.357 | 0.399 | 0.401 | 0.404 | 0.411 | 0.419 | 0.427 | 0.436 | 0.443 | 0.452 | 0.457 | 0.465 | 0.472 | 0.475 | 0.509 |
| Compression ratio | | - | 4.2 | 4.5 | 4.4 | 4.4 | 4.4 | 4.3 | 4.3 | 4.3 | 4.3 | 4.3 | 4.3 | 4.3 | 4.3 | 4.2 | 4.2 | 4.2 |
| COP ratio (relative to R404A) | | % | 100 | 103.8 | 102.9 | 102.9 | 102.8 | 102.7 | 102.5 | 102.4 | 102.2 | 102.1 | 102.0 | 101.9 | 101.8 | 101.7 | 101.6 | 101.3 |
| Refrigerating capacity ratio (relative to R404A) | | % | 100 | 89.8 | 98.7 | 99.1 | 99.8 | 101.2 | 102.8 | 104.5 | 106.2 | 107.7 | 109.6 | 110.8 | 112.3 | 113.8 | 114.5 | 121.7 |

(continued)

| Item | Unit | Reference Example 1-3 (R404A) | Comparative Example 1-5 | Example 1-27 | Example 1-28 | Example 1-29 | Example 1-30 | Example 1-31 | Example 1-32 | Example 1-33 | Example 1-34 | Example 1-35 | Example 1-36 | Example 1-37 | Example 1-38 | Example 1-39 | Comparative Example 1-6 |
|---|---|---|---|---|---|---|---|---|---|---|---|---|---|---|---|---|---|
| ASHRAE flammability classification | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 1-4

The GWP of each of the mixed refrigerants shown in Comparative Examples 1-7 to 1-21 and Reference Example 1-4 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: -80°C
Condensation temperature: 40°C
Superheating temperature: 20 K
Subcooling temperature: 0 K
Compressor efficiency: 70%

The definitions of the terms are the same as those in Test Example 1-1.

Table 62 shows the results of Test Example 1-4. Table 62 shows Comparative Examples with regard to Refrigerant 1 according to the present disclosure. In Table 62, the definitions of the terms are the same as those in Test Example 1-1.

The coefficient of performance (COP) and the compression ratio were determined as in Test Example 1-1.

The flammability of each of the mixed refrigerants was evaluated as in Test Example 1-1. The burning velocity test was performed as in Test Example 1-1.

The flammable range of each of the mixed refrigerants was measured in the same manner and under the same test conditions as in Test Example 1-1 by using a measurement device according to ASTM E681-09 (see Fig. 20).

Table 62

| Item | | Unit | Reference Example 1-4 (R404A) | Comp. Ex. 1-7 | Comp. Ex. 1-8 | Comp. Ex. 1-9 | Comp. Ex. 1-10 | Comp. Ex. 1-11 | Comp. Ex. 1-12 | Comp. Ex. 1-13 | Comp. Ex. 1-14 | Comp. Ex. 1-15 | Comp. Ex. 1-16 | Comp. Ex. 1-17 | Comp. Ex. 1-18 | Comp. Ex. 1-19 | Comp. Ex. 1-20 | Comp. Ex. 1-21 |
|---|---|---|---|---|---|---|---|---|---|---|---|---|---|---|---|---|---|---|
| Composition ratio | HFO-1132 (E) | mass% | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234vf | mass% | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass% | 4.0 | 0 | 0 | 0 | 0 | 0 | 0 | 0 | 0 | 0 | 0 | 0 | 0 | 0 | 0 | 0 |
| | HFC-143a | mass% | 52.0 | 0 | 0 | 0 | 0 | 0 | 0 | 0 | 0 | 0 | 0 | 0 | 0 | 0 | 0 | 0 |
| | HFC-125 | mass% | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 136.7 | 146.0 | 157.7 | 158.1 | 158.8 | 160.4 | 162.1 | 163.9 | 165.8 | 167.4 | 169.6 | 170.9 | 172.6 | 174.3 | 175.2 | 184.0 |
| Saturation pressure (40 °C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2128 |
| Evaporation pressure | | MPa | 0.014 | 0.011 | 0.012 | 0.012 | 0.012 | 0.012 | 0.013 | 0.013 | 0.013 | 0.014 | 0.014 | 0.014 | 0.015 | 0.015 | 0.015 | 0.017 |
| Compression ratio | | - | 134.6 | 149.1 | 150.8 | 150.2 | 149.3 | 147.2 | 145.0 | 142.8 | 140.5 | 138.7 | 136.3 | 134.9 | 133.2 | 131.5 | 130.7 | 123.8 |
| COP ratio (relative to R404A) | | % | 100 | 112.6 | 110.3 | 110.3 | 110.4 | 110.6 | 110.8 | 111.0 | 111.3 | 111.4 | 111.7 | 111.9 | 112.1 | 112.3 | 112.4 | 113.5 |
| Refrigerating capacity ratio (relative to R404A) | | % | 100 | 91.7 | 99.3 | 100.2 | 101.5 | 104.4 | 107.8 | 111.3 | 115.1 | 118.2 | 122.5 | 125.2 | 128.6 | 1321 | 133.8 | 151.0 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 1-5

The GWP of each of the mixed refrigerants shown in Comparative Examples 1-22 to 1-36 and Reference Example 1-5 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: 10°C
Condensation temperature: 40°C
Superheating temperature: 20 K
Subcooling temperature: 0 K
Compressor efficiency: 70%

The definitions of the terms are the same as those in Test Example 1-1.

Table 63 shows the results of Test Example 1-5. Table 63 shows Comparative Examples with regard to Refrigerant 1 according to the present disclosure. In Table 63, the definitions of the terms are the same as those in Test Example 1-1.

The coefficient of performance (COP) and the compression ratio were determined as in Test Example 1-1.

The flammability of each of the mixed refrigerants was evaluated as in Test Example 1-1. The burning velocity test was performed as in Test Example 1-1.

The flammable range of each of the mixed refrigerants was measured in the same manner and under the same test conditions as in Test Example 1-1 by using a measurement device according to ASTM E681-09 (see Fig. 20).

Table 63

| Item | | Unit | Reference Example 1-5 (R404A) | Comp. Ex. 1-22 | Comp. Ex. 1-23 | Comp. Ex. 1-24 | Comp. Ex. 1-25 | Comp. Ex. 1-26 | Comp. Ex. 1-27 | Comp. Ex. 1-28 | Comp. Ex. 1-29 | Comp. Ex. 1-30 | Comp. Ex. 1-31 | Comp. Ex. 1-32 | Comp. Ex. 1-33 | Comp. Ex. 1-34 | Comp. Ex. 1-35 | Comp. Ex. 1-36 |
|---|---|---|---|---|---|---|---|---|---|---|---|---|---|---|---|---|---|---|
| Composition ratio | HFO-1132 (E) | mass% | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass% | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass% | 4.0 | 0 | 0 | 0 | 0 | 0 | 0 | 0 | 0 | 0 | 0 | 0 | 0 | 0 | 0 | 0 |
| | HFC-143a | mass% | 52.0 | 0 | 0 | 0 | 0 | 0 | 0 | 0 | 0 | 0 | 0 | 0 | 0 | 0 | 0 | 0 |
| | HFC-125 | mass% | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 68.5 | 724 | 74.0 | 74.1 | 74.2 | 74.4 | 74.7 | 74.9 | 75.2 | 75.5 | 75.8 | 76.0 | 76.2 | 76.5 | 76.6 | 77.9 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2.128 |
| Evaporation pressure | | MPa | 0.820 | 0.694 | 0.768 | 0.772 | 0.777 | 0.789 | 0.803 | 0.817 | 0.832 | 0.844 | 0.860 | 0.870 | 0.882 | 0.895 | 0.901 | 0.959 |
| Compression ratio | | - | 2.2 | 23 | 2.3 | 2.3 | 2.3 | 23 | 2.3 | 2.3 | 2.3 | 2.2 | 2.2 | 2.2 | 2.2 | 2.2 | 2.2 | 22 |
| COP ratio (relative to R404A) | | % | 100.0 | 103.1 | 101.9 | 101.8 | 101.7 | 101.5 | 101.3 | 101.1 | 100.9 | 100.8 | 100.6 | 100.4 | 100.3 | 100.1 | 100.1 | 99.5 |
| Refrigerating capacity ratio (relative to R404A) | | % | 100.0 | 91.2 | 98.9 | 99.3 | 99.8 | 101.0 | 102.5 | 103.8 | 105.3 | 106.5 | 108.2 | 109.1 | 110.4 | 111.6 | 1123 | 118.2 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 2-1

The GWP of each of the mixed refrigerants shown in Examples 2-1 to 2-6, Comparative Examples 2-1 to 2-9, and Reference Example 2-1 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using the National Institute of Science and Technology (NIST) and Reference Fluid Thermodynamic and Transport Properties Database (Refprop 9.0) under the following conditions.

> Evaporating temperature: -50°C
> Condensation temperature: 40°C
> Superheating temperature: 20 K
> Subcooling temperature: 0 K
> Compressor efficiency: 70%

The "evaporating temperature of -50°C" means that the evaporating temperature of the mixed refrigerant in the evaporator provided in the refrigeration apparatus is -50°C. Further, the "condensation temperature of 40°C" means that the condensation temperature of the mixed refrigerant in the condenser provided in the refrigeration apparatus is 40°C.

Table 64 shows the results of Test Example 2-1. Table 64 shows Examples and Comparative Examples with regard to Refrigerant 2 according to the present disclosure. In Table 64, the "COP ratio" and the "refrigerating capacity ratio" refer to a ratio (%) relative to R404A.

In Table 64, the "saturation pressure (40°C)" refers to a saturation pressure at a saturation temperature of 40°C. In Table 64, the "discharge temperature (°C)" refers to a temperature at which the mixed refrigerant has the highest temperature in the refrigeration cycle according to the theoretical refrigeration cycle calculations of the mixed refrigerant.

The coefficient of performance (COP) was calculated according to the following formula.

$$COP = (refrigerating\ capacity\ or\ heating\ capacity)/power\ consumption$$

The compression ratio was calculated according to the following formula.

$$Compression\ ratio = condensation\ pressure\ (Mpa)/evaporation\ pressure\ (Mpa)$$

The flammability of the mixed refrigerants was determined by adjusting the mixed formulations of the mixed refrigerants to WCF concentrations and measuring the burning velocity according to ANSI/ASHRAE Standard 34-2013. The mixed refrigerant with a burning velocity of 0 cm/s to 10 cm/s was classified as Class 2L (slight flammability), the mixed refrigerant with a burning velocity of more than 10 cm/s was classified as Class 2 (weak flammability), and the mixed refrigerant with no flame propagation was classified as Class 1 (non-flammability). In Table 64, the ASHRAE flammability classification shows the results based on these criteria.

The burning velocity test was performed as follows. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized by using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps, and stored in a PC.

The flammable range of each of the mixed refrigerants was measured using a measurement device according to ASTM E681-09 (see Fig. 20).

More specifically, a 12-L spherical glass flask was used so that the combustion state could be visually observed and photographically recorded. When excessive pressure was generated by combustion in the glass flask, gas was allowed to escape from the upper lid. Ignition was achieved by electric discharge from electrodes disposed at one-third the distance from the bottom.

Test conditions

Test vessel: 280-mm φ spherical (internal volume: 12 liters)
Test temperature: 60°C ±3°C
Pressure: 101.3 kPa ±0.7 kPa
Water: 0.0088 g ±0.0005 g (water content at a relative humidity of 50% at 23°C) per gram of dry air
Mixing ratio of refrigerant composition/air: 1 vol.% increments ±0.2 vol.%
Mixture of refrigerant composition: ±0.1 mass%
Ignition method: AC discharge, voltage: 15 kV, electric current: 30 mA, neon transformer
Electrode spacing: 6.4 mm (1/4 inch)
Spark: 0.4 seconds ±0.05 seconds

Evaluation criteria:

When the flame spread at an angle of more than 90° from the ignition point, it was evaluated that flame propagation was present (flammable).
When the flame spread at an angle of 90° or less from the ignition point, it was evaluated that flame propagation was absent (non-flammable).

Table 64

| Item | | Unit | Reference Example 2-1 (R404A) | Comp. Ex. 2-1 | Comp. Ex. 2-2 | Example 2-1 | Example 2-2 | Example 2-3 | Example 2-4 | Example 2-5 | Example 2-6 | Comp. Ex. 2-3 | Comp. Ex. 2-4 | Comp. Ex. 2-5 | Comp. Ex. 2-6 | Comp. Ex. 2-7 | Comp. Ex. 2-8 | Comp. Ex. 2-9 |
|---|---|---|---|---|---|---|---|---|---|---|---|---|---|---|---|---|---|---|
| Composition ratio | HFO-1132(E) | mass% | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass% | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass% | 4.0 | 0 | 0 | 0 | 0 | 0 | 0 | 0 | 0 | 0 | 0 | 0 | 0 | 0 | 0 | 0 |
| | HFC-143a | mass% | 52.0 | 0 | 0 | 0 | 0 | 0 | 0 | 0 | 0 | 0 | 0 | 0 | 0 | 0 | 0 | 0 |
| | HFC-125 | mass% | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 100.6 | 108.6 | 114.7 | 115.0 | 115.5 | 116.5 | 117.6 | 118.8 | 120.0 | 121.0 | 122.4 | 123.3 | 124.4 | 125.5 | 126.0 | 131.7 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2012 | 2.128 |
| Evaporation pressure | | MPa | 0.082 | 0.063 | 0.072 | 0.073 | 0.074 | 0.075 | 0.077 | 0.079 | 0.081 | 0.083 | 0.085 | 0.086 | 0.088 | 0.090 | 0.091 | 0.099 |
| Compression ratio | | - | 22.2 | 25.3 | 24.1 | 24.0 | 23.9 | 23.8 | 23.6 | 23.4 | 23.1 | 23.0 | 22.8 | 22.6 | 22.5 | 22.3 | 22.2 | 21.6 |
| COP ratio (relative to R404A) | | % | 100 | 106.2 | 106.2 | 106.2 | 106.2 | 106.2 | 106.2 | 106.2 | 106.2 | 106.3 | 106.3 | 106.3 | 106.3 | 106.4 | 106.4 | 106.7 |
| Refrigerating capacity ratio (relative to R404A) | | % | 100 | 86.2 | 98.5 | 99.1 | 100 | 102.1 | 104.5 | 106.9 | 109.5 | 111.7 | 114.6 | 116.4 | 118.7 | 121 | 122.2 | 133.3 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 2-2

The GWP of each of the mixed refrigerants shown in Examples 2-7 to 2-12, Comparative Examples 2-10 to 2-18, and Reference Example 2-2 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: -35°C
Condensation temperature: 40°C
Superheating temperature: 20 K
Subcooling temperature: 0 K
Compressor efficiency: 70%

The definitions of the terms are the same as those in Test Example 2-1.

Table 65 shows the results of Test Example 2-2. Table 65 shows Examples and Comparative Examples with regard to Refrigerant 2 according to the present disclosure. In Table 65, the definitions of the terms are the same as those in Test Example 2-1.

The coefficient of performance (COP) and the compression ratio were determined as in Test Example 2-1.

The flammability of each of the mixed refrigerants was evaluated as in Test Example 2-1. The burning velocity test was performed as in Test Example 2-1.

The flammable range of each of the mixed refrigerants was measured in the same manner and under the same test conditions as in Test Example 2-1 by using a measurement device according to ASTM E681-09 (see Fig. 20).

Table 65

| Item | | Unit | Reference Example 2-2 (R404A) | Comp. Ex. 2-10 | Comp. Ex. 2-11 | Example 2-7 | Example 2-8 | Example 2-9 | Example 2-10 | Example 2-11 | Example 2-12 | Comp. Ex. 2-12 | Comp. Ex. 2-13 | Comp. Ex. 2-14 | Comp. Ex. 2-15 | Comp. Ex. 2-16 | Comp. Ex. 2-17 | Comp. Ex. 2-18 |
|---|---|---|---|---|---|---|---|---|---|---|---|---|---|---|---|---|---|---|
| Composition ratio | HFO-1132 (E) | mass% | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass% | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass% | 4.0 | 0 | 0 | 0 | 0 | 0 | 0 | 0 | 0 | 0 | 0 | 0 | 0 | 0 | 0 | 0 |
| | HFC-143a | mass% | 52.0 | 0 | 0 | 0 | 0 | 0 | 0 | 0 | 0 | 0 | 0 | 0 | 0 | 0 | 0 | 0 |
| | HFC-125 | mass% | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 89.1 | 95.8 | 100.6 | 100.8 | 101.2 | 102.0 | 102.9 | 103.8 | 104.7 | 105.5 | 106.6 | 107.3 | 108.1 | 109.0 | 109.5 | 113.9 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2.128 |
| Evaporation pressure | | MPa | 0.165 | 0.131 | 0.148 | 0.149 | 0.151 | 0.154 | 0.157 | 0.160 | 0.164 | 0.167 | 0.171 | 0.174 | 0.177 | 0.180 | 0.181 | 0.196 |
| Compression ratio | | - | 11.0 | 122 | 11.8 | 11.7 | 11.7 | 11.6 | 11.6 | 11.5 | 11.4 | 11.4 | 11.3 | 11.2 | 11.2 | 11.1 | 11.1 | 10.8 |
| COP ratio (relative to R404A) | | % | 100 | 105.1 | 104.8 | 104.7 | 104.7 | 104.7 | 104.6 | 104.5 | 104.5 | 104.4 | 104.4 | 104.4 | 104.3 | 104.3 | 104.3 | 104.3 |
| Refrigerating capacity ratio (relative to R404A) | | % | 100 | 87.7 | 98.5 | 99.0 | 99.8 | 101.6 | 103.7 | 105.7 | 108.0 | 109.8 | 112.3 | 113.8 | 115.7 | 117.7 | 118.6 | 128.0 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 2-3

The GWP of each of the mixed refrigerants shown in Examples 2-13 to 2-18, Comparative Examples 2-19 to 2-27, and Reference Example 2-3 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: -10°C
Condensation temperature: 40°C
Superheating temperature: 20 K
Subcooling temperature: 0 K
Compressor efficiency: 70%

The definitions of the terms are the same as those in Test Example 2-1.

Table 66 shows the results of Test Example 2-3. Table 66 shows Examples and Comparative Examples with regard to Refrigerant 2 according to the present disclosure. In Table 66, the definitions of the terms are the same as those in Test Example 2-1.

The coefficient of performance (COP) and the compression ratio were determined as in Test Example 2-1.

The flammability of each of the mixed refrigerants was evaluated as in Test Example 2-1. The burning velocity test was performed as in Test Example 2-1.

The flammable range of each of the mixed refrigerants was measured in the same manner and under the same test conditions as in Test Example 2-1 by using a measurement device according to ASTM E681-09 (see Fig. 20).

Table 66

| Item | | Unit | Reference Example 2-3 (R404A) | Comp. Ex. 2-19 | Comp. Ex. 2-20 | Example 2-13 | Example 2-14 | Example 2-15 | Example 2-16 | Example 2-17 | Example 2-18 | Comp. Ex. 2-21 | Comp. Ex. 2-22 | Comp. Ex. 2-23 | Comp. Ex. 2-24 | Comp. Ex. 2-25 | Comp. Ex. 2-26 | Comp. Ex. 2-27 |
|---|---|---|---|---|---|---|---|---|---|---|---|---|---|---|---|---|---|---|
| Composition ratio | HFO-1132 (E) | mass% | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass% | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass% | 4.0 | 0 | 0 | 0 | 0 | 0 | 0 | 0 | 0 | 0 | 0 | 0 | 0 | 0 | 0 | 0 |
| | HFC-143a | mass% | 52.0 | 0 | 0 | 0 | 0 | 0 | 0 | 0 | 0 | 0 | 0 | 0 | 0 | 0 | 0 | 0 |
| | HFC-125 | mass% | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 75.8 | 80.8 | 83.7 | 83.9 | 84.1 | 84.5 | 85.1 | 85.6 | 86.2 | 86.6 | 87.3 | 87.7 | 88.2 | 88.7 | 88.9 | 91.5 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2.128 |
| Evaporation pressure | | MPa | 0.434 | 0.357 | 0.399 | 0.401 | 0.404 | 0.411 | 0.419 | 0.427 | 0.436 | 0.443 | 0.452 | 0.457 | 0.465 | 0.472 | 0.475 | 0.509 |
| Compression ratio | | - | 4.2 | 4.5 | 4.4 | 4.4 | 4.4 | 4.3 | 4.3 | 4.3 | 4.3 | 4.3 | 4.3 | 4.3 | 4.3 | 4.2 | 4.2 | 4.2 |
| COP ratio (relativie to R404A) | | % | 100 | 103.8 | 102.9 | 1029 | 102.8 | 102.7 | 102.5 | 102.4 | 1022 | 102.1 | 102.0 | 101.9 | 101.8 | 101.7 | 101.6 | 101.3 |
| Refrigerating capacity ratio (relative to R404A) | | % | 100 | 89.8 | 98.7 | 99.1 | 99.8 | 101.2 | 102.8 | 104.5 | 106.2 | 107.7 | 109.6 | 110.8 | 112.3 | 113.8 | 114.5 | 121.7 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 2-4

The GWP of each of the mixed refrigerants shown in Examples 2-19 to 2-24, Comparative Examples 2-28 to 2-36, and Reference Example 2-4 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: -80°C
Condensation temperature: 40°C
Superheating temperature: 20 K
Subcooling temperature: 0 K
Compressor efficiency: 70%

The definitions of the terms are the same as those in Test Example 2-1.

Table 67 shows the results of Test Example 2-4. Table 67 shows Examples and Comparative Examples with regard to Refrigerant 2 according to the present disclosure. In Table 67, the definitions of the terms are the same as those in Test Example 2-1.

The coefficient of performance (COP) and the compression ratio were determined as in Test Example 2-1.

The flammability of each of the mixed refrigerants was evaluated as in Test Example 2-1. The burning velocity test was performed as in Test Example 2-1.

The flammable range of each of the mixed refrigerants was measured in the same manner and under the same test conditions as in Test Example 2-1 by using a measurement device according to ASTM E681-09 (see Fig. 20).

Table 67

| Item | | Unit | Reference Example 2-4 (R404A) | Comp. Ex. 2-28 | Comp. Ex. 2-29 | Example 2-19 | Example 2-20 | Example 2-21 | Example 2-22 | Example 2-23 | Example 2-24 | Comp. Ex. 2-30 | Comp. Ex. 2-31 | Comp. Ex. 2-32 | Comp. Ex. 2-33 | Comp. Ex. 2-34 | Comp. Ex. 2-35 | Comp. Ex. 2-36 |
|---|---|---|---|---|---|---|---|---|---|---|---|---|---|---|---|---|---|---|
| Composition ratio | HFO-1132 (E) | mass% | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass% | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass% | 4.0 | 0 | 0 | 0 | 0 | 0 | 0 | 0 | 0 | 0 | 0 | 0 | 0 | 0 | 0 | 0 |
| | HFC-143a | mass% | 52.0 | 0 | 0 | 0 | 0 | 0 | 0 | 0 | 0 | 0 | 0 | 0 | 0 | 0 | 0 | 0 |
| | HFC-125 | mass% | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 136.7 | 146.0 | 157.7 | 158.1 | 158.8 | 160.4 | 1621 | 163.9 | 165.8 | 167.4 | 169.6 | 170.9 | 172.6 | 174.3 | 175.2 | 184.0 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2.012 | 2.128 |
| Evaporation pressure | | MPa | 0.014 | 0.011 | 0.012 | 0.012 | 0.012 | 0.012 | 0.013 | 0.013 | 0.013 | 0.014 | 0.014 | 0.014 | 0.015 | 0.015 | 0.015 | 0.017 |
| Compression ratio | | - | 134.6 | 149.1 | 150.8 | 150.2 | 149.3 | 147.2 | 145.0 | 1428 | 140.5 | 138.7 | 136.3 | 134.9 | 133.2 | 131.5 | 130.7 | 123.8 |
| COP ratio (relative to R404A) | | % | 100 | 112.6 | 110.3 | 110.3 | 110.4 | 110.6 | 110.8 | 111.0 | 111.3 | 111.4 | 111.7 | 111.9 | 112.1 | 112.3 | 1124 | 113.5 |
| Refrigerating capacity ratio (relative to R404A) | | % | 100 | 91.7 | 99.3 | 100.2 | 101.5 | 104.4 | 107.8 | 111.3 | 115.1 | 118.2 | 122.5 | 125.2 | 128.6 | 132.1 | 133.8 | 151.0 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 2-5

The GWP of each of the mixed refrigerants shown in Examples 2-25 to 2-30, Comparative Examples 2-37 to 2-45, and Reference Example 2-5 (R404A) was evaluated based on the values stated in the IPCC, fourth report.

The COP, refrigerating capacity, discharge temperature, saturation pressure at a saturation temperature of 40°C, condensation pressure, and evaporation pressure of each of the mixed refrigerants were determined by performing theoretical refrigeration cycle calculations for the mixed refrigerants by using NIST and Refprop 9.0 under the following conditions.

Evaporating temperature: 10°C
Condensation temperature: 40°C
Superheating temperature: 20 K
Subcooling temperature: 0 K
Compressor efficiency: 70%

The definitions of the terms are the same as those in Test Example 2-1.

Table 68 shows the results of Test Example 2-5. Table 68 shows Examples and Comparative Examples with regard to Refrigerant 2 according to the present disclosure. In Table 68, the definitions of the terms are the same as those in Test Example 2-1.

The coefficient of performance (COP) and the compression ratio were determined as in Test Example 2-1.

The flammability of each of the mixed refrigerants was evaluated as in Test Example 2-1. The burning velocity test was performed as in Test Example 2-1.

The flammable range of each of the mixed refrigerants was measured in the same manner and under the same test conditions as in Test Example 2-1 by using a measurement device according to ASTM E681-09 (see Fig. 20).

Table 68

| Item | | Unit | Reference Example 2-5 (R404A) | Comp. Ex. 2-37 | Comp. Ex. 2-38 | Example 2-25 | Example 2-26 | Example 2-27 | Example 2-28 | Example 2-29 | Example 2-30 | Comp. Ex. 2-39 | Comp. Ex. 2-40 | Comp. Ex. 2-41 | Comp. Ex. 2-42 | Comp. Ex. 2-43 | Comp. Ex. 2-44 | Comp. Ex. 2-45 |
|---|---|---|---|---|---|---|---|---|---|---|---|---|---|---|---|---|---|---|
| Composition ratio | HFO-1132 (E) | mass% | 0 | 30.0 | 40.0 | 40.5 | 41.3 | 43.0 | 45.0 | 47.0 | 49.2 | 51.0 | 53.5 | 55.0 | 57.0 | 59.0 | 60.0 | 70.0 |
| | HFO-1234yf | mass% | 0 | 70.0 | 60.0 | 59.5 | 58.7 | 57.0 | 55.0 | 53.0 | 50.8 | 49.0 | 46.5 | 45.0 | 43.0 | 41.0 | 40.0 | 30.0 |
| | HFC-134a | mass% | 4.0 | 0 | 0 | 0 | 0 | 0 | 0 | 0 | 0 | 0 | 0 | 0 | 0 | 0 | 0 | 0 |
| | HFC-143a | mass% | 52.0 | 0 | 0 | 0 | 0 | 0 | 0 | 0 | 0 | 0 | 0 | 0 | 0 | 0 | 0 | 0 |
| | HFC-125 | mass% | 44.0 | 0 | 0 | 0 | 0 | 0 | 0 | 0 | 0 | 0 | 0 | 0 | 0 | 0 | 0 | 0 |
| GWP (AR4) | | - | 3922 | 6 | 6 | 6 | 6 | 7 | 7 | 7 | 7 | 7 | 7 | 7 | 7 | 8 | 8 | 8 |
| Discharge temperature | | °C | 68.5 | 72.4 | 74.0 | 74.1 | 74.2 | 74.4 | 74.7 | 74.9 | 75.2 | 75.5 | 75.8 | 76.0 | 76.2 | 76.5 | 76.6 | 77.9 |
| Saturation pressure (40°C) | | MPa | 1.822 | 1.592 | 1.745 | 1.752 | 1.764 | 1.788 | 1.817 | 1.844 | 1.874 | 1.898 | 1.931 | 1.950 | 1.975 | 2.000 | 2012 | 2.128 |
| Evaporation pressure | | MPa | 0.820 | 0.694 | 0.768 | 0.772 | 0.777 | 0.789 | 0.803 | 0.817 | 0.832 | 0.844 | 0.860 | 0.870 | 0.882 | 0.895 | 0.901 | 0.959 |
| Compression ratio | | - | 2.2 | 2.3 | 2.3 | 2.3 | 2.3 | 23 | 23 | 2.3 | 2.3 | 22 | 2.2 | 2.2 | 2.2 | 22 | 2.2 | 2.2 |
| COP ratio (relative to R404A) | | % | 100.0 | 103.1 | 101.9 | 101.8 | 101.7 | 101.5 | 101.3 | 101.1 | 100.9 | 100.8 | 100.6 | 100.4 | 100.3 | 100.1 | 100.1 | 99.5 |
| Refrigerating capacity ratio (relative to R404A) | | % | 100.0 | 91.2 | 98.9 | 99.3 | 99.8 | 101.0 | 102.5 | 103.8 | 105.3 | 106.5 | 108.2 | 109.1 | 110.4 | 111.6 | 112.3 | 118.2 |
| ASHRAE flammability classification | | - | Class 1 | Class 2L | Class 2L | Class 2L | Class 2L | Class 2L | Class 2L | Class 2L | Class 2L | Class 2L | Class 2L | Class 2 | Class 2 | Class 2 | Class 2 | Class 2 |

Test Example 3

The GWP of each of the mixed refrigerants shown in Examples 3-1 to 3-5, Comparative Examples 3-1 to 3-5, Reference Example 3-1 (R134a), and Reference Example 3-2 (R404A) was evaluated based on the values stated in the IPCC, fourth report.

The COP, refrigerating capacity, discharge temperature, saturation pressure at a saturation temperature of 45°C, condensation pressure, and evaporation pressure of each of the mixed refrigerants were determined by performing theoretical refrigeration cycle calculations for the mixed refrigerants by using the National Institute of Science and Technology (NIST) and Reference Fluid Thermodynamic and Transport Properties Database (Refprop 9.0) under the following conditions.

Evaporating temperature: -10°C
Condensation temperature: 45°C
Superheating temperature: 20 K
Subcooling temperature: 0 K
Compressor efficiency: 70%

The "evaporating temperature of -10°C" means that the evaporating temperature of the mixed refrigerant in the evaporator provided in the refrigeration apparatus is -10°C. Further, the "condensation temperature of 45°C" means that the condensation temperature of the mixed refrigerant in the condenser provided in the refrigeration apparatus is 45°C.

Table 69 shows the results of Test Example 3. Table 69 shows Examples and Comparative Examples with regard to Refrigerant 3 according to the present disclosure. In Table 69, the "COP ratio" and the "refrigerating capacity ratio" refer to a ratio (%) relative to R134a. In Table 69, the "saturation pressure (45°C)" refers to a saturation pressure at a saturation temperature of 45°C. In Table 69, the "discharge temperature (°C)" refers to a temperature at which the mixed refrigerant has the highest temperature in the refrigeration cycle according to the theoretical refrigeration cycle calculations of the mixed refrigerant.

The coefficient of performance (COP) was calculated according to the following formula.

```
COP = (refrigerating capacity or heating capacity)/power

consumption
```

The critical temperature was determined by performing calculations using the National Institute of Science and Technology (NIST) and Reference Fluid Thermodynamic and Transport Properties Database (Refprop 9.0).

The flammability of the mixed refrigerants was determined by adjusting the mixed formulations of the mixed refrigerants to WCF concentrations and measuring the burning velocity according to ANSI/ASHRAE Standard 34-2013. The mixed refrigerant with a burning velocity of 0 cm/s to 10 cm/s was classified as Class 2L (slight flammability), the mixed refrigerant with a burning velocity of more than 10 cm/s was classified as Class 2 (weak flammability), and the mixed refrigerant with no flame propagation was classified as Class 1 (non-flammability). In Table 69, the ASHRAE flammability classification shows the results based on these criteria.

The burning velocity test was performed as follows. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps, and stored in a PC.

The flammable range of each of the mixed refrigerants was measured using a measurement device according to ASTM E681-09 (see Fig. 20).

More specifically, a 12-L spherical glass flask was used so that the combustion state could be visually observed and photographically recorded. When excessive pressure was generated by combustion in the glass flask, gas was allowed to escape from the upper lid. Ignition was achieved by electric discharge from electrodes disposed at one-third the distance from the bottom.

Test conditions

Test vessel: 280-mm φ spherical (internal volume: 12 liters)
Test temperature: 60°C ±3°C
Pressure: 101.3 kPa ±0.7 kPa
Water: 0.0088 g ±0.0005 g (water content at a relative humidity of 50% at 23°C) per gram of dry air
Mixing ratio of refrigerant composition/air: 1 vol.% increments ±0.2 vol.%
Mixture of refrigerant composition: ±0.1 mass%
Ignition method: AC discharge, voltage: 15 kV, electric current: 30 mA, neon transformer
Electrode spacing: 6.4 mm (1/4 inch)
Spark: 0.4 seconds ±0.05 seconds

Evaluation criteria:

When the flame spread at an angle of more than 90° from the ignition point, it was evaluated that flame propagation was present (flammable).
When the flame spread at an angle of 90° or less from the ignition point, it was evaluated that flame propagation was absent (non-flammable).

Table 69

| Item | | Unit | Reference Example 3-1 (R134a) | Comp. Ex. 3-1 | Comp. Ex. 3-2 | Example 3-1 | Example 3-2 | Example 3-3 | Example 3-4 | Example 3-5 | Comp. Ex. 3-3 | Comp. Ex. 3-4 | Comp. Ex. 3-5 | Reference Example 3-2 (R404A) |
|---|---|---|---|---|---|---|---|---|---|---|---|---|---|---|
| Composition ratio | HFO-1132 (E) | mass% | 0 | 20.0 | 30.0 | 31.1 | 33.0 | 35.0 | 37.9 | 39.8 | 40.0 | 50.0 | 0.0 | 0 |
| | HFO-1234yf | mass% | 0 | 80.0 | 70.0 | 68.9 | 67.0 | 65.0 | 62.1 | 60.2 | 60.0 | 50.0 | 100.0 | 0 |
| | HFC-134a | mass% | 100.0 | 0 | 0 | 0 | 0 | 0 | 0 | 0 | 0 | 0 | 0 | 4.0 |
| | HFC-143a | mass% | 0 | 0 | 0 | 0 | 0 | 0 | 0 | 0 | 0 | 0 | 0 | 52.0 |
| | HFC-125 | mass% | 0 | 0 | 0 | 0 | 0 | 0 | 0 | 0 | 0 | 0 | 0 | 44.0 |
| GWP (AR4) | | - | 1430 | 5 | 6 | 6 | 6 | 6 | 6 | 6 | 6 | 7 | 4 | 3922 |
| Discharge temperature | | °C | 86.9 | 86.3 | 86.9 | 87.2 | 87.9 | 88.5 | 89.4 | 90.0 | 90.1 | 93.0 | 722 | 81.7 |
| Saturation pressure | | MPa | 1.160 | 1.607 | 1.795 | 1.814 | 1.848 | 1.883 | 1.930 | 1.963 | 1.966 | 2123 | 1.154 | 2.052 |
| Evaporation pressure | | MPa | 0.201 | 0.311 | 0.355 | 0.360 | 0.368 | 0.376 | 0.388 | 0.397 | 0.397 | 0.437 | 0.222 | 0.434 |
| Critical temperature | | °C | 101.1 | 84.6 | 83.0 | 82.7 | 82.2 | 81.7 | 81.0 | 80.5 | 80.5 | 78.7 | 94.7 | 72.0 |
| COP ratio (relative to R134a) | | % | 100.0 | 93.6 | 92.7 | 92.6 | 92.4 | 922 | 920 | 91.8 | 91.8 | 91.0 | 95.7 | 88.6 |
| Refrigerating capacity ratio (relative to R134a) | | % | 100.0 | 132.3 | 148.3 | 150.0 | 1528 | 155.8 | 159.8 | 162.7 | 1629 | 176.6 | 96.2 | 164.4 |
| ASHRAE flammability classification | | - | Class 1 | Class 2L | Class 2L | Class 2L | Class 2L | Class 2L | Class 2L | Class 2L | Class 2L | Class 2L | Class 2L | Class 1 |

Test Example 4

The GWP of each of the mixed refrigerants shown in Examples 4-1 to 4-7 and Comparative Examples 4-1 to 4-5 was evaluated based on the values stated in the IPCC, fourth report.

The COP, refrigerating capacity, discharge temperature, and saturation pressure at a saturation temperature of -10°C of each of the mixed refrigerants were determined by performing theoretical refrigeration cycle calculations for the mixed refrigerants by using the National Institute of Science and Technology (NIST) and Reference Fluid Thermodynamic and Transport Properties Database (Refprop 9.0) under the following conditions.

Evaporating temperature: 5°C
Condensation temperature: 45°C
Superheating temperature: 5 K
Subcooling temperature: 5 K
Compressor efficiency: 70%

The "evaporating temperature of 5°C" means that the evaporating temperature of the mixed refrigerant in the evaporator provided in the refrigeration apparatus is 5°C. Further, the "condensation temperature of 45°C" means that the condensation temperature of the mixed refrigerant in the condenser provided in the refrigeration apparatus is 45°C.

Table 70 shows the results of Test Example 4. Table 70 shows Examples and Comparative Examples with regard to Refrigerant 4 according to the present disclosure. In Table 70, the "COP ratio" and the "refrigerating capacity ratio" refer to a ratio (%) relative to R1234yf. In Table 70, the "saturation pressure (-10°C)" refers to a saturation pressure at a saturation temperature of -10°C, which is a typical value of the evaporating temperature in the refrigeration condition. In Table 70, the "discharge temperature (°C)" refers to a temperature at which the mixed refrigerant has the highest temperature in the refrigeration cycle according to the theoretical refrigeration cycle calculations of the mixed refrigerant.

The coefficient of performance (COP) was calculated according to the following formula.

$$\mathrm{COP = (refrigerating\ capacity\ or\ heating\ capacity)/power}$$

$$\mathrm{consumption}$$

The critical temperature was determined by performing calculations using the National Institute of Science and Technology (NIST) and Reference Fluid Thermodynamic and Transport Properties Database (Refprop 9.0).

The flammability of the mixed refrigerants was determined by adjusting the mixed formulations of the mixed refrigerants to WCF concentrations and measuring the burning velocity according to ANSI/ASHRAE Standard 34-2013. The mixed refrigerant with a burning velocity of 0 cm/s to 10 cm/s was classified as Class 2L (slight flammability), the mixed refrigerant with a burning velocity of more than 10 cm/s was classified as Class 2 (weak flammability), and the mixed refrigerant with no flame propagation was classified as Class 1 (non-flammability). In Table 70, the ASHRAE flammability classification shows the results based on these criteria.

The burning velocity test was performed as follows. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps, and stored in a PC.

The flammable range of each of the mixed refrigerants was measured using a measurement device according to ASTM E681-09 (see Fig. 20).

More specifically, a 12-L spherical glass flask was used so that the combustion state could be visually observed and photographically recorded. When excessive pressure was generated by combustion in the glass flask, gas was allowed to escape from the upper lid. Ignition was achieved by electric discharge from electrodes disposed at one-third the distance from the bottom.

Test conditions

Test vessel: 280-mm φ spherical (internal volume: 12 liters)
Test temperature: 60°C ±3°C
Pressure: 101.3 kPa ±0.7 kPa
Water: 0.0088 g ±0.0005 g (water content at a relative humidity of 50% at 23°C) per gram of dry air
Mixing ratio of refrigerant composition/air: 1 vol.% increments ±0.2 vol.%
Mixture of refrigerant composition: ±0.1 mass%
Ignition method: AC discharge, voltage: 15 kV, electric current: 30 mA, neon transformer
Electrode spacing: 6.4 mm (1/4 inch)
Spark: 0.4 seconds ±0.05 seconds

Evaluation criteria:

When the flame spread at an angle of more than 90° from the ignition point, it was evaluated that flame propagation was present (flammable).
When the flame spread at an angle of 90° or less from the ignition point, it was evaluated that flame propagation was absent (non-flammable).

Table 70

| Item | | Unit | Com parative Example 4-1 | Com parative Example 4-2 | Example 4-1 | Example 4-2 | Example 4-3 | Example 4-4 | Example 4-5 | Example 4-6 | Example 4-7 | Com parative Example 4-3 | Com parative Example 4-4 | Com parative Example 4-5 |
|---|---|---|---|---|---|---|---|---|---|---|---|---|---|---|
| Com position ratio | HFO-1132 (E) | mass% | 0 | 15.0 | 21.0 | 23.6 | 24.3 | 25.1 | 26.7 | 27.5 | 28.4 | 30.0 | 40.0 | 50.0 |
| | HFO-1234yf | mass% | 100.0 | 85.0 | 79.0 | 76.4 | 75.7 | 74.9 | 73.3 | 725 | 71.6 | 70.0 | 60.0 | 50.0 |
| GWP (AR4) | | - | 4 | 5 | 5 | 5 | 5 | 6 | 6 | 6 | 6 | 6 | 6 | 7 |
| Discharge temperature | | °C | 54.4 | 61.3 | 63.1 | 63.8 | 64.0 | 64.2 | 64.6 | 64.8 | 65.0 | 65.4 | 67.5 | 69.4 |
| Saturation pressure (-10°C) | | MPa | 0.222 | 0.350 | 0.383 | 0.396 | 0.400 | 0.403 | 0.411 | 0.414 | 0.418 | 0.425 | 0.461 | 0.492 |
| Critical temperature | | °C | 94.7 | 88.1 | 85.9 | 85.0 | 84.8 | 84.5 | 84.0 | 83.8 | 83.5 | 83.0 | 80.5 | 78.7 |
| COP ratio (relative to R1234yf) | | % | 100.0 | 99.1 | 98.8 | 98.6 | 98.5 | 98.4 | 98.3 | 98.2 | 98.2 | 98.0 | 97.2 | 96.6 |
| Refrigerating capacity ratio (relative to R1234yf) | | % | 100.0 | 129.8 | 140.0 | 144.2 | 145.4 | 146.6 | 149.1 | 150.3 | 151.7 | 154.1 | 168.2 | 181.3 |
| ASHRAE flammability classification | | - | Class 2L | Class 2L | Class 2L | Class 2L | Class 2L | Class 2L | Class 2L | Class 2L | Class 2L | Class 2L | Class 2L | Class 2L |

Test Example 5

The GWP of each of the mixed refrigerants shown in Examples 5-1 to 5-13, Comparative Examples 5-1 to 5-3, and Reference Example 5-1 (R134a) was evaluated based on the values stated in the IPCC, fourth report.

The COP, refrigerating capacity, boiling point, and discharge temperature of each of the mixed refrigerants were determined by performing theoretical refrigeration cycle calculations for the mixed refrigerants by using the National Institute of Science and Technology (NIST) and Reference Fluid Thermodynamic and Transport Properties Database (Refprop 9.0) under the following conditions.

Evaporating temperature: -30°C
Condensation temperature: 30°C
Superheating temperature: 5 K
Subcooling temperature: 5 K
Compressor efficiency: 70%

The "evaporating temperature of -30°C" means that the evaporating temperature of the mixed refrigerant in the evaporator provided in the refrigeration apparatus is -30°C. Further, the "condensation temperature of 30°C" means that the condensation temperature of the mixed refrigerant in the condenser provided in the refrigeration apparatus is 30°C.

Table 71 shows the results of Test Example 5. Table 71 shows Examples and Comparative Examples with regard to Refrigerant 5 according to the present disclosure. In Table 71, the "COP ratio" and the "refrigerating capacity ratio" refer to a ratio (%) relative to R1234yf. In Table 71, the "discharge temperature (°C)" refers to a temperature at which the mixed refrigerant has the highest temperature in the refrigeration cycle according to the theoretical refrigeration cycle calculations of the mixed refrigerant. In Table 71, the "boiling point (°C)" means a temperature at which the liquid phase of the mixed refrigerant has atmospheric pressure (101.33 kPa). In Table 71, the "motor power consumption (%)" refers to electrical energy used to enable an electric vehicle to run, and is expressed as a ratio with respect to power consumption when the refrigerant is HFO-1234yf. In Table 71, the "heater power consumption (%)" refers to electrical energy used to operate a heater by an electric vehicle, and is expressed as a ratio with respect to power consumption when the refrigerant is HFO-1234yf. In Table 71, the "drivable distance" refers to a distance drivable by an electric vehicle equipped with a rechargeable battery having a constant electric capacity while having a heater turned on, and is expressed as a ratio (%) relative to a drivable distance (100%) when the electric vehicle is driven without a heater turned on (i.e., heater power consumption is 0).

The coefficient of performance (COP) was calculated according to the following formula.

```
COP = (refrigerating capacity or heating capacity)/power

consumption
```

The flammability of the mixed refrigerants was determined by adjusting the mixed formulations of the mixed refrigerants to WCF concentrations and measuring the burning velocity according to ANSI/ASHRAE Standard 34-2013. The burning velocity was measured as follows. First, the mixed refrigerants used had a purity of 99.5% or more, and were degassed by repeating a cycle of freezing, pumping, and thawing until no traces of air were observed on the vacuum gauge. The burning velocity was measured by the closed method. The initial temperature was ambient temperature. Ignition was performed by generating an electric spark between the electrodes in the center of a sample cell. The duration of the discharge was 1.0 to 9.9 ms, and the ignition energy was typically about 0.1 to 1.0 J. The spread of the flame was visualized using schlieren photographs. A cylindrical container (inner diameter: 155 mm, length: 198 mm) equipped with two light transmission acrylic windows was used as the sample cell, and a xenon lamp was used as the light source. Schlieren images of the flame were recorded by a high-speed digital video camera at a frame rate of 600 fps, and stored in a PC.

Heating was performed by using an electrical heater in the case of a refrigerant having a boiling point of more than -40°C, and using a heat pump in the case of a refrigerant having a boiling point of -40°C or less.

The power consumption when the heater was used was calculated according to the following formula.

```
Power consumption when the heater was used = heating capacity/COP

of heater
```

The COP of the heater refers to heating efficiency.

With regard to the heating efficiency, the COP of the heater is 1 in an electric heater, and the heater consumes an electrode equivalent to the motor power. That is, the power consumption of the heater is E=E/(1+COP). In the case of a heat pump, the COP of the heater was determined by performing theoretical refrigeration cycle calculations for the mixed refrigerants using the National Institute of Science and Technology (NIST) and Reference Fluid Thermodynamic and Transport Properties Database (Refprop 9.0) under the following conditions.

Evaporating temperature: -30°C
Condensation temperature: 30°C
Superheating temperature: 5 K
Subcooling temperature: 5 K
Compressor efficiency: 70%

The drivable distance was calculated according to the following formula.

```
Drivable distance = (battery capacity)/(motor power consumption +
heater power consumption)
```

Table 71

| Item | | Unit | Reference Example 5-1 | Comp. Ex. 5-1 | Comp. Ex. 5-2 | Example 5-1 | Example 5-2 | Example 5-3 | Example 5-4 | Example 5-5 | Example 5-6 | Example 5-7 | Example 5-8 | Example 5-9 | Example 5-10 | Example 5-11 | Example 5-12 | Example 5-13 | Comp. Ex. 5-3 |
|---|---|---|---|---|---|---|---|---|---|---|---|---|---|---|---|---|---|---|---|
| Composition ratio | HFO-1132(E) | mass % | 0.0 | 0 | 10.0 | 12.1 | 15.0 | 20.0 | 25.0 | 30.0 | 35.0 | 40.0 | 45.0 | 50.0 | 55.0 | 60.0 | 65.0 | 72.0 | 75.0 |
| | HFO-1234yf | mass % | 0.0 | 100.0 | 90.0 | 87.9 | 85.0 | 80.0 | 75.0 | 70.0 | 65.0 | 60.0 | 55.0 | 50.0 | 45.0 | 40.0 | 35.0 | 28.0 | 25.0 |
| | HFC-134a | mass % | 100.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| GWP (AR4) | | | 1430 | 4 | 5 | 5 | 5 | 5 | 6 | 6 | 6 | 6 | 7 | 7 | 7 | 8 | 8 | 8 | 9 |
| COP ratio (relative to R1234yf) | | % | 105 | 100 | 100 | 100 | 100 | 100 | 100 | 100 | 100 | 100 | 100 | 100 | 100 | 100 | 100 | 100 | 100 |
| Refrigerating capacity ratio (relative to 1234yf) | | % | 99 | 100 | 123 | 128 | 134 | 145 | 155 | 165 | 175 | 185 | 194 | 203 | 212 | 220 | 229 | 240 | 245 |
| Motor power consumption amount | | % | 100 | 100 | 100 | 100 | 100 | 100 | 100 | 100 | 100 | 100 | 100 | 100 | 100 | 100 | 100 | 100 | 100 |
| Heater power consumption amount | | % | 95 | 100 | 100 | 33 | 33 | 33 | 33 | 33 | 33 | 33 | 33 | 33 | 33 | 33 | 33 | 33 | 33 |
| Drivable distance (without heater) | | % | 100 | 100 | 100 | 100 | 100 | 100 | 100 | 100 | 100 | 100 | 100 | 100 | 100 | 100 | 100 | 100 | 100 |
| Drivable distance (with heater) | | % | 50 | 50 | 50 | 84 | 84 | 84 | 84 | 84 | 84 | 84 | 84 | 84 | 84 | 84 | 84 | 84 | 84 |
| Discharge temperature | | °C | 66.0 | 48.0 | 54.8 | 56.0 | 57.5 | 59.8 | 61.9 | 63.9 | 65.8 | 67.6 | 69.3 | 70.9 | 72.6 | 74.2 | 75.9 | 78.2 | 79.2 |
| Combustion rate | | cm/s | 0.0 | 1.5 | 1.5 | 1.5 | 1.5 | 1.5 | 1.5 | 1.5 | 2.0 | 2.6 | 3.4 | 4.3 | 5.3 | 6.5 | 7.8 | 9.9 | 10.9 |
| Boiling point | | °C | -26.1 | -29.5 | -38.8 | -40.0 | -41.4 | -43.3 | -44.7 | -45.9 | -46.9 | -47.7 | -48.4 | -49.1 | -49.6 | -50.2 | -50.5 | -51.2 | -51.4 |

(continued)

| Item | Unit | Reference Example 5-1 | Comp. Ex. 5-1 | Comp. Ex. 5-2 | Example 5-1 | Example 5-2 | Example 5-3 | Example 5-4 | Example 5-5 | Example 5-6 | Example 5-7 | Example 5-8 | Example 5-9 | Example 5-10 | Example 5-11 | Example 5-12 | Example 5-13 | Comp. Ex. 5-3 |
|---|---|---|---|---|---|---|---|---|---|---|---|---|---|---|---|---|---|---|
| Saturation pressure at -40°C | kPaG | -50.1 | -39 | -4.4 | 0.9 | 7.5 | 17.2 | 25.3 | 323 | 38.4 | 43.9 | 48.8 | 53.4 | 57.5 | 61.4 | 65.0 | 69.6 | 71.5 |
| Heating method | System | Electric heater | Electric heater | Electric heater | Heat pump | Heat pump | Heat pump | Heat pump | Heat pump | Heat pump | Heat pump | Heat pump | Heat pump | Heat pump | Heat pump | Heat pump | Heat pump | Heat pump |

4. Refrigerating machine

The refrigerating machine according to the present disclosure comprises, as a working fluid, a gas phase containing a refrigerant comprising HFO-1132 and oxygen, the concentration of oxygen in the gas phase at a temperature of 25°C being 1000 volume ppm or less.

As mentioned above, the stability of HFO-1132 is improved in the refrigerating machine.

The embodiments are described above; however, it will be understood that various changes in forms and details can be made without departing from the spirit and scope of the claims.

Examples

The present disclosure is described in more detail below with reference to Examples. However, the present disclosure is not limited to the Examples.

Stability Test 1

Examples 1 to 7

The inside of a pressure-resistant container made of SUS316 (internal volume: 30 cc) was evacuated, and a predetermined amount of oxygen was enclosed in the pressure-resistant container. Thereafter, 15 g of liquefied trans-1,2-difluoroethylene having a purity of 99.5% or more was placed therein, and the oxygen concentration in the gas phase was adjusted to the value shown in Table 72 at 25°C. Subsequently, the pressure-resistant container, in which trans-1,2-difluoroethylene was enclosed together with the predetermined concentration of oxygen, was placed in a hot-air-circulating constant-temperature chamber and allowed to stand at a constant temperature of 60°C for 20 days.

After 20 days, the pressure-resistant container was removed from the constant-temperature chamber, and trans-1,2-difluoroethylene was released. The presence or absence of production of solid substance was examined with the naked eye. Moreover, the inside of the container was washed with dichloropentafluoropropane and perfluorohexane, and the solution was dried at 100°C to determine the amount of solid substance produced. Table 72 shows the results. In Table 72, A indicates that the amount of solid substance produced is less than 3 mg; B indicates that the amount of solid substance produced is 3 to 10 mg, but that there is no practical problem; C indicates that the amount of solid substance produced is 11 to 50 mg, but that there is no practical problem; and D indicates that the amount of solid substance produced is 50 mg or more.

Subsequently, with respect to Examples 1 to 7, in which a solid substance was observed with the naked eye, the solid substance after drying was dissolved in deuterated acetone, and $^1$H-NMR, $^{13}$C-NMR, and $^{19}$F-NMR spectra were measured. Identification of the solid substance by peak assignment of the measured NMR spectra revealed that it was a homopolymer of trans-1,2-difluoroethylene. It is presumed that this was produced by polymerization of trans-1,2-difluoroethylene.

Table 72

| | Oxygen concentration in gas (volume ppm) | Presence or absence of solid substance | Amount of solid substance produced (mg) |
|---|---|---|---|
| Example 1 | 4 | A | <3 |
| Example 2 | 10 | A | <3 |
| Example 3 | 200 | B | 4 |
| Example 4 | 400 | B | 10 |
| Example 5 | 500 | C | 15 |
| Example 6 | 1000 | C | 20 |

(continued)

|  | Oxygen concentration in gas (volume ppm) | Presence or absence of solid substance | Amount of solid substance produced (mg) |
|---|---|---|---|
| Example 7 | 4000 | D | 60 |

Stability Test 2

Examples 8 and 9

The inside of a pressure-resistant container made of SUS316 (internal volume: 30 cc) was evacuated, and a predetermined amount of oxygen was enclosed in the pressure-resistant container. Thereafter, 10 g of liquefied trans-1,2-difluoroethylene having a purity of 99.5% or more was placed therein, and the oxygen concentration in the gas phase was adjusted to the value shown in Table 73 at 25°C. Subsequently, the pressure-resistant container, in which trans-1,2-difluoroethylene was enclosed together with the predetermined concentration of oxygen, was placed in a hot-air-circulating constant-temperature chamber and allowed to stand at a constant temperature of 80°C for 5 days.

After 5 days, the pressure-resistant container was removed from the constant-temperature chamber, and trans-1,2-difluoroethylene was released. The presence or absence of production of solid substance was examined with the naked eye. Moreover, the inside of the container was washed with dichloropentafluoropropane and perfluorohexane, and the solution was dried at 100°C to determine the amount of solid substance produced. Table 73 shows the results. In Table 73, A indicates that the amount of solid substance produced is less than 3 mg; B indicates that the amount of solid substance produced is 0 to 3 mg, but that there is no practical problem; C indicates that the amount of solid substance produced is 4 to 50 mg, but that there is no practical problem; and D indicates that the amount of solid substance produced is 50 mg or more.

Subsequently, with respect to Examples 8 and 9, in which a solid substance was observed with the naked eye, the solid substance after drying was dissolved in deuterated acetone, and $^1$H-NMR, $^{13}$C-NMR, and $^{19}$F-NMR spectra were measured. Identification of the solid substance by peak assignment of the measured NMR spectra revealed that it was a homopolymer of trans-1,2-difluoroethylene. It is presumed that this was produced by polymerization of trans-1,2-difluoroethylene.

Table 73

|  | Oxygen concentration in gas (volume ppm) | Presence or absence of solid substance | Amount of solid substance produced (mg) |
|---|---|---|---|
| Example 8 | 4 | A | <3 |
| Example 9 | 300 | D | 60 |

Stability Test 2

Examples 10 to 15

Trans-1,2-difluoroethylene with a purity of 99.5% or more was placed in a glass tube (8 mm ID × 12 mm OD × 300 mm L) sealed on one side. Subsequently, oxygen was enclosed in the tube so that the oxygen concentration in the gas phase was adjusted to the value shown in Table 74 at 25°C. The tube was allowed to stand in a constant-temperature chamber in an atmosphere at 150°C and kept in this state for 1 week. Thereafter, the tube was removed from the constant-temperature chamber and cooled. The acid in the gas inside the tube was analyzed to evaluate the stability of trans-1,2-difluoroethylene.

The acid in the gas was analyzed by the following method. Gas remaining in the above tube after cooling was completely solidified using liquid nitrogen. The tube was then opened and gradually thawed to collect gas into a Tedlar bag. 5 g of pure water was injected into the Tedlar bag to extract the acid into the pure water while efficiently bringing the pure water into contact with the collected gas. The extract was detected by ion chromatography to determine the content (weight ppm) of fluoride ions ($F^-$). Table 74 shows the test results. In Table 74, A indicates that the acid content is less than 1 weight ppm; B indicates that the acid content is 1 to 3 weight ppm, but that there is no practical problem;

C indicates that the acid content is 4 to 10 weight ppm, but that there is no practical problem; and D indicates that the acid content is 100 weight ppm or more.

Table 74

|  | Oxygen concentration in gas (volume ppm) | Presence or absence of acid | Acid content (weight ppm) |
|---|---|---|---|
| Example 10 | 4 | A | <1 |
| Example 11 | 200 | A | <1 |
| Example 12 | 400 | B | 3 |
| Example 13 | 1000 | C | 10 |
| Example 14 | 4000 | D | 100 |

Description of the Reference Numerals

1:  Supply line
2:  Sampling line
3:  Thermometer
4:  Pressure gauge
5:  Electrode
6:  Stirring blade (made of PTFE)

**Claims**

1. A method for allowing a refrigerant comprising 1,2-difluoroethylene (HFO-1132) and oxygen to coexist in a gas phase, the concentration of oxygen in the gas phase at a temperature of 25°C being 1000 volume ppm or less.

2. A method for storing a refrigerant comprising HFO-1132 by allowing the refrigerant and oxygen to coexist in a closed container, the concentration of oxygen in the gas phase at a temperature of 25°C being 1000 volume ppm or less.

3. The method according to claim 1 or 2, wherein the temperature of the gas phase is less than 80°C.

4. The method according to any one of claims 1 to 3, wherein the gas phase coexists with a liquid phase containing the refrigerant.

5. The method according to claim 1 or 2, wherein a refrigerating machine is operated using the refrigerant as a working fluid, the method comprises performing the coexistence in the refrigerating machine, and the concentration of oxygen in the gas phase at a temperature of 25°C is 1000 volume ppm or less.

6. The method according to claim 5, wherein the gas phase coexists with a liquid phase containing the refrigerant in at least part of the refrigerating machine.

7. A method for stabilizing a refrigerant comprising HFO-1132, the method comprising, in a state that allows the refrigerant and oxygen to coexist in a gas phase, maintaining the concentration of oxygen in the gas phase at a temperature of 25°C to 1000 volume ppm or less to thereby stabilize the refrigerant.

8. A storage container of a refrigerant comprising HFO-1132, in which the refrigerant and oxygen coexist in a gas phase, the concentration of oxygen in the gas phase at a temperature of 25°C being 1000 volume ppm or less.

9. A refrigerating machine comprising, as a working fluid, a gas phase containing a refrigerant comprising HFO-1132 and oxygen, the concentration of oxygen in the gas phase at a temperature of 25°C being 1000 volume ppm or less.

10. The method, storage container, or refrigerating machine according to claims 1 to 8, wherein the refrigerant comprises HFO-1132(E) and HFO-1234yf, and
HFO-1132(E) is present in an amount of 35.0 to 65.0 mass%, and HFO-1234yf is present in an amount of 65.0 to 35.0 mass%, based on the total mass of HFO-1132(E) and HFO-1234yf.

11. The method, storage container, or refrigerating machine according to claim 10, wherein in the refrigerant, HFO-1132(E) is present in an amount of 41.3 to 53.5 mass%, and HFO-1234yf is present in an amount of 58.7 to 46.5 mass%, based on the total mass of HFO-1132(E) and HFO-1234yf.

12. The method, storage container, or refrigerating machine according to claims 1 to 8, wherein the refrigerant comprises HFO-1132(E) and HFO-1234yf, and
HFO-1132(E) is present in an amount of 40.5 to 49.2 mass%, and HFO-1234yf is present in an amount of 59.5 to 50.8 mass%, based on the total mass of HFO-1132(E) and HFO-1234yf.

13. The method, storage container, or refrigerating machine according to claims 1 to 8, wherein the refrigerant comprises HFO-1132(E) and HFO-1234yf, and
HFO-1132(E) is present in an amount of 31.1 to 39.8 mass%, and HFO-1234yf is present in an amount of 68.9 to 60.2 mass%, based on the total mass of HFO-1132(E) and HFO-1234yf.

14. The method, storage container, or refrigerating machine according to claim 13, wherein in the refrigerant, HFO-1132(E) is present in an amount of 31.1 to 37.9 mass%, and HFO-1234yf is present in an amount of 68.9 to 62.1 mass%, based on the total mass of HFO-1132(E) and HFO-1234yf.

15. The method, storage container, or refrigerating machine according to any one of claims 10 to 14, wherein the refrigerant consists of HFO-1132(E) and HFO-1234yf.

16. The refrigerating machine according to any one of claims 10 to 15, which has a refrigeration cycle in which the evaporating temperature is -75 to -5°C.

17. The method, storage container, or refrigerating machine according to claims 1 to 8, wherein the refrigerant comprises HFO-1132(E) and HFO-1234yf, and
HFO-1132(E) is present in an amount of 21.0 to 28.4 mass%, and HFO-1234yf is present in an amount of 79.0 to 71.6 mass%, based on the total mass of HFO-1132(E) and HFO-1234yf.

18. The method, storage container, or refrigerating machine according to claim 17, wherein the refrigerant consists of HFO-1132(E) and HFO-1234yf.

19. The method, storage container, or refrigerating machine according to claims 1 to 8, wherein the refrigerant comprises HFO-1132(E) and HFO-1234yf, and
HFO-1132(E) is present in an amount of 12.1 to 72.0 mass%, and HFO-1234yf is present in an amount of 87.9 to 28.0 mass%, based on the total mass of HFO-1132(E) and HFO-1234yf.

20. The method, storage container, or refrigerating machine according to claim 19, wherein the refrigerant consists of HFO-1132(E) and HFO-1234yf.

21. The refrigerating machine according to claim 19 or 20, which is an air-conditioning system for vehicles.

22. The air-conditioning system for vehicles according to claim 21, wherein the air-conditioning system for vehicles is for gasoline vehicles, hybrid vehicles, electric vehicles, or hydrogen vehicles.

Fig. 1

Fig. 2

Fig. 3

Fig. 4

Legend:
- Ternary composition diagram (R32 = 5.0%)
- 95% capacity (relative to 410A)
- 95% COP (relative to 410A)
- Line segment AB
- Line segment CB'

Fig. 5

Ternary composition diagram (R32 = 10.0%)
95% capacity (relative to 410A)
95% COP (relative to 410A)
Line segment AB
Line segment CB'

HFO1132(E)

HFO1123

R1234yf

Fig. 6

HFO1132(E)

C

A

B'

B

O

HFO1123

R1234yf

0    10    20    30    40    50    60    70    80    90    100

- - - - - Ternary composition diagram (R32 = 14.3%)
- - - - - 95% capacity (relative to 410A)
- - - - - 95% COP (relative to 410A)
——— Line segment AB
——— Line segment CB'

Fig. 7

HFO1132(E)
O

C

A

B'

B

HFO1123

R1234yf

0  10  20  30  40  50  60  70  80  90  100

Legend:
— Ternary composition diagram R32 = 16.5%
····· 95% capacity (relative to 410A)
····· 95% COP (relative to 410A)
— Line segment AB
— Line segment CB'

Fig. 8

Legend:
- Ternary composition diagram R32 = 19.2%
- 95% capacity (relative to 410A)
- 95% COP (relative to 410A)
- Line segment AB
- Line segment CB'

Triangle vertices: HFO1132(E), HFO1123, R1234yf
Points labeled: O, C, A, B', B

Fig. 9

HFO1132(E)

HFO1123

R1234yf

| | Ternary composition diagram R32 = 21.8% |
| | 95% capacity (relative to 410A) |
| | 95% COP (relative to 410A) |
| | Line segment AB |
| | Line segment CB′ |

Fig. 10

Fig. 11

Fig. 12

HFO1132(E)

GWP=125
GWP=250
GWP=350
Lower WCF flammability
Lower ASHRAE flammability (WCF & WCFF)
80%Cap. approx. straight line (relative to R410A)
80%Cap.curve (relative to R410A)

R32

R1234yf

Fig. 13

Fig. 14

Fig. 15

Fig. 16

HFO1132(E)

——Triangle diagram (2.5% $CO_2$)
······GWP=125
······GWP=250
······GWP=350
——Lower WCF flammability
__ Lower ASHRAE flammability
(WCF & WCFF)
----80%Cap.(relative to R410A)

A
I
A′
M
J
A″
C
K
N W
L O
P E
D
R32
0  10  20  30  40  B″ 50  60 B′  70  80 B  90  100
R1234yf

Fig. 17

HFO1132(E)

R32

R1234yf

B″        B′        B

| | |
|---|---|
| ▬▬ | Triangle diagram (4% CO₂) |
| ⋯⋯ | GWP=125 |
| ⋯⋯ | GWP=250 |
| ⋯⋯ | GWP=350 |
| ▬▬ | Lower WCF flammability |
| ▬▬ | Lower ASHRAE flammability (WCF & WCFF) |
| ---- | 80%Cap.(relative to R410A) |

Fig. 18

Fig. 19

Fig. 20

**INTERNATIONAL SEARCH REPORT**

| International application No. |
| --- |
| PCT/JP2020/024198 |

A. CLASSIFICATION OF SUBJECT MATTER
C07C 21/18(2006.01)i; F25B 1/00(2006.01)i; C09K 5/04(2006.01)i
FI: C09K5/04 F; F25B1/00 396Z; C07C21/18; F25B1/00 396A

According to International Patent Classification (IPC) or to both national classification and IPC

B. FIELDS SEARCHED

Minimum documentation searched (classification system followed by classification symbols)
C09K5/04; F25B1/00; C07C21/18; F25B1/00

Documentation searched other than minimum documentation to the extent that such documents are included in the fields searched
| | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y<br>A | JP 2016-11423 A (ASAHI GLASS CO., LTD.) 21.01.2016 (2016-01-21) claims 1-5, 7-10, paragraphs [0001], [0008], [0031], [0040]-[0044], [0112], [0113], examples 10-27, tables 5-7 | 1-9<br>10-22 |
| Y<br>A | WO 2015/186557 A1 (ASAHI GLASS CO., LTD.) 10.12.2015 (2015-12-10) claims 1-7, 8-13, paragraphs [0001], [0010], [0026], [0066]-[0069], [0080], [0081], [0094], [0140], [0141], examples 113-140, tables 1-14 | 1-14, 17, 19<br>15, 16, 18,<br>20-22 |
| Y<br>A | WO 2015/115252 A1 (ASAHI GLASS CO., LTD.) 06.08.2015 (2015-08-06) claims 1, 7-10, paragraphs [0009], [0060], [0084], [0143], [0144], examples 29-34, table 11 | 1-14, 19<br>15, 16, 18,<br>20-22 |
| Y<br>A | JP 2019-34972 A (AGC INC.) 07.03.2019 (2019-03-07) claims 1-6, paragraphs [0001], [0003], [0004], [0008], [0013], [0015]-[0022], [0031], examples 1-10, table 1 | 1-14, 17, 19<br>15, 16, 18,<br>20-22 |

☐ Further documents are listed in the continuation of Box C.    ☒ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | |
| "O" | document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" document member of the same patent family |

| Date of the actual completion of the international search | Date of mailing of the international search report |
| --- | --- |
| 24 August 2020 (24.08.2020) | 08 September 2020 (08.09.2020) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**INTERNATIONAL SEARCH REPORT**
Information on patent family members

International application No.

PCT/JP2020/024198

| Patent Documents referred in the Report | Publication Date | Patent Family | Publication Date |
|---|---|---|---|
| JP 2016-11423 A | 21 Jan. 2016 | US 2019/0031934 A1 claims 1, 5-9, paragraphs [0002], [0013], [0116], [0170]-[0172], examples 23-28, tables 8-10 EP 3101082 A1 CN 106029821 A | |
| WO 2015/186557 A1 | 10 Dec. 2015 | US 2017/0058173 A1 claims 1-7, 9-13, paragraphs [0001], [0011], [0028]-[0031], [0069]-[0073], [0084], [0085], [0107], [0162], [0163], examples 113-140, tables 3-14 EP 3153559 A CN 106029821 A | |
| WO 2015/115252 A1 | 06 Aug. 2015 | US 2016/0333243 A1 claims 1, 5-8, paragraphs [0011], [0075], [0101], [0168], [0169], examples 29-34, table 11 EP 3101082 A1 CN 106029821 A | |
| JP 2019-34972 A | 07 Mar. 2019 | US 2015/0051426 A1 claims 1-6, paragraphs [0003], [0005]-[0008], [0014], [0019], [0021]-[0029], examples 1-10, table 1 EP 2842928 A1 CN 104245644 A | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015141678 A